# EUROPEAN PATENT APPLICATION

(11) **EP 4 455 134 A1**
(43) Date of publication of application: **30.10.2024**
(21) Application number: 22911397.2
(22) Date of filing: 23.12.2022
(51) Int. Cl.: C07D 401/14, A61K 9/127, A61K 31/497, A61K 31/519, A61K 31/5377, A61K 45/00, A61K 47/24, A61K 47/28, A61K 47/34, A61K 47/44, A61P 35/00, A61P 35/02, A61P 43/00, C07D 405/14, C07D 413/14, C07D 471/04, C07D 487/04, C07D 491/048

(54) **1H-PYRAZOLE-3-AMINE DERIVATIVE HAVING BICYCLIC BACKBONE**

(30) Priority: 24.12.2021 JP 2021210843; 27.05.2022 JP 2022087175
(71) Applicant: Sumitomo Pharma Co., Ltd., Osaka-shi, Osaka 541-0045 (JP)
(72) Inventor: KAMIOKA, Seiji, Osaka-shi, Osaka 554-0022 (JP); HAYASHI, Kento, Osaka-shi, Osaka 554-0022 (JP); BAN, Hitoshi, Osaka-shi, Osaka 554-0022 (JP); SHIMADA, Naoaki, Osaka-shi, Osaka 554-0022 (JP); MATSUOKA, Makoto, Osaka-shi, Osaka 554-0022 (JP); HIROSE, Wataru, Tokyo 103-6012 (JP)
(74) Representative: Vossius & Partner Patentanwälte Rechtsanwälte mbB
(86) International application number: PCT/JP2022/047583
(87) International publication number: WO 2023/120696

(57) **Abstract**

Provided is a compound that exerts an anti-cancer action based on checkpoint kinase 1 (CHK1) inhibition. It was found that the following compound represented by formula (1) or a pharmaceutically acceptable salt thereof has a potent inhibitory action against CHK1, and thereby exhibits an excellent anti-tumor action, which resulted in the completion of the present disclosure: 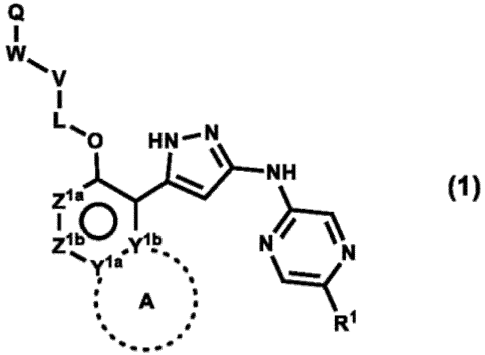 wherein R¹, L, V, W, Q, Ring A, Y^{1a}, Y^{1b}, Z^{1a}, and Z^{1b} is as defined in the description.

## Description

### [Technical Field]

The present disclosure relates to a 1H-pyrazol-3-amine derivative having a bicyclic skeleton which is useful as a medicament for inhibiting checkpoint kinase 1 (CHK1) to treat or prevent cancer characterized by a malfunction in deoxyribonucleic acid (DNA) replication, chromosome segregation or cell division, and a pharmaceutically acceptable salt thereof, a pharmaceutical composition comprising the same, a liposome comprising the same, and a therapeutic agent or prophylactic agent for a pathological condition associated with CHK1, comprising the composition or the liposome.

### [Background Art]

CHK1 is a serine/threonine protein kinase downstream of ATR in a checkpoint signaling pathway of DNA damage during a cell cycle. In mammals, CHK1 is ATR-dependently phosphorylated in response to a DNA damage induced by ionizing radiation or the like, or DNA replication stress due to excessive cell growth or genome instability in cancer cells or the like (Non Patent Literatures 1 to 5). Through such phosphorylation that activates CHK1, CHK1 phosphorylates CDC25A, and dephosphorylation of cyclin E/CDK2 by CDC25A is inhibited, and progression from the S phase is discontinued. CHK1 also phosphorylates CDC25C, and dephosphorylation of cyclin B/CDK1 by CDC25C is inhibited, and progression from the G2M phase is discontinued. In either case, regulation of CDK activity induces discontinuation of the cell cycle and obstruction of cell division in the presence of a DNA damage to promote repair of the DNA damage. In a cancer cell, inhibition of CHK1 suppresses a checkpoint function of a DNA damage in a cell cycle, inducing DNA repair deficiency, uncontrollable DNA synthesis, etc. in the presence of a DNA damage, resulting in induction of a DNA fragmentation, replication catastrophe, and cell death (Patent Literatures 1 to 2).

Various CHK1 inhibitors (Patent Literatures 1 to 4) have been reported.

### [Citation List]

### [Patent Literature]

[PTL 1]
   WO 2010/077758
[PTL 2]
   WO 2012/064548
[PTL 3]
   WO 2017/132928
[PTL 4]
   WO 2021/043208

### [Non Patent Literature]

[NPL 1]
   Dai Y and Grant S, Clin Cancer Res, 16(2):376-383 (2010)
[NPL 2]
   Benada J and Macurek L, Biomolecules, 5:1912-1937 (2015)
[NPL 3]
   King C, Mol Cancer Ther, 14(9):2004-2013 (2015)
[NPL 4]
   Dent P, Expert Opinion on Investigational drugs, 28(12):1095-1100 (2019)
[NPL 5]
   Evangelisti G. et al, Expert Opinion on Investigational Drugs, 29(8):779-792 (2020)
[NPL 6]
   David H. et al, J Clin Oncol, 34:1764-1771 (2016)

### [Summary of the Invention]

### [Solution to Problem]

The present disclosure provides a compound that exerts an anti-cancer action based on CHK1 inhibition. Preferably, the present disclosure provides a compound having a certain difference between a concentration of a compound that suppresses the growth of CHK1-expressing cancer cells and a concentration of a compound that suppresses hERG current, a compound having a certain difference between a concentration of a compound that suppresses a CHK1 inhibitory activity and a concentration of a compound that induces toxicity to hemocytes, and/or a compound, which is encapsulated in a liposome and is released in a sustained manner from the liposome. Specifically, an anti-tumor agent with a high therapeutic effect while reducing side effects is provided.

The present inventors have conducted intensive studies in an attempt to solve the above-mentioned problem and found that the following compound represented by formula (1) or a pharmaceutically acceptable salt thereof (hereinafter to be also referred to as "the compound of the present disclosure") has a potent inhibitory action against CHK1, and thereby exhibits an excellent anti-tumor action, which resulted in the completion of the present disclosure.

Accordingly, the present disclosure is as follows.

### [Item 1]

A compound represented by 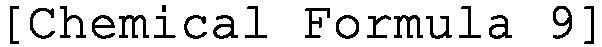 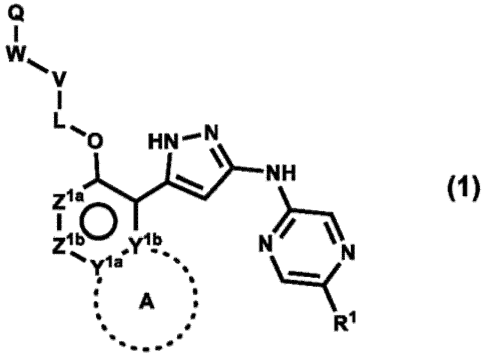 or a pharmaceutically acceptable salt thereof, wherein
R¹ represents a hydrogen atom, a halogen atom, cyano, nitro, carboxyl, a sulfonic acid, a phosphoric acid, -OR², -SR², -COR³, -CO₂R³, -CONR⁴R⁵, -SO₂R³, -SO₂NR⁴R⁵, -OCOR³, -OCO₂R³, -OCONR⁴R⁵, -NR⁴R⁵, -NR⁶COR³, -NR⁶CO₂R³, - NR⁶CONR⁴R⁵, -NR⁶SO₂R³, -NR⁶SO₂NR⁴R⁵, optionally substituted C₁₋₆ alkyl, optionally substituted C₂₋₆ alkenyl, optionally substituted C₂₋₆ alkynyl, optionally substituted C₃₋₁₀ cycloalkyl, an optionally substituted 3- to 10-membered saturated heterocyclic group, optionally substituted C₆₋₁₀ aryl, or optionally substituted 5- to 12-membered heteroaryl,
R² represents a hydrogen atom or C₁₋₆ alkyl,
R³ represents C₁₋₆ alkyl,
R⁴, R⁵ and R⁶ represent each independently a hydrogen atom, or C₁₋₆ alkyl, wherein when R⁴ and R⁵ attached to the same nitrogen atom are both C₁₋₆ alkyl, then they may form a 3- to 8-membered nitrogen-containing saturated heterocycle together with the nitrogen atom to which each is attached,
Y^{1a} and Y^{1b} represent each independently a carbon atom, or a nitrogen atom,
Z^{1a} represents a nitrogen atom, or CR^{7a},
Z^{1b} represents a nitrogen atom, or CR^{7b},
R^{7a} and R^{7b} represent each independently a hydrogen atom, a halogen atom, cyano, nitro, carboxyl, a sulfonic acid, a phosphoric acid, -OR⁸, -SR⁸, -COR⁹, -CO₂R⁹, - CONR¹⁰R¹¹, -SO₂R⁹, -SO₂NR¹⁰R¹¹, -OCOR⁹, -OCO₂R⁹, -OCONR¹⁰R¹¹, - NR¹⁰R¹¹, -NR¹²COR⁹, -NR¹²CO₂R⁹, -NR¹²CONR¹⁰R¹¹, -NR¹²SO₂R⁹, - NR¹²SO₂NR¹⁰R¹¹, optionally substituted C₁₋₆ alkyl, optionally substituted C₂₋₆ alkenyl, optionally substituted C₂₋₆ alkynyl, optionally substituted C₃₋₁₀ cycloalkyl, an optionally substituted 3- to 10-membered saturated heterocyclic group, optionally substituted C₆₋₁₀ aryl, or optionally substituted 5- to 12-membered heteroaryl,
R⁸ represents a hydrogen atom or C₁₋₆ alkyl,
R⁹ represents C₁₋₆ alkyl,
R¹⁰, R¹¹ and R¹² represent each independently a hydrogen atom or C₁₋₆ alkyl, wherein when R¹¹ and R¹² attached to the same nitrogen atom are both C₁₋₆ alkyl, then they may form a 3- to 8-membered nitrogen-containing saturated heterocycle together with the nitrogen atom to which each is attached,
Ring A represents optionally substituted 3- to 10-membered cycloalkane, optionally substituted 6- to 10-membered arene, an optionally substituted 4- to 10-membered aromatic heterocycle, or an optionally substituted 4- to 10-membered non-aromatic heterocycle,
L represents a single bond or optionally substituted C₁₋₆ alkylene,
V represents a single bond, carbonyl, thiocarbonyl, optionally substituted C₂₋₆ alkenylene, optionally substituted C₃₋₁₀ cycloalkylene, optionally substituted C₃₋₁₀ cycloalkenylene, an optionally substituted 3- to 10-membered divalent saturated heterocyclic group, or - C(=NR¹³)-,
R¹³ represents a hydrogen atom, or C₁₋₆ alkyl,
W represents a single bond, or optionally substituted C₁₋₆ alkylene,
Q represents a hydrogen atom, or NHR¹⁴, and
R¹⁴ represents a hydrogen atom, optionally substituted C₁₋₆ alkyl, optionally substituted C₃₋₁₀ cycloalkyl, or an optionally substituted 3- to 10-membered saturated heterocyclic group.

### [Item 2]

The compound or the pharmaceutically acceptable salt thereof of item 1, wherein the optionally substituted C₁₋₆ alkyl, the optionally substituted C₂₋₆ alkenyl, the optionally substituted C₂₋₆ alkynyl, the optionally substituted C₂₋₆ alkenylene, the optionally substituted C₃₋₁₀ cycloalkyl, the optionally substituted 3- to 10-membered saturated heterocyclic group, the optionally substituted 6- to 10-membered arene, the optionally substituted 4- to 10-membered aromatic heterocycle, the optionally substituted 3- to 10-membered cycloalkane, the optionally substituted 4- to 10-membered non-aromatic heterocycle, the optionally substituted C₆₋₁₀ aryl, the optionally substituted 5- to 12-membered heteroaryl, the optionally substituted C₁₋₆ alkylene, the optionally substituted C₃₋₁₀ cycloalkylene, the optionally substituted C₃₋₁₀ cycloalkenylene, or the optionally substituted 3- to 10-membered divalent saturated heterocyclic group in R¹, R^{7a}, R^{7b}, R¹⁴, Ring A, L, V, and W are each independently optionally substituted with 1 to 5 of the same or different substituents selected from the group consisting of
(1) a halogen atom,
(2) a hydroxyl group,
(3) C₆₋₁₀ aryl,
(4) 5- to 12-membered heteroaryl,
(5) C₁₋₆ alkyl,
(6) C₂₋₆ alkenyl,
(7) C₂₋₆ alkynyl,
(8) C₁₋₆ alkoxy,
(9) C₁₋₆ alkylthio
(10) C₃₋₁₀ cycloalkyl,
(11) a 3- to 10-membered saturated heterocyclic group,
(12) carboxyl,
(13) -COR¹⁵,
(14) -CO₂R¹⁵,
(15) -CONR¹⁶R¹⁷,
(16) -NR¹⁶R¹⁷,
(17) -NR¹⁸COR¹⁵,
(18) -NR¹⁸CO₂R¹⁵,
(19) -NR¹⁸SO₂R¹⁵,
(20) -NR¹⁸CONR¹⁶R¹⁷,
(21) -NR¹⁸SO₂NR¹⁶R¹⁷,
(22) -SO₂R¹⁵,
(23) -SO₂NR¹⁶R¹⁷,
(24) -OCOR¹⁵,
(25) -OCO₂R¹⁵,
(26) -OCONR¹⁶R¹⁷,
(27) a sulfonic acid,
(28) a phosphoric acid,
(29) cyano, and
(30) nitro,

wherein the groups represented by the (3) C₆₋₁₀ aryl, (4) 5- to 12-membered heteroaryl, (5) C₁₋₆ alkyl, (6) C₂₋₆ alkenyl, (7) C₂₋₆ alkynyl, (8) C₁₋₆ alkoxy, (9) C₁₋₆ alkylthio, (10) C₃₋₁₀ cycloalkyl and (11) a 3- to 10-membered saturated heterocyclic group are optionally substituted with 1 to 5 of the same or different substituents selected from the group consisting of
   (a) a halogen atom,
   (b) a hydroxyl group,
   (c) C₆₋₁₀ aryl,
   (d) 5- to 12-membered heteroaryl,
   (e) C₁₋₆ alkyl,
   (f) C₂₋₆ alkenyl,
   (g) C₂₋₆ alkynyl,
   (h) C₁₋₆ alkoxy,
   (i) C₃₋₁₀ cycloalkyl,
   (j) a 3- to 10-membered saturated heterocyclic group,
   (k) carboxyl,
   (l) -COR¹⁵,
   (m) -CO₂R¹⁵,
   (n) -CONR¹⁶R¹⁷,
   (o) -NR¹⁶R¹⁷,
   (p) -NR¹⁸COR¹⁵,
   (q) -NR¹⁸SO₂R¹⁵,
   (r) -SO₂R¹⁵,
   (s) -SO₂NR¹⁶R¹⁷,
   (t) a sulfonic acid,
   (u) a phosphoric acid,
   (v) cyano, and
   (w) nitro,
R¹⁵ is C₁₋₆ alkyl, and when plural, then each is independent,
R¹⁶ and R¹⁷ represent each independently a hydrogen atom or C₁₋₆ alkyl, and when R¹⁶ or R¹⁷ is plural, then each of R¹⁶ or R¹⁷ may be the same or different, wherein when R¹⁶ and R¹⁷ attached to the same nitrogen atom are both C₁₋₆ alkyl, then they may form a 3- to 8-membered nitrogen-containing saturated heterocycle together with the nitrogen atom to which each is attached, and
R¹⁸ is a hydrogen atom or C₁₋₆ alkyl.

### [Item 3]

The compound or the pharmaceutically acceptable salt thereof of any one of items 1 to 2, wherein the optionally substituted C₁₋₆ alkyl, the optionally substituted C₂₋₆ alkenyl, the optionally substituted C₂₋₆ alkynyl, the optionally substituted C₂₋₆ alkenylene, the optionally substituted C₃₋₁₀ cycloalkyl, the optionally substituted 3- to 10-membered saturated heterocyclic group, the optionally substituted 6- to 10-membered arene, the optionally substituted 4- to 10-membered aromatic heterocycle, the optionally substituted 3- to 10-membered cycloalkane, the optionally substituted 4- to 10-membered non-aromatic heterocycle, the optionally substituted C₆₋₁₀ aryl, the optionally substituted 5- to 12-membered heteroaryl, the optionally substituted C₁₋₆ alkylene, the optionally substituted C₃₋₁₀ cycloalkylene, the optionally substituted C₃₋₁₀ cycloalkenylene, or the optionally substituted 3- to 10-membered divalent saturated heterocyclic group in R¹, R^{7a}, R^{7b}, R¹⁴, Ring A, L, V, and W are each independently optionally substituted with 1 to 5 of the same or different substituents selected from the group consisting of
(1) a halogen atom,
(2) a hydroxyl group,
(3) C₆₋₁₀ aryl,
(4) 5- to 12-membered heteroaryl,
(5) C₁₋₆ alkyl optionally substituted with 1 to 3 halogen atoms,
(6) C₂₋₆ alkenyl,
(7) C₂₋₆ alkynyl,
(8) C₁₋₆ alkoxy,
(9) C₃₋₁₀ cycloalkyl,
(10) a 3- to 10-membered saturated heterocyclic group,
(11) carboxyl,
(12) -COR¹⁵,
(13) -CO₂R¹⁵,
(14) -CONR¹⁶R¹⁷,
(15) -NR¹⁶R¹⁷,
(16) -SO₂R¹⁵,
(17) -SO₂NR¹⁶R¹⁷,
(18) a sulfonic acid,
(19) a phosphoric acid,
(20) cyano, and
(21) nitro,

R¹⁵ is C₁₋₆ alkyl, and when plural, then each is independent, and
R¹⁶ and R¹⁷ represent each independently a hydrogen atom or C₁₋₆ alkyl, and when R¹⁶ or R¹⁷ is plural, then each of R¹⁶ or R¹⁷ may be the same or different, wherein when R¹⁶ and R¹⁷ attached to the same nitrogen atom are both C₁₋₆ alkyl, then they may form a 3- to 8-membered nitrogen-containing saturated heterocycle together with the nitrogen atom to which each is attached.

### [Item 4]

The compound or the pharmaceutically acceptable salt thereof of any one of items 1 to 3, wherein the optionally substituted C₁₋₆ alkyl, the optionally substituted C₂₋₆ alkenyl, the optionally substituted C₂₋₆ alkynyl, the optionally substituted C₂₋₆ alkenylene, the optionally substituted C₃₋₁₀ cycloalkyl, the optionally substituted 3- to 10-membered saturated heterocyclic group, the optionally substituted 6- to 10-membered arene, the optionally substituted 4- to 10-membered aromatic heterocycle, the optionally substituted 3- to 10-membered cycloalkane, the optionally substituted 4- to 10-membered non-aromatic heterocycle, the optionally substituted C₆₋₁₀ aryl, the optionally substituted 5- to 12-membered heteroaryl, the optionally substituted C₁₋₆ alkylene, the optionally substituted C₃₋₁₀ cycloalkylene, the optionally substituted C₃₋₁₀ cycloalkenylene, or the optionally substituted 3- to 10-membered divalent saturated heterocyclic group in R¹, R^{7a}, R^{7b}, R¹⁴, Ring A, L, V, and W are each independently optionally substituted with 1 to 5 of the same or different substituents selected from the group consisting of
(1) a halogen atom,
(2) a hydroxyl group,
(3) phenyl,
(4) 5- to 6-membered heteroaryl,
(5) C₁₋₆ alkyl optionally substituted with 1 to 3 halogen atoms,
(6) C₁₋₆ alkoxy,
(7) C₃₋₇ cycloalkyl,
(8) a 3- to 7-membered saturated heterocyclic group,
(9) -COR¹⁵,
(10) -CO₂R¹⁵,
(11) -CONR¹⁶R¹⁷,
(12) -NR¹⁶R¹⁷,
(13) -SO₂R¹⁵,
(14) -SO₂NR¹⁶R¹⁷, and
(15) cyano,

R¹⁵ is C₁₋₆ alkyl, and when plural, then each is independent, and
R¹⁶ and R¹⁷ represent each independently a hydrogen atom or C₁₋₆ alkyl, and when R¹⁶ or R¹⁷ is plural, then each of R¹⁶ or R¹⁷ may be the same or different, wherein when R¹⁶ and R¹⁷ attached to the same nitrogen atom are both C₁₋₆ alkyl, then they may form a 3- to 8-membered nitrogen-containing saturated heterocycle together with the nitrogen atom to which each is attached.

### [Item 5]

The compound or the pharmaceutically acceptable salt thereof of any one of items 1 to 4, wherein R¹ is a hydrogen atom, a halogen atom, cyano, -OR², -CO₂R³, C₁₋₆ alkyl, C₃₋₁₀ cycloalkyl, or a 3- to 10-membered saturated heterocyclic group.

### [Item 6]

The compound or the pharmaceutically acceptable salt thereof of any one of items 1 to 4, wherein R¹ is a hydrogen atom, a halogen atom, cyano, -CO₂R³ or C₁₋₆ alkyl.

### [Item 6A]

The compound or the pharmaceutically acceptable salt thereof of any one of items 1 to 4, wherein R¹ is a hydrogen atom, a halogen atom, cyano, or -CO₂R³.

### [Item 7]

The compound or the pharmaceutically acceptable salt thereof of any one of items 1 to 4, wherein R¹ is cyano.

### [Item 8]

The compound or the pharmaceutically acceptable salt thereof of any one of items 1 to 7, wherein
R^{7a} and R^{7b} are each independently a hydrogen atom, a halogen atom, cyano, -OR⁸, -CO₂R⁹, -CONR¹⁰R¹¹, -NR¹⁰R¹¹, - NR¹²COR⁹, C₁₋₆ alkyl (the alkyl is optionally substituted with 1 to 3 of the same or different substituents selected from the group consisting of a fluorine atom, a chlorine atom, a bromine atom, a hydroxyl group, phenyl, 5- to 6-membered heteroaryl, C₁₋₆ alkoxy, C₃₋₇ cycloalkyl, a 3- to 7-membered saturated heterocyclic group, - CONR¹⁶R¹⁷, -NR¹⁶R¹⁷, and cyano), C₃₋₁₀ cycloalkyl (the cycloalkyl is optionally substituted with 1 to 3 of the same or different substituents selected from the group consisting of a fluorine atom, a chlorine atom, a bromine atom, a hydroxyl group, phenyl, 5- to 6-membered heteroaryl, C₁₋₆ alkyl, C₁₋₆ alkoxy, C₃₋₇ cycloalkyl, a 3-to 7-membered saturated heterocyclic group, -CONR¹⁶R¹⁷, - NR¹⁶R¹⁷, and cyano), a 3- to 10-membered saturated heterocyclic group (the saturated heterocyclic group is optionally substituted with 1 to 3 of the same or different substituents selected from the group consisting of a fluorine atom, a chlorine atom, a bromine atom, a hydroxyl group, phenyl, 5- to 6-membered heteroaryl, C₁₋₆ alkyl, C₁₋₆ alkoxy, C₃₋₇ cycloalkyl, a 3- to 7-membered saturated heterocyclic group, -CONR¹⁶R¹⁷, -NR¹⁶R¹⁷, and cyano), phenyl (the phenyl is optionally substituted with 1 to 3 of the same or different substituents selected from the group consisting of a fluorine atom, a chlorine atom, a bromine atom, a hydroxyl group, phenyl, 5- to 6-membered heteroaryl, C₁₋₆ alkyl, C₁₋₆ alkoxy, C₃₋₇ cycloalkyl, a 3- to 7-membered saturated heterocyclic group, -CONR¹⁶R¹⁷, -NR¹⁶R¹⁷, and cyano), or 5- to 6-membered heteroaryl (the heteroaryl is optionally substituted with 1 to 3 of the same or different substituents selected from the group consisting of a fluorine atom, a chlorine atom, a bromine atom, a hydroxyl group, phenyl, 5- to 6-membered heteroaryl, C₁₋₆ alkyl, C₁₋₆ alkoxy, C₃₋₇ cycloalkyl, a 3- to 7-membered saturated heterocyclic group, -CONR¹⁶R¹⁷, -NR¹⁶R¹⁷, and cyano), and
R¹⁶ and R¹⁷ represent each independently a hydrogen atom or C₁₋₆ alkyl, and when R¹⁶ or R¹⁷ is plural, then each of R¹⁶ or R¹⁷ may be the same or different, wherein when R¹⁶ and R¹⁷ attached to the same nitrogen atom are both C₁₋₆ alkyl, then they may form a 3- to 8-membered nitrogen-containing saturated heterocycle together with the nitrogen atom to which each is attached.

### [Item 9]

The compound or the pharmaceutically acceptable salt thereof of any one of items 1 to 7, wherein
R^{7a} and R^{7b} are each independently
   a hydrogen atom,
   a halogen atom
   -OR⁹,
   C₁₋₆ alkyl (the alkyl is optionally substituted with 1 to 3 of the same or different substituents selected from the group consisting of a fluorine atom, a hydroxyl group, C₁₋₃ alkoxy, -NR¹⁶R¹⁷, and cyano),
   C₃₋₇ cycloalkyl (the cycloalkyl is optionally substituted with 1 to 3 of the same or different substituents selected from the group consisting of a fluorine atom, a hydroxyl group, C₁₋₃ alkyl, C₁₋₃ alkoxy, -NR¹⁶R¹⁷, and cyano),
   a 3- to 7-membered saturated heterocyclic group (the saturated heterocyclic group is optionally substituted with 1 to 3 of the same or different substituents selected from the group consisting of a fluorine atom, a hydroxyl group, C₁₋₃ alkyl, C₁₋₃ alkoxy, -NR¹⁶R¹⁷, and cyano),
   phenyl (the phenyl is optionally substituted with 1 to 3 of the same or different substituents selected from the group consisting of a fluorine atom, a hydroxyl group, C₁₋₃ alkyl, C₁₋₃ alkoxy, -NR¹⁶R¹⁷, and cyano), or 5- to 6-membered heteroaryl (the heteroaryl is optionally substituted with 1 to 3 of the same or different substituents selected from the group consisting of a fluorine atom, a hydroxyl group, C₁₋₃ alkyl, C₁₋₃ alkoxy, - NR¹⁶R¹⁷, and cyano), and
R¹⁶ and R¹⁷ represent each independently a hydrogen atom or C₁₋₆ alkyl, and when R¹⁶ or R¹⁷ is plural, then each of R¹⁶ or R¹⁷ may be the same or different, wherein when R¹⁶ and R¹⁷ attached to the same nitrogen atom are both C₁₋₆ alkyl, then they may form a 3- to 8-membered nitrogen-containing saturated heterocycle together with the nitrogen atom to which each is attached.

### [Item 10]

The compound or the pharmaceutically acceptable salt thereof of any one of items 1 to 7, wherein
R^{7a} and R^{7b} are each independently
   a hydrogen atom,
   a fluorine atom,
   a chlorine atom,
   a bromine atom,
   C₁₋₆ alkyl (the alkyl is optionally substituted with 1 to 2 of the same or different substituents selected from the group consisting of a fluorine atom, a hydroxyl group, C₁₋₃ alkoxy, -NR¹⁶R¹⁷, and cyano), or
   5- to 6-membered heteroaryl (the heteroaryl is optionally substituted with 1 to 2 of the same or different substituents selected from the group consisting of a fluorine atom, a hydroxyl group, C₁₋₃ alkyl, C₁₋₃ alkoxy, - NR¹⁶R¹⁷, and cyano), and
R¹⁶ and R¹⁷ represent each independently a hydrogen atom or C₁₋₆ alkyl, and when R¹⁶ or R¹⁷ is plural, then each of R¹⁶ or R¹⁷ may be the same or different, wherein when R¹⁶ and R¹⁷ attached to the same nitrogen atom are both C₁₋₆ alkyl, then they may form a 3- to 8-membered nitrogen-containing saturated heterocycle together with the nitrogen atom to which each is attached.

### [Item 11]

The compound or the pharmaceutically acceptable salt thereof of any one of items 1 to 10, wherein
Ring A is
   3- to 10-membered cycloalkane (the cycloalkane is optionally substituted with 1 to 3 of the same or different substituents selected from the group consisting of a fluorine atom, a chlorine atom, a bromine atom, a hydroxyl group, phenyl, 5- to 6-membered heteroaryl, C₁₋₆ alkyl, C₁₋₆ alkoxy, C₃₋₇ cycloalkyl, a 3- to 7-membered saturated heterocyclic group, -CONR¹⁶R¹⁷, -NR¹⁶R¹⁷, and cyano),
   6- to 10-membered arene (the arene is optionally substituted with 1 to 3 of the same or different substituents selected from the group consisting of a fluorine atom, a chlorine atom, a bromine atom, a hydroxyl group, phenyl, 5- to 6-membered heteroaryl, C₁₋₆ alkyl, C₁₋₆ alkoxy, C₃₋₇ cycloalkyl, a 3- to 7-membered saturated heterocyclic group, -CONR¹⁶R¹⁷, -NR¹⁶R¹⁷, and cyano),
   a 4- to 10-membered aromatic heterocycle (the aromatic heterocycle is optionally substituted with 1 to 3 of the same or different substituents selected from the group consisting of a fluorine atom, a chlorine atom, a bromine atom, a hydroxyl group, phenyl, 5- to 6-membered heteroaryl, C₁₋₆ alkyl, C₁₋₆ alkoxy, C₃₋₇ cycloalkyl, a 3-to 7-membered saturated heterocyclic group, -CONR¹⁶R¹⁷, - NR¹⁶R¹⁷, and cyano), or
   a 4- to 10-membered non-aromatic heterocycle (the non-aromatic heterocycle is optionally substituted with 1 to 3 of the same or different substituents selected from the group consisting of a fluorine atom, a chlorine atom, a bromine atom, a hydroxyl group, phenyl, 5- to 6-membered heteroaryl, C₁₋₆ alkyl, C₁₋₆ alkoxy, C₃₋₇ cycloalkyl, a 3-to 7-membered saturated heterocyclic group, -CONR¹⁶R¹⁷, - NR¹⁶R¹⁷, and cyano), and
R¹⁶ and R¹⁷ represent each independently a hydrogen atom or C₁₋₆ alkyl, and when R¹⁶ or R¹⁷ is plural, then each of R¹⁶ or R¹⁷ may be the same or different, wherein when R¹⁶ and R¹⁷ attached to the same nitrogen atom are both C₁₋₆ alkyl, then they may form a 3- to 8-membered nitrogen-containing saturated heterocycle together with the nitrogen atom to which each is attached.

### [Item 12]

The compound or the pharmaceutically acceptable salt thereof of any one of items 1 to 10, wherein
Ring A is
   3- to 10-membered cycloalkane (the cycloalkane is optionally substituted with 1 to 3 of the same or different substituents selected from the group consisting of a fluorine atom, a hydroxyl group, C₁₋₃ alkyl, C₁₋₃ alkoxy, -NR¹⁶R¹⁷, and cyano),
   6- to 10-membered arene (the arene is optionally substituted with 1 to 3 of the same or different substituents selected from the group consisting of a fluorine atom, a hydroxyl group, C₁₋₃ alkyl, C₁₋₃ alkoxy, - NR¹⁶R¹⁷, and cyano),
   a 4- to 10-membered aromatic heterocycle (the aromatic heterocycle is optionally substituted with 1 to 3 of the same or different substituents selected from the group consisting of a fluorine atom, a hydroxyl group, C₁₋₃ alkyl, C₁₋₃ alkoxy, -NR¹⁶R¹⁷, and cyano), or
   a 4- to 10-membered non-aromatic heterocycle (the non-aromatic heterocycle is optionally substituted with 1 to 3 of the same or different substituents selected from the group consisting of a fluorine atom, a hydroxyl group, C₁₋₃ alkyl, C₁₋₃ alkoxy, -NR¹⁶R¹⁷, and cyano), and
R¹⁶ and R¹⁷ represent each independently a hydrogen atom or C₁₋₆ alkyl, and when R¹⁶ or R¹⁷ is plural, then each of R¹⁶ or R¹⁷ may be the same or different, wherein when R¹⁶ and R¹⁷ attached to the same nitrogen atom are both C₁₋₆ alkyl, then they may form a 3- to 8-membered nitrogen-containing saturated heterocycle together with the nitrogen atom to which each is attached.

### [Item 13]

The compound or the pharmaceutically acceptable salt thereof of any one of items 1 to 10, wherein
Ring A is
   a 4- to 10-membered aromatic heterocycle (the aromatic heterocycle is optionally substituted with 1 to 2 of the same or different substituents selected from the group consisting of a fluorine atom, a hydroxyl group, C₁₋₃ alkyl, C₁₋₃ alkoxy, -NR¹⁶R¹⁷, and cyano), or
   a 4- to 10-membered non-aromatic heterocycle (the non-aromatic heterocycle is optionally substituted with 1 to 2 of the same or different substituents selected from the group consisting of a fluorine atom, a hydroxyl group, C₁₋₃ alkyl, C₁₋₃ alkoxy, -NR¹⁶R¹⁷, and cyano), and
R¹⁶ and R¹⁷ represent each independently a hydrogen atom or C₁₋₆ alkyl, and when R¹⁶ or R¹⁷ is plural, then each of R¹⁶ or R¹⁷ may be the same or different, wherein when R¹⁶ and R¹⁷ attached to the same nitrogen atom are both C₁₋₆ alkyl, then they may form a 3- to 8-membered nitrogen-containing saturated heterocycle together with the nitrogen atom to which each is attached.

### [Item 14]

The compound or the pharmaceutically acceptable salt thereof of any one of items 1 to 13, wherein
L is
   a single bond, or
   C₁₋₆ alkylene (the alkylene is optionally substituted with 1 to 3 of the same or different substituents selected from the group consisting of a fluorine atom, a hydroxyl group, C₁₋₃ alkyl, C₁₋₃ alkoxy, -NR¹⁶R¹⁷, and cyano), and
R¹⁶ and R¹⁷ represent each independently a hydrogen atom or C₁₋₆ alkyl, and when R¹⁶ or R¹⁷ is plural, then each of R¹⁶ or R¹⁷ may be the same or different, wherein when R¹⁶ and R¹⁷ attached to the same nitrogen atom are both C₁₋₆ alkyl, then they may form a 3- to 8-membered nitrogen-containing saturated heterocycle together with the nitrogen atom to which each is attached.

### [Item 15]

The compound or the pharmaceutically acceptable salt thereof of any one of items 1 to 14, wherein
V is
   a single bond,
   carbonyl,
   thiocarbonyl,
   C₂₋₆ alkenylene (the alkenylene is optionally substituted with 1 to 3 of the same or different substituents selected from the group consisting of a fluorine atom, a hydroxyl group, C₁₋₃ alkyl, C₁₋₃ alkoxy, -NR¹⁶R¹⁷, and cyano),
   C₃₋₁₀ cycloalkylene (the cycloalkylene is optionally substituted with 1 to 3 of the same or different substituents selected from the group consisting of a fluorine atom, a hydroxyl group, C₁₋₃ alkyl, C₁₋₃ alkoxy, - NR¹⁶R¹⁷, and cyano),
   C₃₋₁₀ cycloalkenylene (the cycloalkenylene is optionally substituted with 1 to 3 of the same or different substituents selected from the group consisting of a fluorine atom, a hydroxyl group, C₁₋₃ alkyl, C₁₋₃ alkoxy, - NR¹⁶R¹⁷, and cyano),
   a 3- to 10-membered divalent saturated heterocyclic group (the saturated heterocyclic group is optionally substituted with 1 to 3 of the same or different substituents selected from the group consisting of a fluorine atom, a hydroxyl group, C₁₋₃ alkyl optionally substituted with 1 to 2 hydroxyl groups or fluorine atoms, C₁₋₃ alkoxy, -NR¹⁶R¹⁷, and cyano), or
   -C(=NR¹³)-, and
R¹⁶ and R¹⁷ represent each independently a hydrogen atom or C₁₋₆ alkyl, and when R¹⁶ or R¹⁷ is plural, then each of R¹⁶ or R¹⁷ may be the same or different, wherein when R¹⁶ and R¹⁷ attached to the same nitrogen atom are both C₁₋₆ alkyl, then they may form a 3- to 8-membered nitrogen-containing saturated heterocycle together with the nitrogen atom to which each is attached.

### [Item 16]

The compound or the pharmaceutically acceptable salt thereof of any one of items 1 to 15, wherein
W is
   a single bond, or
   C₁₋₆ alkylene (the alkylene is optionally substituted with 1 to 3 of the same or different substituents selected from the group consisting of a fluorine atom, a hydroxyl group, C₁₋₃ alkoxy, -NR¹⁶R¹⁷, and cyano), and
R¹⁶ and R¹⁷ represent each independently a hydrogen atom or C₁₋₆ alkyl, and when R¹⁶ or R¹⁷ is plural, then each of R¹⁶ or R¹⁷ may be the same or different, wherein when R¹⁶ and R¹⁷ attached to the same nitrogen atom are both C₁₋₆ alkyl, then they may form a 3- to 8-membered nitrogen-containing saturated heterocycle together with the nitrogen atom to which each is attached.

### [Item 17]

The compound or the pharmaceutically acceptable salt thereof of any one of items 1 to 16, wherein
R¹⁴ is
   a hydrogen atom,
   C₁₋₆ alkyl (the alkyl is optionally substituted with 1 to 3 of the same or different substituents selected from the group consisting of a fluorine atom, a hydroxyl group, C₁₋₃ alkoxy, -NR¹⁶R¹⁷, and cyano),
   C₃₋₁₀ cycloalkyl (the cycloalkyl is optionally substituted with 1 to 3 of the same or different substituents selected from the group consisting of a fluorine atom, a hydroxyl group, C₁₋₃ alkyl, C₁₋₃ alkoxy, -NR¹⁶R¹⁷, and cyano), or
   a 3- to 10-membered saturated heterocyclic group (the saturated heterocyclic group is optionally substituted with 1 to 3 of the same or different substituents selected from the group consisting of a fluorine atom, a hydroxyl group, C₁₋₃ alkyl, C₁₋₃ alkoxy, -NR¹⁶R¹⁷, and cyano), and
R¹⁶ and R¹⁷ represent each independently a hydrogen atom or C₁₋₆ alkyl, and when R¹⁶ or R¹⁷ is plural, then each of R¹⁶ or R¹⁷ may be the same or different, wherein when R¹⁶ and R¹⁷ attached to the same nitrogen atom are both C₁₋₆ alkyl, then they may form a 3- to 8-membered nitrogen-containing saturated heterocycle together with the nitrogen atom to which each is attached.

### [Item 18]

The compound or the pharmaceutically acceptable salt thereof of item 1, wherein formula (1) is represented by the following formula (2): 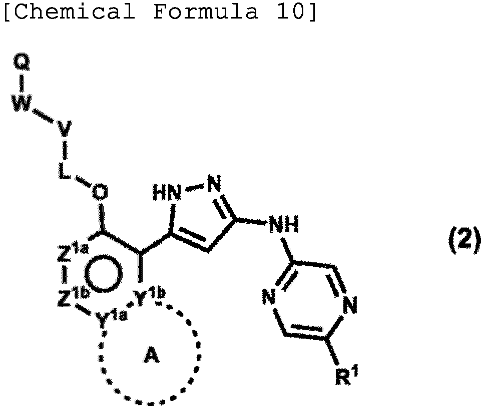 wherein
R¹ represents a hydrogen atom, a halogen atom, cyano, or -CO₂R³,
R³ represents C₁₋₆ alkyl,
Y^{1a} and Y^{1b} represent each independently a carbon atom, or a nitrogen atom,
Z^{1a} represents a nitrogen atom, or CR^{7a},
Z^{1b} represents a nitrogen atom, or CR^{7b},
R^{7a} and R^{7b} represent each independently
   a hydrogen atom,
   a fluorine atom,
   a chlorine atom,
   a bromine atom,
   C₁₋₆ alkyl (the alkyl is optionally substituted with 1 to 2 of the same or different substituents selected from the group consisting of a fluorine atom, a hydroxyl group, C₁₋₃ alkoxy, -NR¹⁶R¹⁷, and cyano), or
   5- to 6-membered heteroaryl (the heteroaryl is optionally substituted with 1 to 2 of the same or different substituents selected from the group consisting of a fluorine atom, a hydroxyl group, C₁₋₃ alkyl, C₁₋₃ alkoxy, - NR¹⁶R¹⁷, and cyano),
Ring A represents
   a 5- to 6-membered aromatic heterocycle (the aromatic heterocycle is optionally substituted with 1 to 2 of the same or different substituents selected from the group consisting of a fluorine atom, a hydroxyl group, C₁₋₃ alkyl, C₁₋₃ alkoxy, -NR¹⁶R¹⁷, and cyano), or
   a 5- to 8-membered non-aromatic heterocycle (the non-aromatic heterocycle is optionally substituted with 1 to 2 of the same or different substituents selected from the group consisting of a fluorine atom, a hydroxyl group, C₁₋₃ alkyl, C₁₋₃ alkoxy, -NR¹⁶R¹⁷, and cyano),
L represents
   a single bond, or
   C₁₋₆ alkylene (the alkylene is optionally substituted with 1 to 3 of the same or different substituents selected from the group consisting of a fluorine atom, a hydroxyl group, C₁₋₃ alkyl, C₁₋₃ alkoxy, -NR¹⁶R¹⁷, and cyano),
V represents
   a single bond,
   C₂₋₆ alkenylene (the alkenylene is optionally substituted with 1 to 3 of the same or different substituents selected from the group consisting of a fluorine atom, a hydroxyl group, C₁₋₃ alkyl, C₁₋₃ alkoxy, -NR¹⁶R¹⁷, and cyano),
   C₃₋₁₀ cycloalkylene (the cycloalkylene is optionally substituted with 1 to 3 of the same or different substituents selected from the group consisting of a fluorine atom, a hydroxyl group, C₁₋₃ alkyl, C₁₋₃ alkoxy, - NR¹⁶R¹⁷, and cyano),
   C₃₋₁₀ cycloalkenylene (the cycloalkenylene is optionally substituted with 1 to 3 of the same or different substituents selected from the group consisting of a fluorine atom, a hydroxyl group, C₁₋₃ alkyl, C₁₋₃ alkoxy, - NR¹⁶R¹⁷, and cyano), or
   a 3- to 10-membered divalent saturated heterocyclic group (the saturated heterocyclic group is optionally substituted with 1 to 3 of the same or different substituents selected from the group consisting of a fluorine atom, a hydroxyl group, C₁₋₃ alkyl optionally substituted with 1 to 2 fluorine atoms, C₁₋₃ alkoxy, - NR¹⁶R¹⁷, and cyano),
W represents
   a single bond, or
   C₁₋₆ alkylene (the alkylene is optionally substituted with 1 to 3 of the same or different substituents selected from the group consisting of a fluorine atom, a hydroxyl group, C₁₋₃ alkoxy, -NR¹⁶R¹⁷, and cyano),
Q represents
   a hydrogen atom, or
   NHR¹⁴,
R¹⁴ represents
   a hydrogen atom,
   C₁₋₆ alkyl (the alkyl is optionally substituted with 1 to 3 of the same or different substituents selected from the group consisting of a fluorine atom, a hydroxyl group, C₁₋₃ alkoxy, -NR¹⁶R¹⁷, and cyano),
   C₃₋₁₀ cycloalkyl (the cycloalkyl is optionally substituted with 1 to 3 of the same or different substituents selected from the group consisting of a fluorine atom, a hydroxyl group, C₁₋₃ alkyl, C₁₋₃ alkoxy, -NR¹⁶R¹⁷, and cyano), or
   a 3- to 10-membered saturated heterocyclic group (the saturated heterocyclic group is optionally substituted with 1 to 3 of the same or different substituents selected from the group consisting of a fluorine atom, a hydroxyl group, C₁₋₃ alkyl, C₁₋₃ alkoxy, -NR¹⁶R¹⁷, and cyano), and
R¹⁶ and R¹⁷ represent each independently a hydrogen atom, or C₁₋₆ alkyl, and when R¹⁶ or R¹⁷ is plural, then each of R¹⁶ or R¹⁷ may be the same or different, wherein when R¹⁶ and R¹⁷ attached to the same nitrogen atom are both C₁₋₆ alkyl, then they may form a 3- to 8-membered nitrogen-containing saturated heterocycle together with the nitrogen atom to which each is attached.

### [Item 18A]

The compound or the pharmaceutically acceptable salt thereof of any one of items 1 to 18, wherein R¹ is cyano.

### [Item 19]

The compound or the pharmaceutically acceptable salt thereof of any one of items 1 to 18 and 18A, wherein Z^{1a} is CR^{7a}.

### [Item 20]

The compound or the pharmaceutically acceptable salt thereof of any one of items 1 to 19, wherein Z^{1b} is CR^{7b}.

### [Item 21]

The compound or the pharmaceutically acceptable salt thereof of any one of items 1 to 19, wherein Z^{1b} is a nitrogen atom.

### [Item 22]

The compound or the pharmaceutically acceptable salt thereof of any one of items 1 to 21, wherein R^{7a} and R^{7b} are each independently
a hydrogen atom,
a fluorine atom,
a chlorine atom,
a bromine atom, or
C₁₋₆ alkyl (the alkyl is optionally substituted with 1 to 2 of the same or different substituents selected from the group consisting of a fluorine atom, a hydroxyl group, and C₁₋₃ alkoxy) .

### [Item 23]

The compound or the pharmaceutically acceptable salt thereof of any one of items 1 to 21, wherein R^{7a} and R^{7b} are each independently a hydrogen atom, a fluorine atom, a chlorine atom, a bromine atom, or C₁₋₃ alkyl.

### [Item 24]

The compound or the pharmaceutically acceptable salt thereof of any one of items 1 to 21, wherein R^{7a} and R^{7b} are hydrogen atoms.

### [Item 25]

The compound or the pharmaceutically acceptable salt thereof of any one of items 1 to 24, wherein L is a single bond, or
C₁₋₆ alkylene (the alkylene is optionally substituted with 1 to 2 of the same or different substituents selected from the group consisting of a fluorine atom, a hydroxyl group, C₁₋₃ alkyl, and cyano) .

### [Item 26]

The compound or the pharmaceutically acceptable salt thereof of any one of items 1 to 25, wherein V is a single bond,
C₂₋₆ alkenylene (the alkenylene is optionally substituted with 1 to 3 of the same or different substituents selected from the group consisting of a fluorine atom, a hydroxyl group, C₁₋₃ alkyl, and cyano),
C₃₋₁₀ cycloalkylene (the cycloalkylene is optionally substituted with 1 to 3 of the same or different substituents selected from the group consisting of a fluorine atom, a hydroxyl group, C₁₋₃ alkyl, and cyano), C₃₋₁₀ cycloalkenylene (the cycloalkenylene is optionally substituted with 1 to 3 of the same or different substituents selected from the group consisting of a fluorine atom, a hydroxyl group, C₁₋₃ alkyl, and cyano), or
a 3- to 10-membered divalent saturated heterocyclic group (the saturated heterocyclic group is optionally substituted with 1 to 3 of the same or different substituents selected from the group consisting of a fluorine atom, a hydroxyl group, C₁₋₃ alkyl optionally substituted with 1 to 2 fluorine atoms, and cyano).

### [Item 27]

The compound or the pharmaceutically acceptable salt thereof of any one of items 1 to 26, wherein W is a single bond, or
C₁₋₆ alkylene (the alkylene is optionally substituted with 1 to 2 of the same or different substituents selected from the group consisting of a fluorine atom, a hydroxyl group, and cyano).

### [Item 28]

The compound or the pharmaceutically acceptable salt thereof of any one of items 1 to 27, wherein R¹⁴ is a hydrogen atom, or
C₁₋₆ alkyl (the alkyl is optionally substituted with 1 to 2 of the same or different substituents selected from the group consisting of a fluorine atom, a hydroxyl group, and cyano).

### [Item 29]

The compound or the pharmaceutically acceptable salt thereof of any one of items 1 to 28, wherein Q is a hydrogen atom,
NH₂, or
NMeH.

### [Item 30]

The compound or the pharmaceutically acceptable salt thereof of any one of items 1 to 29, wherein Ring A is a 5- to 6-membered aromatic heterocycle (the aromatic heterocycle is optionally substituted with 1 to 2 of the same or different substituents selected from the group consisting of a fluorine atom, a hydroxyl group, C₁₋₃ alkyl, C₁₋₃ alkoxy, and cyano) .

### [Item 31]

The compound or the pharmaceutically acceptable salt thereof of any one of items 1 to 29, wherein Ring A is a 5- to 8-membered non-aromatic heterocycle (the non-aromatic heterocycle is optionally substituted with 1 to 2 of the same or different substituents selected from the group consisting of a fluorine atom, a hydroxyl group, C₁₋₃ alkyl, C₁₋₃ alkoxy, and cyano) .

### [Item 32]

The compound or the pharmaceutically acceptable salt thereof of any one of items 1 to 31, wherein
-L-V-W- is C₁₋₆ alkylene (the alkylene is optionally substituted with 1 to 3 of the same or different substituents selected from the group consisting of a fluorine atom, a hydroxyl group, C₁₋₃ alkyl, C₁₋₃ alkoxy, - NR¹⁶R¹⁷, and cyano),
Q is NHR¹⁴, and
R¹⁶ and R¹⁷ represent each independently a hydrogen atom or C₁₋₆ alkyl, and when R¹⁶ or R¹⁷ is plural, then each of R¹⁶ or R¹⁷ may be the same or different, wherein when R¹⁶ and R¹⁷ attached to the same nitrogen atom are both C₁₋₆ alkyl, then they may form a 3- to 8-membered nitrogen-containing saturated heterocycle together with the nitrogen atom to which each is attached.

### [Item 33]

The compound or the pharmaceutically acceptable salt thereof of any one of items 1 to 31, wherein
L is C₁₋₆ alkylene (the alkylene is optionally substituted with 1 to 3 of the same or different substituents selected from the group consisting of a fluorine atom, a hydroxyl group, C₁₋₃ alkyl, C₁₋₃ alkoxy, - NR¹⁶R¹⁷, and cyano),
V is a 3- to 10-membered divalent saturated heterocyclic group (the saturated heterocyclic group is optionally substituted with 1 to 3 of the same or different substituents selected from the group consisting of a fluorine atom, C₁₋₃ alkyl optionally substituted with 1 to 2 fluorine atoms, C₁₋₃ alkoxy, -NR¹⁶R¹⁷, and cyano),
W is a single bond,
Q is a hydrogen atom, and
R¹⁶ and R¹⁷ represent each independently a hydrogen atom or C₁₋₆ alkyl, and when R¹⁶ or R¹⁷ is plural, then each of R¹⁶ or R¹⁷ may be the same or different, wherein when R¹⁶ and R¹⁷ attached to the same nitrogen atom are both C₁₋₆ alkyl, then they may form a 3- to 8-membered nitrogen-containing saturated heterocycle together with the nitrogen atom to which each is attached.

### [Item 34]

The compound or the pharmaceutically acceptable salt thereof of any one of items 1 to 31, wherein
L is C₁₋₆ alkylene (the alkylene is optionally substituted with 1 to 3 of the same or different substituents selected from the group consisting of a fluorine atom, a hydroxyl group, C₁₋₃ alkyl, C₁₋₃ alkoxy, - NR¹⁶R¹⁷, and cyano),
V is C₃₋₁₀ cycloalkylene (the cycloalkylene is optionally substituted with 1 to 3 of the same or different substituents selected from the group consisting of a fluorine atom, a hydroxyl group, C₁₋₃ alkyl, C₁₋₃ alkoxy, -NR¹⁶R¹⁷, and cyano),
W is a single bond, or C₁₋₆ alkylene (the alkylene is optionally substituted with 1 to 3 of the same or different substituents selected from the group consisting of a fluorine atom, a hydroxyl group, C₁₋₃ alkoxy, - NR¹⁶R¹⁷, and cyano),
Q is NHR¹⁴, and
R¹⁶ and R¹⁷ represent each independently a hydrogen atom or C₁₋₆ alkyl, and when R¹⁶ or R¹⁷ is plural, then each of R¹⁶ or R¹⁷ may be the same or different, wherein when R¹⁶ and R¹⁷ attached to the same nitrogen atom are both C₁₋₆ alkyl, then they may form a 3- to 8-membered nitrogen-containing saturated heterocycle together with the nitrogen atom to which each is attached.

### [Item 34A]

The compound or the pharmaceutically acceptable salt thereof of any one of items 1 to 34, wherein Y^{1b} is a carbon atom.

### [Item 35]

The compound or the pharmaceutically acceptable salt thereof of item 1, wherein formula (1) is represented by the following formula (3): 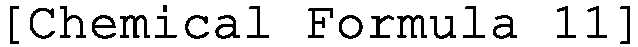 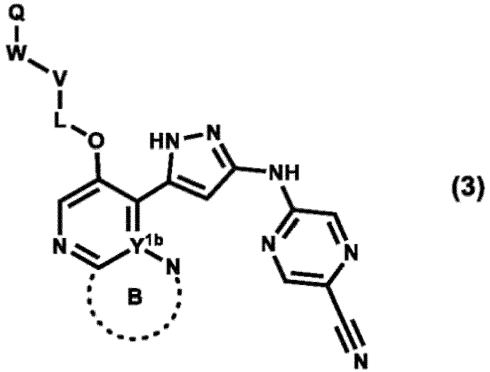 wherein
Y^{1b} represents
   a carbon atom, or
   a nitrogen atom,
Ring B represents a 5- to 6-membered aromatic heterocycle (the aromatic heterocycle is optionally substituted with 1 to 2 of the same or different substituents selected from the group consisting of a fluorine atom, a hydroxyl group, C₁₋₃ alkyl, C₁₋₃ alkoxy, and cyano),
L represents
   a single bond, or
   C₁₋₆ alkylene (the alkylene is optionally substituted with 1 to 2 substituents selected from the group consisting of a fluorine atom, a hydroxyl group, C₁₋₃ alkyl, and cyano),
V represents
   a single bond,
   C₂₋₆ alkenylene (the alkenylene is optionally substituted with 1 to 3 of the same or different substituents selected from the group consisting of a fluorine atom, a hydroxyl group, C₁₋₃ alkyl, and cyano),
   C₃₋₁₀ cycloalkylene (the cycloalkylene is optionally substituted with 1 to 3 of the same or different substituents selected from the group consisting of a fluorine atom, a hydroxyl group, C₁₋₃ alkyl, and cyano), C₃₋₁₀ cycloalkenylene (the cycloalkenylene is optionally substituted with 1 to 3 of the same or different substituents selected from the group consisting of a fluorine atom, a hydroxyl group, C₁₋₃ alkyl, and cyano), or
   a 3- to 10-membered divalent saturated heterocyclic group (the saturated heterocyclic group is optionally substituted with 1 to 3 of the same or different substituents selected from the group consisting of a fluorine atom, a hydroxyl group, C₁₋₃ alkyl optionally substituted with 1 to 2 hydroxyl groups or fluorine atoms, and cyano),
W represents
   a single bond, or
   C₁₋₃ alkylene (the alkylene is optionally substituted with 1 to 2 substituents selected from the group consisting of a fluorine atom, a hydroxyl group, and cyano),
Q represents
   a hydrogen atom, or
   NHR¹⁴, and
R¹⁴ is
   a hydrogen atom, or
   C₁₋₃ alkyl (the alkyl is optionally substituted with one substituent selected from the group consisting of a fluorine atom, a hydroxyl group, and cyano).

### [Item 36]

The compound or the pharmaceutically acceptable salt thereof of item 35, wherein Ring B is an unsubstituted 5- to 6-membered aromatic heterocycle.

### [Item 37]

The compound or the pharmaceutically acceptable salt thereof of item 35 or 36, wherein Y^{1b} is a carbon atom.

### [Item 38]

The compound or the pharmaceutically acceptable salt thereof of any one of items 1 to 31 and 35 to 37, wherein L is
a single bond, or
C₁₋₆ alkylene (the alkylene is optionally substituted with 1 to 2 of the same or different substituents selected from the group consisting of a fluorine atom or C₁₋₃ alkyl).

### [Item 39]

The compound or the pharmaceutically acceptable salt thereof of any one of items 1 to 31 and 35 to 38, wherein V is
a single bond,
C₂₋₆ alkenylene (the alkenylene is optionally substituted with one substituent selected from the group consisting of a hydroxyl group, and C₁₋₃ alkyl),
C₃₋₁₀ cycloalkylene (the cycloalkylene is optionally substituted with 1 to 3 substituents selected from the group consisting of a fluorine atom, a hydroxyl group, and C₁₋₃ alkyl),
C₃₋₁₀ cycloalkenylene (the cycloalkenylene is optionally substituted with one substituent selected from the group consisting of a hydroxyl group, and C₁₋₃ alkyl), or
a 3- to 10-membered divalent saturated heterocyclic group (the saturated heterocyclic group is optionally substituted with one substituent selected from the group consisting of a fluorine atom, a hydroxyl group, and C₁₋₃ alkyl optionally substituted with 1 to 2 fluorine atoms).

### [Item 40]

The compound or the pharmaceutically acceptable salt thereof of any one of items 1 to 31 and 35 to 39, wherein W is
a single bond, or
C₁₋₃ alkylene optionally substituted with 1 to 2 fluorine atoms.

### [Item 41]

The compound or the pharmaceutically acceptable salt thereof of any one of items 1 to 31 and 35 to 40, wherein R¹⁴ is
a hydrogen atom, or
C₁₋₆ alkyl.

### [Item 42]

The compound or the pharmaceutically acceptable salt thereof of any one of items 1 to 31 and 35 to 41, wherein Q is
a hydrogen atom, or
NH₂.

### [Item 42A]

The compound or the pharmaceutically acceptable salt thereof of any one of items 35 to 42, wherein
-L-V-W- is C₁₋₆ alkylene (the alkylene is optionally substituted with 1 to 3 of the same or different substituents selected from the group consisting of a fluorine atom, a hydroxyl group, C₁₋₃ alkyl, C₁₋₃ alkoxy, - NR¹⁶R¹⁷, and cyano),
Q is NHR¹⁴, and
R¹⁶ and R¹⁷ represent each independently a hydrogen atom or C₁₋₆ alkyl, and when R¹⁶ or R¹⁷ is plural, then each of R¹⁶ or R¹⁷ may be the same or different, wherein when R¹⁶ and R¹⁷ attached to the same nitrogen atom are both C₁₋₆ alkyl, then they may form a 3- to 8-membered nitrogen-containing saturated heterocycle together with the nitrogen atom to which each is attached.

### [Item 42B]

The compound or the pharmaceutically acceptable salt thereof of any one of items 35 to 42, wherein
L is C₁₋₆ alkylene (the alkylene is optionally substituted with 1 to 3 of the same or different substituents selected from the group consisting of a fluorine atom, a hydroxyl group, C₁₋₃ alkyl, C₁₋₃ alkoxy, - NR¹⁶R¹⁷, and cyano),
V is a 3- to 10-membered divalent saturated heterocyclic group (the saturated heterocyclic group is optionally substituted with 1 to 3 of the same or different substituents selected from the group consisting of a fluorine atom, C₁₋₃ alkyl optionally substituted with 1 to 2 fluorine atoms, C₁₋₃ alkoxy, -NR¹⁶R¹⁷, and cyano),
W is a single bond,
Q is a hydrogen atom, and
R¹⁶ and R¹⁷ represent each independently a hydrogen atom or C₁₋₆ alkyl, and when R¹⁶ or R¹⁷ is plural, then each of R¹⁶ or R¹⁷ may be the same or different, wherein when R¹⁶ and R¹⁷ attached to the same nitrogen atom are both C₁₋₆ alkyl, then they may form a 3- to 8-membered nitrogen-containing saturated heterocycle together with the nitrogen atom to which each is attached.

### [Item 42C]

The compound or the pharmaceutically acceptable salt thereof of any one of items 35 to 42, wherein
L is a single bond, or C₁₋₆ alkylene (the alkylene is optionally substituted with 1 to 3 of the same or different substituents selected from the group consisting of a fluorine atom, a hydroxyl group, C₁₋₃ alkyl, C₁₋₃ alkoxy, -NR¹⁶R¹⁷, and cyano),
V is C₃₋₁₀ cycloalkylene (the cycloalkylene is optionally substituted with 1 to 3 of the same or different substituents selected from the group consisting of a fluorine atom, a hydroxyl group, C₁₋₃ alkyl, C₁₋₃ alkoxy, -NR¹⁶R¹⁷, and cyano),
W is a single bond, or C₁₋₆ alkylene (the alkylene is optionally substituted with 1 to 3 of the same or different substituents selected from the group consisting of a fluorine atom, a hydroxyl group, C₁₋₃ alkoxy, - NR¹⁶R¹⁷, and cyano),
Q is NHR¹⁴, and
R¹⁶ and R¹⁷ represent each independently a hydrogen atom or C₁₋₆ alkyl, and when R¹⁶ or R¹⁷ is plural, then each of R¹⁶ or R¹⁷ may be the same or different, wherein when R¹⁶ and R¹⁷ attached to the same nitrogen atom are both C₁₋₆ alkyl, then they may form a 3- to 8-membered nitrogen-containing saturated heterocycle together with the nitrogen atom to which each is attached.

### [Item 43]

The compound or the pharmaceutically acceptable salt thereof of item 1, wherein formula (1) is represented by the following formula (4): 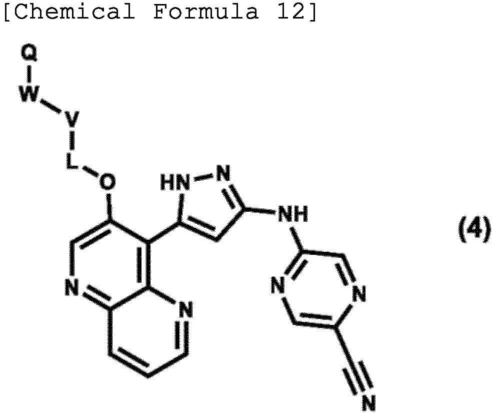 wherein
L represents
   a single bond, or
   C₁₋₆ alkylene (the alkylene is optionally substituted with 1 to 2 of the same or different substituents selected from the group consisting of a fluorine atom or C₁₋₃ alkyl),
V represents
   a single bond,
   C₂₋₆ alkenylene (the alkenylene is optionally substituted with one substituent selected from the group consisting of a hydroxyl group, and C₁₋₃ alkyl),
   C₃₋₁₀ cycloalkylene (the cycloalkylene is optionally substituted with 1 to 3 substituents selected from the group consisting of a fluorine atom, a hydroxyl group, and C₁₋₃ alkyl),
   C₃₋₁₀ cycloalkenylene (the cycloalkenylene is optionally substituted with one substituent selected from the group consisting of a hydroxyl group, and C₁₋₃ alkyl), or
   a 3- to 10-membered divalent saturated heterocyclic group (the saturated heterocyclic group is optionally substituted with one substituent selected from the group consisting of a fluorine atom, a hydroxyl group, and C₁₋₃ alkyl optionally substituted with 1 to 2 fluorine atoms),
W represents
   a single bond, or
   C₁₋₃ alkylene optionally substituted with 1 to 2 fluorine atoms, and
Q represents
   a hydrogen atom, or
   NH₂.

### [Item 44]

The compound or the pharmaceutically acceptable salt thereof of any one of items 1 to 31 and 43, wherein V is
a single bond,
C₂₋₆ alkenylene,
C₃₋₁₀ cycloalkylene (the cycloalkylene is optionally substituted with 1 to 3 fluorine atoms),
C₃₋₁₀ cycloalkenylene, or
a 3- to 10-membered divalent saturated heterocyclic group (the saturated heterocyclic group is optionally substituted with one substituent selected from the group consisting of a fluorine atom, and C₁₋₃ alkyl optionally substituted with 1 to 2 fluorine atoms).

### [Item 45]

The compound or the pharmaceutically acceptable salt thereof of any one of items 1 to 31 and 43 to 44, wherein Q is a hydrogen atom.

### [Item 46]

The compound or the pharmaceutically acceptable salt thereof of any one of items 1 to 31 and 43 to 44, wherein Q is NH₂.

### [Item 46A]

The compound or the pharmaceutically acceptable salt thereof of any one of items 43 to 46, wherein
-L-V-W- is C₁₋₆ alkylene (the alkylene is optionally substituted with 1 to 3 of the same or different substituents selected from the group consisting of a fluorine atom, a hydroxyl group, C₁₋₃ alkyl, C₁₋₃ alkoxy, - NR¹⁶R¹⁷, and cyano),
Q is NHR¹⁴, and
R¹⁶ and R¹⁷ represent each independently a hydrogen atom or C₁₋₆ alkyl, and when R¹⁶ or R¹⁷ is plural, then each of R¹⁶ or R¹⁷ may be the same or different, wherein when R¹⁶ and R¹⁷ attached to the same nitrogen atom are both C₁₋₆ alkyl, then they may form a 3- to 8-membered nitrogen-containing saturated heterocycle together with the nitrogen atom to which each is attached.

### [Item 46B]

The compound or the pharmaceutically acceptable salt thereof of any one of items 43 to 46, wherein
L is C₁₋₆ alkylene (the alkylene is optionally substituted with 1 to 3 of the same or different substituents selected from the group consisting of a fluorine atom, a hydroxyl group, C₁₋₃ alkyl, C₁₋₃ alkoxy, - NR¹⁶R¹⁷, and cyano),
V is a 3- to 10-membered divalent saturated heterocyclic group (the saturated heterocyclic group is optionally substituted with 1 to 3 of the same or different substituents selected from the group consisting of a fluorine atom, C₁₋₃ alkyl optionally substituted with 1 to 2 fluorine atoms, C₁₋₃ alkoxy, -NR¹⁶R¹⁷, and cyano),
W is a single bond,
Q is a hydrogen atom, and
R¹⁶ and R¹⁷ represent each independently a hydrogen atom or C₁₋₆ alkyl, and when R¹⁶ or R¹⁷ is plural, then each of R¹⁶ or R¹⁷ may be the same or different, wherein when R¹⁶ and R¹⁷ attached to the same nitrogen atom are both C₁₋₆ alkyl, then they may form a 3- to 8-membered nitrogen-containing saturated heterocycle together with the nitrogen atom to which each is attached.

### [Item 46C]

The compound or the pharmaceutically acceptable salt thereof of any one of items 43 to 46, wherein
L is a single bond, or C₁₋₆ alkylene (the alkylene is optionally substituted with 1 to 3 of the same or different substituents selected from the group consisting of a fluorine atom, a hydroxyl group, C₁₋₃ alkyl, C₁₋₃ alkoxy, -NR¹⁶R¹⁷, and cyano),
V is C₃₋₁₀ cycloalkylene (the cycloalkylene is optionally substituted with 1 to 3 of the same or different substituents selected from the group consisting of a fluorine atom, a hydroxyl group, C₁₋₃ alkyl, C₁₋₃ alkoxy, -NR¹⁶R¹⁷, and cyano),
W is a single bond, or C₁₋₆ alkylene (the alkylene is optionally substituted with 1 to 3 of the same or different substituents selected from the group consisting of a fluorine atom, a hydroxyl group, C₁₋₃ alkoxy, - NR¹⁶R¹⁷, and cyano),
Q is NHR¹⁴, and
R¹⁶ and R¹⁷ represent each independently a hydrogen atom or C₁₋₆ alkyl, and when R¹⁶ or R¹⁷ is plural, then each of R¹⁶ or R¹⁷ may be the same or different, wherein when R¹⁶ and R¹⁷ attached to the same nitrogen atom are both C₁₋₆ alkyl, then they may form a 3- to 8-membered nitrogen-containing saturated heterocycle together with the nitrogen atom to which each is attached.

### [Item 47]

The compound or the pharmaceutically acceptable salt thereof of item 1, wherein formula (1) is represented by the following formula (5): 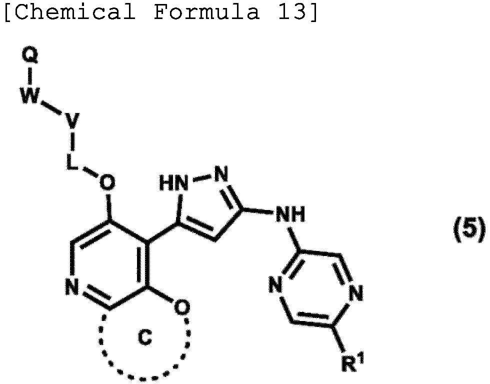 wherein
R¹ represents a hydrogen atom, a halogen atom, cyano, or -CO₂R³,
R³ represents C₁₋₆ alkyl,
Ring C represents a 5- to 8-membered non-aromatic heterocycle (the non-aromatic heterocycle is optionally substituted with 1 to 2 of the same or different substituents selected from the group consisting of a fluorine atom, a hydroxyl group, C₁₋₃ alkyl, C₁₋₃ alkoxy, and cyano),
L represents
   a single bond, or
   C₁₋₆ alkylene (the alkylene is optionally substituted with 1 to 2 substituents selected from the group consisting of a fluorine atom, a hydroxyl group, C₁₋₃ alkyl, and cyano),
V represents
   a single bond,
   C₂₋₆ alkenylene (the alkenylene is optionally substituted with 1 to 3 of the same or different substituents selected from the group consisting of a fluorine atom, a hydroxyl group, C₁₋₃ alkyl, and cyano),
   C₃₋₁₀ cycloalkylene (the cycloalkylene is optionally substituted with 1 to 3 of the same or different substituents selected from the group consisting of a fluorine atom, a hydroxyl group, C₁₋₃ alkyl, and cyano), C₃₋₁₀ cycloalkenylene (the cycloalkenylene is optionally substituted with 1 to 3 of the same or different substituents selected from the group consisting of a fluorine atom, a hydroxyl group, C₁₋₃ alkyl, and cyano), or
   a 3- to 10-membered divalent saturated heterocyclic group (the saturated heterocyclic group is optionally substituted with 1 to 3 of the same or different substituents selected from the group consisting of a fluorine atom, a hydroxyl group, C₁₋₃ alkyl optionally substituted with 1 to 2 hydroxyl groups or fluorine atoms, and cyano),
W represents
   a single bond, or
   C₁₋₃ alkylene (the alkylene is optionally substituted with 1 to 2 substituents selected from the group consisting of a fluorine atom, a hydroxyl group, and cyano),
Q represents
   a hydrogen atom, or
   NHR¹⁴, and
R¹⁴ is
   a hydrogen atom, or
   C₁₋₃ alkyl (the alkyl is optionally substituted with one substituent selected from the group consisting of a fluorine atom, a hydroxyl group, and cyano).

### [Item 48]

The compound or the pharmaceutically acceptable salt thereof of item 47, wherein R¹ is cyano.

### [Item 48]

The compound or the pharmaceutically acceptable salt thereof of item 47, wherein Ring C is an unsubstituted 5- to 8-membered non-aromatic heterocycle.

### [Item 49]

The compound or the pharmaceutically acceptable salt thereof of any one of items 1 to 31 and 48, wherein L is
a single bond, or
C₁₋₆ alkylene (the alkylene is optionally substituted with 1 to 2 of the same or different substituents selected from the group consisting of a fluorine atom, or C₁₋₃ alkyl).

### [Item 50]

The compound or the pharmaceutically acceptable salt thereof of any one of items 1 to 31 and 48 to 49, wherein V is
a single bond,
C₂₋₆ alkenylene (the alkenylene is optionally substituted with one substituent selected from the group consisting of a hydroxyl group, and C₁₋₃ alkyl),
C₃₋₁₀ cycloalkylene (the cycloalkylene is optionally substituted with one substituent selected from the group consisting of a hydroxyl group, and C₁₋₃ alkyl),
C₃₋₁₀ cycloalkenylene (the cycloalkenylene is optionally substituted with one substituent selected from the group consisting of a hydroxyl group, and C₁₋₃ alkyl), or
a 3- to 10-membered divalent saturated heterocyclic group (the saturated heterocyclic group is optionally substituted with one substituent selected from the group consisting of a fluorine atom, a hydroxyl group, and C₁₋₃ alkyl optionally substituted with 1 to 2 hydroxyl groups or fluorine atoms).

### [Item 51]

The compound or the pharmaceutically acceptable salt thereof of any one of items 1 to 31 and 48 to 50, wherein W is
a single bond, or
C₁₋₃ alkylene.

### [Item 52]

The compound or the pharmaceutically acceptable salt thereof of any one of items 46 to 51, wherein R¹⁴ is a hydrogen atom, or
C₁₋₆ alkyl.

### [Item 53]

The compound or the pharmaceutically acceptable salt thereof of any one of items 1 to 31 and 48 to 52, wherein Q is
a hydrogen atom
NH₂, or
NMeH.

### [Item 53A]

The compound or the pharmaceutically acceptable salt thereof of any one of items 47 to 53, wherein
-L-V-W- is C₁₋₆ alkylene (the alkylene is optionally substituted with 1 to 3 of the same or different substituents selected from the group consisting of a fluorine atom, a hydroxyl group, C₁₋₃ alkyl, C₁₋₃ alkoxy, - NR¹⁶R¹⁷, and cyano),
Q is NHR¹⁴, and
R¹⁶ and R¹⁷ represent each independently a hydrogen atom or C₁₋₆ alkyl, and when R¹⁶ or R¹⁷ is plural, then each of R¹⁶ or R¹⁷ may be the same or different, wherein when R¹⁶ and R¹⁷ attached to the same nitrogen atom are both C₁₋₆ alkyl, then they may form a 3- to 8-membered nitrogen-containing saturated heterocycle together with the nitrogen atom to which each is attached.

### [Item 53B]

The compound or the pharmaceutically acceptable salt thereof of any one of items 47 to 53, wherein
L is C₁₋₆ alkylene (the alkylene is optionally substituted with 1 to 3 of the same or different substituents selected from the group consisting of a fluorine atom, a hydroxyl group, C₁₋₃ alkyl, C₁₋₃ alkoxy, - NR¹⁶R¹⁷, and cyano),
V is a 3- to 10-membered divalent saturated heterocyclic group (the saturated heterocyclic group is optionally substituted with 1 to 3 of the same or different substituents selected from the group consisting of a fluorine atom, C₁₋₃ alkyl optionally substituted with 1 to 2 fluorine atoms, C₁₋₃ alkoxy, -NR¹⁶R¹⁷, and cyano),
W is a single bond,
Q is a hydrogen atom, and
R¹⁶ and R¹⁷ represent each independently a hydrogen atom or C₁₋₆ alkyl, and when R¹⁶ or R¹⁷ is plural, then each of R¹⁶ or R¹⁷ may be the same or different, wherein when R¹⁶ and R¹⁷ attached to the same nitrogen atom are both C₁₋₆ alkyl, then they may form a 3- to 8-membered nitrogen-containing saturated heterocycle together with the nitrogen atom to which each is attached.

### [Item 53C]

The compound or the pharmaceutically acceptable salt thereof of any one of items 47 to 53, wherein
L is a single bond, or C₁₋₆ alkylene (the alkylene is optionally substituted with 1 to 3 of the same or different substituents selected from the group consisting of a fluorine atom, a hydroxyl group, C₁₋₃ alkyl, C₁₋₃ alkoxy, -NR¹⁶R¹⁷, and cyano),
V is C₃₋₁₀ cycloalkylene (the cycloalkylene is optionally substituted with 1 to 3 of the same or different substituents selected from the group consisting of a fluorine atom, a hydroxyl group, C₁₋₃ alkyl, C₁₋₃ alkoxy, -NR¹⁶R¹⁷, and cyano),
W is a single bond, or C₁₋₆ alkylene (the alkylene is optionally substituted with 1 to 3 of the same or different substituents selected from the group consisting of a fluorine atom, a hydroxyl group, C₁₋₃ alkoxy, - NR¹⁶R¹⁷, and cyano),
Q is NHR¹⁴, and
R¹⁶ and R¹⁷ represent each independently a hydrogen atom or C₁₋₆ alkyl, and when R¹⁶ or R¹⁷ is plural, then each of R¹⁶ or R¹⁷ may be the same or different, wherein when R¹⁶ and R¹⁷ attached to the same nitrogen atom are both C₁₋₆ alkyl, then they may form a 3- to 8-membered nitrogen-containing saturated heterocycle together with the nitrogen atom to which each is attached.

### [Item 54]

The compound or the pharmaceutically acceptable salt thereof of item 1, wherein formula (1) is represented by the following formula (6): 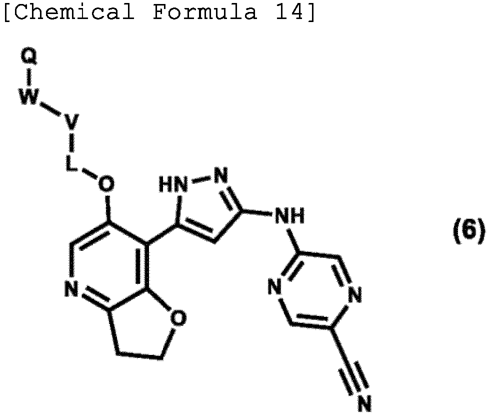 wherein
L represents
   a single bond, or
   C₁₋₆ alkylene (the alkylene is optionally substituted with 1 to 2 of the same or different substituents selected from the group consisting of a fluorine atom, or C₁₋₃ alkyl),
V represents
   a single bond,
   C₂₋₆ alkenylene (the alkenylene is optionally substituted with one substituent selected from the group consisting of a hydroxyl group, and C₁₋₃ alkyl),
   C₃₋₁₀ cycloalkylene (the cycloalkylene is optionally substituted with one substituent selected from the group consisting of a hydroxyl group, and C₁₋₃ alkyl),
   C₃₋₁₀ cycloalkenylene (the cycloalkenylene is optionally substituted with one substituent selected from the group consisting of a hydroxyl group, and C₁₋₃ alkyl), or
   a 3- to 10-membered divalent saturated heterocyclic group (the saturated heterocyclic group is optionally substituted with one substituent selected from the group consisting of a fluorine atom, a hydroxyl group, and C₁₋₃ alkyl optionally substituted with 1 to 2 fluorine atoms),
W represents
   a single bond, or
   C₁₋₃ alkylene optionally substituted with 1 to 2 fluorine atoms, and
Q represents
   a hydrogen atom
   NH₂, or
   NMeH.

### [Item 55]

The compound or the pharmaceutically acceptable salt thereof of item 54, wherein V is
a single bond,
C₂₋₆ alkenylene,
C₃₋₁₀ cycloalkylene,
C₃₋₁₀ cycloalkenylene, or
a 3- to 10-membered divalent saturated heterocyclic group (the saturated heterocyclic group is optionally substituted with one substituent selected from the group consisting of a fluorine atom, and C₁₋₃ alkyl optionally substituted with 1 to 2 fluorine atoms).

### [Item 56]

The compound or the pharmaceutically acceptable salt thereof of any one of items 1 to 31 and 55, wherein W is
a single bond, or
C₁₋₃ alkylene.

### [Item 57]

The compound or the pharmaceutically acceptable salt thereof of any one of items 1 to 31 and 55 to 56, wherein Q is a hydrogen atom.

### [Item 58]

The compound or the pharmaceutically acceptable salt thereof of any one of items 1 to 31 and 55 to 56, wherein Q is NH₂.

### [Item 59]

The compound or the pharmaceutically acceptable salt thereof of any one of items 1 to 31 and 55 to 56, wherein Q is NHMe.

### [Item 59A]

The compound or the pharmaceutically acceptable salt thereof of any one of items 54 to 59, wherein
-L-V-W- is C₁₋₆ alkylene (the alkylene is optionally substituted with 1 to 3 of the same or different substituents selected from the group consisting of a fluorine atom, a hydroxyl group, C₁₋₃ alkyl, C₁₋₃ alkoxy, - NR¹⁶R¹⁷, and cyano),
Q is NHR¹⁴, and
R¹⁶ and R¹⁷ represent each independently a hydrogen atom or C₁₋₆ alkyl, and when R¹⁶ or R¹⁷ is plural, then each of R¹⁶ or R¹⁷ may be the same or different, wherein when R¹⁶ and R¹⁷ attached to the same nitrogen atom are both C₁₋₆ alkyl, then they may form a 3- to 8-membered nitrogen-containing saturated heterocycle together with the nitrogen atom to which each is attached.

### [Item 59B]

The compound or the pharmaceutically acceptable salt thereof of any one of items 54 to 59, wherein
L is C₁₋₆ alkylene (the alkylene is optionally substituted with 1 to 3 of the same or different substituents selected from the group consisting of a fluorine atom, a hydroxyl group, C₁₋₃ alkyl, C₁₋₃ alkoxy, - NR¹⁶R¹⁷, and cyano),
V is a 3- to 10-membered divalent saturated heterocyclic group (the saturated heterocyclic group is optionally substituted with 1 to 3 of the same or different substituents selected from the group consisting of a fluorine atom, C₁₋₃ alkyl optionally substituted with 1 to 2 fluorine atoms, C₁₋₃ alkoxy, -NR¹⁶R¹⁷, and cyano),
W is a single bond,
Q is a hydrogen atom, and
R¹⁶ and R¹⁷ represent each independently a hydrogen atom or C₁₋₆ alkyl, and when R¹⁶ or R¹⁷ is plural, then each of R¹⁶ or R¹⁷ may be the same or different, wherein when R¹⁶ and R¹⁷ attached to the same nitrogen atom are both C₁₋₆ alkyl, then they may form a 3- to 8-membered nitrogen-containing saturated heterocycle together with the nitrogen atom to which each is attached.

### [Item 59C]

The compound or the pharmaceutically acceptable salt thereof of any one of items 54 to 59, wherein
L is a single bond, or C₁₋₆ alkylene (the alkylene is optionally substituted with 1 to 3 of the same or different substituents selected from the group consisting of a fluorine atom, a hydroxyl group, C₁₋₃ alkyl, C₁₋₃ alkoxy, -NR¹⁶R¹⁷, and cyano),
V is C₃₋₁₀ cycloalkylene (the cycloalkylene is optionally substituted with 1 to 3 of the same or different substituents selected from the group consisting of a fluorine atom, a hydroxyl group, C₁₋₃ alkyl, C₁₋₃ alkoxy, -NR¹⁶R¹⁷, and cyano),
W is a single bond, or C₁₋₆ alkylene (the alkylene is optionally substituted with 1 to 3 of the same or different substituents selected from the group consisting of a fluorine atom, a hydroxyl group, C₁₋₃ alkoxy, - NR¹⁶R¹⁷, and cyano),
Q is NHR¹⁴, and
R¹⁶ and R¹⁷ represent each independently a hydrogen atom or C₁₋₆ alkyl, and when R¹⁶ or R¹⁷ is plural, then each of R¹⁶ or R¹⁷ may be the same or different, wherein when R¹⁶ and R¹⁷ attached to the same nitrogen atom are both C₁₋₆ alkyl, then they may form a 3- to 8-membered nitrogen-containing saturated heterocycle together with the nitrogen atom to which each is attached.

### [Item 60]

The compound or the pharmaceutically acceptable salt thereof of item 1, wherein formula (1) is represented by the following formula (7): 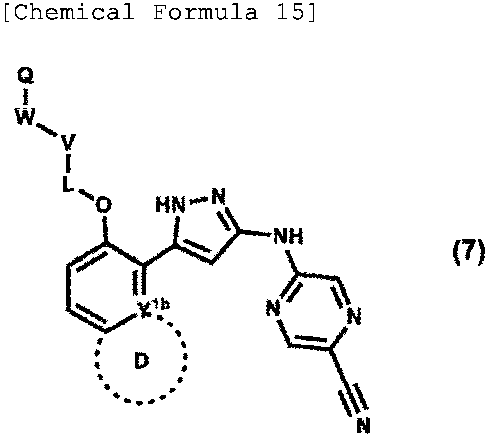 wherein
Y^{1b} represents
   a carbon atom, or
   a nitrogen atom,
Ring D represents
   a 5- to 8-membered non-aromatic heterocycle, or
   a 5- to 6-membered aromatic heterocycle (the non-aromatic heterocycle and aromatic heterocycle are optionally substituted with 1 to 2 of the same or different substituents selected from the group consisting of a fluorine atom, a hydroxyl group, C₁₋₃ alkyl, C₁₋₃ alkoxy, and cyano),
L represents
   a single bond, or
   C₁₋₆ alkylene (the alkylene is optionally substituted with 1 to 2 substituents selected from the group consisting of a fluorine atom, a hydroxyl group, C₁₋₃ alkyl, and cyano),
V represents
   a single bond,
   C₂₋₆ alkenylene (the alkenylene is optionally substituted with 1 to 3 of the same or different substituents selected from the group consisting of a fluorine atom, a hydroxyl group, C₁₋₃ alkyl, and cyano),
   C₃₋₁₀ cycloalkylene (the cycloalkylene is optionally substituted with 1 to 3 of the same or different substituents selected from the group consisting of a fluorine atom, a hydroxyl group, C₁₋₃ alkyl, and cyano), C₃₋₁₀ cycloalkenylene (the cycloalkenylene is optionally substituted with 1 to 3 of the same or different substituents selected from the group consisting of a fluorine atom, a hydroxyl group, C₁₋₃ alkyl, and cyano), or
   a 3- to 10-membered divalent saturated heterocyclic group (the saturated heterocyclic group is optionally substituted with 1 to 3 of the same or different substituents selected from the group consisting of a fluorine atom, a hydroxyl group, C₁₋₃ alkyl optionally substituted with 1 to 2 hydroxyl groups or fluorine atoms, and cyano),
W represents
   a single bond, or
   C₁₋₃ alkylene (the alkylene is optionally substituted with one substituent selected from the group consisting of a fluorine atom, a hydroxyl group, and cyano),
Q represents
   a hydrogen atom, or
   NHR¹⁴, and
R¹⁴ is
   a hydrogen atom, or
   C₁₋₃ alkyl (the alkyl is optionally substituted with one substituent selected from the group consisting of a fluorine atom, a hydroxyl group, and cyano).

### [Item 61]

The compound or the pharmaceutically acceptable salt thereof of item 60, wherein Y^{1b} is a carbon atom.

### [Item 62]

The compound or the pharmaceutically acceptable salt thereof of item 60, wherein Y^{1b} is a nitrogen atom.

### [Item 63]

The compound or the pharmaceutically acceptable salt thereof of any one of items 60 to 62, wherein Ring D is a 5- to 8-membered non-aromatic heterocycle.

### [Item 64]

The compound or the pharmaceutically acceptable salt thereof of any one of items 60 to 63, wherein Ring D is a 5- to 6-membered aromatic heterocycle.

### [Item 65]

The compound or the pharmaceutically acceptable salt thereof of any one of items 1 to 31 and 60 to 64, wherein L is
a single bond, or
C₁₋₆ alkylene (the alkylene is optionally substituted with 1 to 2 of the same or different substituents selected from the group consisting of a fluorine atom, or C₁₋₃ alkyl).

### [Item 66]

The compound or the pharmaceutically acceptable salt thereof of any one of items 1 to 31 and 60 to 65, wherein V is
a single bond,
C₂₋₆ alkenylene,
C₃₋₁₀ cycloalkylene (the cycloalkylene is optionally substituted with 1 to 3 fluorine atoms),
C₃₋₁₀ cycloalkenylene, or
a 3- to 10-membered divalent saturated heterocyclic group (the saturated heterocyclic group is optionally substituted with one substituent selected from the group consisting of a fluorine atom, and C₁₋₃ alkyl optionally substituted with 1 to 2 hydroxyl groups or fluorine atoms).

### [Item 67]

The compound or the pharmaceutically acceptable salt thereof of any one of items 1 to 31 and 60 to 66, wherein W is
a single bond, or
C₁₋₃ alkylene.

### [Item 68]

The compound or the pharmaceutically acceptable salt thereof of any one of items 1 to 31 and 60 to 67, wherein R¹⁴ is
a hydrogen atom, or
C₁₋₆ alkyl.

### [Item 69]

The compound or the pharmaceutically acceptable salt thereof of any one of items 1 to 31 and 60 to 68, wherein Q is
a hydrogen atom, or
NH₂.

### [Item 69A]

The compound or the pharmaceutically acceptable salt thereof of any one of items 60 to 69, wherein
-L-V-W- is C₁₋₆ alkylene (the alkylene is optionally substituted with 1 to 3 of the same or different substituents selected from the group consisting of a fluorine atom, a hydroxyl group, C₁₋₃ alkyl, C₁₋₃ alkoxy, - NR¹⁶R¹⁷, and cyano),
Q is NHR¹⁴, and
R¹⁶ and R¹⁷ represent each independently a hydrogen atom or C₁₋₆ alkyl, and when R¹⁶ or R¹⁷ is plural, then each of R¹⁶ or R¹⁷ may be the same or different, wherein when R¹⁶ and R¹⁷ attached to the same nitrogen atom are both C₁₋₆ alkyl, then they may form a 3- to 8-membered nitrogen-containing saturated heterocycle together with the nitrogen atom to which each is attached.

### [Item 69B]

The compound or the pharmaceutically acceptable salt thereof of any one of items 60 to 69, wherein
L is C₁₋₆ alkylene (the alkylene is optionally substituted with 1 to 3 of the same or different substituents selected from the group consisting of a fluorine atom, a hydroxyl group, C₁₋₃ alkyl, C₁₋₃ alkoxy, - NR¹⁶R¹⁷, and cyano),
V is a 3- to 10-membered divalent saturated heterocyclic group (the saturated heterocyclic group is optionally substituted with 1 to 3 of the same or different substituents selected from the group consisting of a fluorine atom, C₁₋₃ alkyl optionally substituted with 1 to 2 fluorine atoms, C₁₋₃ alkoxy, -NR¹⁶R¹⁷, and cyano),
W is a single bond,
Q is a hydrogen atom, and
R¹⁶ and R¹⁷ represent each independently a hydrogen atom or C₁₋₆ alkyl, and when R¹⁶ or R¹⁷ is plural, then each of R¹⁶ or R¹⁷ may be the same or different, wherein when R¹⁶ and R¹⁷ attached to the same nitrogen atom are both C₁₋₆ alkyl, then they may form a 3- to 8-membered nitrogen-containing saturated heterocycle together with the nitrogen atom to which each is attached.

### [Item 69C]

The compound or the pharmaceutically acceptable salt thereof of any one of items 60 to 69, wherein
L is C₁₋₆ alkylene (the alkylene is optionally substituted with 1 to 3 of the same or different substituents selected from the group consisting of a fluorine atom, a hydroxyl group, C₁₋₃ alkyl, C₁₋₃ alkoxy, - NR¹⁶R¹⁷, and cyano),
V is C₃₋₁₀ cycloalkylene (the cycloalkylene is optionally substituted with 1 to 3 of the same or different substituents selected from the group consisting of a fluorine atom, a hydroxyl group, C₁₋₃ alkyl, C₁₋₃ alkoxy, -NR¹⁶R¹⁷, and cyano),
W is C₁₋₆ alkylene (the alkylene is optionally substituted with 1 to 3 of the same or different substituents selected from the group consisting of a fluorine atom, a hydroxyl group, C₁₋₃ alkoxy, -NR¹⁶R¹⁷, and cyano),
Q is NHR¹⁴, and
R¹⁶ and R¹⁷ represent each independently a hydrogen atom or C₁₋₆ alkyl, and when R¹⁶ or R¹⁷ is plural, then each of R¹⁶ or R¹⁷ may be the same or different, wherein when R¹⁶ and R¹⁷ attached to the same nitrogen atom are both C₁₋₆ alkyl, then they may form a 3- to 8-membered nitrogen-containing saturated heterocycle together with the nitrogen atom to which each is attached.

### [Item 70]

The compound or the pharmaceutically acceptable salt thereof of item 1, wherein formula (1) is represented by the following formula (8): 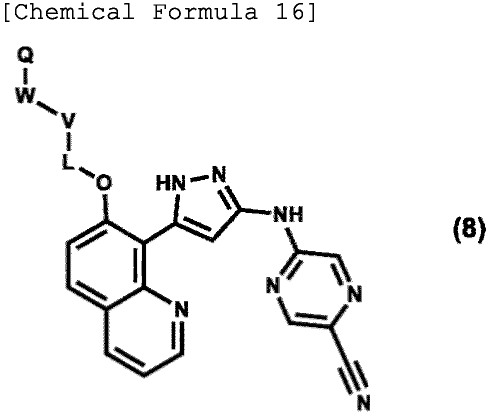 wherein
L represents
   a single bond, or
   C₁₋₆ alkylene (the alkylene is optionally substituted with 1 to 2 substituents selected from the group consisting of a fluorine atom, and C₁₋₃ alkyl),
V represents
   a single bond,
   C₂₋₆ alkenylene (the alkenylene is optionally substituted with one substituent selected from the group consisting of a hydroxyl group, and C₁₋₃ alkyl),
   C₃₋₁₀ cycloalkylene (the cycloalkylene is optionally substituted with one substituent selected from the group consisting of a hydroxyl group, and C₁₋₃ alkyl),
   C₃₋₁₀ cycloalkenylene (the cycloalkenylene is optionally substituted with one substituent selected from the group consisting of a hydroxyl group, and C₁₋₃ alkyl), or
   a 3- to 10-membered divalent saturated heterocyclic group (the saturated heterocyclic group is optionally substituted with one substituent selected from the group consisting of a fluorine atom, a hydroxyl group, and C₁₋₃ alkyl optionally substituted with 1 to 2 fluorine atoms),
W represents
   a single bond, or
   C₁₋₃ alkylene optionally substituted with 1 to 2 fluorine atoms, and
Q represents
   a hydrogen atom
   NH₂, or
   NHMe.

### [Item 71]

The compound or the pharmaceutically acceptable salt thereof of any one of items 1 to 31 and 70, wherein L is C₁₋₆ alkylene.

### [Item 72]

The compound or the pharmaceutically acceptable salt thereof of any one of items 1 to 31 and 70 to 71, wherein V is a single bond.

### [Item 73]

The compound or the pharmaceutically acceptable salt thereof of any one of items 1 to 31 and 70 to 72, wherein W is
a single bond, or
C₁₋₃ alkylene.

### [Item 74]

The compound or the pharmaceutically acceptable salt thereof of any one of items 1 to 31 and 70 to 73, wherein Q is NH₂.

### [Item 74A]

The compound or the pharmaceutically acceptable salt thereof of any one of items 70 to 74, wherein
-L-V-W- is C₁₋₆ alkylene (the alkylene is optionally substituted with 1 to 3 of the same or different substituents selected from the group consisting of a fluorine atom, a hydroxyl group, C₁₋₃ alkyl, C₁₋₃ alkoxy, - NR¹⁶R¹⁷, and cyano),
Q is NHR¹⁴, and
R¹⁶ and R¹⁷ represent each independently a hydrogen atom or C₁₋₆ alkyl, and when R¹⁶ or R¹⁷ is plural, then each of R¹⁶ or R¹⁷ may be the same or different, wherein when R¹⁶ and R¹⁷ attached to the same nitrogen atom are both C₁₋₆ alkyl, then they may form a 3- to 8-membered nitrogen-containing saturated heterocycle together with the nitrogen atom to which each is attached.

### [Item 74B]

The compound or the pharmaceutically acceptable salt thereof of any one of items 70 to 74, wherein
L is C₁₋₆ alkylene (the alkylene is optionally substituted with 1 to 3 of the same or different substituents selected from the group consisting of a fluorine atom, a hydroxyl group, C₁₋₃ alkyl, C₁₋₃ alkoxy, - NR¹⁶R¹⁷, and cyano),
V is a 3- to 10-membered divalent saturated heterocyclic group (the saturated heterocyclic group is optionally substituted with 1 to 3 of the same or different substituents selected from the group consisting of a fluorine atom, C₁₋₃ alkyl optionally substituted with 1 to 2 fluorine atoms, C₁₋₃ alkoxy, -NR¹⁶R¹⁷, and cyano),
W is a single bond,
Q is a hydrogen atom, and
R¹⁶ and R¹⁷ represent each independently a hydrogen atom or C₁₋₆ alkyl, and when R¹⁶ or R¹⁷ is plural, then each of R¹⁶ or R¹⁷ may be the same or different, wherein when R¹⁶ and R¹⁷ attached to the same nitrogen atom are both C₁₋₆ alkyl, then they may form a 3- to 8-membered nitrogen-containing saturated heterocycle together with the nitrogen atom to which each is attached.

### [Item 74C]

The compound or the pharmaceutically acceptable salt thereof of any one of items 70 to 74, wherein
L is C₁₋₆ alkylene (the alkylene is optionally substituted with 1 to 3 of the same or different substituents selected from the group consisting of a fluorine atom, a hydroxyl group, C₁₋₃ alkyl, C₁₋₃ alkoxy, - NR¹⁶R¹⁷, and cyano),
V is C₃₋₁₀ cycloalkylene (the cycloalkylene is optionally substituted with 1 to 3 of the same or different substituents selected from the group consisting of a fluorine atom, a hydroxyl group, C₁₋₃ alkyl, C₁₋₃ alkoxy, -NR¹⁶R¹⁷, and cyano),
W is C₁₋₆ alkylene (the alkylene is optionally substituted with 1 to 3 of the same or different substituents selected from the group consisting of a fluorine atom, a hydroxyl group, C₁₋₃ alkoxy, -NR¹⁶R¹⁷, and cyano),
Q is NHR¹⁴, and
R¹⁶ and R¹⁷ represent each independently a hydrogen atom or C₁₋₆ alkyl, and when R¹⁶ or R¹⁷ is plural, then each of R¹⁶ or R¹⁷ may be the same or different, wherein when R¹⁶ and R¹⁷ attached to the same nitrogen atom are both C₁₋₆ alkyl, then they may form a 3- to 8-membered nitrogen-containing saturated heterocycle together with the nitrogen atom to which each is attached.

### [Item 75]

The compound or the pharmaceutically acceptable salt thereof of item 1, which is selected from the following compounds:
5-({5-[7-(3-aminopropoxy)quinolin-8-yl]-1H-pyrazol-3-yl}amino)pyrazine-2-carbonitrile (Example 1),
5-({5-[6-(3-aminopropoxy)-1-benzofuran-7-yl]-1H-pyrazol-3-yl}amino)pyrazine-2-carbonitrile (Example 2),
5-({5-[6-(3-aminopropoxy)-2,3-dihydro-1-benzofuran-7-yl]-1H-pyrazol-3-yl}amino)pyrazine-2-carbonitrile (Example 3),
5-{[5-(6-{ [1-(aminomethyl)cyclopropyl]methoxy}-2,3-dihydro-1-benzofuran-7-yl)-1H-pyrazol-3-yl]amino}pyrazine-2-carbonitrile (Example 4),
5-[(5-{6-[(3-methylazetidin-3-yl)methoxy]-2,3-dihydro-1-benzofuran-7-yl}-1H-pyrazol-3-yl)amino]pyrazine-2-carbonitrile (Example 5),
5-({5-[6-(3-aminopropoxy)pyrazolo[1,5-a]pyridin-7-yl]-1H-pyrazol-3-yl}amino)pyrazine-2-carbonitrile (Example 6),
5-({5-[6-(3-aminopropoxy)pyrazolo[1,5-a]pyrimidin-7-yl]-1H-pyrazol-3-yl}amino)pyrazine-2-carbonitrile (Example 7),
5-({5-[3-(3-aminopropoxy)-1,5-naphthyridin-4-yl]-1H-pyrazol-3-yl}amino)pyrazine-2-carbonitrile (Example 8),
5-({5-[3-(3-amino-2,2-difluoropropoxy)-1,5-naphthyridin-4-yl]-1H-pyrazol-3-yl}amino)pyrazine-2-carbonitrile (Example 9),
5-{[5-(3-{[1-(aminomethyl)cyclopropyl]methoxy}-1,5-naphthyridin-4-yl)-1H-pyrazol-3-yl]amino}pyrazine-2-carbonitrile (Example 10),
5-[(5-{3-[(3-methylazetidin-3-yl)methoxy]-1,5-naphthyridin-4-yl}-1H-pyrazol-3-yl)amino]pyrazine-2-carbonitrile (Example 11),
5-{[5-(3-{ [(2S)-morpholin-2-yl]methoxy}-1,5-naphthyridin-4-yl)-1H-pyrazol-3-yl]amino}pyrazine-2-carbonitrile (Example 12),
5-({5-[6-(3-aminopropoxy)-2,3-dihydrofuro[3,2-b]pyridin-7-yl]-1H-pyrazol-3-yl}amino)pyrazine-2-carbonitrile (Example 13),
5-{[5-(6-{ [1-(aminomethyl)cyclopropyl]methoxy}-2,3-dihydrofuro[3,2-b]pyridin-7-yl)-1H-pyrazol-3-yl]amino}pyrazine-2-carbonitrile (Example 14),
5-[(5-{6-[(3-methylazetidin-3-yl)methoxy]-2,3-dihydrofuro[3,2-b]pyridin-7-yl}-1H-pyrazol-3-yl)amino]pyrazine-2-carbonitrile (Example 15),
5-({5-[6-(3-aminopropoxy)-2-methyl-1,3-benzoxazol-7-yl]-1H-pyrazol-3-yl}amino)pyrazine-2-carbonitrile (Example 16),
5-{[5-(6-{[1-(aminomethyl)cyclopropyl]methoxy}pyrazolo[1,5-a]pyridin-7-yl)-1H-pyrazol-3-yl]amino}pyrazine-2-carbonitrile (Example 17),
5-[(5-{6-[(3-methylazetidin-3-yl)methoxylpyrazolo[1,5-a]pyridin-7-yl}-1H-pyrazol-3-yl)amino]pyrazine-2-carbonitrile (Example 18),
5-{[5-(6-{[1-(aminomethyl)cyclopropyl]methoxy}-1-benzofuran-7-yl)-1H-pyrazol-3-yl]amino}pyrazine-2-carbonitrile (Example 19),
5-{[5-(6-{[1-(aminomethyl)-2,2-difluorocyclopropyl]methoxy}-1-benzofuran-7-yl)-1H-pyrazol-3-yl]amino}pyrazine-2-carbonitrile (Example 20),
5-{[5-(6-{[(1R,3R)-3-aminocyclopentyl]oxy}-2,3-dihydrofuro[3,2-b]pyridin-7-yl)-1H-pyrazol-3-yl]amino}pyrazine-2-carbonitrile (Example 21),
5-{[5-(6-{[3-(difluoromethyl)azetidin-3-yl]methoxy}-2,3-dihydrofuro[3,2-b]pyridin-7-yl)-1H-pyrazol-3-yl]amino}pyrazine-2-carbonitrile (Example 22),
5-[(5-{6-[(3-fluoroazetidin-3-yl)methoxy]-2,3-dihydrofuro[3,2-b]pyridin-7-yl}-1H-pyrazol-3-yl)amino]pyrazine-2-carbonitrile (Example 23),
5-{[5-(6-{[(1R,3S)-3-aminocyclohexyl]oxy}-2,3-dihydrofuro[3,2-b]pyridin-7-yl)-1H-pyrazol-3-yl]amino}pyrazine-2-carbonitrile (Example 24),
5-[(5-{6-[(1R)-1-(azetidin-3-yl)ethoxy]-2,3-dihydrofuro[3,2-b]pyridin-7-yl}-1H-pyrazol-3-yl)amino]pyrazine-2-carbonitrile (Example 25),
5-{[5-(6-{[(3S)-pyrrolidin-3-yl]methoxy}-2,3-dihydrofuro[3,2-b]pyridin-7-yl)-1H-pyrazol-3-yl]amino}pyrazine-2-carbonitrile (Example 26),
5-[(5-{6-[3-(methylamino)propoxy]-2,3-dihydrofuro[3,2-b]pyridin-7-yl}-1H-pyrazol-3-yl)amino]pyrazine-2-carbonitrile (Example 27),
5-[(5-{6-[(2R)-3-amino-2-methylpropoxy]-2,3-dihydrofuro[3,2-b]pyridin-7-yl}-1H-pyrazol-3-yl)amino]pyrazine-2-carbonitrile (Example 28),
5-{[5-(6-{[(1R,2S,4S,5S)-4-aminobicyclo[3.1.0]hexan-2-yl]oxy}-2,3-dihydrofuro[3,2-b]pyridin-7-yl)-1H-pyrazol-3-yl]amino}pyrazine-2-carbonitrile (Example 29),
5-[(5-{6-[(2S)-3-amino-2-methylpropoxy]-2,3-dihydrofuro[3,2-b]pyridin-7-yl}-1H-pyrazol-3-yl)amino]pyrazine-2-carbonitrile (Example 30),
5-{[5-(6-{[2-(aminomethyl)prop-2-en-1-yl]oxy}-2,3-dihydrofuro[3,2-b]pyridin-7-yl)-1H-pyrazol-3-yl]amino}pyrazine-2-carbonitrile (Example 31),
N-{5-[6-(3-aminopropoxy)-2,3-dihydrofuro[3,2-b]pyridin-7-yl]-1H-pyrazol-3-yl}-5-chloropyrazin-2-amine (Example 32),
methyl 5-({5-[6-(3-aminopropoxy)-2,3-dihydrofuro[3,2-b]pyridin-7-yl]-1H-pyrazol-3-yl}amino)pyrazine-2-carboxylate (Example 33),
5-{[5-(3-{[(1R,2S)-2-(aminomethyl)cyclopentyl]oxy}-1,5-naphthyridin-4-yl)-1H-pyrazol-3-yl]amino}pyrazine-2-carbonitrile (Example 34),
5-[(5-{3-[(3-fluoroazetidin-3-yl)methoxy]-1,5-naphthyridin-4-yl}-1H-pyrazol-3-yl)amino]pyrazine-2-carbonitrile (Example 35),
5-{[5-(3-{[(1R,3S)-3-aminocyclohexyl]oxy}-1,5-naphthyridin-4-yl)-1H-pyrazol-3-yl]amino}pyrazine-2-carbonitrile (Example 36),
5-{[5-(3-{[(1R,3R)-3-aminocyclopentyl]oxy}-1,5-naphthyridin-4-yl)-1H-pyrazol-3-yl]amino}pyrazine-2-carbonitrile (Example 37), and
5-{[5-(3-{[1-(aminomethyl)-3,3-difluorocyclobutyl]methoxy}-1,5-naphthyridin-4-yl)-1H-pyrazol-3-yl]amino}pyrazine-2-carbonitrile (Example 38).

### [Item 76]

The compound or the pharmaceutically acceptable salt thereof of item 1, which is the following:
5-({5-[7-(3-aminopropoxy)quinolin-8-yl]-1H-pyrazol-3-yl}amino)pyrazine-2-carbonitrile (Example 1),
5-({5-[3-(3-aminopropoxy)-1,5-naphthyridin-4-yl]-1H-pyrazol-3-yl}amino)pyrazine-2-carbonitrile (Example 8),
5-({5-[3-(3-amino-2,2-difluoropropoxy)-1,5-naphthyridin-4-yl]-1H-pyrazol-3-yl}amino)pyrazine-2-carbonitrile (Example 9),
5-{[5-(3-{[1-(aminomethyl)cyclopropyl]methoxy}-1,5-naphthyridin-4-yl)-1H-pyrazol-3-yl]amino}pyrazine-2-carbonitrile (Example 10),
5-[(5-{3-[(3-methylazetidin-3-yl)methoxy]-1,5-naphthyridin-4-yl}-1H-pyrazol-3-yl)amino]pyrazine-2-carbonitrile (Example 11),
5-({5-[6-(3-aminopropoxy)-2,3-dihydrofuro[3,2-b]pyridin-7-yl]-1H-pyrazol-3-yl}amino)pyrazine-2-carbonitrile (Example 13),
5-{[5-(6-{[1-(aminomethyl)cyclopropyl]methoxy}-2,3-dihydrofuro[3,2-b]pyridin-7-yl)-1H-pyrazol-3-yl]amino}pyrazine-2-carbonitrile (Example 14),
5-[(5-{6-[(1R)-1-(azetidin-3-yl)ethoxy]-2,3-dihydrofuro[3,2-b]pyridin-7-yl}-1H-pyrazol-3-yl)amino]pyrazine-2-carbonitrile (Example 25),
5-{[5-(6-{[(3S)-pyrrolidin-3-yl]methoxy}-2,3-dihydrofuro[3,2-b]pyridin-7-yl)-1H-pyrazol-3-yl]amino}pyrazine-2-carbonitrile (Example 26),
5-[(5-{6-[3-(methylamino)propoxy]-2,3-dihydrofuro[3,2-b]pyridin-7-yl}-1H-pyrazol-3-yl)amino]pyrazine-2-carbonitrile (Example 27),
5-[(5-{6-[(2R)-3-amino-2-methylpropoxy]-2,3-dihydrofuro[3,2-b]pyridin-7-yl}-1H-pyrazol-3-yl)amino]pyrazine-2-carbonitrile (Example 28),
5-[(5-{3-[(3-fluoroazetidin-3-yl)methoxy]-1,5-naphthyridin-4-yl}-1H-pyrazol-3-yl)amino]pyrazine-2-carbonitrile (Example 35), or
5-{[5-(3-{[1-(aminomethyl)-3,3-difluorocyclobutyl]methoxy}-1,5-naphthyridin-4-yl)-1H-pyrazol-3-yl]amino}pyrazine-2-carbonitrile (Example 38).

### [Item 77]

The compound or the pharmaceutically acceptable salt thereof of item 1, which is the following:
5-({5-[7-(3-aminopropoxy)quinolin-8-yl]-1H-pyrazol-3-yl}amino)pyrazine-2-carbonitrile (Example 1),
5-({5-[3-(3-aminopropoxy)-1,5-naphthyridin-4-yl]-1H-pyrazol-3-yl}amino)pyrazine-2-carbonitrile (Example 8),
5-{ [5-(3-{ [1-(aminomethyl)cyclopropyl]methoxy}-1,5-naphthyridin-4-yl)-1H-pyrazol-3-yl]amino}pyrazine-2-carbonitrile (Example 10),
5-({5-[6-(3-aminopropoxy)-2,3-dihydrofuro[3,2-b]pyridin-7-yl]-1H-pyrazol-3-yl}amino)pyrazine-2-carbonitrile (Example 13),
5-{[5-(6-{[1-(aminomethyl)cyclopropyl]methoxy}-2,3-dihydrofuro[3,2-b]pyridin-7-yl)-1H-pyrazol-3-yl]amino}pyrazine-2-carbonitrile (Example 14),
5-[(5-{6-[(1R)-1-(azetidin-3-yl)ethoxy]-2,3-dihydrofuro[3,2-b]pyridin-7-yl}-1H-pyrazol-3-yl)amino]pyrazine-2-carbonitrile (Example 25), or
5-[(5-{6-[(2R)-3-amino-2-methylpropoxy]-2,3-dihydrofuro[3,2-b]pyridin-7-yl}-1H-pyrazol-3-yl)amino]pyrazine-2-carbonitrile (Example 28).

### [Item 78]

A liposome comprising the compound or the pharmaceutically acceptable salt thereof of any one of items 1 to 77.

### [Item 79]

A pharmaceutical composition comprising the compound or the pharmaceutically acceptable salt thereof of any one of items 1 to 77 as an active ingredient.

### [Item 80]

A pharmaceutical composition comprising a liposome encapsulating the compound or the pharmaceutically acceptable salt thereof of any one of items 1 to 77.

### [Item 81]

The pharmaceutical composition of item 80, wherein the liposome further comprises a phospholipid.

### [Item 82]

The pharmaceutical composition of item 80 or 81, wherein the liposome comprises
(1) the compound or the pharmaceutically acceptable salt thereof of any one of items 1 to 77, and
(2) a phospholipid.

### [Item 83]

The pharmaceutical composition of item 81 or 82, wherein the phospholipid is one phospholipid selected from the group consisting of phosphatidylcholine, phosphatidylglycerol, phosphatidic acid, phosphatidylethanolamine, phosphatidylserine, phosphatidylinositol, sphingomyelin, soybean lecithin, egg yolk lecithin, hydrogenated egg yolk lecithin, and hydrogenated soybean lecithin, or a combination of two or more thereof.

### [Item 84]

The pharmaceutical composition of any one of item 80 to 83, wherein the liposome further comprises a sterol.

### [Item 85]

The pharmaceutical composition of item 84, wherein the sterol is a cholesterol.

### [Item 86]

The pharmaceutical composition of any one of item 80 to 85, wherein the liposome further comprises a polymer-modified lipid.

### [Item 87]

The pharmaceutical composition of item 86, wherein a polymer moiety of the polymer-modified lipid is polyethylene glycol, polypropylene glycol, polyvinyl alcohol, polyvinylpyrrolidone, methoxypolyethylene glycol, methoxypolypropylene glycol, methoxypolyvinyl alcohol, methoxypolyvinylpyrrolidone, ethoxypolyethylene glycol, ethoxypolypropylene glycol, ethoxypolyvinyl alcohol, ethoxypolyvinylpyrrolidone, propoxypolyethylene glycol, propoxypolypropylene glycol, propoxypolyvinyl alcohol, or propoxypolyvinylpyrrolidone.

### [Item 88]

The pharmaceutical composition of item 86 or 87, wherein a lipid moiety of the polymer-modified lipid is phosphatidylethanolamine or diacylglycerol.

### [Item 89]

The pharmaceutical composition of any one of item 86 to 88, wherein the polymer-modified lipid comprises polyethylene glycol, polypropylene glycol, polyvinyl alcohol, polyvinylpyrrolidone, methoxypolyethylene glycol, methoxypolypropylene glycol, methoxypolyvinyl alcohol, methoxypolyvinylpyrrolidone, ethoxypolyethylene glycol, ethoxypolypropylene glycol, ethoxypolyvinyl alcohol, ethoxypolyvinylpyrrolidone, propoxypolyethylene glycol, propoxypolypropylene glycol, propoxypolyvinyl alcohol, or propoxypolyvinylpyrrolidone as a polymer moiety, and comprises phosphatidylethanolamine or diacylglycerol as a lipid moiety.

### [Item 90]

The pharmaceutical composition of any one of item 80 to 89, wherein the liposome comprises
(1) the compound or the pharmaceutically acceptable salt thereof of any one of items 1 to 77,
(2) 40 to 70 mol% of a phospholipid,
(3) 30 to 50 mol% of a cholesterol, and
(4) 1 to 10 mol% of a polymer-modified lipid.

### [Item 91]

The pharmaceutical composition of any one of item 80 to 90, wherein the liposome further comprises an additive selected from the group consisting of an inorganic acid, an inorganic acid salt, an organic acid, an organic acid salt, a saccharide, a buffer, an antioxidant, and a polymer.

### [Item 92]

A therapeutic agent and/or prophylactic agent for cancer, comprising the compound or the pharmaceutically acceptable salt thereof of any one of items 1 to 77 as an active ingredient.

### [Item 93]

The therapeutic agent and/or prophylactic agent of item 92, wherein the cancer is at least one type of cancer selected from the group consisting of acute leukemia, chronic lymphocytic leukemia, chronic myelogenous leukemia, myelodysplastic syndrome, polycythemia vera, malignant lymphoma, plasma cell tumor, multiple myeloma, brain tumor, head and neck cancer, esophageal cancer, thyroid cancer, small cell lung cancer, non-small cell lung cancer, thymoma/thymic carcinoma, breast cancer, gastric cancer, gallbladder/bile duct cancer, liver cancer, hepatocellular carcinoma, pancreatic cancer, colon cancer, rectal cancer, anal cancer, gastrointestinal stromal tumor, choriocarcinoma, endometrial cancer, cervical cancer, ovarian cancer, bladder cancer, urothelial cancer, renal cancer, renal cell cancer, prostate cancer, testicular tumor, testicular germ cell tumor, ovarian germ cell tumor, Wilms tumor, malignant melanoma, neuroblastoma, osteosarcoma, Ewing sarcoma, chondrosarcoma, soft tissue sarcoma, or skin cancer.

### [Item 94]

A method for treating and/or preventing cancer, comprising administering a therapeutically and/or prophylactically effective amount of the compound or the pharmaceutically acceptable salt thereof of any one of items 1 to 77, or the liposome of item 78, or the pharmaceutical composition of items 79 to 91, or the therapeutic agent and/or prophylactic agent of item 92 or 93, to a patient in need thereof.

### [Item 95]

The method of item 94, wherein the cancer is at least one type of cancer selected from the group consisting of acute leukemia, chronic lymphocytic leukemia, chronic myelogenous leukemia, myelodysplastic syndrome, polycythemia vera, malignant lymphoma, plasma cell tumor, multiple myeloma, brain tumor, head and neck cancer, esophageal cancer, thyroid cancer, small cell lung cancer, non-small cell lung cancer, thymoma/thymic carcinoma, breast cancer, gastric cancer, gallbladder/bile duct cancer, liver cancer, hepatocellular carcinoma, pancreatic cancer, colon cancer, rectal cancer, anal cancer, gastrointestinal stromal tumor, choriocarcinoma, endometrial cancer, cervical cancer, ovarian cancer, bladder cancer, urothelial cancer, renal cancer, renal cell cancer, prostate cancer, testicular tumor, testicular germ cell tumor, ovarian germ cell tumor, Wilms tumor, malignant melanoma, neuroblastoma, osteosarcoma, Ewing sarcoma, chondrosarcoma, soft tissue sarcoma, or skin cancer.

### [Item 96]

Use of the compound or the pharmaceutically acceptable salt thereof of any one of items 1 to 77, or the liposome of item 78, or the pharmaceutical composition of items 79 to 91, or the therapeutic agent and/or prophylactic agent of item 92 or 93, for the manufacture of a therapeutic agent and/or prophylactic agent for cancer.

### [Item 97]

The use of the compound or the pharmaceutically acceptable salt thereof, or the pharmaceutical composition, or the liposome, or the therapeutic agent and/or prophylactic agent of item 96, wherein the cancer is at least one type of cancer selected from the group consisting of acute leukemia, chronic lymphocytic leukemia, chronic myelogenous leukemia, myelodysplastic syndrome, polycythemia vera, malignant lymphoma, plasma cell tumor, multiple myeloma, brain tumor, head and neck cancer, esophageal cancer, thyroid cancer, small cell lung cancer, non-small cell lung cancer, thymoma/thymic carcinoma, breast cancer, gastric cancer, gallbladder/bile duct cancer, liver cancer, hepatocellular carcinoma, pancreatic cancer, colon cancer, rectal cancer, anal cancer, gastrointestinal stromal tumor, choriocarcinoma, endometrial cancer, cervical cancer, ovarian cancer, bladder cancer, urothelial cancer, renal cancer, renal cell cancer, prostate cancer, testicular tumor, testicular germ cell tumor, ovarian germ cell tumor, Wilms tumor, malignant melanoma, neuroblastoma, osteosarcoma, Ewing sarcoma, chondrosarcoma, soft tissue sarcoma, or skin cancer.

### [Item 98]

The compound or the pharmaceutically acceptable salt thereof of any one of items 1 to 77, or the liposome of item 78, or the pharmaceutical composition of items 79 to 91, or the therapeutic agent and/or prophylactic agent of item 92 or 93, for use in the treatment and/or prophylaxis of cancer.

### [Item 99]

The compound or the pharmaceutically acceptable salt thereof, or the pharmaceutical composition, or the liposome, or the therapeutic agent and/or prophylactic agent of item 98, wherein the cancer is at least one type of cancer selected from the group consisting of acute leukemia, chronic lymphocytic leukemia, chronic myelogenous leukemia, myelodysplastic syndrome, polycythemia vera, malignant lymphoma, plasma cell tumor, multiple myeloma, brain tumor, head and neck cancer, esophageal cancer, thyroid cancer, small cell lung cancer, non-small cell lung cancer, thymoma/thymic carcinoma, breast cancer, gastric cancer, gallbladder/bile duct cancer, liver cancer, hepatocellular carcinoma, pancreatic cancer, colon cancer, rectal cancer, anal cancer, gastrointestinal stromal tumor, choriocarcinoma, endometrial cancer, cervical cancer, ovarian cancer, bladder cancer, urothelial cancer, renal cancer, renal cell cancer, prostate cancer, testicular tumor, testicular germ cell tumor, ovarian germ cell tumor, Wilms tumor, malignant melanoma, neuroblastoma, osteosarcoma, Ewing sarcoma, chondrosarcoma, soft tissue sarcoma, or skin cancer.

### [Item 100]

The compound or the pharmaceutically acceptable salt thereof of any one of items 1 to 77, or the liposome of item 78, for treating cancer, used in combination with a concomitant drug or a pharmaceutically acceptable salt thereof, wherein the concomitant drug is at least one selected from the group consisting of a hormonal therapy agent, a chemotherapeutic agent, an immunotherapeutic agent, and an agent that inhibits a cell growth factor and its receptor action.

### [Item 101]

The compound or the pharmaceutically acceptable salt thereof of any one of items 1 to 77, or the liposome of item 78, for treating cancer, used in combination with a concomitant drug or a pharmaceutically acceptable salt thereof, wherein the concomitant drug is at least one selected from the group consisting of a 5-FU type drug, Cytarabine, Doxorubicin hydrochloride, Gemcitabine, Methotrexate, Pemetrexed, Etoposide, Irinotecan, Topotecan, Cisplatin, Carboplatin, Oxaliplatin, Paclitaxel, Docetaxel, ionizing radiation, Bevacizumab, liposomal Doxorubicin, Rucaparib, Olaparib, Niraparib, Trabectedin, Pazopanib, Pembrolizumab, Nivolumab, Ipilimumab, Durvalumab, Avelumab, Atezolizumab, Larotrectinib, Entrectinib, nab-Paclitaxel, Erlotinib, liposomal Irinotecan, Leucovorin, Cetuximab, Eribulin, Ifosfamide, and Dacarbazine.

### [Item 102]

The compound or the pharmaceutically acceptable salt thereof of any one of items 1 to 77, or the liposome of item 78, for treating cancer, used in combination with a concomitant drug or a pharmaceutically acceptable salt thereof, wherein the concomitant drug is at least one selected from the group consisting of a 5-FU type drug, Irinotecan, Gemcitabine, Cisplatin, Carboplatin, Oxaliplatin, Paclitaxel, ionizing radiation, Rucaparib, Olaparib, Niraparib, Pembrolizumab, and Nivolumab.

### [Item 103]

A pharmaceutical composition comprising the compound or the pharmaceutically acceptable salt thereof of any one of items 1 to 77, or the pharmaceutical composition of any one of items 79 to 91, in combination with a concomitant drug, wherein the concomitant drug is at least one selected from the group consisting of a hormonal therapy agent, a chemotherapeutic agent, an immunotherapeutic agent, and an agent that inhibits a cell growth factor and its receptor action.

### [Item 104]

A pharmaceutical composition comprising the compound or the pharmaceutically acceptable salt thereof of any one of items 1 to 77, or the pharmaceutical composition of any one of items 79 to 91, in combination with a concomitant drug, wherein the concomitant drug is at least one selected from the group consisting of a 5-FU type drug, Cytarabine, Doxorubicin hydrochloride, Gemcitabine, Methotrexate, Pemetrexed, Etoposide, Irinotecan, Topotecan, Cisplatin, Carboplatin, Oxaliplatin, Paclitaxel, Docetaxel, ionizing radiation, Bevacizumab, liposomal Doxorubicin, Rucaparib, Olaparib, Niraparib, Trabectedin, Pazopanib, Pembrolizumab, Nivolumab, Ipilimumab, Durvalumab, Avelumab, Atezolizumab, Larotrectinib, Entrectinib, nab-Paclitaxel, Erlotinib, liposomal Irinotecan, Leucovorin, Cetuximab, Eribulin, Ifosfamide, and Dacarbazine.

### [Item 105]

A pharmaceutical composition comprising the compound or the pharmaceutically acceptable salt thereof of any one of items 1 to 77, or the pharmaceutical composition of any one of items 79 to 91, in combination with a concomitant drug, wherein the concomitant drug is at least one selected from the group consisting of a 5-FU type drug, Irinotecan, Gemcitabine, Cisplatin, Carboplatin, Oxaliplatin, Paclitaxel, ionizing radiation, Rucaparib, Olaparib, Niraparib, Pembrolizumab, and Nivolumab.

### [Effect of the Invention]

The present disclosure provides a CHK1 inhibitor comprising a 1H-pyrazol-3-amine derivatives having a bicyclic skeleton or a pharmaceutically acceptable salt thereof. The compound of the present disclosure has both an excellent CHK1 inhibitory activity as well as high safety. The compound of the present disclosure is encapsulated in a liposome and is released in a sustained manner from the liposome. The compound of the present disclosure is useful as a therapeutic drug for a CHK1-related diseases. Specifically, the compound of the present disclosure can be applied to patients with pancreatic cancer, ovarian cancer, osteosarcoma, Ewing sarcoma, chondrosarcoma, soft tissue sarcoma, or the like.

### [Description of Embodiments]

The present disclosure is described hereinafter in more detail. Throughout the entire specification, a singular expression should be understood as encompassing the concept thereof in the plural form, unless specifically noted otherwise. Thus, singular articles (e.g., "a", "an", "the" and the like in the case of English) should also be understood as encompassing the concept thereof in the plural form, unless specifically noted otherwise. The terms used herein should also be understood as being used in the meaning that is commonly used in the art, unless specifically noted otherwise. Thus, unless defined otherwise, all terminologies and scientific technical terms that are used herein have the same meaning as the general understanding of those skilled in the art to which the present disclosure pertains. In case of a contradiction, the present specification (including the definitions) takes precedence.

The terms used herein are described hereinafter.

As used herein, the number of substituents in a group defined as "optionally substituted" is not particularly limited, as long as they are substitutable. The description of each group is also applicable when the group is a substituent or a part of another group, unless specifically noted otherwise.

"Halogen atom" includes a fluorine atom, a chlorine atom, a bromine atom, an iodine atom, and the like. The preferred is a fluorine atom or a chlorine atom.

"C₁₋₆ alkyl" refers to a linear or branched saturated hydrocarbon group with 1 to 6 carbon atoms, and "C₆ alkyl" refers to alkyl with 6 carbon atoms. The same applies to other numbers. C₁₋₆ alkyl is preferably "C₁₋₄ alkyl", and more preferably "C₁₋₃ alkyl". Specific examples of "C₁₋₃ alkyl" include methyl, ethyl, propyl, 1-methylethyl, and the like. Specific examples of "C₁₋₄ alkyl" include, in addition to the specific examples for the "C₁₋₃ alkyl" described above, butyl, 1,1-dimethylethyl, 1-methylpropyl, 2-methylpropyl, and the like. Specific examples of "C₁₋₆ alkyl" include, in addition to the specific examples for the "C₁₋₄ alkyl" described above, pentyl, 1,1-dimethylpropyl, 1,2-dimethylpropyl, 1-methylbutyl, 2-methylbutyl, 4-methylpentyl, 3-methylpentyl, 2-methylpentyl, 1-methylpentyl, hexyl, and the like.

"C₂₋₆ alkenyl" refers to a linear or branched unsaturated hydrocarbon group with 2 to 6 carbon atoms, comprising 1 to 3 double bonds. "C₂₋₆ alkenyl" is preferably "C₂₋₄ alkenyl". Specific examples of "C₂₋₄ alkenyl" include vinyl, propenyl, methylpropenyl, butenyl, and the like. Specific examples of "C₂₋₆ alkenyl" include, in addition to the specific examples for the "C₂₋₄ alkenyl" described above, pentenyl, hexenyl, and the like.

"C₂₋₆ alkynyl" refers to a linear or branched unsaturated hydrocarbon group with 2 to 6 carbon atoms, comprising one triple bonds. "C₂₋₆ alkynyl" is preferably "C₂₋₄ alkynyl". Specific examples of "C₂₋₄ alkynyl" include propynyl, methylpropynyl, butynyl, and the like. Specific examples of "C₂₋₆ alkynyl" include, in addition to the specific examples for the "C₂₋₄ alkynyl" described above, methylbutynyl, pentynyl, hexynyl, and the like.

"C₁₋₆ alkoxy" is "C₁₋₆ alkyloxy", and the "C₁₋₆ alkyl" moiety is defined the same as the "C₁₋₆ alkyl". "C₁₋₆ alkoxy" is preferably "C₁₋₄ alkoxy", and more preferably "C₁₋₃ alkoxy". Specific examples of "C₁₋₃ alkoxy" include methoxy, ethoxy, propoxy, 1-methylethoxy, and the like. Specific examples of "C₁₋₄ alkoxy" include, in addition to the specific examples for the "C₁₋₃ alkoxy" described above, butoxy, 1,1-dimethylethoxy, 1-methylpropoxy, 2-methylpropoxy, and the like. Specific examples of "C₁₋₆ alkoxy" include, in addition to the specific examples for the "C₁₋₄ alkoxy" described above, pentyloxy, 1,1-dimethylpropoxy, 1,2-dimethylpropoxy, 1-methylbutoxy, 2-methylbutoxy, 4-methylpentyloxy, 3-methylpentyloxy, 2-methylpentyloxy, 1-methylpentyloxy, hexyloxy, and the like.

The "C₁₋₆ alkyl" moiety of "C₁₋₆ alkylthio" is defined by the same as the "C₁₋₆ alkyl". "C₁₋₆ alkylthio" is preferably "C₁₋₄ alkylthio", and more preferably "C₁₋₃ alkylthio". Specific examples of "C₁₋₃ alkylthio" include methylthio, ethylthio, propylthio, 1-methylethylthio, and the like. Specific examples of "C₁₋₄ alkylthio" include, in addition to the specific examples for the "C₁₋₃ alkylthio" described above, butylthio, 1,1-dimethylethylthio, 1-methylpropylthio, 2-methylpropylthio, and the like. Specific examples of "C₁₋₆ alkylthio" include, in addition to the specific examples for the "C₁₋₄ alkylthio" described above, pentylthio, 1,1-dimethylpropylthio, 1,2-dimethylpropylthio, 1-methylbutylthio, 2-methylbutylthio, 4-methylpentylthio, 3-methylpentylthio, 2-methylpentylthio, 1-methylpentylthio, hexylthio, and the like.

"C₁₋₆ alkylene" refers to a linear or branched divalent saturated hydrocarbon group with 1 to 6 carbon atoms. "C₁₋₆ alkylene" is preferably "C₁₋₄ alkylene", and more preferably "C₁₋₃ alkylene". Specific examples of "C₁₋₃ alkylene" include a methylene group, an ethylene group, a propylene group, and the like. Specific examples of "C₁₋₄ alkylene" include, in addition to the specific examples for the "C₁₋₃ alkylene" described above, a butylene group, a 1,1-dimethylethylene group, a 1,2-dimethylethylene group, a 1-methyltrimethylene group, a 2-methyltrimethylene group, and the like. Specific examples of "C₁₋₆ alkylene" include, in addition to the specific examples for the "C₁₋₄ alkylene" described above, a pentylene group, a 1,1-dimethyltrimethylene group, a 1,2-dimethyltrimethylene group, a 1-methylbutylene group, a 2-methylbutylene group, a 1-methylpentylene group, a 2-methylpentylene group, a 3-methylpentylene group, a hexylene group, and the like.

"C₂₋₆ alkenylene" refers to a linear or branched divalent unsaturated hydrocarbon group with 2 to 6 carbon atoms, containing 1 to 3 double bonds. "C₂₋₆ alkenylene" is preferably "C₂₋₄ alkenylene". Specific examples of "C₂₋₄ alkenylene" include a vinylene group, a vinylidene group, a propenylene group, a methylpropenylene group, a butenylene group and the like. Specific examples of "C₂₋₆ alkenyl" include, in addition to the specific examples for the "C₂₋₄ alkenyl" described above, a pentenylene group, a hexenylene group and the like.

"C₃₋₁₀ cycloalkyl" refers to a cyclic saturated hydrocarbon group with 3 to 10 carbon atoms, including those with partially an unsaturated bond and those with a crosslinked structure. "C₃₋₁₀ cycloalkyl" is preferably "C₃₋₇ cycloalkyl". Specific examples of "C₃₋₇ cycloalkyl" include cyclopropyl, cyclobutyl, cyclopentyl, cyclohexyl, cycloheptyl, and the like. Specific examples of "C₃₋₁₀ cycloalkyl" include, in addition to the specific examples for the "C₃₋₇ cycloalkyl" described above, cyclooctyl, cyclononyl, cyclodecyl, adamantyl, and the like.

"C₃₋₁₀ cycloalkyl" also encompasses those with a bicyclic structure in which the C₃₋₁₀ cycloalkyl described above is fused with an aromatic hydrocarbon ring. Specific examples of such a fused compound include the structures represented by the following formulas, and the like.

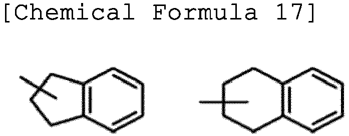

Specific examples of the crosslinked structure described above include the structures represented by the following formulas, and the like.

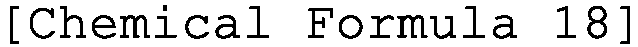

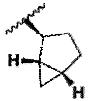

"C₃₋₁₀ cycloalkylene" refers to a cyclic divalent saturated hydrocarbon group with 3 to 10 carbon atoms, including those with partially an unsaturated bond and those with a crosslinked structure. "C₃₋₁₀ cycloalkylene" is preferably "C₃₋₇ cycloalkylene". Specific examples of "C₃₋₇ cycloalkylene" include cyclopropylene, cyclobutylene, cyclopentylene, cyclohexylene, cycloheptylene, and the like. Specific examples of "C₃₋₁₀ cycloalkyl" include, in addition to the specific examples for the "C₃₋₇ cycloalkyl" described above, cyclooctylene, cyclononylene, cyclodecylene, adamantylene, and the like.

Specific examples of the crosslinked structure described above include the structures represented by the following formulas, and the like.

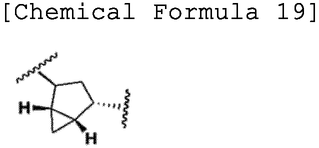

"C₃₋₁₀ cycloalkenylene" refers to a cyclic divalent unsaturated hydrocarbon group with 3 to 10 carbon atoms, including those with a crosslinked structure. Specific examples of "C₃₋₁₀ cycloalkenylene" include cyclobutenylene, cyclopentenylene, cyclohexenylene and the like.

"3- to 10-membered saturated carbocyclic ring" refers to a cyclic saturated hydrocarbon with 3 to 10 carbon atoms. "3- to 10-membered saturated carbocyclic ring" is preferably "4- to 6-membered saturated carbocyclic ring". Specific examples of "4- to 6-membered saturated carbocyclic ring" include a cyclobutane ring, a cyclopentane ring, a cyclohexane ring, and the like. Specific examples of "3- to 10-membered saturated carbocyclic ring" include, in addition to the specific examples for the "4- to 6-membered saturated carbocyclic ring", a cyclopropane ring, a cycloheptane ring, cyclooctane, cyclononane, cyclodecane, and the like.

"3- to 10-membered saturated heterocyclic group" refers to a monovalent saturated heterocyclic group comprised of 1 to 2 atoms independently selected from the group consisting of a nitrogen atom, an oxygen atom and a sulfur atom, and 2 to 9 carbon atoms, including those with partially an unsaturated bond and those with a crosslinked structure. Ring-constituting atoms may include oxidized atoms such as -C(O)-, -S(O)-, or -SO₂-. "3- to 10-membered saturated heterocyclic group" is preferably "4- to 7-membered monocyclic saturated heterocyclic group". Specific examples of "4- to 7-membered monocyclic saturated heterocyclic group" include oxetanyl, azetidinyl, tetrahydrofuryl, pyrrolidinyl, imidazolidinyl, piperidinyl, morpholinyl, thiomorpholinyl, dioxothiomorpholinyl, hexamethyleniminyl, oxazolidinyl, thiazolidinyl, oxoimidazolidinyl, dioxoimidazolidinyl, oxooxazolidinyl, dioxooxazolidinyl, dioxothiazolidinyl, tetrahydrofuranyl, tetrahydropyranyl, and the like. Examples of "3- to 10-membered saturated heterocyclic group" include, in addition to the specific examples for the "4- to 7-membered monocyclic saturated heterocyclic group" described above, oxiranyl, aziridinyl, and the like.

"3- to 10-membered saturated heterocyclic group" also encompasses those with a bicyclic structure in which the 3- to 10-membered saturated heterocyclic group is fused with a 6-membered aromatic hydrocarbon ring or a 6-membered aromatic heterocycle. Examples of the 6-membered aromatic hydrocarbon ring for forming a fused ring include a benzene ring and the like. Examples of the 6-membered aromatic heterocycle for forming a fused ring include pyridine, pyrimidine, pyridazine, and the like. Specific examples of bicyclic "3- to 10-membered saturated heterocyclic group" forming a fused ring include dihydroindolyl, dihydroisoindolyl, dihydropurinyl, dihydrothiazolopyrimidinyl, dihydrobenzodioxanyl, isoindolyl, indazolyl, tetrahydroquinolinyl, tetrahydroisoquinolinyl, tetrahydronaphthyridinyl, and the like.

"3- to 8-membered nitrogen-containing saturated heterocycle" refers to a saturated heterocycle comprised of 1 nitrogen atom and 2 to 7 carbon atoms. "3- to 8-membered nitrogen-containing saturated heterocycle" is preferably "4- to 6-membered nitrogen-containing saturated heterocycle". Specific examples of "4- to 6-membered nitrogen-containing saturated heterocycle" include an azetidine ring, a pyrrolidine ring, a piperidine ring, and the like. Specific examples of "3-to 8-membered nitrogen-containing saturated heterocycle" include, in addition to the specific examples for the "4-to 6-membered nitrogen-containing saturated heterocycle" described above, an aziridine ring, an azepane ring, an azocane ring, and the like.

"3- to 10-membered divalent saturated heterocyclic group" refers to a divalent saturated heterocyclic group comprised of 1 to 2 atoms independently selected from the group consisting of a nitrogen atom, an oxygen atom and a sulfur atom, and 2 to 9 carbon atoms, including those with partially an unsaturated bond and those with a crosslinked structure. Ring-constituting atoms may include oxidized atoms such as -C(O)-, -S(O)-, or -SO₂-. "3- to 10-membered saturated heterocyclic group" is preferably "4- to 7-membered monocyclic saturated heterocyclic group". Specific examples of "4- to 7-membered monocyclic saturated heterocyclic group" include oxetanylene, azetidinylene, tetrahydrofurylene, pyrrolidinylene, imidazolidinylene, piperidinylene, morpholinylene, thiomorpholinylene, dioxothiomorpholinylene, hexamethyleniminylene, oxazolidinylene, thiazolidinylene, oxoimidazolidinylene, dioxooimidazolidinylene, oxooxazolidinylene, dioxooxazolidinylene, dioxothiazolidinylene, tetrahydrofuranylene, tetrahydropyranylene, and the like. Examples of "3- to 10-membered saturated heterocyclic group" include, in addition to the specific examples for the "4- to 7-membered monocyclic saturated heterocyclic group" described above, oxiranylene, aziridinylene, and the like.

"3- to 10-membered saturated heterocyclic group" also encompasses those with a bicyclic structure in which the 3- to 10-membered saturated heterocyclic group is fused with a 6-membered aromatic hydrocarbon ring or a 6-membered aromatic heterocycle. Examples of the 6-membered aromatic hydrocarbon ring for forming a fused ring include a benzene ring and the like. Examples of the 6-membered aromatic heterocycle for forming a fused ring include pyridine, pyrimidine, pyridazine, and the like. Specific examples of bicyclic "3- to 10-membered saturated heterocyclic group" forming a fused ring include dihydroindolylene, dihydroisoindolylene, dihydropurinylene, dihydrothiazolopyrimidinylene, dihydrobenzodioxanylene, isoindolylene, indazolylene, tetrahydroquinolylene, tetrahydroisoquinolinylene, tetrahydronaphthylidinylene, and the like.

"C₆₋₁₀ aryl" refers to an aromatic hydrocarbon ring group with 6 to 10 carbon atoms. Specific examples of "C₆₋₁₀ aryl" include phenyl, 1-naphthyl, 2-naphthyl, and the like. Preferred examples thereof include phenyl.

"C₆₋₁₀ aryl" also encompasses those with a bicyclic structure in which the C₆₋₁₀ aryl is fused with C₄₋₆ cycloalkyl or a 5- to 6-membered saturated heterocycle. Specific examples of bicyclic "C₆₋₁₀ aryl" forming a fused ring include the groups represented by the following and the like.

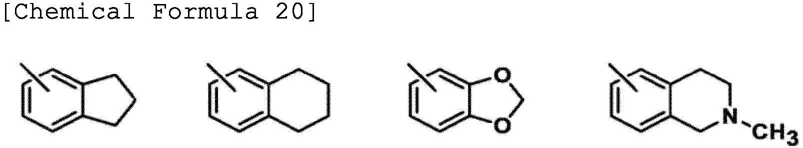

"Aromatic hydrocarbon ring" refers to the cyclic moiety of the "C₆₋₁₀ aryl" described above.

"5- to 12-membered heteroaryl" refers to a cyclic group derived from a monocyclic 5- to 7-membered aromatic heterocycle or a cyclic group derived from a bicyclic 8-to 12-membered aromatic heterocycle, each containing 1 to 4 atoms independently selected from the group consisting of a nitrogen atom, an oxygen atom and a sulfur atom. This is preferably "5- to 7-membered monocyclic heteroaryl", more preferably pyridyl, pyrimidinyl, quinolyl, or isoquinolyl, and more preferably pyridyl. Specific examples of "5- to 7-membered monocyclic heteroaryl" include pyridyl, pyridazinyl, isothiazolyl, pyrrolyl, furyl, thienyl, thiazolyl, imidazolyl, pyrimidinyl, thiadiazolyl, pyrazolyl, oxazolyl, isoxazolyl, pyrazinyl, triazinyl, triazolyl, oxadiazolyl, triazolyl, tetrazolyl, and the like. Specific examples of "5- to 12-membered heteroaryl" include, in addition to the specific examples for the "5- to 7-membered monocyclic heteroaryl" described above, indolyl, indazolyl, chromenyl, quinolyl, isoquinolyl, benzofuranyl, benzothienyl, benzoxazolyl, benzothiazolyl, benzisoxazolyl, benzisothiazolyl, benzotriazolyl, benzimidazolyl, and the like.

"Aromatic heterocycle" refers to a cyclic moiety of the "5- to 12-membered heteroaryl" described above.

"3- to 10-membered cycloalkane" refers to a cyclic saturated hydrocarbon with 3 to 10 carbon atoms, including those with partially an unsaturated bond and those with a crosslinked structure, and a part of which may be fused with another ring. Specific examples of "3-to 10-membered cycloalkane" include cyclobutane, cyclopentane, cyclohexane, cycloheptane and the like.

"6- to 10-membered arene" refers to a cyclic aromatic hydrocarbon with 6 to 10 carbon atoms, and a part of which may be fused with another ring. Specific examples of "6- to 10-membered arene" include benzene, naphthalene and the like.

"4- to 10-membered aromatic heterocycle" refers to a monocyclic 4- to 7-membered aromatic heterocycle, or a bicyclic 8- to 12-membered aromatic heterocycle, each containing 1 to 4 atoms independently selected from the group consisting of a nitrogen atom, an oxygen atom and a sulfur atom, and a part of which may be fused with another ring. This is preferably "5- to 6-membered aromatic heterocycle", "5-membered aromatic heterocycle", or "6-membered aromatic heterocycle". Specific examples of "5- to 6-membered aromatic heterocycle" include furan, thiophene, oxazole, pyridine, pyrazine, pyrimidine, pyridazine and the like. Specific examples of "5-membered aromatic heterocycle" include furan, thiophene, oxazole and the like. Specific examples of "6-membered aromatic heterocycle" include pyridine, pyrazine, pyrimidine, pyridazine and the like.

"4- to 10-membered non-aromatic heterocycle" refers to a non-aromatic heterocycle comprised of 1 to 2 atoms independently selected from the group consisting of a nitrogen atom, an oxygen atom and a sulfur atom, and 2 to 9 carbon atoms, including those with partially an unsaturated bond and those with a crosslinked structure, and a part of which may be fused with another ring. This is preferably "5- to 8-membered non-aromatic heterocycle", and more preferably "5- to 6-membered non-aromatic heterocycle", "5-membered non-aromatic heterocycle" or "6-membered non-aromatic heterocycle". Specific examples of "5- to 8-membered non-aromatic heterocycle" include tetrahydrofuran, dihydrofuran, tetrahydropyran, tetrahydrothiophene, dihydrothiophene, pyrrolidine, piperidine, oxepane, and azepane. Specific examples of "5- to 6-membered non-aromatic heterocycle" include tetrahydrofuran, dihydrofuran, tetrahydropyran, tetrahydrothiophene, dihydrothiophene, pyrrolidine, piperidine, and the like. Specific examples of "5-membered non-aromatic heterocycle" include tetrahydrofuran, dihydrofuran, tetrahydrothiophene, dihydrothiophene and the like. Specific examples of "6-membered non-aromatic heterocycle" include tetrahydropyran.

"Cancer" and "tumor" are defined the same in the present disclosure, both referring to malignant tumor and encompassing cancer, sarcoma, and hematological malignancy. Specific examples of "cancer" and "tumor" include acute leukemia, chronic lymphocytic leukemia, chronic myelogenous leukemia, myelodysplastic syndrome, polycythemia vera, malignant lymphoma, plasma cell tumor, multiple myeloma, brain tumor, head and neck cancer, esophageal cancer, thyroid cancer, small cell lung cancer, non-small cell lung cancer, thymoma/thymic carcinoma, breast cancer, gastric cancer, gallbladder/bile duct cancer, liver cancer, hepatocellular carcinoma, pancreatic cancer, colon cancer, rectal cancer, anal cancer, gastrointestinal stromal tumor, choriocarcinoma, endometrial cancer, cervical cancer, ovarian cancer, bladder cancer, urothelial cancer, renal cancer, renal cell cancer, prostate cancer, testicular tumor, testicular germ cell tumor, ovarian germ cell tumor, Wilms tumor, malignant melanoma, neuroblastoma, osteosarcoma, Ewing sarcoma, chondrosarcoma, soft tissue sarcoma, skin cancer and the like.

Prexasertib is a compound with the following structure.

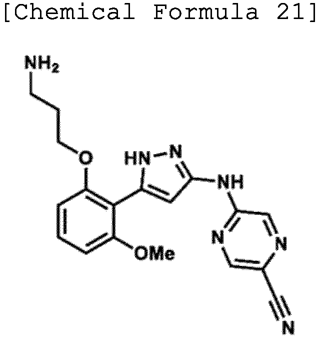

In the compound of the present disclosure represented by formula (1), (2), (3), (4), (5), (6), (7) or (8), preferred R¹, R², R³, R⁴ R⁵, R⁶, R^{7a}, R^{7b}, R⁸, R⁹, R¹⁰, R¹¹, R¹², R¹³, R¹⁴, R¹⁵, R¹⁶,R¹⁷, R¹⁸, Ring A, Ring B, Ring C, Ring D, Y^{1a}, Y^{1b}, Z^{1a}, Z^{1b}, L, V, W and Q are as follows, but the technical scope of the present disclosure is not limited to the range of the compounds listed below.

In a preferred embodiment, R¹ is a hydrogen atom, a halogen atom, cyano, -OR², -CO₂R³, C₁₋₆ alkyl, C₃₋₁₀ cycloalkyl, or a 3- to 10-membered saturated heterocyclic group.

In a more preferred embodiment, R¹ is a hydrogen atom, a halogen atom, cyano or -CO₂R³.

In a further more preferred embodiment, R¹ is a hydrogen atom or cyano.

In a still more preferred embodiment, R¹ is cyano.

In a preferred embodiment, R² is a hydrogen atom or C₁₋₆ alkyl.

In a more preferred embodiment, R² is C₁₋₆ alkyl.

In a preferred embodiment, R³ is C₁₋₆ alkyl.

In a more preferred embodiment, R³ is a methyl group.

In a preferred embodiment, R⁴, R⁵ and R⁶ are each a hydrogen atom, or C₁₋₆ alkyl, wherein when R⁴ and R⁵ attached to the same nitrogen atom are both C₁₋₆ alkyl, then they may form a 3- to 8-membered nitrogen-containing saturated heterocycle together with the nitrogen atom to which each is attached.

In a preferred embodiment, R^{7a} and R^{7b} are each a hydrogen atom, a halogen atom, -OR⁹, C₁₋₆ alkyl (the alkyl is optionally substituted with 1 to 3 of the same or different substituents selected from the group consisting of a fluorine atom, a hydroxyl group, C₁₋₃ alkoxy, - NR¹⁶R¹⁷, and cyano), C₃₋₇ cycloalkyl (the cycloalkyl is optionally substituted with 1 to 3 of the same or different substituents selected from the group consisting of a fluorine atom, a hydroxyl group, C₁₋₃ alkyl, C₁₋₃ alkoxy, -NR¹⁶R¹⁷, and cyano), a 3- to 7-membered saturated heterocyclic group (the saturated heterocyclic group is optionally substituted with 1 to 3 of the same or different substituents selected from the group consisting of a fluorine atom, a hydroxyl group, C₁₋₃ alkyl, C₁₋₃ alkoxy, -NR¹⁶R¹⁷, and cyano), phenyl (the phenyl is optionally substituted with 1 to 3 of the same or different substituents selected from the group consisting of a fluorine atom, a hydroxyl group, C₁₋₃ alkyl, C₁₋₃ alkoxy, -NR¹⁶R¹⁷, and cyano), or 5- to 6-membered heteroaryl (the heteroaryl is optionally substituted with 1 to 3 of the same or different substituents selected from the group consisting of a fluorine atom, a hydroxyl group, C₁₋₃ alkyl, C₁₋₃ alkoxy, -NR¹⁶R¹⁷, and cyano) .

In a more preferred embodiment, R^{7a} and R^{7b} are each a hydrogen atom, a fluorine atom, a chlorine atom, a bromine atom, C₁₋₆ alkyl (the alkyl is optionally substituted with 1 to 2 of the same or different substituents selected from the group consisting of a fluorine atom, a hydroxyl group, C₁₋₃ alkoxy, -NR¹⁶R¹⁷, and cyano), or 5- to 6-membered heteroaryl (the heteroaryl is optionally substituted with 1 to 2 of the same or different substituents selected from the group consisting of a fluorine atom, a hydroxyl group, C₁₋₃ alkyl, C₁₋₃ alkoxy, -NR¹⁶R¹⁷, and cyano) .

In a further more preferred embodiment, R^{7a} and R^{7b} are each a hydrogen atom, a fluorine atom, a chlorine atom, a bromine atom, or C₁₋₃ alkyl.

In a still more preferred embodiment, R^{7a} and R^{7b} are each a hydrogen atom.

In a preferred embodiment, R⁸ is a hydrogen atom, or C₁₋₆ alkyl.

In a preferred embodiment, R⁹ is C₁₋₆ alkyl.

In a preferred embodiment, R¹⁰, R¹¹ and R¹² are each a hydrogen atom or C₁₋₆ alkyl, wherein when R¹¹ and R¹² attached to the same nitrogen atom are both C₁₋₆ alkyl, then they may form a 3- to 8-membered nitrogen-containing saturated heterocycle together with the nitrogen atom to which each is attached.

In a preferred embodiment, R¹³ is a hydrogen atom, or C₁₋₆ alkyl.

In a preferred embodiment, R¹⁴ is a hydrogen atom, or C₁₋₆ alkyl.

In a preferred embodiment, R⁹ is C₁₋₃ alkyl.

In a more preferred embodiment, R⁹ is a methyl group.

In a preferred embodiment, R¹⁰ is C₁₋₃ alkyl.

In a more preferred embodiment, R¹⁰ is a methyl group.

In a preferred embodiment, R¹¹, R¹² and R¹³ are each a hydrogen atom, or C₁₋₆ alkyl.

In a more preferred embodiment, R¹¹, R¹² and R¹³ are each C₁₋₃ alkyl.

In a still more preferred embodiment, R¹¹, R¹² and R¹³ are each a methyl group.

In a preferred embodiment, R¹⁴ is a hydrogen atom, C₁₋₆ alkyl (the alkyl is optionally substituted with 1 to 3 of the same or different substituents selected from the group consisting of a fluorine atom, a hydroxyl group, C₁₋₃ alkoxy, -NR¹⁶R¹⁷, and cyano), C₃₋₁₀ cycloalkyl (the cycloalkyl is optionally substituted with 1 to 3 of the same or different substituents selected from the group consisting of a fluorine atom, a hydroxyl group, C₁₋₃ alkyl, C₁₋₃ alkoxy, -NR¹⁶R¹⁷, and cyano), or a 3- to 10-membered saturated heterocyclic group (the saturated heterocyclic group is optionally substituted with 1 to 3 of the same or different substituents selected from the group consisting of a fluorine atom, a hydroxyl group, C₁₋₃ alkyl, C₁₋₃ alkoxy, -NR¹⁶R¹⁷, and cyano) .

In a more preferred embodiment, R¹⁴ is a hydrogen atom, or C₁₋₆ alkyl (the alkyl is optionally substituted with 1 to 2 of the same or different substituents selected from the group consisting of a fluorine atom, a hydroxyl group, and cyano).

In a further more preferred embodiment, R¹⁴ is a hydrogen atom, or a methyl group (the alkyl is optionally substituted with 1 to 2 of the same or different substituents selected from the group consisting of a fluorine atom, a hydroxyl group, and cyano).

In a still more preferred embodiment, R¹⁴ is a hydrogen atom.

In a preferred embodiment, R¹⁵ is C₁₋₆ alkyl.

In a more preferred embodiment, R¹⁵ is C₁₋₃ alkyl.

In a preferred embodiment, R¹⁶ and R¹⁷ are each a hydrogen atom, or C₁₋₆ alkyl, wherein when R¹⁶ and R¹⁷ attached to the same nitrogen atom are both C₁₋₆ alkyl, then they may form a 3- to 8-membered nitrogen-containing saturated heterocycle together with the nitrogen atom to which each is attached.

In a more preferred embodiment, R¹⁶ and R¹⁷ are each C₁₋₆ alkyl.

In a still more preferred embodiment, R¹⁶ and R¹⁷ are each C₁₋₃ alkyl.

In a preferred embodiment, R¹⁸ is a hydrogen atom, or C₁₋₆ alkyl.

In a more preferred embodiment, R¹⁸ is C₁₋₆ alkyl.

In a still more preferred embodiment, R¹⁸ is C₁₋₃ alkyl.

In a preferred embodiment, Ring A is 3- to 10-membered cycloalkane (the cycloalkane is optionally substituted with 1 to 3 of the same or different substituents selected from the group consisting of a fluorine atom, a hydroxyl group, C₁₋₃ alkyl, C₁₋₃ alkoxy, - NR¹⁶R¹⁷, and cyano), 6- to 10-membered arene (the arene is optionally substituted with 1 to 3 of the same or different substituents selected from the group consisting of a fluorine atom, a hydroxyl group, C₁₋₃ alkyl, C₁₋₃ alkoxy, -NR¹⁶R¹⁷, and cyano), a 4- to 10-membered aromatic heterocycle or a 4- to 10-membered non-aromatic heterocycle (the aromatic heterocycle, or non-aromatic heterocycle is optionally substituted with 1 to 3 of the same or different substituents selected from the group consisting of a fluorine atom, a hydroxyl group, C₁₋₃ alkyl, C₁₋₃ alkoxy, -NR¹⁶R¹⁷, and cyano) .

In a more preferred embodiment, Ring A is a 4- to 10-membered aromatic heterocycle, or a 4- to 10-membered non-aromatic heterocycle (the aromatic heterocycle, or non-aromatic heterocycle is optionally substituted with 1 to 2 of the same or different substituents selected from the group consisting of a fluorine atom, a hydroxyl group, C₁₋₃ alkyl, C₁₋₃ alkoxy, -NR¹⁶R¹⁷, and cyano) .

In a still more preferred embodiment, Ring A is a 5- to 6-membered aromatic heterocycle, or a 5- to 8-membered non-aromatic heterocycle.

In a preferred embodiment, Ring B is a 5- to 6-membered aromatic heterocycle (the aromatic heterocycle is optionally substituted with 1 to 2 of the same or different substituents selected from the group consisting of a fluorine atom, a hydroxyl group, C₁₋₃ alkyl, C₁₋₃ alkoxy, and cyano).

In a more preferred embodiment, Ring B is a 5- to 6-membered aromatic heterocycle.

In a preferred embodiment, Ring C is a 5- to 8-membered non-aromatic heterocycle, or a 5- to 6-membered aromatic heterocycle (the non-aromatic heterocycle and aromatic heterocycle are each optionally substituted with 1 to 2 of the same or different substituents selected from the group consisting of a fluorine atom, a hydroxyl group, C₁₋₃ alkyl, C₁₋₃ alkoxy, and cyano) .

In a more preferred embodiment, Ring C is a 5- to 8-membered non-aromatic heterocycle.

In a preferred embodiment, Ring D is a 5- to 8-membered non-aromatic heterocycle, or a 5- to 6-membered aromatic heterocycle (the non-aromatic heterocycle and aromatic heterocycle are each optionally substituted with 1 to 2 of the same or different substituents selected from the group consisting of a fluorine atom, a hydroxyl group, C₁₋₃ alkyl, C₁₋₃ alkoxy, and cyano) .

In a more preferred embodiment, Ring D is a 5- to 8-membered non-aromatic heterocycle, or a 5- to 6-membered aromatic heterocycle.

In a preferred embodiment, L is a single bond, or C₁₋₆ alkylene (the alkylene is optionally substituted with 1 to 3 of the same or different substituents selected from the group consisting of a fluorine atom, a hydroxyl group, C₁₋₃ alkyl, C₁₋₃ alkoxy, -NR¹⁶R¹⁷, and cyano) .

In a more preferred embodiment, L is a single bond, or C₁₋₆ alkylene (the alkylene is optionally substituted with 1 to 2 of the same or different substituents selected from the group consisting of a fluorine atom, a hydroxyl group, C₁₋₃ alkyl, and cyano) .

In a still more preferred embodiment, L is a single bond, or C₁₋₆ alkylene (the alkylene is optionally substituted with 1 to 2 of the same or different substituents selected from the group consisting of a fluorine atom or C₁₋₃ alkyl) .

In a preferred embodiment, V is a single bond, carbonyl, thiocarbonyl, optionally substituted C₂₋₆ alkenylene, C₃₋₁₀ cycloalkylene, C₃₋₁₀ cycloalkenylene (the alkenylene, cycloalkylene, or cycloalkenylene is optionally substituted with 1 to 3 of the same or different substituents selected from the group consisting of a fluorine atom, a hydroxyl group, C₁₋₃ alkyl, C₁₋₃ alkoxy, -NR¹⁶R¹⁷, and cyano), or a 3- to 10-membered divalent saturated heterocyclic group (the saturated heterocyclic group is optionally substituted with 1 to 3 of the same or different substituents selected from the group consisting of a fluorine atom, a hydroxyl group, C₁₋₃ alkyl optionally substituted with 1 to 2 hydroxyl groups or fluorine atoms, C₁₋₃ alkoxy, -NR¹⁶R¹⁷, and cyano), or -C(=NR¹³).

In a more preferred embodiment, V is a single bond, C₂₋₆ alkenylene, C₃₋₁₀ cycloalkylene, C₃₋₁₀ cycloalkenylene (the alkenylene, cycloalkylene, or cycloalkenylene is optionally substituted with 1 to 3 of the same or different substituents selected from the group consisting of a fluorine atom, a hydroxyl group, C₁₋₃ alkyl, and cyano), or a 3- to 10-membered divalent saturated heterocyclic group (the saturated heterocyclic group is optionally substituted with 1 to 3 of the same or different substituents selected from the group consisting of a fluorine atom, a hydroxyl group, C₁₋₃ alkyl optionally substituted with 1 to 2 fluorine atoms, and cyano).

In a further more preferred embodiment, V is a single bond, C₂₋₆ alkenylene, C₃₋₁₀ cycloalkylene, C₃₋₁₀ cycloalkenylene (the alkenylene, cycloalkylene, and cycloalkenylene are each optionally substituted with 1 to 3 substituents selected from the group consisting of a fluorine atom, a hydroxyl group, and C₁₋₃ alkyl), or a 3-to 10-membered divalent saturated heterocyclic group (the saturated heterocyclic group is optionally substituted with one substituent selected from the group consisting of a fluorine atom, a hydroxyl group, and C₁₋₃ alkyl optionally substituted with 1 to 2 fluorine atoms).

In a still more preferred embodiment, V is a single bond, C₂₋₆ alkenylene, C₃₋₁₀ cycloalkylene (the cycloalkylene is optionally substituted with 1 to 3 fluorine atoms), C₃₋₁₀ cycloalkenylene, or a 3- to 10-membered divalent saturated heterocyclic group (the saturated heterocyclic group is optionally substituted with one substituent selected from the group consisting of a fluorine atom, and C₁₋₃ alkyl optionally substituted with 1 to 2 fluorine atoms).

In a preferred embodiment, W is a single bond, or C₁₋₆ alkylene (the alkylene is optionally substituted with 1 to 3 of the same or different substituents selected from the group consisting of a fluorine atom, a hydroxyl group, C₁₋₃ alkoxy, -NR¹⁶R¹⁷, and cyano) .

In a more preferred embodiment, W is a single bond, or C₁₋₆ alkylene (the alkylene is optionally substituted with 1 to 2 of the same or different substituents selected from the group consisting of a fluorine atom, a hydroxyl group, and cyano).

In a still more preferred embodiment, W is a single bond, or C₁₋₃ alkylene optionally substituted with 1 to 2 fluorine atoms.

In another still more preferred embodiment, W is a single bond, or C₁₋₃ alkylene.

In a preferred embodiment, Q is a hydrogen atom, or NHR¹⁴.

In a more preferred embodiment, Q is a hydrogen atom, NH₂, or NHMe.

In a further more preferred embodiment, Q is a hydrogen atom, or NH₂.

In a still more preferred embodiment, Q is a hydrogen atom.

In another still more preferred embodiment, Q is NH₂.

One embodiment of the compound represented by formula (1) includes the following (A).
(A) A compound or a pharmaceutically acceptable salt thereof, wherein
   R¹ is a hydrogen atom, a halogen atom, cyano, -CO₂R³ or C₁₋₆ alkyl,
   R³ is C₁₋₆ alkyl,
   Y^{1a} and Y^{1b} are each independently a carbon atom, or a nitrogen atom,
   Z^{1a} is a nitrogen atom, or CR^{7a},
   Z^{1b} is a nitrogen atom, or CR^{7b},
   R^{7a} and R^{7b} are each independently
      a hydrogen atom,
      a halogen atom
      -OR⁹,
      C₁₋₆ alkyl (the alkyl is optionally substituted with 1 to 3 of the same or different substituents selected from the group consisting of a fluorine atom, a hydroxyl group, C₁₋₃ alkoxy, -NR¹⁶R¹⁷, and cyano),
      C₃₋₇ cycloalkyl (the cycloalkyl is optionally substituted with 1 to 3 of the same or different substituents selected from the group consisting of a fluorine atom, a hydroxyl group, C₁₋₃ alkyl, C₁₋₃ alkoxy, -NR¹⁶R¹⁷, and cyano),
      a 3- to 7-membered saturated heterocyclic group (the saturated heterocyclic group is optionally substituted with 1 to 3 of the same or different substituents selected from the group consisting of a fluorine atom, a hydroxyl group, C₁₋₃ alkyl, C₁₋₃ alkoxy, -NR¹⁶R¹⁷, and cyano),
      phenyl (the phenyl is optionally substituted with 1 to 3 of the same or different substituents selected from the group consisting of a fluorine atom, a hydroxyl group, C₁₋₃ alkyl, C₁₋₃ alkoxy, -NR¹⁶R¹⁷, and cyano), or
      5- to 6-membered heteroaryl (the heteroaryl is optionally substituted with 1 to 3 of the same or different substituents selected from the group consisting of a fluorine atom, a hydroxyl group, C₁₋₃ alkyl, C₁₋₃ alkoxy, - NR¹⁶R¹⁷, and cyano),
   R⁹ is C₁₋₆ alkyl,
   R¹⁶ and R¹⁷ represent each independently a hydrogen atom or C₁₋₆ alkyl, and when R¹⁶ or R¹⁷ is plural, then each of R¹⁶ or R¹⁷ may be the same or different, wherein when R¹⁶ and R¹⁷ attached to the same nitrogen atom are both C₁₋₆ alkyl, then they may form a 3- to 8-membered nitrogen-containing saturated heterocycle together with the nitrogen atom to which each is attached,
   Ring A is
      3- to 10-membered cycloalkane (the cycloalkane is optionally substituted with 1 to 3 of the same or different substituents selected from the group consisting of a fluorine atom, a hydroxyl group, C₁₋₃ alkyl, C₁₋₃ alkoxy, -NR¹⁶R¹⁷, and cyano),
      6- to 10-membered arene (the arene is optionally substituted with 1 to 3 of the same or different substituents selected from the group consisting of a fluorine atom, a hydroxyl group, C₁₋₃ alkyl, C₁₋₃ alkoxy, - NR¹⁶R¹⁷, and cyano),
      a 4- to 10-membered aromatic heterocycle (the aromatic heterocycle is optionally substituted with 1 to 3 of the same or different substituents selected from the group consisting of a fluorine atom, a hydroxyl group, C₁₋₃ alkyl, C₁₋₃ alkoxy, -NR¹⁶R¹⁷, and cyano), or
      a 4- to 10-membered non-aromatic heterocycle (the non-aromatic heterocycle is optionally substituted with 1 to 3 of the same or different substituents selected from the group consisting of a fluorine atom, a hydroxyl group, C₁₋₃ alkyl, C₁₋₃ alkoxy, -NR¹⁶R¹⁷, and cyano),
   L is
      a single bond, or
      C₁₋₆ alkylene (the alkylene is optionally substituted with 1 to 3 of the same or different substituents selected from the group consisting of a fluorine atom, a hydroxyl group, C₁₋₃ alkyl, C₁₋₃ alkoxy, -NR¹⁶R¹⁷, and cyano),
   V is
      a single bond,
      carbonyl,
      thiocarbonyl,
      C₂₋₆ alkenylene (the alkenylene is optionally substituted with 1 to 3 of the same or different substituents selected from the group consisting of a fluorine atom, a hydroxyl group, C₁₋₃ alkyl, C₁₋₃ alkoxy, -NR¹⁶R¹⁷, and cyano),
      C₃₋₁₀ cycloalkylene (the cycloalkylene is optionally substituted with 1 to 3 of the same or different substituents selected from the group consisting of a fluorine atom, a hydroxyl group, C₁₋₃ alkyl, C₁₋₃ alkoxy, - NR¹⁶R¹⁷, and cyano),
      C₃₋₁₀ cycloalkenylene (the cycloalkenylene is optionally substituted with 1 to 3 of the same or different substituents selected from the group consisting of a fluorine atom, a hydroxyl group, C₁₋₃ alkyl, C₁₋₃ alkoxy, - NR¹⁶R¹⁷, and cyano),
      a 3- to 10-membered divalent saturated heterocyclic group (the saturated heterocyclic group is optionally substituted with 1 to 3 of the same or different substituents selected from the group consisting of a fluorine atom, a hydroxyl group, C₁₋₃ alkyl optionally substituted with 1 to 2 hydroxyl groups or fluorine atoms, C₁₋₃ alkoxy, -NR¹⁶R¹⁷, and cyano), or
      -C(=NR¹³)-,
   W is
      a single bond, or
      C₁₋₆ alkylene (the alkylene is optionally substituted with 1 to 3 of the same or different substituents selected from the group consisting of a fluorine atom, a hydroxyl group, C₁₋₃ alkoxy, -NR¹⁶R¹⁷, and cyano),
   Q is
      a hydrogen atom, or
      NHR¹⁴, and
   R¹⁴ is
      a hydrogen atom,
      C₁₋₆ alkyl (the alkyl is optionally substituted with 1 to 3 of the same or different substituents selected from the group consisting of a fluorine atom, a hydroxyl group, C₁₋₃ alkoxy, -NR¹⁶R¹⁷, and cyano),
      C₃₋₁₀ cycloalkyl (the cycloalkyl is optionally substituted with 1 to 3 of the same or different substituents selected from the group consisting of a fluorine atom, a hydroxyl group, C₁₋₃ alkyl, C₁₋₃ alkoxy, -NR¹⁶R¹⁷, and cyano), or
      a 3- to 10-membered saturated heterocyclic group (the saturated heterocyclic group is optionally substituted with 1 to 3 of the same or different substituents selected from the group consisting of a fluorine atom, a hydroxyl group, C₁₋₃ alkyl, C₁₋₃ alkoxy, -NR¹⁶R¹⁷, and cyano).

One embodiment of the compound represented by formula (2) includes the following (B).

A compound or a pharmaceutically acceptable salt thereof, wherein
R¹ is a hydrogen atom, a halogen atom, cyano, or - CO₂R³,
R³ is C₁₋₆ alkyl,
Z^{1a} is CR^{7a},
Z^{1b} is a nitrogen atom, or CR^{7b},
R^{7a} and R^{7b} are each independently a hydrogen atom, a fluorine atom, a chlorine atom, a bromine atom, or C₁₋₃ alkyl,
Ring A is a 5- to 6-membered aromatic heterocycle (the aromatic heterocycle is optionally substituted with 1 to 2 of the same or different substituents selected from the group consisting of a fluorine atom, a hydroxyl group, C₁₋₃ alkyl, C₁₋₃ alkoxy, and cyano),
L is
   a single bond, or
   C₁₋₆ alkylene (the alkylene is optionally substituted with 1 to 2 of the same or different substituents selected from the group consisting of a fluorine atom, a hydroxyl group, C₁₋₃ alkyl, and cyano),
V is
   a single bond,
   C₂₋₆ alkenylene (the alkenylene is optionally substituted with 1 to 3 of the same or different substituents selected from the group consisting of a fluorine atom, a hydroxyl group, C₁₋₃ alkyl, and cyano),
   C₃₋₁₀ cycloalkylene (the cycloalkylene is optionally substituted with 1 to 3 of the same or different substituents selected from the group consisting of a fluorine atom, a hydroxyl group, C₁₋₃ alkyl, and cyano), C₃₋₁₀ cycloalkenylene (the cycloalkenylene is optionally substituted with 1 to 3 of the same or different substituents selected from the group consisting of a fluorine atom, a hydroxyl group, C₁₋₃ alkyl, and cyano), or
   a 3- to 10-membered divalent saturated heterocyclic group (the saturated heterocyclic group is optionally substituted with 1 to 3 of the same or different substituents selected from the group consisting of a fluorine atom, a hydroxyl group, C₁₋₃ alkyl optionally substituted with 1 to 2 fluorine atoms, and cyano),
W is
   a single bond, or
   C₁₋₆ alkylene (the alkylene is optionally substituted with 1 to 2 of the same or different substituents selected from the group consisting of a fluorine atom, a hydroxyl group, and cyano),
Q is
   a hydrogen atom, or
   NHR¹⁴, and
R¹⁴ is
   a hydrogen atom, or
   C₁₋₆ alkyl (the alkyl is optionally substituted with 1 to 2 of the same or different substituents selected from the group consisting of a fluorine atom, a hydroxyl group, and cyano).

One embodiment of the compound represented by formula (2) includes the following (C).

A compound or a pharmaceutically acceptable salt thereof, wherein
R¹ is a hydrogen atom, a halogen atom, cyano, or - CO₂R³,
R³ is C₁₋₆ alkyl,
Z^{1a} is CR^{7a},
Z^{1b} is a nitrogen atom, or CR^{7b},
R^{7a} and R^{7b} are each independently a hydrogen atom, a fluorine atom, a chlorine atom, a bromine atom, or C₁₋₃ alkyl,
Ring A is a 5- to 8-membered non-aromatic heterocycle (the non-aromatic heterocycle is optionally substituted with 1 to 2 of the same or different substituents selected from the group consisting of a fluorine atom, a hydroxyl group, C₁₋₃ alkyl, C₁₋₃ alkoxy, and cyano),
L is
   a single bond, or
   C₁₋₆ alkylene (the alkylene is optionally substituted with 1 to 2 of the same or different substituents selected from the group consisting of a fluorine atom, a hydroxyl group, C₁₋₃ alkyl, and cyano),
V is
   a single bond,
   C₂₋₆ alkenylene (the alkenylene is optionally substituted with 1 to 3 of the same or different substituents selected from the group consisting of a fluorine atom, a hydroxyl group, C₁₋₃ alkyl, and cyano),
   C₃₋₁₀ cycloalkylene (the cycloalkylene is optionally substituted with 1 to 3 of the same or different substituents selected from the group consisting of a fluorine atom, a hydroxyl group, C₁₋₃ alkyl, and cyano), C₃₋₁₀ cycloalkenylene (the cycloalkenylene is optionally substituted with 1 to 3 of the same or different substituents selected from the group consisting of a fluorine atom, a hydroxyl group, C₁₋₃ alkyl, and cyano), or
   a 3- to 10-membered divalent saturated heterocyclic group (the saturated heterocyclic group is optionally substituted with 1 to 3 of the same or different substituents selected from the group consisting of a fluorine atom, a hydroxyl group, C₁₋₃ alkyl optionally substituted with 1 to 2 fluorine atoms, and cyano),
W is
   a single bond, or
   C₁₋₆ alkylene (the alkylene is optionally substituted with 1 to 2 of the same or different substituents selected from the group consisting of a fluorine atom, a hydroxyl group, and cyano),
Q is
   a hydrogen atom, or
   NHR¹⁴, and
R¹⁴ is
   a hydrogen atom, or
   C₁₋₆ alkyl (the alkyl is optionally substituted with 1 to 2 of the same or different substituents selected from the group consisting of a fluorine atom, a hydroxyl group, and cyano).

One embodiment of the compound represented by formula (2) includes the following (D).

A compound or a pharmaceutically acceptable salt thereof, wherein
R¹ is a hydrogen atom, a halogen atom, cyano, or - CO₂R³,
R³ is C₁₋₆ alkyl,
Z^{1a} is CR^{7a},
Z^{1b} is a nitrogen atom, or CR^{7b},
R^{7a} and R^{7b} are each independently a hydrogen atom, a fluorine atom, a chlorine atom, a bromine atom, or C₁₋₃ alkyl,
Ring A is
   a 5- to 6-membered aromatic heterocycle (the aromatic heterocycle is optionally substituted with 1 to 2 of the same or different substituents selected from the group consisting of a fluorine atom, a hydroxyl group, C₁₋₃ alkyl, C₁₋₃ alkoxy, and cyano), or
   a 5- to 8-membered non-aromatic heterocycle (the non-aromatic heterocycle is optionally substituted with 1 to 2 of the same or different substituents selected from the group consisting of a fluorine atom, a hydroxyl group, C₁₋₃ alkyl, C₁₋₃ alkoxy, and cyano),
L is
   a single bond, or
   C₁₋₆ alkylene (the alkylene is optionally substituted with 1 to 2 of the same or different substituents selected from the group consisting of a fluorine atom, a hydroxyl group, C₁₋₃ alkyl, and cyano),
V is
   a single bond,
   C₂₋₆ alkenylene (the alkenylene is optionally substituted with 1 to 3 of the same or different substituents selected from the group consisting of a fluorine atom, a hydroxyl group, C₁₋₃ alkyl, and cyano),
   C₃₋₁₀ cycloalkylene (the cycloalkylene is optionally substituted with 1 to 3 of the same or different substituents selected from the group consisting of a fluorine atom, a hydroxyl group, C₁₋₃ alkyl, and cyano), C₃₋₁₀ cycloalkenylene (the cycloalkenylene is optionally substituted with 1 to 3 of the same or different substituents selected from the group consisting of a fluorine atom, a hydroxyl group, C₁₋₃ alkyl, and cyano), or
   a 3- to 10-membered divalent saturated heterocyclic group (the saturated heterocyclic group is optionally substituted with 1 to 3 of the same or different substituents selected from the group consisting of a fluorine atom, a hydroxyl group, C₁₋₃ alkyl optionally substituted with 1 to 2 fluorine atoms, and cyano),
W is
   a single bond, or
   C₁₋₆ alkylene (the alkylene is optionally substituted with 1 to 2 of the same or different substituents selected from the group consisting of a fluorine atom, a hydroxyl group, and cyano),
Q is
   a hydrogen atom, or
   NHR¹⁴, and
R¹⁴ is
   a hydrogen atom, or
   C₁₋₆ alkyl (the alkyl is optionally substituted with 1 to 2 of the same or different substituents selected from the group consisting of a fluorine atom, a hydroxyl group, and cyano).

One embodiment of the compound represented by formula (3) includes the following (E).

A compound or a pharmaceutically acceptable salt thereof, wherein
Y^{1b} is a carbon atom,
Ring B is a 5- to 6-membered aromatic heterocycle,
L is
   a single bond, or
   C₁₋₆ alkylene (the alkylene is optionally substituted with 1 to 2 of the same or different substituents selected from the group consisting of a fluorine atom or C₁₋₃ alkyl),
V is
   a single bond,
   C₂₋₆ alkenylene (the alkenylene is optionally substituted with one substituent selected from the group consisting of a hydroxyl group, and C₁₋₃ alkyl),
   C₃₋₁₀ cycloalkylene (the cycloalkylene is optionally substituted with one substituent selected from the group consisting of a fluorine atom, a hydroxyl group, and C₁₋₃ alkyl),
   C₃₋₁₀ cycloalkenylene (the cycloalkenylene is optionally substituted with one substituent selected from the group consisting of a hydroxyl group, and C₁₋₃ alkyl), or a 3- to 10-membered divalent saturated heterocyclic group (the saturated heterocyclic group is optionally substituted with one substituent selected from the group consisting of a fluorine atom, a hydroxyl group, and C₁₋₃ alkyl optionally substituted with 1 to 2 fluorine atoms),
W is
   a single bond, or
   C₁₋₃ alkylene optionally substituted with 1 to 2 fluorine atoms, and
Q is
   a hydrogen atom, or
   NH₂.

One embodiment of the compound represented by formula (4) includes the following (F).

A compound or a pharmaceutically acceptable salt thereof, wherein
L is
   a single bond, or
   C₁₋₆ alkylene (the alkylene is optionally substituted with 1 to 2 of the same or different substituents selected from the group consisting of a fluorine atom or C₁₋₃ alkyl),
V is
   a single bond,
   C₂₋₆ alkenylene,
   C₃₋₁₀ cycloalkylene (the cycloalkylene is optionally substituted with 1 to 3 fluorine atoms),
   C₃₋₁₀ cycloalkenylene, or
   a 3- to 10-membered divalent saturated heterocyclic group (the saturated heterocyclic group is optionally substituted with one substituent selected from the group consisting of a fluorine atom, and C₁₋₃ alkyl optionally substituted with 1 to 2 fluorine atoms),
W is
   a single bond, or
   C₁₋₃ alkylene optionally substituted with 1 to 2 fluorine atoms, and
Q is
   a hydrogen atom.

One embodiment of the compound represented by formula (4) includes the following (G).

A compound or a pharmaceutically acceptable salt thereof, wherein
L is
   a single bond, or
   C₁₋₆ alkylene (the alkylene is optionally substituted with 1 to 2 of the same or different substituents selected from the group consisting of a fluorine atom or C₁₋₃ alkyl),
V is
   a single bond,
   C₂₋₆ alkenylene,
   C₃₋₁₀ cycloalkylene (the cycloalkylene is optionally substituted with 1 to 3 fluorine atoms),
   C₃₋₁₀ cycloalkenylene, or
   a 3- to 10-membered divalent saturated heterocyclic group (the saturated heterocyclic group is optionally substituted with one substituent selected from the group consisting of a fluorine atom, and C₁₋₃ alkyl optionally substituted with 1 to 2 fluorine atoms),
W is
   a single bond, or
   C₁₋₃ alkylene optionally substituted with 1 to 2 fluorine atoms, and
Q is NH₂.

One embodiment of the compound represented by formula (5) includes the following (H).

A compound or a pharmaceutically acceptable salt thereof, wherein
R¹ is a hydrogen atom, a halogen atom, cyano, or - CO₂R³,
R³ is C₁₋₆ alkyl,
Ring C is a 5- to 8-membered non-aromatic heterocycle,
L is
   a single bond, or
   C₁₋₆ alkylene (the alkylene is optionally substituted with 1 to 2 of the same or different substituents selected from the group consisting of a fluorine atom, or C₁₋₃ alkyl),
V is
   a single bond,
   C₂₋₆ alkenylene (the alkenylene is optionally substituted with one substituent selected from the group consisting of a hydroxyl group, and C₁₋₃ alkyl),
   C₃₋₁₀ cycloalkylene (the cycloalkylene is optionally substituted with one substituent selected from the group consisting of a hydroxyl group, and C₁₋₃ alkyl),
   C₃₋₁₀ cycloalkenylene (the cycloalkenylene is optionally substituted with one substituent selected from the group consisting of a hydroxyl group, and C₁₋₃ alkyl), or
   a 3- to 10-membered divalent saturated heterocyclic group (the saturated heterocyclic group is optionally substituted with one substituent selected from the group consisting of a fluorine atom, a hydroxyl group, and C₁₋₃ alkyl optionally substituted with 1 to 2 hydroxyl groups or fluorine atoms),
W is
   a single bond, or
   C₁₋₃ alkylene, and
Q is
   a hydrogen atom
   NH₂, or
   NMeH.

One embodiment of the compound represented by formula (6) includes the following (I).

A compound or a pharmaceutically acceptable salt thereof, wherein
L is
   a single bond, or
   C₁₋₆ alkylene (the alkylene is optionally substituted with 1 to 2 of the same or different substituents selected from the group consisting of a fluorine atom, or C₁₋₃ alkyl),
V is
   a single bond,
   C₂₋₆ alkenylene,
   C₃₋₁₀ cycloalkylene,
   C₃₋₁₀ cycloalkenylene, or
   a 3- to 10-membered divalent saturated heterocyclic group (the saturated heterocyclic group is optionally substituted with one substituent selected from the group consisting of a fluorine atom, and C₁₋₃ alkyl optionally substituted with 1 to 2 fluorine atoms),
W is
   a single bond, or
   C₁₋₃ alkylene, and
Q is
   a hydrogen atom
   NH₂, or
   NMeH.

One embodiment of the compound represented by formula (7) includes the following (J).

A compound or a pharmaceutically acceptable salt thereof, wherein
Y^{1b} is a carbon atom,
Ring D is a 5- to 8-membered non-aromatic heterocycle,
L is
   a single bond, or
   C₁₋₆ alkylene (the alkylene is optionally substituted with 1 to 2 of the same or different substituents selected from the group consisting of a fluorine atom, or C₁₋₃ alkyl),
V is
   a single bond,
   C₂₋₆ alkenylene,
   C₃₋₁₀ cycloalkylene,
   C₃₋₁₀ cycloalkenylene, or
   a 3- to 10-membered divalent saturated heterocyclic group (the saturated heterocyclic group is optionally substituted with one substituent selected from the group consisting of a fluorine atom, and C₁₋₃ alkyl optionally substituted with 1 to 2 hydroxyl groups or fluorine atoms),
W is
   a single bond, or
   C₁₋₃ alkylene, and
Q is
   a hydrogen atom, or
   NH₂.

One embodiment of the compound represented by formula (7) includes the following (K).

A compound or a pharmaceutically acceptable salt thereof, wherein
Y^{1b} is a carbon atom, or a nitrogen atom,
Ring D is a 5- to 6-membered aromatic heterocycle,
L is
   a single bond, or
   C₁₋₆ alkylene (the alkylene is optionally substituted with 1 to 2 of the same or different substituents selected from the group consisting of a fluorine atom, or C₁₋₃ alkyl),
V is
   a single bond,
   C₂₋₆ alkenylene,
   C₃₋₁₀ cycloalkylene (the cycloalkylene is optionally substituted with 1 to 3 fluorine atoms),
   C₃₋₁₀ cycloalkenylene, or
   a 3- to 10-membered divalent saturated heterocyclic group (the saturated heterocyclic group is optionally substituted with one substituent selected from the group consisting of a fluorine atom, and C₁₋₃ alkyl optionally substituted with 1 to 2 hydroxyl groups or fluorine atoms),
W is
   a single bond, or
   C₁₋₃ alkylene, and
Q is
   a hydrogen atom, or
   NH₂.

One embodiment of the compound represented by formula (7) includes the following (K).

A compound or a pharmaceutically acceptable salt thereof, wherein
Y^{1b} is a carbon atom,
Ring D is a 5- to 6-membered aromatic heterocycle,
L is
   a single bond, or
   C₁₋₆ alkylene (the alkylene is optionally substituted with 1 to 2 of the same or different substituents selected from the group consisting of a fluorine atom, or C₁₋₃ alkyl),
V is
   a single bond,
   C₂₋₆ alkenylene,
   C₃₋₁₀ cycloalkylene (the cycloalkylene is optionally substituted with 1 to 3 fluorine atoms),
   C₃₋₁₀ cycloalkenylene, or
   a 3- to 10-membered divalent saturated heterocyclic group (the saturated heterocyclic group is optionally substituted with one substituent selected from the group consisting of a fluorine atom, and C₁₋₃ alkyl optionally substituted with 1 to 2 hydroxyl groups or fluorine atoms),
W is
   a single bond, or
   C₁₋₃ alkylene, and
Q is
   hydrogen atom, or
   NH₂.

One embodiment of the compound represented by formula (8) includes the following (L).

A compound or a pharmaceutically acceptable salt thereof, wherein
L is C₁₋₆ alkylene,
V is a single bond,
W is
   a single bond, or
   C₁₋₃ alkylene, and
Q is NH₂.

When the compound of the present disclosure is administered, the amount of the compound used varies depending on symptoms, age, administration method, and the like. For example, in the case of intravenous injection, an effect is expected by administering to an adult 0.01 mg as the lower limit (preferably 0.1 mg) and 1000 mg as the upper limit (preferably 100 mg) once or in several batches daily depending on the symptoms. Examples of its dosing schedule include single-dose administration, once a day administration for three days in a row, twice a day for one week in a row, and the like. Further, each administration described above can be repeated at intervals of about 1 days to about 60 days.

The compounds of the present disclosure may be administered parenterally or orally, preferably administered parenterally, more preferably administered by intravenous injection. Furthermore, the compound of the present disclosure is preferably formulated and administered as a pharmaceutically acceptable carrier such as a liposome.

The liposome encapsulating the compound of the present disclosure comprises at least one phospholipid. Examples of phospholipids include phosphatidylcholine, phosphatidylglycerol, phosphatidic acid, phosphatidylethanolamine, phosphatidylserine, phosphatidylinositol, sphingomyelin, and the like.

The fatty acid residue in the phospholipid is not particularly limited, and examples thereof include saturated or unsaturated fatty acid residues having 14 to 18 carbon atoms, specifically, acyl groups derived from fatty acids such as myristic acid, palmitic acid, stearic acid, oleic acid and linoleic acid. Phospholipids derived from natural products such as egg yolk lecithin and soybean lecithin, as well as hydrogenated egg yolk lecithin and hydrogenated soybean lecithin (also called hydrogenated soybean phospholipid or hydrogenated soybean phosphatidylcholine), in which the unsaturated fatty acid residues are hydrogenated, can also be used.

The amount (mole fraction) of phospholipids in the total liposome membrane components is not particularly limited, and is preferably 30-80%, more preferably 40-70%.

The liposome encapsulating the compound of the present disclosure can contain a sterol. Examples of sterols include cholesterol, β-sitosterol, stigmasterol, campesterol, brassicasterol, ergosterol, and fucosterol, and the like. Preferred sterol is cholesterol. The amount (molar fraction) of sterols in the total liposome membrane components is not particularly limited, and is preferably 0 to 60%, more preferably 10 to 50%, still more preferably 30 to 50%.

The liposome encapsulating the compound of the present disclosure can contain a polymer-modified lipid to improve in vivo retention. The amount (molar fraction) of polymer-modified lipid in the total liposome membrane components is not particularly limited, and is preferably 0 to 20%, more preferably 1 to 10%. A polymer moiety of the polymer-modified lipid is preferably a hydrophilic polymer, more preferably a hydrophilic polymer in which the terminal end of the polymer that is not bound to lipids is alkoxylated. The polymer moiety of the polymer-modified lipid is not particularly limited, and specifically includes polyethylene glycol, polypropylene glycol, polyvinyl alcohol, polyvinylpyrrolidone, methoxypolyethylene glycol, methoxypolypropylene glycol, methoxypolyvinyl alcohol, methoxypolyvinylpyrrolidone, ethoxypolyethylene glycol, ethoxypolypropylene glycol, ethoxypolyvinyl alcohol, ethoxypolyvinylpyrrolidone, propoxypolyethylene glycol, propoxypolypropylene glycol, propoxypolyvinyl alcohol, and propoxypolyvinylpyrrolidone. The polymer moiety of the polymer-modified lipid preferably includes polyethylene glycol, methoxypolyethylene glycol, methoxypolypropylene glycol, ethoxypolyethylene glycol, ethoxypolypropylene glycol, propoxypolyethylene glycol, and propoxypolypropylene glycol. The polymer moiety of the polymer-modified lipid more preferably includes polyethylene glycol, methoxypolyethylene glycol, ethoxypolyethylene glycol, ethoxypolypropylene glycol, and propoxypolyethylene glycol. The polymer moiety of the polymer-modified lipid still more preferably includes polyethylene glycol, and methoxypolyethylene glycol. The polymer moiety of the polymer-modified lipid most preferably includes methoxypolyethylene glycol. The molecular weight of the polymer moiety of the polymer-modified lipid is not particularly limited, and, for example, it is 100 to 10000 Daltons, preferably 1000 to 7000 Daltons, more preferably 1500 to 5000 Daltons, most preferably 1500 to 3000 Daltons.

A lipid moiety of the polymer-modified lipid is not particularly limited, and specifically includes phosphatidylethanolamines, and diacylglycerols. The lipid moiety of the polymer-modified lipid preferably includes phosphatidylethanolamines having a saturated or unsaturated fatty acid residue with 14 to 18 carbon atoms, and diacylglycerols having saturated or unsaturated fatty acid residue with 14 to 18 carbon atoms, more preferably includes phosphatidylethanolamines having a saturated fatty acid residue with 14 to 18 carbon atoms, and diacylglycerols having a saturated fatty acid residue with 14 to 18 carbon atoms, still more preferably includes phosphatidylethanolamines having a palmitoyl group or stearoyl group, and diacylglycerols having palmitoyl group or stearoyl group. The lipid moiety of the polymer-modified lipid most preferably includes distearoylphosphatidylethanolamine.

The liposome encapsulating the compound of the present disclosure can contain a pharmaceutically acceptable additive. Additives include inorganic acids, inorganic acid salts, organic acids, organic acid salts, saccharides, buffers, antioxidants, and polymers.

Examples of inorganic acids include phosphoric acid, hydrochloric acid, and sulfuric acid.

Examples of inorganic acid salts include sodium hydrogenphosphate, sodium chloride, ammonium sulfate, and magnesium sulfate.

Examples of organic acids include citric acid, acetic acid, succinic acid, and tartaric acid.

Examples of organic acid salts include sodium citrate, sodium acetate, disodium succinate, and sodium tartrate.

Examples of saccharides include glucose, sucrose, mannitol, sorbitol, and trehalose.

Examples of buffers include L-arginine, L-histidine, trometamol (trishydroxymethylaminomethane, Tris), and salts thereof.

Examples of antioxidants include sodium sulfite, L-cysteine, sodium thioglycolate, sodium thiosulfate, ascorbic acid, and tocopherol.

Examples of polymers include polyvinyl alcohol, polyvinylpyrrolidone, carboxyvinyl polymer, and sodium carboxymethyl cellulose.

The production method of the compound of the present disclosure represented by formula (1) will be described below with examples, but the production method of the compound of the present disclosure is not limited thereto. The compounds used in the following production method may form a salt, as long as they do not adversely affect the reaction.

The compound of the present disclosure can be produced from a known compound as a starting material by, for example, Production Method A, B, C, D, E, F, G, H, I, J, K, L, M, N, O, P, Q, R, or S described below, a method analogous thereto, or in appropriate combination with a synthetic method well known to those skilled in the art. The compound of the present disclosure other than formula (a2) can also be produced by a method analogous thereto or in appropriate combination with a synthetic method well known to those skilled in the art.

### Production Method A

The compound of the present disclosure represented by formula (a2) can be produced, for example, by the following method. 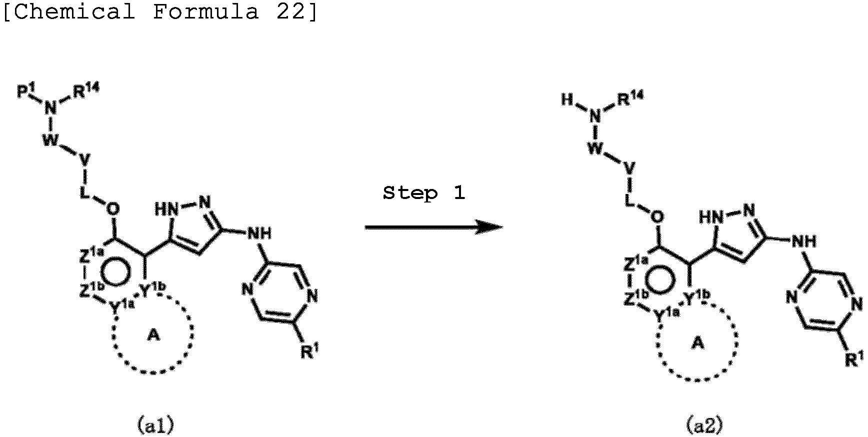 wherein R¹, R¹⁴, Y^{1a}, Y^{1b}, Z^{1a}, Z^{1b}, Ring A, L, V and W are as defined in item 1, and P¹ is an amino-protecting group. Examples of P¹ include amino-protecting groups described in Protective Groups in Organic Synthesis (Theodora W. Greene, Peter G. M. Wuts, published by John Wiley & Sons, Inc., 1999), and the like.

### [Step 1]

Compound (a2) can be produced by removing the protecting group P¹ of compound (a1) obtained by the production method described below. This step can be performed, for example, according to the method described in Protective Groups in Organic Synthesis (Theodora W. Greene, Peter G. M. Wuts, published by John Wiley & Sons, Inc., 1999), and the like.

### Production Method B

The compound of the present disclosure represented by formula (a1) can be produced, for example, by the following method. 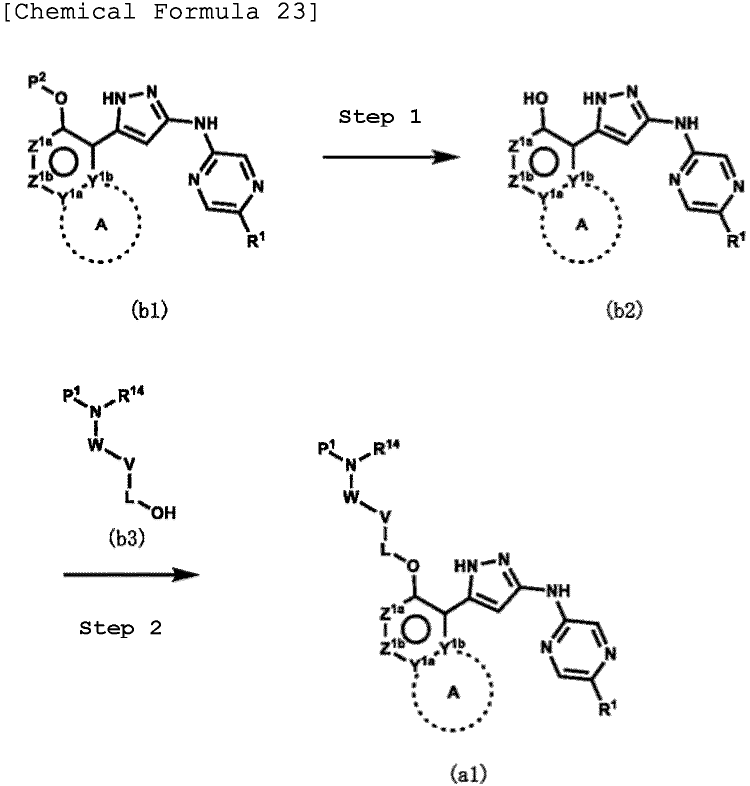 wherein R¹, R¹⁴, Y^{1a}, Y^{1b}, Z^{1a}, Z^{1b}, Ring A, L, V and W are as defined in item 1, P¹ is an amino-protecting group, and P² is a phenol-protecting group. Examples of P¹ include amino-protecting groups described in Protective Groups in Organic Synthesis (Theodora W. Greene, Peter G. M. Wuts, published by John Wiley & Sons, Inc., 1999), and the like. Examples of P² include phenol-protecting groups described in Protective Groups in Organic Synthesis (Theodora W. Greene, Peter G. M. Wuts, published by John Wiley & Sons, Inc., 1999), and the like.

Compound (b3) may be a commercially available product.

### [Step 1]

Compound (b2) can be produced by removing the protecting group P² of compound (b1) obtained by the production method described below. This step can be performed, for example, according to the method described in Protective Groups in Organic Synthesis (Theodora W. Greene, Peter G. M. Wuts, published by John Wiley & Sons, Inc., 1999), and the like.

### [Step 2]

Compound (a1) can be produced by subjecting compound (b2) to Mitsunobu reaction with compound (b3) in the presence of Mitsunobu reagent, in a suitable solvent.

Examples of the Mitsunobu reagent include a combination of diethyl azodicarboxylate (DEAD), diisopropyl azodicarboxylate (DIAD), N,N,N',N'-tetraisopropylazodicarboxamide (TIPA), 1,1'-(azodicarbonyl)dipiperidine (ADDP) or N,N,N',N'-tetramethylazodicarboxamide (TMAD) and triphenylphosphine or tributylphosphine, and the like. Cyanomethylenetrimethylphosphorane (CMMP) or cyanomethylenetributylphosphorane (CMBP) can be used.

The solvent is not particularly limited as long as it does not react under the reaction condition of this step. Examples thereof include ether solvents such as diethyl ether, diisopropyl ether, tetrahydrofuran, methyl cyclopentyl ether, anisole, 1,4-dioxane and the like; aromatic hydrocarbon solvents such as benzene, toluene, chlorobenzene, xylene and the like; ester solvents such as ethyl acetate, methyl acetate and the like; aprotic solvents such as N,N-dimethylformamide, N,N-dimethylacetamide, N-methyl-2-pyrrolidinone, 1,3-dimethyl-2-imidazolidinone, dimethyl sulfoxide and the like; or mixed solvents thereof, and the like. Preferred examples of the solvent include toluene, benzene, THF, 1,4-dioxane and mixed solvents thereof, and the like.

The reaction time is usually 5 min to 72 hr, preferably 12 hr to 24 hr.

The reaction temperature is usually 0°C to 100°C, preferably 0°C to 50°C.

### Production Method C

The compound of the present disclosure represented by formula (b1) can be produced, for example, by the following method. 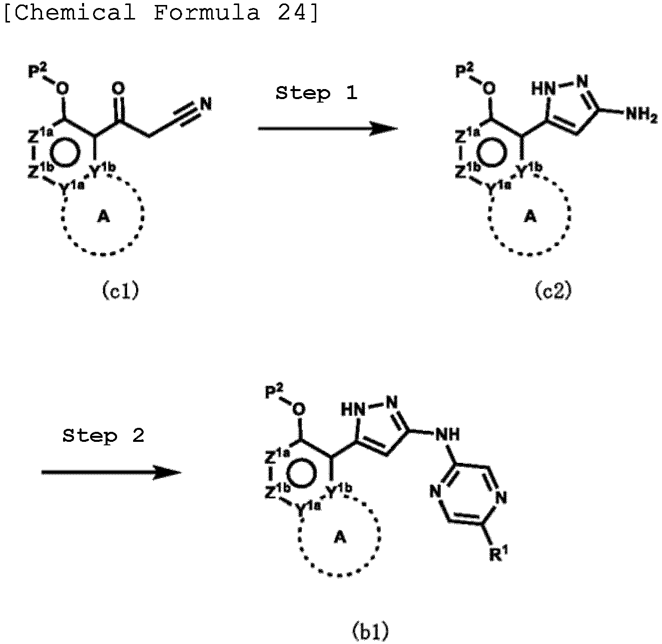 wherein R¹, Y^{1a}, Y^{1b}, Z^{1a}, Z^{1b} and Ring A are as defined in item 1, and P² is a phenol-protecting group. Examples of P² include phenol-protecting groups described in Protective Groups in Organic Synthesis (Theodora W. Greene, Peter G. M. Wuts, published by John Wiley & Sons, Inc., 1999), and the like.

### [Step 1]

Compound (c2) can be produced by reacting compound (c1) with hydrazine monohydrate in the presence or absence of a suitable acid, in a suitable solvent.

Examples of the acid include acetic acid, propionic acid, p-toluenesulfonic acid, methanesulfonic acid, ethanesulfonic acid, hydrochloric acid, sulfuric acid, camphorsulfonic acid and the like. Preferred examples of the acid include acetic acid, p-toluenesulfonic acid and the like.

The solvent is not particularly limited as long as it does not react under the reaction condition of this step. Examples thereof include ether solvents such as diethyl ether, diisopropyl ether, tetrahydrofuran, methyl cyclopentyl ether, anisole, 1,4-dioxane and the like; aromatic hydrocarbon solvents such as benzene, toluene, chlorobenzene, xylene and the like; ester solvents such as ethyl acetate, methyl acetate and the like; aprotic solvents such as N,N-dimethylformamide, N,N-dimethylacetamide, N-methyl-2-pyrrolidinone, 1,3-dimethyl-2-imidazolidinone, dimethyl sulfoxide and the like; alcohol solvents such as methanol, ethanol, isopropyl alcohol, butanol and the like; or mixtures thereof. Preferred examples of the solvent include 1,4-dioxane, toluene, ethanol and the like.

The reaction time is usually 5 min to 72 hr, preferably 12 hr to 24 hr.

The reaction temperature is usually 0°C to 200°C, preferably 50°C to 100°C.

### [Step 2]

Compound (b1) can be produced by reacting compound (c2) with a 5-chloropyrazine derivative in the presence of a suitable base, in a suitable solvent.

Examples of the base include organic bases such as triethylamine, diisopropylethylamine, tributylamine, 1,5-diazabicyclo[4.3.0]non-5-ene (DBN), 1,8-diazabicyclo[5.4.0]undec-7-ene (DBU), pyridine, dimethylaminopyridine, picoline, N-methylmorpholine (NMM), N-ethylmorpholine and the like, or inorganic bases such as sodium hydrogencarbonate, potassium hydrogencarbonate, sodium carbonate, potassium carbonate, sodium hydroxide, potassium hydroxide and the like. Preferred examples of the base include triethylamine, diisopropylethylamine, N-ethylmorpholine and the like.

The solvent is not particularly limited as long as it does not react under the reaction condition of this step. Examples thereof include ether solvents such as diethyl ether, diisopropyl ether, tetrahydrofuran, methyl cyclopentyl ether, anisole, 1,4-dioxane and the like; aromatic hydrocarbon solvents such as benzene, toluene, chlorobenzene, xylene and the like; ester solvents such as ethyl acetate, methyl acetate and the like; aprotic solvents such as N,N-dimethylformamide, N,N-dimethylacetamide, N-methyl-2-pyrrolidinone, 1,3-dimethyl-2-imidazolidinone, dimethyl sulfoxide and the like; or mixtures thereof. Preferred examples of the solvent include tetrahydrofuran, toluene, dimethyl sulfoxide and the like.

The reaction time is usually 5 min to 48 hr, preferably 1 hr to 6 hr.

The reaction temperature is usually 0°C to 200°C, preferably 50°C to 100°C.

### Production Method D

The compound of the present disclosure represented by formula (c1) can be produced, for example, by the following method. 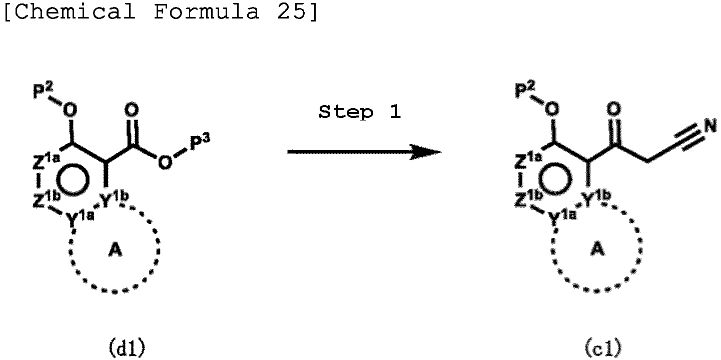 wherein Y^{1a}, Y^{1b}, Z^{1a}, Z^{1b} and Ring A are as defined in item 1, P² is a phenol-protecting group, and P³ is a carboxyl-protecting group. Examples of P² include phenol-protecting groups described in Protective Groups in Organic Synthesis (Theodora W. Greene, Peter G. M. Wuts, published by John Wiley & Sons, Inc., 1999), and the like. Examples of P³ include carboxyl-protecting groups described in Protective Groups in Organic Synthesis (Theodora W. Greene, Peter G. M. Wuts, published by John Wiley & Sons, Inc., 1999), and the like.

### [Step 1]

Compound (c1) can be produced by reacting compound (d1) with acetonitrile in the presence of a suitable base, in a suitable solvent.

Examples of the base include alkali metal hydrides such as sodium hydride and the like, alkali metal halides such as potassium fluoride and the like, alkali metal hydroxides such as sodium hydroxide, potassium hydroxide and the like, alkali carbonates such as sodium carbonate, potassium carbonate, cesium carbonate, sodium hydrogencarbonate and the like, alkali metal alkoxides such as sodium ethoxide, sodium tert-butoxide, potassium tert-butoxide and the like, alkali metal alkoxides such as lithiumdiisopropylamide, n-butyllithium, methyllithium, isopropylmagnesium bromide and the like. Preferred examples of the base include sodium ethoxide, sodium tert-butoxide, potassium tert-butoxide, n-butyllithium and the like.

The solvent is not particularly limited as long as it does not react under the reaction condition of this step. Examples thereof include ether solvents such as diethyl ether, diisopropyl ether, tetrahydrofuran, methyl cyclopentyl ether, anisole, 1,4-dioxane and the like; hydrocarbon solvents such as benzene, toluene, chlorobenzene, xylene and the like aromatic; ester solvents such as ethyl acetate, methyl acetate and the like; aprotic solvents such as N,N-dimethylformamide, N,N-dimethylacetamide, N-methyl-2-pyrrolidinone, 1,3-dimethyl-2-imidazolidinone, dimethyl sulfoxide and the like; alcohol solvents such as methanol, ethanol, isopropyl alcohol, butanol and the like; or mixtures thereof. Preferred examples of the solvent include tetrahydrofuran, toluene and the like.

The reaction time is usually 5 min to 72 hr, preferably 30 min to 6 hr.

The reaction temperature is usually -100°C to 100°C, preferably -100°C to 30°C.

### Production Method E

The compound of the present disclosure represented by formula (b1) can be produced, for example, by the following method. 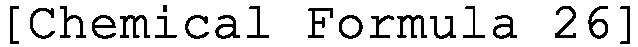 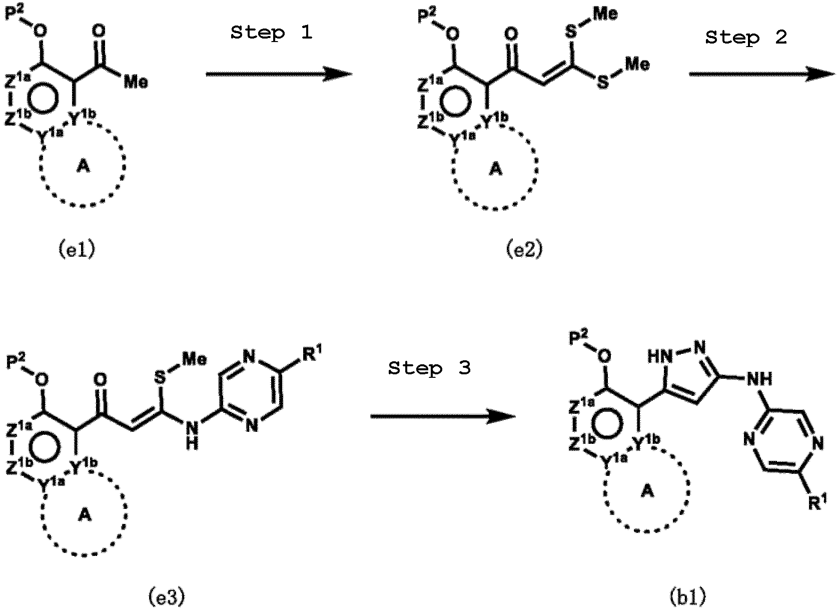 wherein R¹, Y^{1a}, Y^{1b}, Z^{1a}, Z^{1b} and Ring A are as defined in item 1, and P² is a phenol-protecting group. Examples of P² include phenol-protecting groups described in Protective Groups in Organic Synthesis (Theodora W. Greene, Peter G. M. Wuts, published by John Wiley & Sons, Inc., 1999), and the like.

### [Step 1]

Compound (e2) can be produced by reacting compound (e1) with carbon disulfide and iodomethane in the presence of a suitable base, in a suitable solvent.

Examples of the base include alkali metal hydrides such as sodium hydride and the like, alkali metal halides such as potassium fluoride and the like, alkali metal hydroxides such as sodium hydroxide, potassium hydroxide and the like, alkali carbonates such as sodium carbonate, potassium carbonate, cesium carbonate, sodium hydrogencarbonate and the like, alkali metal alkoxides such as sodium ethoxide, sodium tert-butoxide, potassium tert-butoxide and the like, alkali metal alkoxides such as lithiumdiisopropylamide, n-butyllithium, methyllithium, isopropylmagnesium bromide and the like. Preferred examples of the base include sodium hydride, potassium tert-butoxide and the like.

The solvent is not particularly limited as long as it does not react under the reaction condition of this step. Examples thereof include ether solvents such as diethyl ether, diisopropyl ether, tetrahydrofuran, methyl cyclopentyl ether, anisole, 1,4-dioxane and the like; aromatic hydrocarbon solvents such as benzene, toluene, chlorobenzene, xylene and the like; ester solvents such as ethyl acetate, methyl acetate and the like; aprotic solvents such as N,N-dimethylformamide, N,N-dimethylacetamide, N-methyl-2-pyrrolidinone, 1,3-dimethyl-2-imidazolidinone, dimethyl sulfoxide and the like; alcohol solvents such as methanol, ethanol, isopropyl alcohol, butanol and the like; or mixtures thereof. Preferred examples of the solvent include tetrahydrofuran, toluene, N,N-dimethylformamide and the like.

The reaction time is usually 5 min to 72 hr, preferably 30 min to 2 hr.

The reaction temperature is usually -78°C to 200°C, preferably 0°C to 25°C.

### [Step 2]

Compound (e3) can be produced by reacting compound (e2) with a 5-aminopyrazine derivative in the presence of a suitable base, in a suitable solvent.

Examples of the base include organic bases such as triethylamine, diisopropylethylamine, tributylamine, 1,5-diazabicyclo[4.3.0]non-5-ene (DBN), 1,8-diazabicyclo[5.4.0]undec-7-ene (DBU), pyridine, dimethylaminopyridine, picoline, N-methylmorpholine (NMM), N-ethylmorpholine and the like, or inorganic bases such as sodium hydrogencarbonate, potassium hydrogencarbonate, sodium carbonate, potassium carbonate, sodium hydroxide, potassium hydroxide and the like. Preferred examples of the base include triethylamine, diisopropylethylamine and the like.

The solvent is not particularly limited as long as it does not react under the reaction condition of this step. Examples thereof include ether solvents such as diethyl ether, diisopropyl ether, tetrahydrofuran, methyl cyclopentyl ether, anisole, 1,4-dioxane and the like; aromatic hydrocarbon solvents such as benzene, toluene, chlorobenzene, xylene and the like; ester solvents such as ethyl acetate, methyl acetate and the like; aprotic solvents such as N,N-dimethylformamide, N,N-dimethylacetamide, N-methyl-2-pyrrolidinone, 1,3-dimethyl-2-imidazolidinone, dimethyl sulfoxide and the like; or mixtures thereof. Preferred examples of the solvent include tetrahydrofuran, toluene, dimethyl sulfoxide and the like.

The reaction time is usually 5 min to 48 hr, preferably 2 hr to 8 hr.

The reaction temperature is usually 0°C to 200°C, preferably 50°C to 100°C.

### [Step 3]

Compound (b1) can be produced by reacting compound (e3) with hydrazine monohydrate in the presence or absence of a suitable acid, in a suitable solvent.

Examples of the acid include acetic acid, propionic acid, p-toluenesulfonic acid, methanesulfonic acid, ethanesulfonic acid, hydrochloric acid, sulfuric acid, camphorsulfonic acid and the like. Preferred examples of the acid include acetic acid.

The solvent is not particularly limited as long as it does not react under the reaction condition of this step. Examples thereof include ether solvents such as diethyl ether, diisopropyl ether, tetrahydrofuran, methyl cyclopentyl ether, anisole, 1,4-dioxane and the like; aromatic hydrocarbon solvents such as benzene, toluene, chlorobenzene, xylene and the like; ester solvents such as ethyl acetate, methyl acetate and the like; aprotic solvents such as N,N-dimethylformamide, N,N-dimethylacetamide, N-methyl-2-pyrrolidinone, 1,3-dimethyl-2-imidazolidinone, dimethyl sulfoxide and the like; alcohol solvents such as methanol, ethanol, isopropyl alcohol, butanol and the like; or mixtures thereof. Preferred examples of the solvent include 1,4-dioxane, ethanol and the like.

The reaction time is usually 5 min to 72 hr, preferably 12 hr to 24 hr.

The reaction temperature is usually 0°C to 200°C, preferably 50°C to 100°C.

### Production Method F

The compound of the present disclosure represented by formula (c1) can be produced, for example, by the following method. 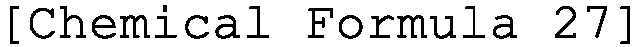 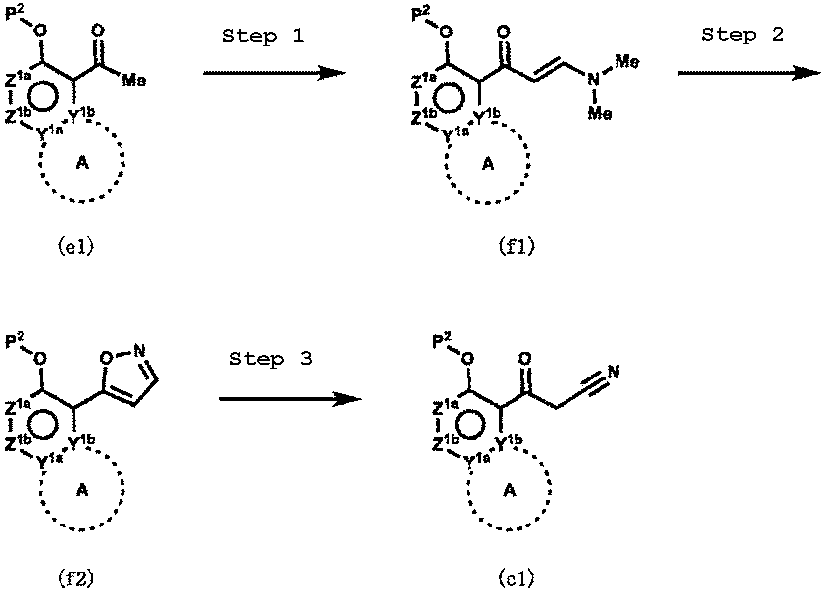 wherein Y^{1a}, Y^{1b}, Z^{1a}, Z^{1b} and Ring A are as defined in item 1, and P² is a phenol-protecting group. Examples of P² include phenol-protecting groups described in Protective Groups in Organic Synthesis (Theodora W. Greene, Peter G. M. Wuts, published by John Wiley & Sons, Inc., 1999), and the like.

### [Step 1]

Compound (f1) can be produced by reacting compound (e1) with dimethylformamide dimethyl acetal in a suitable solvent.

The solvent is not particularly limited as long as it does not react under the reaction condition of this step. Examples thereof include ether solvents such as diethyl ether, diisopropyl ether, tetrahydrofuran, methyl cyclopentyl ether, anisole, 1,4-dioxane and the like; aromatic hydrocarbon solvents such as benzene, toluene, chlorobenzene, xylene and the like; ester solvents such as ethyl acetate, methyl acetate and the like; aprotic solvents such as N,N-dimethylformamide, N,N-dimethylacetamide, N-methyl-2-pyrrolidinone, 1,3-dimethyl-2-imidazolidinone, dimethyl sulfoxide and the like; or mixtures thereof. Preferred examples of the solvent include N,N-dimethylformamide and the like.

The reaction time is usually 5 min to 48 hr, preferably 24 hr to 48 hr.

The reaction temperature is usually 0°C to 200°C, preferably 60°C to 120°C.

### [Step 2]

Compound (f2) can be produced by reacting compound (f1) with hydroxylamine hydrochloride in a suitable solvent.

The solvent is not particularly limited as long as it does not react under the reaction condition of this step. Examples thereof include ether solvents such as diethyl ether, diisopropyl ether, tetrahydrofuran, methyl cyclopentyl ether, anisole, 1,4-dioxane and the like; aromatic hydrocarbon solvents such as benzene, toluene, chlorobenzene, xylene and the like; ester solvents such as ethyl acetate, methyl acetate and the like; aprotic solvents such as N,N-dimethylformamide, N,N-dimethylacetamide, N-methyl-2-pyrrolidinone, 1,3-dimethyl-2-imidazolidinone, dimethyl sulfoxide and the like; alcohol solvents such as methanol, ethanol, isopropyl alcohol, butanol and the like; or mixtures thereof. Preferred examples of the solvent include ethanol, isopropyl alcohol and the like.

The reaction time is usually 5 min to 48 hr, preferably 6 hr to 12 hr.

The reaction temperature is usually 0°C to 200°C, preferably 40°C to 80°C.

### [Step 3]

Compound (c1) can be produced by reacting compound (f2) with a base in a suitable solvent.

Examples of the base include inorganic bases such as sodium hydrogencarbonate, potassium hydrogencarbonate, sodium carbonate, potassium carbonate, sodium hydroxide, potassium hydroxide and the like. Preferred examples of the base include sodium hydroxide, potassium hydroxide and the like.

The solvent is not particularly limited as long as it does not react under the reaction condition of this step. Examples thereof include ether solvents such as diethyl ether, diisopropyl ether, tetrahydrofuran, methyl cyclopentyl ether, anisole, 1,4-dioxane and the like; aromatic hydrocarbon solvents such as benzene, toluene, chlorobenzene, xylene and the like; ester solvents such as ethyl acetate, methyl acetate and the like; aprotic solvents such as N,N-dimethylformamide, N,N-dimethylacetamide, N-methyl-2-pyrrolidinone, 1,3-dimethyl-2-imidazolidinone, dimethyl sulfoxide and the like; protic solvents such as water, methanol, ethanol, isopropyl alcohol, butanol and the like; or mixtures thereof. Preferred examples of the solvent include a mixed solvent of water and ethanol, and the like.

The reaction time is usually 5 min to 48 hr, preferably 1 hr to 6 hr.

The reaction temperature is usually 0°C to 200°C, preferably 20°C to 50°C.

### Production Method G

The compound of the present disclosure represented by formula (a1) can be produced, for example, by the following method. 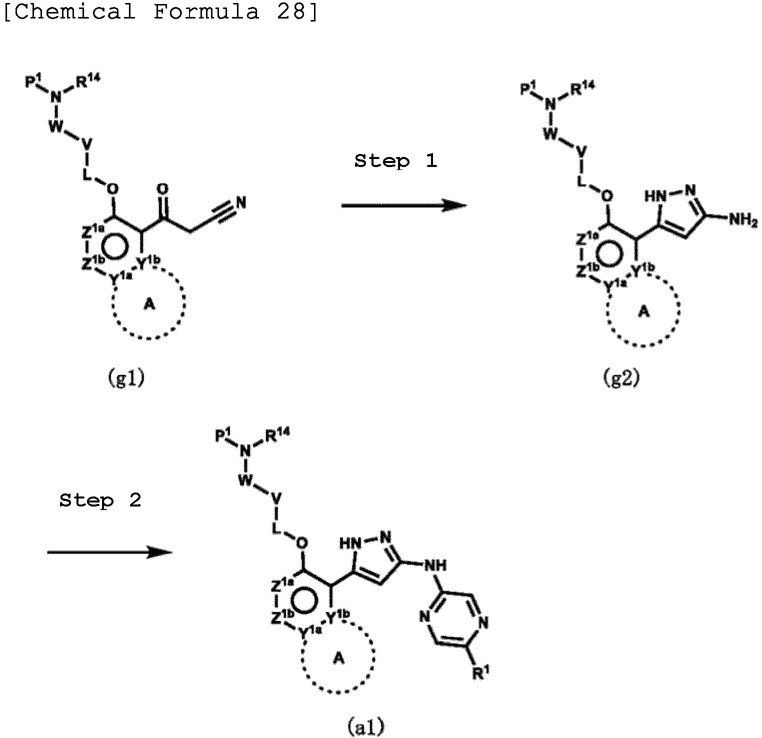 wherein R¹, R¹⁴, Y^{1a}, Y^{1b}, Z^{1a}, Z^{1b}, Ring A, L, V and W are as defined in item 1, and P¹ is an amino-protecting group. Examples of P¹ include amino-protecting groups described in Protective Groups in Organic Synthesis (Theodora W. Greene, Peter G. M. Wuts, published by John Wiley & Sons, Inc., 1999), and the like.

### [Step 1]

Compound (g2) can be produced from compound (g1) obtained by the production method described below, by the method described in Step 1 of Production Method C or a method analogous thereto.

### [Step 2]

Compound (a1) can be produced from compound (g2), by the method described in Step 2 of Production Method C or a method analogous thereto.

### Production Method H

The compound represented by formula (a1) can be produced, for example, by the following method. 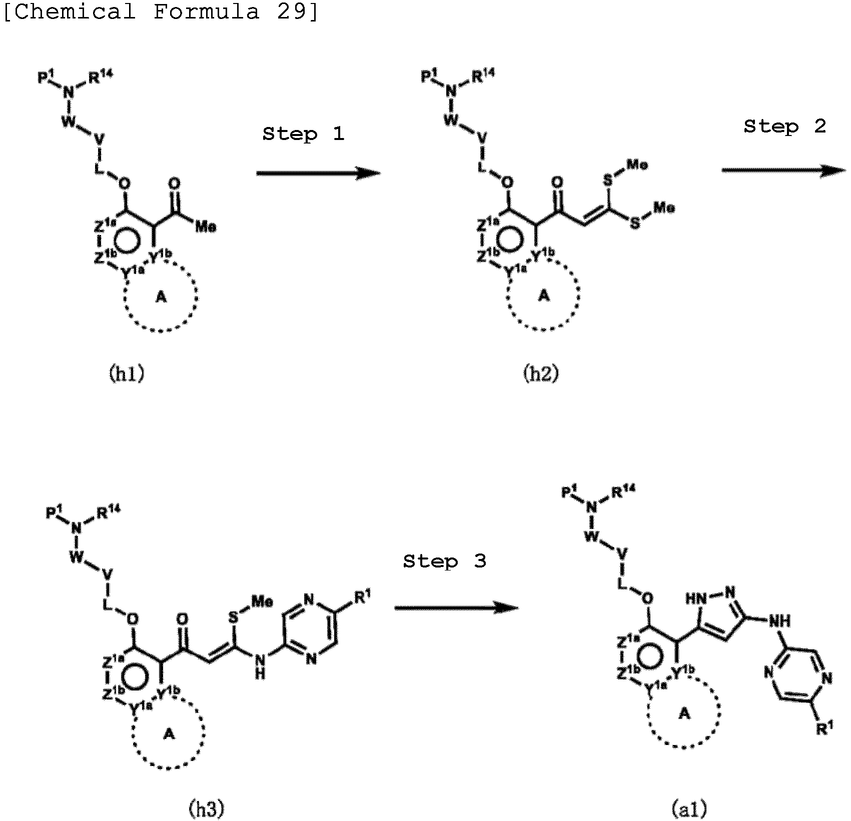 wherein R¹, R¹⁴, Y^{1a}, Y^{1b}, Z^{1a}, Z^{1b}, Ring A, L, V and W are as defined in item 1, and P¹ is an amino-protecting group. Examples of P¹ include amino-protecting groups described in Protective Groups in Organic Synthesis (Theodora W. Greene, Peter G. M. Wuts, published by John Wiley & Sons, Inc., 1999), and the like.

### [Step 1]

Compound (h2) can be produced from compound (h1) obtained by the production method described below, by the method described in Step 1 of Production Method E or a method analogous thereto.

### [Step 2]

Compound (h3) can be produced from compound (h2), by the method described in Step 2 of Production Method E or a method analogous thereto.

### [Step 3]

Compound (a1) can be produced from compound (h3), by the method described in Step 3 of Production Method E or a method analogous thereto.

### Production Method I

The compound represented by formula (g1) can be produced, for example, by the following method. 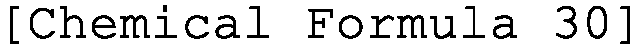 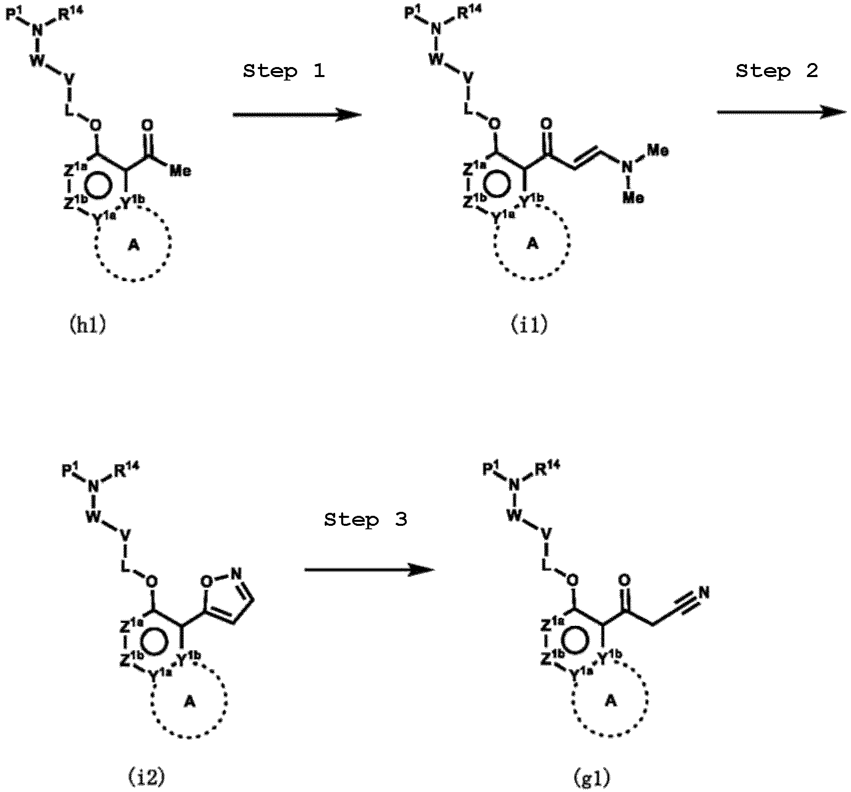 wherein R¹⁴, Y^{1a}, Y^{1b}, Z^{1a}, Z^{1b}, Ring A, L, V and W are as defined in item 1, and P¹ is an amino-protecting group. Examples of P¹ include amino-protecting groups described in Protective Groups in Organic Synthesis (Theodora W. Greene, Peter G. M. Wuts, published by John Wiley & Sons, Inc., 1999), and the like.

### [Step 1]

Compound (i1) can be produced from compound (h1) obtained by the production method described below, by the method described in Step 1 of Production Method F or a method analogous thereto.

### [Step 2]

Compound (i2) can be produced from compound (i1), by the method described in Step 2 of Production Method F or a method analogous thereto.

### [Step 3]

Compound (g1) can be produced from compound (i2), by the method described in Step 3 of Production Method F or a method analogous thereto.

### Production Method J

The compound represented by formula (g1) can also be produced, for example, by the following method. 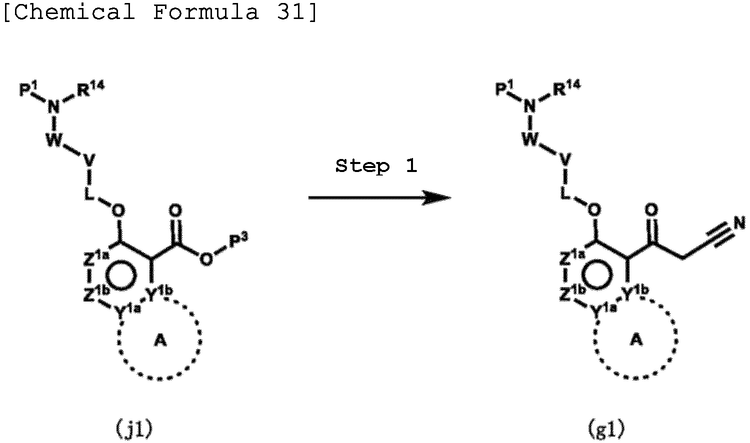 wherein R¹⁴, Y^{1a}, Y^{1b}, Z^{1a}, Z^{1b}, Ring A, L, V and W are as defined in item 1, P¹ is an amino-protecting group, and P³ is a carboxyl-protecting group. Examples of P¹ include amino-protecting groups described in Protective Groups in Organic Synthesis (Theodora W. Greene, Peter G. M. Wuts, published by John Wiley & Sons, Inc., 1999), and the like. Examples of P³ include amino-protecting groups described in Protective Groups in Organic Synthesis (Theodora W. Greene, Peter G. M. Wuts, published by John Wiley & Sons, Inc., 1999), and the like.

### [Step 1]

Compound (g1) can be produced from compound (j1) obtained by the production method described below, by the method described in Step 1 of Production Method D or a method analogous thereto.

### Production Method K

The compound of the present disclosure represented by formula (d1) can be produced, for example, by the following method. 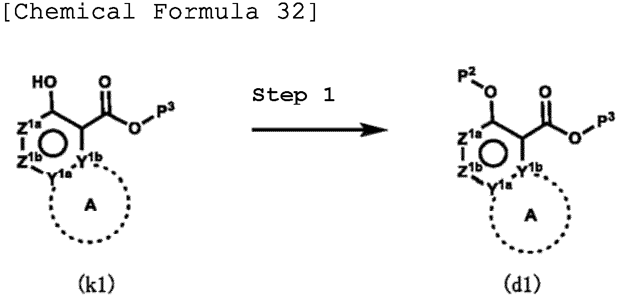 wherein Y^{1a}, Y^{1b}, Z^{1a}, Z^{1b} and Ring A are as defined in item 1, P² is a phenol-protecting group, and P³ is a carboxyl-protecting group. Examples of P² include phenol-protecting groups described in Protective Groups in Organic Synthesis (Theodora W. Greene, Peter G. M. Wuts, published by John Wiley & Sons, Inc., 1999), and the like. Examples of P³ include carboxyl-protecting groups described in Protective Groups in Organic Synthesis (Theodora W. Greene, Peter G. M. Wuts, published by John Wiley & Sons, Inc., 1999), and the like.

Compound (k1) may be a commercially available product.

### [Step 1]

Compound (d1) can be produced, for example, from compound (k1) by the method described in Protective Groups in Organic Synthesis (Theodora W. Greene, Peter G. M. Wuts, published by John Wiley & Sons, Inc., 1999).

### Production Method L

The compound of the present disclosure represented by formula (e1) can be produced, for example, by the following method. 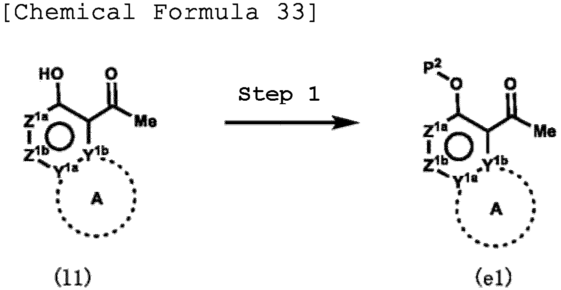 wherein Y^{1a}, Y^{1b}, Z^{1a}, Z^{1b} and Ring A are as defined in item 1, and P² is a phenol-protecting group. Examples of P² include phenol-protecting groups described in Protective Groups in Organic Synthesis (Theodora W. Greene, Peter G. M. Wuts, published by John Wiley & Sons, Inc., 1999), and the like.

Compound (11) may be a commercially available product.

### [Step 1]

Compound (e1) can be produced, for example, from compound (11) by the method described in Protective Groups in Organic Synthesis (Theodora W. Greene, Peter G. M. Wuts, published by John Wiley & Sons, Inc., 1999).

### Production Method M

The compound of the present disclosure represented by formula (j1) can be produced, for example, by the following method. 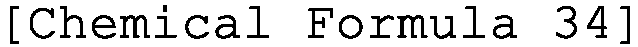 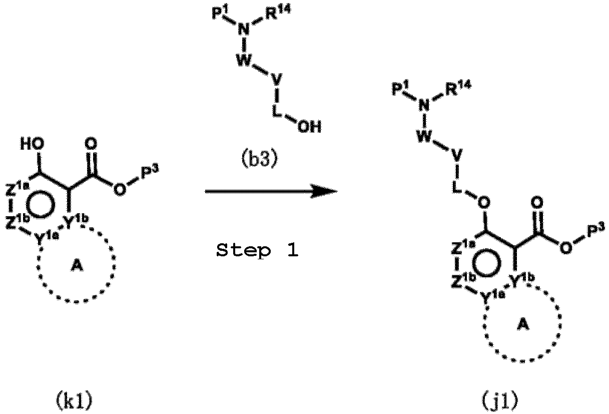 wherein R¹⁴, Y^{1a}, Y^{1b}, Z^{1a}, Z^{1b}, Ring A, L, V and W are as defined in item 1, P¹ is an amino-protecting group, and P³ is a carboxyl-protecting group. Examples of P¹ include amino-protecting groups described in Protective Groups in Organic Synthesis (Theodora W. Greene, Peter G. M. Wuts, published by John Wiley & Sons, Inc., 1999), and the like. Examples of P³ include amino-protecting groups described in Protective Groups in Organic Synthesis (Theodora W. Greene, Peter G. M. Wuts, published by John Wiley & Sons, Inc., 1999), and the like.

Compound (k1) and compound (b3) may be commercially available products.

### [Step 1]

Compound (j1) can be produced from compound (k1) and compound (b3), by the method described in Step 2 of Production Method B or a method analogous thereto.

### Production Method N

The compound of the present disclosure represented by formula (j1) can be produced, for example, by the following method. 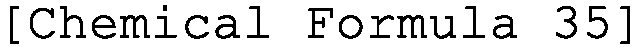 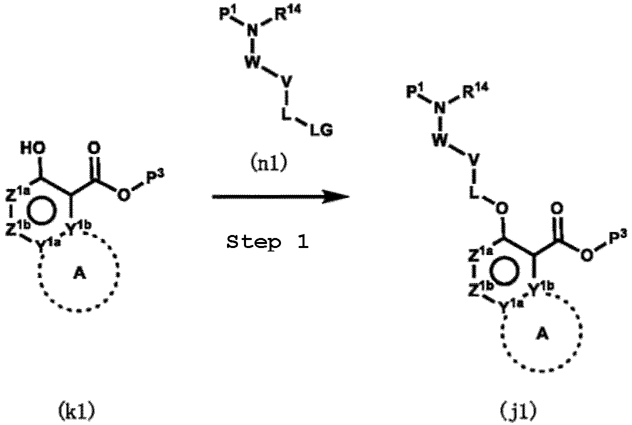 wherein R¹⁴, Y^{1a}, Y^{1b}, Z^{1a}, Z^{1b}, Ring A, L, V and W are as defined in item 1, P¹ is an amino-protecting group, P³ is a carboxyl-protecting group, and LG is a leaving group. Examples of P¹ include amino-protecting groups described in Protective Groups in Organic Synthesis (Theodora W. Greene, Peter G. M. Wuts, published by John Wiley & Sons, Inc., 1999), and the like. Examples of P³ include amino-protecting groups described in Protective Groups in Organic Synthesis (Theodora W. Greene, Peter G. M. Wuts, published by John Wiley & Sons, Inc., 1999), and the like. Examples of LG include halogen, methanesulfonyloxy, p-toluenesulfonic acidoxy, trifluoromethanesulfonic acidoxy and the like.

Compound (k1) and compound (n1) may be commercially available products.

### [Step 1]

Compound (j1) can be produced by reacting compound (k1) with compound (n1) in the presence or absence of a suitable base, in a suitable solvent.

Examples of the base include organic bases such as triethylamine, diisopropylethylamine, tributylamine, 1.5-diazabicyclo[4.3.0]non-5-ene (DBN), 1,8-diazabicyclo[5.4.0]undec-7-ene (DBU), pyridine, 4-dimethylaminopyridine, picoline, N-methylmorpholine (NMM) and the like, or inorganic bases such as sodium hydrogencarbonate, potassium hydrogencarbonate, sodium carbonate, potassium carbonate, sodium hydroxide, potassium hydroxide and the like. Preferred examples of the base include triethylamine, diisopropylethylamine, potassium carbonate, sodium hydroxide and the like.

The solvent is not particularly limited as long as it does not react under the reaction condition of this step. Examples thereof include alcohol solvents such as methanol, ethanol, 2-propanol (isopropyl alcohol), tert-butanol and the like; ether solvents such as diethyl ether, diisopropyl ether, tetrahydrofuran, methyl cyclopentyl ether, 1,4-dioxane and the like; aromatic hydrocarbon solvents such as benzene, toluene, chlorobenzene, anisole, xylene and the like; ester solvents such as ethyl acetate, methyl acetate and the like; aprotic solvents such as acetonitrile, N,N-dimethylformamide, N,N-dimethylacetamide, N-methyl-2-pyrrolidinone, 1,3-dimethyl-2-imidazolidinone, dimethyl sulfoxide and the like; or mixtures thereof. Preferred examples of the solvent include 2-propanol, tetrahydrofuran, toluene, acetonitrile, N,N-dimethylformamide and the like.

The reaction temperature is usually -80°C to under heating with reflux, preferably 25°C to 90°C.

The reaction time is usually 30 min to 48 hr, preferably 6 to 12 hr.

### production method O

The compound of the present disclosure represented by formula (h1) can be produced, for example, by the following method. 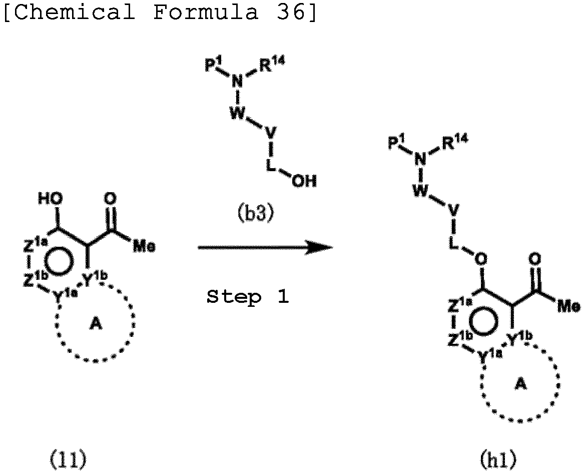 wherein R¹⁴, Y^{1a}, Y^{1b}, Z^{1a}, Z^{1b}, Ring A, L, V and W are as defined in item 1, and P¹ is an amino-protecting group. Examples of P¹ include amino-protecting groups described in Protective Groups in Organic Synthesis (Theodora W. Greene, Peter G. M. Wuts, published by John Wiley & Sons, Inc., 1999), and the like.

Compound (11) and compound (b3) may be commercially available products.

### [Step 1]

Compound (h1) can be produced from compound (11) and compound (b3), by the method described in Step 2 of Production Method B or a method analogous thereto.

### Production Method P

The compound of the present disclosure represented by formula (h1) can be produced, for example, by the following method. 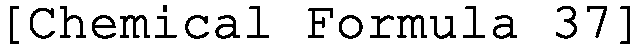 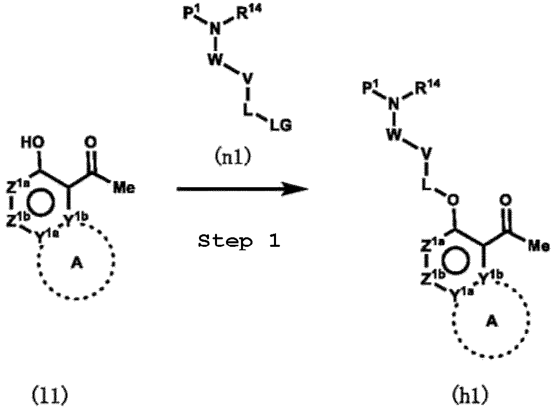 wherein R¹⁴, Y^{1a}, Y^{1b}, Z^{1a}, Z^{1b}, Ring A, L, V and W are as defined in item 1, P¹ is an amino-protecting group, and LG is a leaving group. Examples of P¹ include amino-protecting groups described in Protective Groups in Organic Synthesis (Theodora W. Greene, Peter G. M. Wuts, published by John Wiley & Sons, Inc., 1999), and the like. Examples of LG include halogen, methanesulfonyloxy, p-toluenesulfonic acidoxy, trifluoromethanesulfonic acidoxy and the like.

Compound (11) and compound (n1) may be commercially available products.

### [Step 1]

Compound (h1) can be produced from compound (11) and compound (n1), by the method described in Step 2 of Production Method N or a method analogous thereto.

### Production Method Q

The compound of the present disclosure represented by formula (d1) can be produced, for example, by the following method. 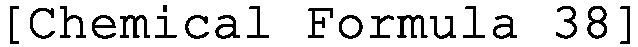 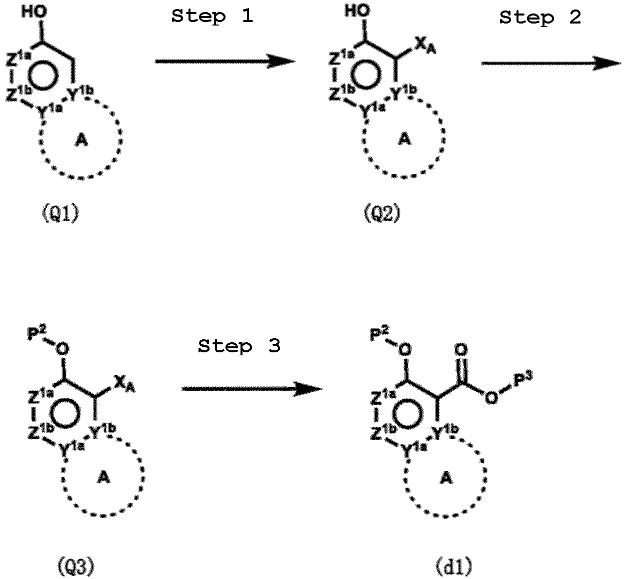 wherein Y^{1a}, Y^{1b}, Z^{1a}, Z^{1b} and Ring A are as defined in item 1, X_{A} is a halogen atom, P² is a phenol-protecting group, and P³ is a carboxyl-protecting group. Examples of P² include phenol-protecting groups described in Protective Groups in Organic Synthesis (Theodora W. Greene, Peter G. M. Wuts, published by John Wiley & Sons, Inc., 1999), and the like. Examples of P³ include carboxyl-protecting groups described in Protective Groups in Organic Synthesis (Theodora W. Greene, Peter G. M. Wuts, published by John Wiley & Sons, Inc., 1999), and the like.

Compound (q1) may be a commercially available product.

### [Step 1]

Compound (q2) can be produced by reacting compound (q1) with an N-halosuccinimide in the presence of a suitable acid, in a suitable solvent.

Examples of the acid include acetic acid, propionic acid, p-toluenesulfonic acid, methanesulfonic acid, ethanesulfonic acid, hydrochloric acid, sulfuric acid, camphorsulfonic acid and the like. Preferred examples of the acid include p-toluenesulfonic acid.

The solvent is not particularly limited as long as it does not react under the reaction condition of this step. Examples thereof include ether solvents such as diethyl ether, diisopropyl ether, tetrahydrofuran, methyl cyclopentyl ether, anisole, 1,4-dioxane and the like; aromatic hydrocarbon solvents such as benzene, toluene, chlorobenzene, xylene and the like; ester solvents such as ethyl acetate, methyl acetate and the like; aprotic solvents such as N,N-dimethylformamide, N,N-dimethylacetamide, N-methyl-2-pyrrolidinone, 1,3-dimethyl-2-imidazolidinone, dimethyl sulfoxide and the like; or mixtures thereof. Preferred examples of the solvent include acetonitrile.

The reaction time is usually 5 min to 48 hr, preferably 1 hr to 6 hr.

The reaction temperature is usually 0°C to 200°C, preferably 50°C to 100°C.

### [Step 2]

Compound (q3) can be produced, for example, from compound (q2) by the method described in Protective Groups in Organic Synthesis (Theodora W. Greene, Peter G. M. Wuts, published by John Wiley & Sons, Inc., 1999).

### [Step 3]

Compound (d1) can be produced by reacting compound (q3) with ethyl chloroformate in the presence of a suitable base, in a suitable solvent.

Examples of the base include alkali metal hydrides such as sodium hydride and the like, alkali metal halides such as potassium fluoride and the like, alkali metal hydroxides such as sodium hydroxide, potassium hydroxide and the like, alkali carbonates such as sodium carbonate, potassium carbonate, cesium carbonate, sodium hydrogencarbonate and the like, alkali metal alkoxides such as sodium ethoxide, sodium tert-butoxide, potassium tert-butoxide and the like, alkali metal alkoxides such as lithiumdiisopropylamide, n-butyllithium, methyllithium, isopropylmagnesium bromide and the like. Preferred examples of the base include n-butyllithium.

The solvent is not particularly limited as long as it does not react under the reaction condition of this step. Examples thereof include ether solvents such as diethyl ether, diisopropyl ether, tetrahydrofuran, methyl cyclopentyl ether, anisole, 1,4-dioxane and the like; aromatic hydrocarbon solvents such as benzene, toluene, chlorobenzene, xylene and the like; ester solvents such as ethyl acetate, methyl acetate and the like; aprotic solvents such as N,N-dimethylformamide, N,N-dimethylacetamide, N-methyl-2-pyrrolidinone, 1,3-dimethyl-2-imidazolidinone, dimethyl sulfoxide and the like; alcohol solvents such as methanol, ethanol, isopropyl alcohol, butanol and the like; or mixtures thereof. Preferred examples of the solvent include tetrahydrofuran, toluene and the like.

The reaction time is usually 5 min to 72 hr, preferably 30 min to 6 hr.

The reaction temperature is usually -100°C to 100°C, preferably -100°C to -50°C.

### Production Method R

The compound of the present disclosure represented by formula (d1) can be produced, for example, by the following method. 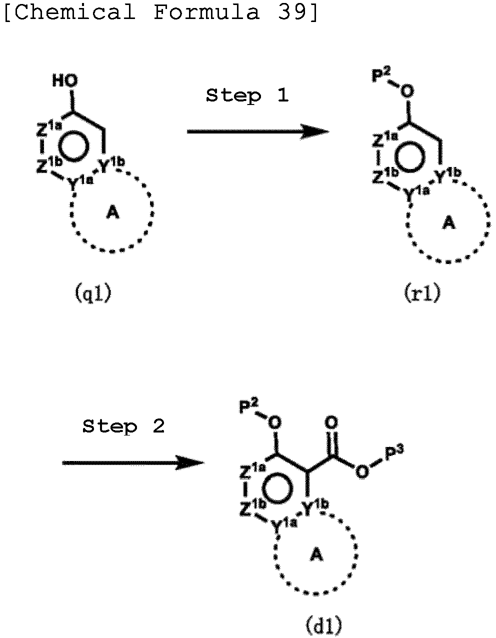 wherein Y^{1a}, Y^{1b}, Z^{1a}, Z^{1b} and Ring A are as defined in item 1, P² is a phenol-protecting group, and P³ is a carboxyl-protecting group. Examples of P² include phenol-protecting groups described in Protective Groups in Organic Synthesis (Theodora W. Greene, Peter G. M. Wuts, published by John Wiley & Sons, Inc., 1999), and the like. Examples of P³ include carboxyl-protecting groups described in Protective Groups in Organic Synthesis (Theodora W. Greene, Peter G. M. Wuts, published by John Wiley & Sons, Inc., 1999), and the like.

Compound (q1) may be a commercially available product.

### [Step 1]

Compound (r1) can be produced, for example, from compound (q1) by the method described in Protective Groups in Organic Synthesis (Theodora W. Greene, Peter G. M. Wuts, published by John Wiley & Sons, Inc., 1999).

### [Step 2]

Compound (d1) can be produced by reacting compound (r1) with an alkyl chloroformate in the presence of a suitable base, in a suitable solvent.

Examples of the base include alkali metal hydrides such as sodium hydride and the like, alkali metal halides such as potassium fluoride and the like, alkali metal hydroxides such as sodium hydroxide, potassium hydroxide and the like, alkali carbonates such as sodium carbonate, potassium carbonate, cesium carbonate, sodium hydrogencarbonate and the like, alkali metal alkoxides such as sodium ethoxide, sodium tert-butoxide, potassium tert-butoxide and the like, alkali metal alkoxides such as lithiumdiisopropylamide, n-butyllithium, methyllithium, isopropylmagnesium bromide and the like. Preferred examples of the base include lithiumdiisopropylamide.

The solvent is not particularly limited as long as it does not react under the reaction condition of this step. Examples thereof include ether solvents such as diethyl ether, diisopropyl ether, tetrahydrofuran, methyl cyclopentyl ether, anisole, 1,4-dioxane and the like; aromatic hydrocarbon solvents such as benzene, toluene, chlorobenzene, xylene and the like; ester solvents such as ethyl acetate, methyl acetate and the like; aprotic solvents such as N,N-dimethylformamide, N,N-dimethylacetamide, N-methyl-2-pyrrolidinone, 1,3-dimethyl-2-imidazolidinone, dimethyl sulfoxide and the like; alcohol solvents such as methanol, ethanol, isopropyl alcohol, butanol and the like; or mixtures thereof. Preferred examples of the solvent include tetrahydrofuran, toluene and the like.

The reaction time is usually 5 min to 72 hr, preferably 30 min to 6 hr.

The reaction temperature is usually -100°C to 100°C, preferably -100°C to -50°C.

### Production Method S

The compound of the present disclosure represented by formula (e1) can be produced, for example, by the following method. 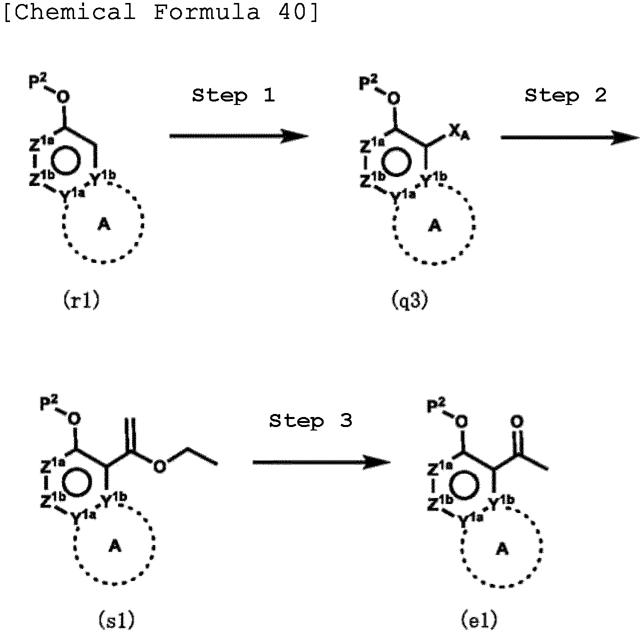 wherein Y^{1a}, Y^{1b}, Z^{1a}, Z^{1b} and Ring A are as defined in item 1, and P² is a phenol-protecting group. Examples of P² include phenol-protecting groups described in Protective Groups in Organic Synthesis (Theodora W. Greene, Peter G. M. Wuts, published by John Wiley & Sons, Inc., 1999), and the like.

### [Step 1]

Compound (q3) can be produced by reacting compound (r1) with a halogenating agent in the presence of a suitable base, in a suitable solvent.

Examples of the halogenating agent include N-chlorosuccinimide, Br₂, N-bromosuccinimide, I₂, N-iodosuccinimide and the like. Preferred examples of the halogenating agent include Br₂, and I₂.

Examples of the base include alkali metal hydrides such as sodium hydride and the like, alkali metal halides such as potassium fluoride and the like, alkali metal hydroxides such as sodium hydroxide, potassium hydroxide and the like, alkali carbonates such as sodium carbonate, potassium carbonate, cesium carbonate, sodium hydrogencarbonate and the like, alkali metal alkoxides such as sodium ethoxide, sodium tert-butoxide, potassium tert-butoxide and the like, alkali metal alkoxides such as lithiumdiisopropylamide, n-butyllithium, methyllithium, isopropylmagnesium bromide and the like. Preferred examples of the base include n-butyllithium.

The solvent is not particularly limited as long as it does not react under the reaction condition of this step. Examples thereof include ether solvents such as diethyl ether, diisopropyl ether, tetrahydrofuran, methyl cyclopentyl ether, anisole, 1,4-dioxane and the like; aromatic hydrocarbon solvents such as benzene, toluene, chlorobenzene, xylene and the like; ester solvents such as ethyl acetate, methyl acetate and the like; aprotic solvents such as N,N-dimethylformamide, N,N-dimethylacetamide, N-methyl-2-pyrrolidinone, 1,3-dimethyl-2-imidazolidinone, dimethyl sulfoxide and the like; alcohol solvents such as methanol, ethanol, isopropyl alcohol, butanol and the like; or mixtures thereof. Preferred examples of the solvent include tetrahydrofuran, toluene and the like.

The reaction time is usually 5 min to 72 hr, preferably 30 min to 6 hr.

The reaction temperature is usually -100°C to 100°C, preferably -100°C to -50°C.

### [Step 2]

Compound (s1) can be produced by reacting compound (q3) with tributyl(1-ethoxyvinyl)stannane in the presence of a suitable palladium catalyst, in a suitable solvent.

As the palladium catalyst, palladium-phosphine complexes can be used. Examples of the palladium-phosphine complex include tetrakis(triphenylphosphine)palladium(0), bis(tri-tert-butylphosphine)palladium(0), bis(triphenylphosphine)palladium(II)dichloride, bis(trio-tolylphosphine)palladium(II), [1,2-bis(diphenylphosphino)ethane]palladium(II), bis(diphenylphosphino)ferrocene]dichloropalladium(II), and [1,3-bis(diphenylphosphino)propane]palladium(II). Preferred examples of the palladium catalysts include bis(diphenylphosphino)ferrocene]dichloropalladium(II).

The solvent is not particularly limited as long as it does not react under the reaction condition of this step. Examples thereof include ether solvents such as diethyl ether, diisopropyl ether, tetrahydrofuran, methyl cyclopentyl ether, anisole, 1,4-dioxane and the like; aromatic hydrocarbon solvents such as benzene, toluene, chlorobenzene, xylene and the like; ester solvents such as ethyl acetate, methyl acetate and the like; aprotic solvents such as N,N-dimethylformamide, N,N-dimethylacetamide, N-methyl-2-pyrrolidinone, 1,3-dimethyl-2-imidazolidinone, dimethyl sulfoxide and the like; alcohol solvents such as methanol, ethanol, isopropyl alcohol, butanol and the like; or mixtures thereof. Preferred examples of the solvent include 1,4-dioxane, tetrahydrofuran, toluene and the like.

The reaction time is usually 5 min to 72 hr, preferably 6 hr to 30 hr.

The reaction temperature is usually 0°C to 200°C, preferably 100°C to 150°C.

In the production method described above, the starting raw materials and intermediates for which the production method is not described are available as commercial products, or can be synthesized from a commercially available product by a method known to those skilled in the art or a method analogous thereto.

In each reaction of the production methods described above, protecting groups can be used as necessary, even if the use of protecting groups is not explicitly described. For example, the target compound can be obtained by protecting the portion other than the reaction point as necessary and deprotecting the portion after the completion of the reaction or series of reactions, if any functional group other than the reaction point changes under the described reaction condition or if the absence of a protecting group is unsuitable for carrying out the described method.

As the protecting group, the protecting group described in Protective Groups in Organic Synthesis (Theodora W. Greene, Peter G. M. Wuts, published by John Wiley & Sons, Inc., 1999) etc., or the like can be used. Specific examples of amino-protecting group include benzyloxycarbonyl, tert-butoxycarbonyl, acetyl, benzyl and the like. Specific examples of hydroxyl-protecting group include trialkyl silyl such as trimethylsilyl, tert-butyldimethylsilyl and the like, acetyl, benzyl and the like.

The introduction or removal of protecting groups can be carried out by a method commonly used in synthetic organic chemistry (see, for example, the aforementioned Protective Groups in Organic Synthesis) or a method analogous thereto.

As used herein, protecting groups, condensing agents, or the like may be denoted by the nomenclature of IUPAC-IUB (Biochemical Nomenclature Committees) that is commonly used in the art. It should be noted that compound names used herein do not necessarily follow the IUPAC nomenclature.

The intermediates or target compounds in the production methods described above can also be introduced into another compound contained in the present disclosure by appropriately converting its functional group (e.g., various conversions using amino, a hydroxyl group, carbonyl, halogen, or the like while protecting or deprotecting a functional group as needed). The conversion of functional groups can be carried out by a common method usually used (see, for example, Comprehensive Organic Transformations, R. C. Larock, John Wiley & Sons Inc. (1999), etc.).

The intermediates or target compounds in the production methods described above can be isolated and purified by a purification method commonly used in organic synthetic chemistry (e.g., neutralization, filtration, extraction, washing, drying, concentration, recrystallization, various chromatographies). The intermediates can also be used in the subsequent reaction without any particular purification.

As the protecting group, the protecting group described in Protective Groups in Organic Synthesis (Theodora W. Greene, Peter G. M. Wuts, published by John Wiley & Sons, Inc., 1999) etc., or the like can be used. Specific examples of amino-protecting group include benzyloxycarbonyl, tert-butoxycarbonyl, acetyl, benzyl and the like. Specific examples of hydroxyl-protecting group include trialkyl silyl such as trimethylsilyl, tert-butyldimethylsilyl and the like, acetyl, benzyl and the like.

The introduction or removal of protecting groups can be carried out by a method commonly used in synthetic organic chemistry (see, for example, the aforementioned Protective Groups in Organic Synthesis) or a method analogous thereto.

As used herein, protecting groups, condensing agents, or the like may be denoted by the nomenclature of IUPAC-IUB (Biochemical Nomenclature Committees) that is commonly used in the art. It should be noted that compound names used herein do not necessarily follow the IUPAC nomenclature.

The intermediates or target compounds in the production methods described above can also be introduced into another compound contained in the present disclosure by appropriately converting its functional group (e.g., various conversions using amino, a hydroxyl group, carbonyl, halogen, or the like while protecting or deprotecting a functional group as needed). The conversion of functional groups can be carried out by a common method usually used (see, for example, Comprehensive Organic Transformations, R. C. Larock, John Wiley & Sons Inc. (1999), etc.).

The intermediates or target compounds in the production methods described above can be isolated and purified by a purification method commonly used in organic synthetic chemistry (e.g., neutralization, filtration, extraction, washing, drying, concentration, recrystallization, various chromatographies). The intermediates can also be used in the subsequent reaction without any particular purification.

Examples of the "pharmaceutically acceptable salt" include acid addition salts and base addition salts. Examples of the acid addition salt include inorganic acid salts such as hydrochloride, hydrobromide, sulfurate, hydroiodide, nitrate, phosphorate and the like, or organic acid salts such as citrate, oxalate, phthalic acid salt, fumarate, maleate, succinic acid salt, malic acid salt, acetate, formate, propionate, benzoate, trifluoroacetate, methanesulfonate, benzenesulfonate, para-toluenesulfonate, camphorsulfonate and the like. Examples of the base addition salt include inorganic base salts such as sodium salt, potassium salt, calcium salt, magnesium salt, barium salt, aluminium salt and the like, or salts with organic base such as trimethylamine, triethylamine, pyridine, picoline, 2,6-lutidine, ethanolamine, diethanolamine, triethanolamine, tromethamine [tris(hydroxymethyl)methylamine], tert-butylamine, cyclohexylamine, dicyclohexylamine, N,N-dibenzyl ethylamine and the like. Examples of the "pharmaceutically acceptable salt" also include amino acid salts with basic or acidic amino acids such as arginine, lysine, ornithine, aspartic acid, or glutamic acid and the like.

Salts suitable for the starting raw materials and intermediates and salts acceptable as raw materials for pharmaceutical products are conventionally used nontoxic salts. Examples thereof include acid addition salts such as organic acid salts (e.g., acetate, trifluoroacetate, maleate, fumarate, citrate, tartrate, methanesulfonate, benzenesulfonate, formate, toluenesulfonate, etc.) and inorganic acid salts (e.g., hydrochloride, hydrobromide, hydroiodide, sulfurate, nitrate, phosphate, etc.), salts with amino acids (e.g., arginine, aspartic acid, glutamic acid, etc.), metal salts such as alkali metal salts (e.g., sodium salt, potassium salt, etc.), alkaline-earth metal salts (e.g., calcium salt, magnesium salt, etc.) and the like, ammonium salts, organic base salts (e.g., trimethylamine salt, triethylamine salt, pyridine salt, picoline salt, dicyclohexylamine salt, N,N'-dibenzyl ethylene diamine salt, etc.) and the like. Those skilled in the art can appropriately select these salts.

Deuterated compounds prepared by converting any one or more of ¹H of the compounds represented by formulas (1) to (8) to ²H(D) are also encompassed by the compounds represented by formulas (1) to (8) in the present disclosure.

The present disclosure encompasses the compounds represented by formulas (1) to (8) and pharmaceutically acceptable salts thereof. The compound of the present disclosure can also be in a form of a hydrate and/or solvate of various solvents (ethanolate, etc.). Thus, such hydrates and/or solvates are also encompassed by the compound of the present disclosure.

The compound of the present disclosure also encompasses all other possible isomers such as optical isomers based on an optically-active center, atropisomers based on axial or planar chirality resulting from restriction of intramolecular rotation, other stereoisomers, tautomers, geometric isomers, and crystalline forms in any form, and mixtures thereof.

In particular, an optical isomer and an atropisomer can be obtained as a racemate, or an optically-active form if an optically-active starting material or intermediate is used, respectively. If necessary, the corresponding raw material, intermediate, or final product racemate can be physically or chemically resolved, during an appropriate step of the production method described above, into their optical enantiomers by a known separation method such as a method using an optically active column or a fractional crystallization method. Examples of the resolution method include a diastereomer method in which two types of diastereomers are synthesized by reacting a racemate with an optically-active resolving agent, and the diastereomers are then resolved by a method such as a fractional crystallization method, utilizing the difference in their physical properties.

If it is desirable to obtain a pharmaceutically acceptable salt of the compound of the present disclosure, a compound represented by formula (1) to (8), when obtained in a form of a pharmaceutically acceptable salt, may be directly purified, or when obtained in a free form, a salt may be formed by dissolving or suspending the compound in a suitable organic solvent and adding an acid or a base according to a common method.

The liposome encapsulating the compound of the present disclosure can be produced, for example, by the following method.

### [Step 1]

Membrane components such as a phospholipid, cholesterol and the like are dissolved in an organic solvent such as chloroform, and the organic solvent is evaporated from a flask to form a thin film of the lipid mixture on the inner wall of the flask. As an alternative to the above, membrane components are dissolved in tert-butyl alcohol, and the solution is lyophilized to obtain a lipid mixture as a lyophilized product. Also, membrane components such as a phospholipid, cholesterol and the like are dissolved in an organic solvent, and dried using an instantaneous vacuum dryer CRUX (Hosokawa Micron Corporation) to obtain a powderized lipid mixture. This is available from Nippon Fine Chemical Co., Ltd. under the name Presome (registered trademark).

### [Step 2]

An inner aqueous phase solution such as aqueous ammonium sulfate solution and the like is added to the lipid mixture obtained in Step 1, and the mixture is dispersed to obtain a crude liposome dispersion.

### [Step 3]

The crude liposome dispersion obtained in Step 2 is passed through a filter using an extruder to obtain liposomes of the desired particle size. Alternatively, the crude liposome dispersion obtained in Step 2 is discharged through a nozzle at high pressure using a high-pressure homogenizer to obtain liposomes of the desired particle size. The particle size of the liposomes is not particularly limited, and is, for example, 10 nm to 200 nm, preferably 30 nm to 150 nm, more preferably 40 nm to 140 nm, still more preferably 50 to 120 nm, most preferably 60 to 100 nm. The particle size of the liposomes is an average value measured by a dynamic light scattering method, and can be measured, for example, using Zetasizer Nano ZS (Malvern Instruments).

### [Step 4]

The outer aqueous phase of the liposome solution obtained in Step 3 is replaced by gel filtration, dialysis, tangential flow filtration, ultracentrifugation, or the like.

### [Step 5]

The liposome solution with the replaced outer aqueous phase obtained in Step 4 is incubated with the compound to be encapsulated to encapsulate the compound in the liposomes.

### [Step 6]

The resulting liposomes encapsulating the compound are subjected to gel filtration, dialysis, tangential flow filtration, or ultracentrifugation, or the like to remove unencapsulated compound. If a desired encapsulation rate is achieved in Step 5, Step 6 can be omitted.

The compound of the present disclosure can be used in combination with other drugs for purpose of enhancing its effect. Specifically, the compound of the present disclosure can be used in combination with a drug such as a hormonal therapy agent, a chemotherapeutic agent, an immunotherapeutic agent or an agent that inhibits a cell growth factor and its receptor action, and the like. Hereinafter, a drug that may be used in combination with the compound of the present disclosure is abbreviated to a concomitant drug.

The compound of the present disclosure, even when used as a single agent, exhibits excellent anticancer effect, and further the combination use with one or several of the above-described concomitant drugs (polypharmacy) can further enhance its effect or improve the QOL of a patient.

Examples of "hormonal therapy agents" include Fosfestrol, Diethylstilbestrol, Chlorotrianisene, Medroxyprogesterone acetate, Megestrol acetate, Chlormadinone acetate, Cyproterone acetate, Danazol, Dienogest, Asoprisnil, Allylestrenol, Gestrinone, Nomegestrol, Tadenan, Mepartricin, Raloxifene, Ormeloxifene, Levormeloxifene, antiestrogens (e.g., Tamoxifen citrate, Toremifene citrate, and the like), pill preparation, Mepitiostane, Testololactone, aminoglutethimide, LH-RH derivatives (LH-RH agonist (e.g., Goserelin acetate, Buserelin, Leuprorelin, and the like), LH-RH antagonist), Droloxifene, Epitiostanol, ethynyl estradiol sulfonate, aromatase inhibitors (e.g., Fadrozole hydrochloride, Anastrozole, Letrozole, exemestane, vorozole, formestane, and the like), antiandrogens (e.g., Flutamide, Enzalutamide, Apalutamide, Bicalutamide, Nilutamide, and the like), adrenocortical hormone drugs (e.g., Dexamethasone, Prednisolone, Betamethasone, Triamcinolone, and the like), androgen synthesis inhibitors (e.g., Abiraterone, and the like), retinoid, and drugs to delay the metabolism of retinoid (e.g., Liarozole, and the like), and the like.

As "chemotherapeutic agents", for example, alkylating agents, antimetabolites, anticancer antibiotics, plant-derived anticancer agents, molecularly targeted therapeutic agents, immunomodulators, other chemotherapeutic agents, and the like are used. Representative examples are described below.

Examples of "alkylating agents" include Nitrogen mustard, Nitrogen mustard N-oxide hydrochloride, Chlorambucil, Cyclophosphamide, Ifosfamide, Thiotepa, Carboquone, Improsulfan tosylate, Busulfan, Nimustine hydrochloride, Mitobronitol, Melphalan, Dacarbazine, Ranimustine, Estramustine phosphate sodium, Triethylenemelamine, Carmustine, Lomustine, Streptozocin, Pipobroman, Etoglucide, Carboplatin, Cisplatin, Miriplatin, Nedaplatin, Oxaliplatin, Altretamine, Ambamustine, Dibrospidium chloride, Fotemustine, Prednimustine, Pumitepa, Ribomustin, Temozolomide, Treosulfan, Trofosfamide, Zinostatin stimalamer, Adozelesin, Cystemustine, Bizelesin, Trabectedin, and DDS preparations thereof, and the like.

Examples of "antimetabolites" include Mercaptopurine, 6-Mercaptopurine riboside, Thioinosine, Methotrexate, Pemetrexed, Enocitabine, Cytarabine, Cytarabin ocfosfate, Ancitabine hydrochloride, 5-FU type drugs (e.g., Fluorouracil, Tegafur, UFT, Doxifluridine, Carmofur, Galocitabine, Emitefur, Capecitabine, and the like), Aminopterin, Nelarabine, Leucovorin calcium, tabloid, Butocin, calcium folinate, calcium levofolinate, Cladribine, Emitefur, Fludarabine, Gemcitabine, hydroxycarbamide, Pentostatin, Piritrexim, Idoxuridine, Mitoguazone, Tiazofurin, Ambamustine, Bendamustine, and DDS preparations thereof, and the like.

Examples of "anticancer antibiotics" include Actinomycin D, Actinomycin C, Mitomycin C, Chromomycin A3, Bleomycin hydrochloride, Bleomycin sulfate, Peplomycin sulfate, Daunorubicin hydrochloride, Doxorubicin hydrochloride, Aclarubicin hydrochloride, Pirarubicin hydrochloride, Epirubicin hydrochloride, Neocarzinostatin, Mithramycin, Sarkomycin, Carzinophilin, Mitotane, Zorubicin hydrochloride, Mitoxantrone hydrochloride, Idarubicin hydrochloride, Eribulin, and DDS preparations thereof, and the like.

Examples of "plant-derived anticancer agents" include Etoposide, Etoposide phosphate, Vinblastine sulfate, Vincristine sulfate, Vindesine sulfate, Teniposide, Paclitaxel, Docetaxel, DJ-927, Vinorelbine, Irinotecan, Topotecan, and DDS preparations thereof, and the like.

Examples of "molecularly targeted therapeutic agents" include Imatinib, Gefitinib, Erlotinib, Sorafenib, Dasatinib, Sunitinib, Nilotinib, Lapatinib, Pazopanib, Ruxolitinib, Crizotinib, Vemurafenib, Vandetanib, Ponatinib, Cabozantinib, Tofacitinib, Regorafenib, Bosutinib, Axitinib, Dabrafenib, Trametinib, Nintedanib, Idelalisib, Ceritinib, Lenvatinib, Palbociclib, Alectinib, Afatinib, Osimertinib, Ribociclib, Abemaciclib, Brigatinib, Neratinib, Copanlisib, Cobimetinib, Ibrutinib, Acalabrutinib, Encorafenib, Binimetinib, Baricitinib, Fostamatinib, Lorlatinib, Erdafitinib, Entrectinib, Dacomitinib, Sirolimus, Everolimus, Temsirolimus, Olaparib, Rucaparib, Niraparib, Venetoclax, Azacitidine, Decitabine, Vorinostat, Panobinostat, Romidepsin, Bortezomib, Carfilzomib, Larotrectinib, Ixazomib, and the like.

Examples of "immunomodulators" include Lenalidomide and Pomalidomide, and the like.

Examples of "other chemotherapeutic agents" include Sobuzoxane, and the like.

Examples of "immunotherapeutic agents (BRM)" include Picibanil, Krestin, Sizofiran, Lentinan, Ubenimex, interferon, interleukin, macrophage colony stimulating factor, granulocyte-colony stimulating factor, Erythropoietin, Lymphotoxin, BCG vaccine, Corynebacterium parvum, Levamisole, polysaccharide K, Procodazole, anti-CTLA4 antibody, anti-PD-1 antibody, anti-PD-L1 antibody, Toll-like Receptors agonists (e.g., TLR7 agonist, TLR8 agonist, TLR9 agonist, and the like).

The cell growth factor in an agent that inhibits a cell growth factor and its receptor action may be any substance as long as it promotes cell growth, and generally includes a factor that is a peptide having a molecular weight of 20,000 or less and exhibits an effect at a low concentration by binding with a receptor. Specific examples thereof include EGF (epidermal growth factor) or substances having substantially the same activity as it (e.g., TGFalpha, and the like), insulin or substances having substantially the same activity as it (e.g., insulin, IGF (insulin-like growth factor)-1, IGF-2, and the like), FGF (fibroblast growth factor) or substances having substantially the same activity as it (e.g., acidic FGF, basic FGF, KGK (keratinocyte growth factor), FGF-10, and the like), and other cell growth factors (e.g., CSF (colony stimulating factor), EPO (erythropoietin), IL-2 (interleukin-2), NGF (nerve growth factor), PDGF (platelet-derived growth factor), TGF-beta (transforming growth factor beta), HGF (hepatocyte growth factor), VEGF (vascular endothelial growth factor), heregulin, angiopoietin, and the like).

The period of administration of the compound of the present disclosure and a concomitant drug is not limited, and these may be administered concurrently or at intervals to a subject to be administered. In addition, a mixture of the compound of the present disclosure and a concomitant drug may be made. The dosage of a concomitant drug can be appropriately selected using clinically used dose as criteria. In addition, the mixing ratio of the compound of the present disclosure and a concomitant drug can be appropriately selected depending on a subject to be administered, an administration route, target disease, symptoms, combinations, and the like. For example, when a subject to be administered is a human, 0.01 to 100 parts by weight of a concomitant drug may be used relative to one part by weight of the compound of the present disclosure. In addition, for purpose of inhibiting its side effect, they can be used in combination with a drug (a concomitant drug) such as an antiemetic agent, a sleep-inducing agent, an anticonvulsant, and the like.

As used herein, "or" is used when "at least one or more" of the listed matters in the sentence can be employed. When explicitly described herein as "within the range of two values", the range also includes the two values themselves.

Reference literatures such as scientific literatures, patents, and patent applications cited herein are incorporated herein by reference to the same extent that the entirety of each document is specifically described.

The present disclosure has been described while showing preferred embodiments to facilitate understanding. While the present disclosure is described hereinafter based on the Examples, the above descriptions and the following Examples are provided for the sole purpose of exemplification, not limitation of the present disclosure. Thus, the scope of the present disclosure is not limited to the embodiments and Examples that are specifically described herein and is limited only by the scope of claims.

### [Examples]

The present disclosure is explained in more detail below by referring to Reference Examples, Examples and Test Examples, but it is not limited thereto.

The following abbreviations may be used herein.
Ts: p-toluenesulfonyl
THF: tetrahydrofuran
TFA: trifluoroacetic acid
DMF: N,N-dimethylformamide
DMSO: dimethyl sulfoxide
MeCN: acetonitrile
Me: methyl
Boc: tert-butoxycarbonyl
Dess-Martin reagent: Dess-Martin periodinane (1,1,1-triacetoxy-1,1-dihydro-1,2-benziodoxol-3-(1H)-one)
SEMC1: 2-(trimethylsilyl)ethoxymethyl chloride
DIEA: N,N-diisopropylethylamine
PTSA: p-toluenesulfonic acid
NIS: N-iodosuccinimide
DMAP: 4-dimethylaminopyridine
MOMCl: chloromethyl methyl ether
p-TsCl: p-toluenesulfonyl chloride
TBAI: tetrabutylammonium iodide

NMR (Nuclear Magnetic Resonance) data used for compound identification was obtained by JNM-ECS400 nuclear magnetic resonance system (400 MHz) manufactured by JEOL.

The symbols used in the NMR mean the following: s is singlet, d is doublet, dd is doublet of doublets, t is triplet, td is triplet of doublets, q is quartet, m is multiplet, br is broad, brs is broad singlet, brm is broad multiplet, and J is bond constant.

### LC/MS (Liquid Chromatography-Mass Spectrometry) analysis used for compound identification

The conditions are shown below. Among the observed mass spectrometry values [MS (m/z)], the value corresponding to the monoisotopic mass (accurate mass consisting only of the main isotope) is indicated by [M+H]⁺, [M-H]⁻ or [M+2H]²⁺ or the like, and the retention time is indicated by Rt (min).

### LC/MS Measurement Method:

### Analysis Condition A

Detection equipment: ACQUITY (registered trademark) SQ detector (Waters)
HPLC: ACQUITY UPLC (registered trademark) system
Column: Waters ACQUITY UPLC (registered trademark) BEH C18 (1.7 µm, 2.1 mm × 30mm)
Solvent: Solution A: 0.06% formic acid/H₂O, Solution B: 0.06% formic acid/MeCN
Gradient condition: 0.0-1.3 min Linear gradient from B 2% to 96%
Flow rate: 0.8 mL/min
UV: 220 nm and 254 nm
Column temperature: 40°C

### Measurement Condition B

Detection equipment: Shimadzu LCMS-2020 system
Column: L-column-2 CDS (4.6 mm × 35 mm)
Gradient condition: MeCN/H₂O/HCO₂H=10/90/0.1_{→}100/0/0.1 (0-2 min), 100/0/0.1 (2-4 min)
Flow rate: 2 mL/min
Column temperature: 40°C

### Reference Example 1:

### tert-butyl (3-{[8-(3-amino-1H-pyrazol-5-yl)quinolin-7-yl]oxy)propyl)carbamate

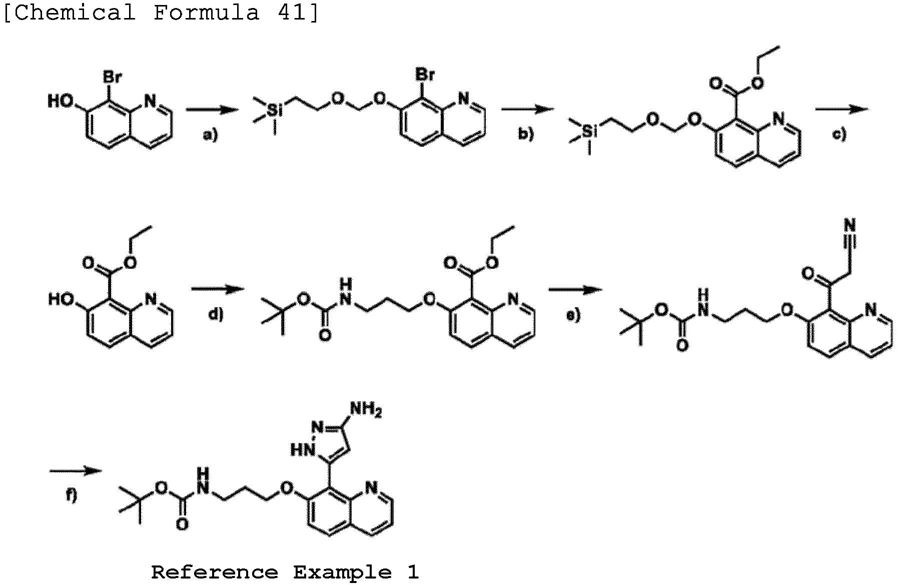

### a) Production of 8-bromo-7-{[2-(trimethylsilyl)ethoxy]methoxy}quinoline

To a solution of 8-bromo-7-quinolinol (1.06 g) in DMF (23.7 mL) were added SEMCl (1.25 mL) and DIEA (1.66 mL) under ice-cooling, and the mixture was stirred at 0°C for 1 hr. To the reaction solution was added water, and the mixture was subjected to extraction with ethyl acetate. The organic layer was washed with water and saturated brine, dried over anhydrous sodium sulfate, and filtered, and the solvent was evaporated under reduced pressure. The residue was purified by silica gel column chromatography (hexane/ethyl acetate) to give the title compound (1.66 g).
LC-MS; 354.1, 356.1/Rt (min) 1.170 (Measurement Condition A)

### b) Production of ethyl 7-{[2-(trimethylsilyl)ethoxy]methoxy}quinoline-8-carboxylate

To a solution of 8-bromo-7-{[2-(trimethylsilyl)ethoxy]methoxy}quinoline (1.65 g) in THF (23.3 mL) was added dropwise n-butyllithium (2.69 M, 2.60 mL) at -78°C, and the mixture was stirred for 30 min. Chloroethyl formate (2.22 mL) was added thereto at -78°C, and the mixture was stirred at -78°C for additional 15 min. To the reaction solution was added saturated aqueous ammonium chloride solution, and the mixture was subjected to extraction with chloroform. The organic layer was washed with saturated brine, dried over anhydrous sodium sulfate, and filtered, and the solvent was evaporated under reduced pressure. The residue was purified by silica gel column chromatography (hexane/ethyl acetate) to give the title compound (1.25 g).
LC-MS;[M+H]⁺ 348.0/Rt (min) 1.146 (Measurement Condition A)

### c) Production of ethyl 7-hydroxyquinoline-8-carboxylate

To a solution of ethyl 7-{[2-(trimethylsilyl)ethoxy]methoxy}quinoline-8-carboxylate (1.25 g) in ethanol (18.0 mL) was added PTSA monohydrate (137 mg) at room temperature, and the mixture was stirred at 50°C for 5 hr. The reaction solution was concentrated, and the residue was purified by silica gel column chromatography (chloroform/methanol) to the title compound (781 mg) as a crude product.
LC-MS;[M+H]⁺ 218.1/Rt (min) 0.441 (Measurement Condition A)

### d) Production of ethyl 7-[3-{(tert-butoxycarbonyl)amino}propoxy]quinoline-8-carboxylate

To a solution of ethyl 7-hydroxyquinoline-8-carboxylate (781 mg) in DMF (18.0 mL) were added potassium carbonate (994 mg) and t-butyl N-(3-bromopropyl)carbamate (1.28 g) at room temperature, and the mixture was stirred at room temperature for 6 hr. To the reaction solution was added water, and the mixture was subjected to extraction with ethyl acetate. The organic layer was washed with water and saturated brine, dried over anhydrous sodium sulfate, and filtered, and the solvent was evaporated under reduced pressure. The residue was purified by amino silica gel column chromatography (hexane/ethyl acetate) to give the title compound (1.29 g)
LC-MS;[M+H]⁺ 375.0/Rt (min) 0.882 (Measurement Condition A)

### e) Production of tert-butyl (3-{[8-(2-cyanoacetyl)quinolin-7-yl]oxy}propyl)carbamate

To a solution of MeCN (0.283 mL) in THF (13.6 mL) was added dropwise n-butyllithium (1.58M, 2.58mL) at - 78°C, and the mixture was stirred at -78°C for 1 hr. Ethyl 7-[3-{(tert-butoxycarbonyl)amino}propoxy]quinoline-8-carboxylate (1.02 g) in THF (13.6 mL) was added thereto at -78°C, and the mixture was stirred at -78°C for additional 2.5 hr. To the reaction solution was added saturated aqueous ammonium chloride solution, and the mixture was subjected to extraction with chloroform. The organic layer was washed with saturated brine, dried over anhydrous sodium sulfate, and filtered, and the solvent was evaporated under reduced pressure to the title compound (1.00 g) as a crude product.
LC-MS;[M+H]⁺ 370.3/Rt (min) 0.808 (Measurement Condition A)

### f) Production of tert-butyl (3-{[8-(3-amino-1H-pyrazol-5-yl)quinolin-7-yl]oxy}propyl)carbamate (Reference Example 1)

To a solution of tert-butyl (3-{[8-(2-cyanoacetyl)quinolin-7-yl]oxy}propyl)carbamate (1.00 g) in ethanol (13.6 mL) were added acetic acid (1.32 mL) and hydrazine monohydrate (1.55 mL) at room temperature, and the mixture was stirred under reflux for 2.5 hr. After allowing to cool, to the reaction solution was added water, and the mixture was subjected to extraction with ethyl acetate. The organic layer was washed with saturated brine, dried over anhydrous sodium sulfate, and filtered, and the solvent was evaporated under reduced pressure. The residue was purified by amino silica gel column chromatography (chloroform/methanol) to give Reference Example 1 (224 mg).
LC-MS;[M+H]⁺ 384.3/Rt (min) 0.685 (Measurement Condition A)

### Reference Example 2:

### tert-butyl {[1-({[4-(3-amino-1H-pyrazol-5-yl)-1,5-naphthyridin-3-yl]oxy}methyl)cyclopropyl]methyl}carbamate

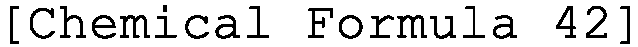

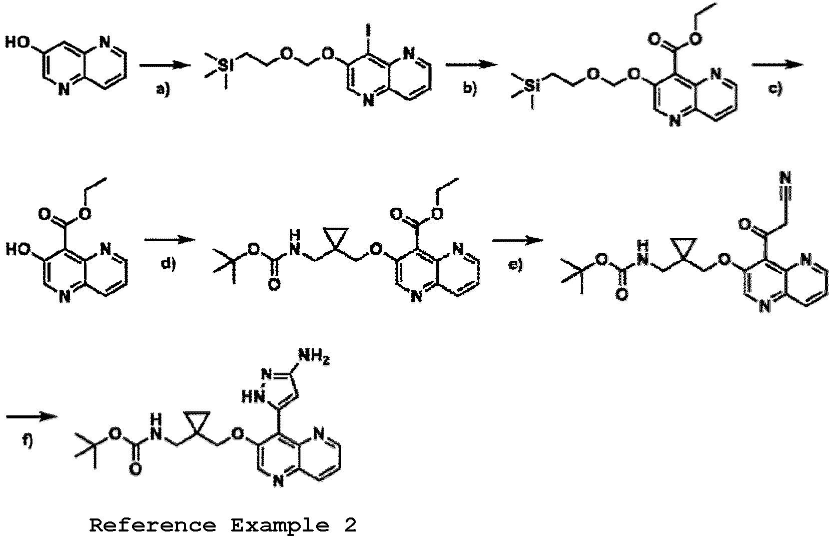

### a) Production of 4-iodo-3-{[2-(trimethylsilyl)ethoxy]methoxy}-1,5-naphthyridine

To a solution of 1,5-naphthyridin-3-ol (5.00 g) in MeCN (86.0 mL) were added PTSA monohydrate (6.51 g) and NIS (11.6 g) at room temperature, and the mixture was stirred at 60°C for 2 hr. The reaction solution was concentrated, and the residue was dissolved in DMF (50.0 mL). SEMC1 (6.00 mL) and DIEA (29.8 mL) were added thereto under ice-cooling, and the mixture was stirred at room temperature for 15 min. To the reaction solution was added water, and the mixture was subjected to extraction with ethyl acetate. The organic layer was washed with water and saturated brine, dried over anhydrous sodium sulfate, and filtered, and the solvent was evaporated under reduced pressure. The residue was purified by silica gel column chromatography (hexane/ethyl acetate) to give the title compound (10.7 g).
LC-MS;[M+H]⁺ 403.2/Rt (min) 1.168 (Measurement Condition A)

### b) Production of ethyl 3-{[2-(trimethylsilyl)ethoxy]methoxy}-1,5-naphthyridine-4-carboxylate

To a solution of 4-iodo-3-{[2-(trimethylsilyl)ethoxy]methoxy}-1,5-naphthyridine (805 mg) in THF (10.0 mL) was added dropwise n-butyllithium (2.69 M, 1.12 mL) at -78°C, and the mixture was stirred for 30 min. Chloroethyl formate (0.950 mL) was added thereto at -78°C, and the mixture was stirred at -78°C for additional 15 min. To the reaction solution was added saturated aqueous ammonium chloride solution, and the mixture was subjected to extraction with chloroform. The organic layer was washed with saturated brine, dried over anhydrous sodium sulfate, and filtered, and the solvent was evaporated under reduced pressure. The residue was purified by silica gel column chromatography (hexane/ethyl acetate) to give the title compound (492 mg) .
LC-MS;[M+H]⁺ 350.2/Rt (min) 1.109 (Measurement Condition A)

### c) Production of ethyl 3-hydroxy-1,5-naphthyridine-4-carboxylate

To a solution of ethyl 3-{[2-(trimethylsilyl)ethoxy]methoxy}-1,5-naphthyridine-4-carboxylate (845 mg) in ethanol (12.1 mL) was added PTSA monohydrate (461 mg) at room temperature, and the mixture was stirred at room temperature for 30 min. The reaction solution was concentrated, and the residue was purified by silica gel column chromatography (chloroform/methanol) to give the title compound (324 mg).
LC-MS;[M+H]⁺ 219.1/Rt (min) 0.453 (Measurement Condition A)

### d) Production of ethyl 3-[(1-{[(tert-butoxycarbonyl)amino]methyl}cyclopropyl)methoxy]-1,5-naphthyridine-4-carboxylate

To a solution of ethyl 3-hydroxy-1,5-naphthyridine-4-carboxylate (162 mg) and 1,1'-dimethylethyl N-[[1-(hydroxymethyl)cyclopropyl]methyl]carbamate (299 mg) in THF (3.71 mL) was added 1,1'-(azodicarbonyl)dipiperidine (375 mg) at room temperature, and then tributylphosphine (0.366 mL) was added thereto dropwise under ice-cooling, and the mixture was stirred at room temperature for 3 hr. The reaction solution was filtered through Celite, to the filtrate was added water, and the mixture was subjected to extraction with chloroform. The organic layer was washed with saturated brine, dried over anhydrous sodium sulfate, and filtered, and the solvent was evaporated under reduced pressure. The residue was purified by amino silica gel column chromatography (hexane/ethyl acetate) to give the title compound (298 mg)
LC-MS;[M+H]⁺ 403.3/Rt (min) 0.930 (Measurement Condition A)

### e) Production of tert-butyl {[1-({[4-(2-cyanoacetyl)-1,5-naphthyridin-3-yl]oxy}methyl)cyclopropyl]methyl}carbamate

To a solution of MeCN (0.116 mL) in THF (3.71 mL) was added dropwise n-butyllithium (1.58M, 1.41mL) at - 78°C, and the mixture was stirred for 1 hr. Ethyl 3-[(1-{[(tert-butoxycarbonyl)amino]methyl}cyclopropyl)methoxy]-1,5-naphthyridine-4-carboxylate (298 mg) in THF (3.71 mL) was added thereto at -78°C, and the mixture was stirred at -78°C for 2 hr. To the reaction solution was added saturated aqueous ammonium chloride solution, and the mixture was subjected to extraction with chloroform. The organic layer was washed with saturated brine, dried over anhydrous sodium sulfate, and filtered, and the solvent was evaporated under reduced pressure to the title compound (294 mg) as a crude product.
LC-MS;[M+H]⁺ 397.3/Rt (min) 0.890 (Measurement Condition A)

### f) Production of tert-butyl {[1-({[4-(3-amino-1H-pyrazol-5-yl)-1,5-naphthyridin-3-yl]oxy}methyl)cyclopropyl]methyl}carbamate (Reference Example 2)

To a solution of tert-butyl {[1-({[4-(2-cyanoacetyl)-1,5-naphthyridin-3-yl]oxy}methyl)cyclopropyl]methyl}carbamate (294 mg) in ethanol (3.71 mL) were added acetic acid (0.425 mL) and hydrazine monohydrate (0.360 mL) at room temperature, and the mixture was stirred under reflux for 5 hr. After allowing to cool, to the reaction solution was added water, and the mixture was subjected to extraction with chloroform. The organic layer was washed with saturated brine, dried over anhydrous sodium sulfate, and filtered, and the solvent was evaporated under reduced pressure. The residue was purified by amino silica gel column chromatography (hexane/ethyl acetate) to give Reference Example 2 (87.2 mg).
LC-MS;[M+H]⁺ 411.4/Rt (min) 0.712

### Reference Example 3:

### tert-butyl 3-methyl-3-[(tosyloxy)methyl]azetidine-1-carboxylate

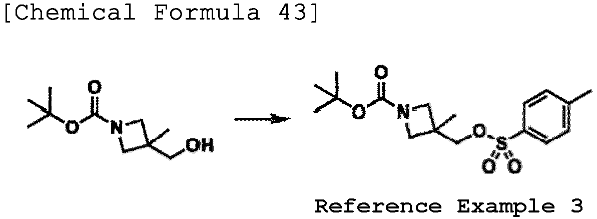

### Production of tert-butyl 3-methyl-3-[(tosyloxy)methyl]azetidine-1-carboxylate (Reference Example 3)

To a solution of t-butyl 3-(hydroxymethyl)-3-methylazetidine-1-carboxylate (1.00 g) in dichloromethane (16.6 mL) were added DMAP (0.182 g), triethylamine (1.39 mL) and p-TsCl (1.42 g) at room temperature, and the mixture was stirred at room temperature for 2 hr. To the reaction solution was added water, and the mixture was subjected to extraction with chloroform. The organic layer was washed with saturated brine, dried over anhydrous sodium sulfate, and filtered, and the solvent was evaporated under reduced pressure. The residue was purified by silica gel column chromatography (hexane/ethyl acetate) to give Reference Example 3 (1.04 g).
LC-MS;[M+H]⁺ 356.2/Rt (min) 1.066 (Measurement Condition A)

### Reference Example 4:

### tert-butyl 3-({[7-(3-amino-1H-pyrazol-5-yl)-2,3-dihydrofuro[3,2-b]pyridin-6-yl]oxy}methyl)-3-methylazetidine-1-carboxylate

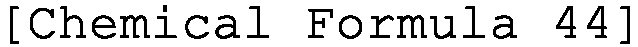

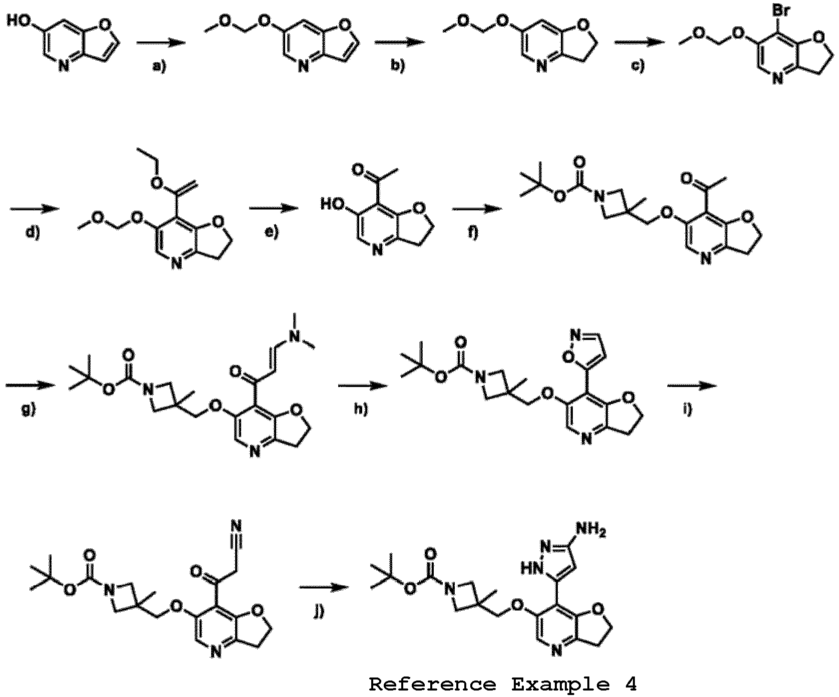

### a) Production of 6-(methoxymethoxy)furo[3,2-b]pyridine

To a solution of 1-(7-hydroxyquinoline-8-yl)ethan-1-one (3.00 g) in DMF (40.0 mL) were added dropwise potassium carbonate (6.14 g) and MOMCl (1.84 mL) under ice-cooling, and the mixture was stirred at room temperature for 4 hr. To the reaction solution was added saturated brine, and the mixture was subjected to extraction with ethyl acetate. The organic layer was dried over anhydrous magnesium sulfate, and filtered, and the solvent was evaporated under reduced pressure. The residue was purified by amino silica gel column chromatography (chloroform/methanol) to give the title compound (2.00 g).
LC-MS;[M+H]⁺ 181.1/Rt (min) 0.602 (Measurement Condition A)

### b) Production of 6-(methoxymethoxy)-2,3-dihydrofuro[3,2-b]pyridine

To a solution of 6-(methoxymethoxy)furo[3,2-b]pyridine (5.00 g) in ethanol (20.0 mL) was added 10% palladium-carbon (2.97 g) at room temperature, and the mixture was stirred under hydrogen atmosphere at room temperature for 8 hr. The reaction solution was filtered through Celite, the filtrate was concentrated, and the residue was purified by amino silica gel column chromatography (chloroform/methanol) to give the title compound (4.00 g).
LC-MS;[M+H]⁺ 183.1/Rt (min) 0.525 (Measurement Condition A)

### c) Production of 7-bromo-6-(methoxymethoxy)-2,3-dihydrofuro[3,2-b]pyridine

To a solution of 6-(methoxymethoxy)-2,3-dihydrofuro[3,2-b]pyridine (1.37 g) in THF (20.0 mL) was added dropwise n-butyllithium (2.60M, 3.49mL) at -78°C, and the mixture was stirred for 1 hr. Br2 (0.584 mL) was added thereto at -78°C, and the mixture was stirred at - 78°C for additional 2 hr. To the reaction solution was added 5% aqueous potassium hydrogen sulfate solution, and the mixture was subjected to extraction with ethyl acetate. The organic layer was washed with saturated brine, dried over anhydrous magnesium sulfate, and filtered, and the solvent was evaporated under reduced pressure. The residue was purified by silica gel column chromatography (hexane/ethyl acetate) to give the title compound (1.00 g).
LC-MS;260.1, 262.1/Rt (min) 0.720 (Measurement Condition A)

### d) Production of 7-(1-ethoxyvinyl)-6-(methoxymethoxy)-2,3-dihydrofuro[3,2-b]pyridine

To a solution of 7-bromo-6-(methoxymethoxy)-2,3-dihydrofuro[3,2-b]pyridine (1.44 g) in 1,4-dioxane (20.0 mL) were added tributyl(1-ethoxyvinyl)stannane (2.75 mL) and [1,1'-bis(diphenylphosphino)ferrocene]dichloropalladium (II) (0.904 g) under ice-cooling, and the mixture was stirred at 120°C for 15 hr. To the reaction solution was added saturated brine, and the mixture was subjected to extraction with ethyl acetate. The organic layer was dried over anhydrous magnesium sulfate, and filtered, and the solvent was evaporated under reduced pressure. The residue was purified by silica gel column chromatography (hexane/ethyl acetate) to give the title compound (1.14 g).
LC-MS;[M+H]⁺ 252.2/Rt (min) 0.741 (Measurement Condition A)

### e) Production of 1-(6-hydroxy-2,3-dihydrofuro[3,2-b]pyridin-7-yl)ethan-1-one

To a solution of 7-(1-ethoxyvinyl)-6-(methoxymethoxy)-2,3-dihydrofuro[3,2-b]pyridine (1.50 g) in methanol (20.0 mL) was added 5M HCl aqueous solution (2.00 mL) under ice-cooling, and the mixture was stirred at room temperature for 2 hr. The reaction solution was concentrated, and the residue was purified by silica gel column chromatography (chloroform/methanol) to give the title compound (567 mg).
LC-MS;[M+H]⁺ 180.0/Rt (min) 0.618 (Measurement Condition A)

### f) Production of tert-butyl 3-{[(7-acetyl-2,3-dihydrofuro[3,2-b]pyridin-6-yl)oxy]methyl}-3-methylazetidine-1-carboxylate

To a solution of 1-(6-hydroxy-2,3-dihydrofuro[3,2-b]pyridin-7-yl)ethan-1-one (420 mg) in DMF (20.0 mL) were added Reference Example 3 (1.25 g), TBAI (43.3 mg) and cesium carbonate (1.53 g) under ice-cooling, and the mixture was stirred at room temperature for 12 hr. To the reaction solution was added saturated brine, and the mixture was subjected to extraction with ethyl acetate. The organic layer was dried over anhydrous magnesium sulfate, and filtered, and the solvent was evaporated under reduced pressure. The residue was purified by silica gel column chromatography (hexane/ethyl acetate) to give the title compound (400 mg).
LC-MS;[M+H]⁺ 363.3/Rt (min) 0.886 (Measurement Condition A)

### g) Production of tert-butyl (E)-3-[({7-[3-(dimethylamino)acryloyl]-2,3-dihydrofuro[3,2-b]pyridin-6-yl}oxy)methyl]-3-methylazetidine-1-carboxylate

To a solution of tert-butyl 3-{[(7-acetyl-2,3-dihydrofuro[3,2-b]pyridin-6-yl)oxy]methyl}-3-methylazetidine-1-carboxylate (400 mg) in DMF (10.0 mL) was added N,N-dimethylformamide dimethylacetal (1.32 g) under ice-cooling, and the mixture was stirred at 115°C for 3 hr. The reaction solution was concentrated to give the title compound (440 mg) as a crude product.
LC-MS;[M+H]⁺ 419.4/Rt (min) 0.726 (Measurement Condition A)

### h) Production of tert-butyl 3-({[7-(isoxazol-5-yl)-2,3-dihydrofuro[3,2-b]pyridin-6-yl)oxy]methyl}-3-methylazetidine-1-carboxylate

To a solution of tert-butyl (E)-3-[({7-[3-(dimethylamino)acryloyl]-2,3-dihydrofuro[3,2-b]pyridin-6-yl}oxy)methyl]-3-methylazetidine-1-carboxylate (440 mg) in ethanol (30.0 mL) was added hydroxylamine hydrochloride (880 mg) under ice-cooling, and the mixture was stirred at 65°C for 2 hr. To the reaction solution was added saturated brine, and the mixture was subjected to extraction with ethyl acetate. The organic layer was dried over anhydrous magnesium sulfate, and filtered, and the solvent was evaporated under reduced pressure. The residue was purified by silica gel column chromatography (hexane/ethyl acetate) to give the title compound (200 mg) .
LC-MS;[M+H]⁺ 388.3/Rt (min) 0.918 (Measurement Condition A)

### i) Production of tert-butyl 3-({[7-(2-cyanoacetyl)-2,3-dihydrofuro[3,2-b]pyridin-6-yl)oxy]methyl}-3-methylazetidine-1-carboxylate

To a solution of tert-butyl 3-({[7-(isoxazol-5-yl)-2,3-dihydrofuro[3,2-b]pyridin-6-yl)oxy]methyl}-3-methylazetidine-1-carboxylate (260 mg) in ethanol/H2O (4:1, 25 mL) was added potassium hydroxide (75.0 mg) under ice-cooling, and the mixture was stirred at room temperature for 2 hr. To the reaction solution was added saturated brine, and the mixture was subjected to extraction with ethyl acetate. The organic layer was dried over anhydrous magnesium sulfate, and filtered to give the title compound (250 mg) as a crude product. LC-MS;[M+H]⁺ 388.3/Rt (min) 0.921 (Measurement Condition A)

### j) Production of tert-butyl 3-({[7-(3-amino-1H-pyrazol-5-yl)-2,3-dihydrofuro[3,2-b]pyridin-6-yl]oxy}methyl)-3-methylazetidine-1-carboxylate (Reference Example 4)

To a solution of tert-butyl 3-({[7-(2-cyanoacetyl)-2,3-dihydrofuro[3,2-b]pyridin-6-yl)oxy]methyl}-3-methylazetidine-1-carboxylate (250 mg) in ethanol (10.0 mL) were added acetic acid (0.369 mL) and hydrazine monohydrate (0.314 mL) at 0°C, and the mixture was stirred under reflux for 2 hr. After allowing to cool, to the reaction solution was added saturated aqueous sodium hydrogencarbonate solution, and the mixture was subjected to extraction with chloroform. The organic layer was dried over anhydrous magnesium sulfate, and filtered to give Reference Example 4 (100 mg) as a crude product.
LC-MS;[M+H]⁺ 402.4/Rt (min) 0.720 (Measurement Condition A)

### Reference Example 5:

### 1-(6-hydroxy-2-methylbenzo[d]oxazol-7-yl)ethan-1-one

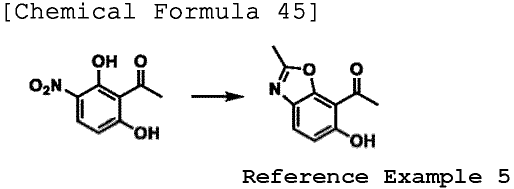

### Production of 1-(6-hydroxy-2-methylbenzo[d]oxazol-7-yl)ethan-1-one (Reference Example 5)

To a solution of 1-(2,6-dihydroxy-3-nitrophenyl)ethan-1-one (2.40 g) in ethanol (30.0 mL) was added 4.5% palladium (1.44 g) at room temperature, and the mixture was stirred under hydrogen atmosphere at room temperature for 3 hr. The reaction solution was filtered through Celite, and the filtrate was concentrated. The residue was dissolved in methanol (30.0 mL), and then trimethyl orthoacetate (3.05 mL) and hydrochloric acid (0.051 mL) were added thereto, and the mixture was stirred under reflux for 5 hr. The reaction solution was concentrated, to the residue was added water, and the mixture was subjected to extraction with chloroform. The organic layer was dried over anhydrous sodium sulfate, and filtered, and the solvent was evaporated under reduced pressure. The residue was purified by silica gel column chromatography (hexane/ethyl acetate) to give Reference Example 5 (0.550 g).
LC-MS;[M+H]⁺ 192.1/Rt (min) 0.728 (Measurement Condition A)

### Reference Example 6:

ethyl 6-(methoxymethoxy)pyrazolo[1,5-a]pyrimidine-7-carboxylate

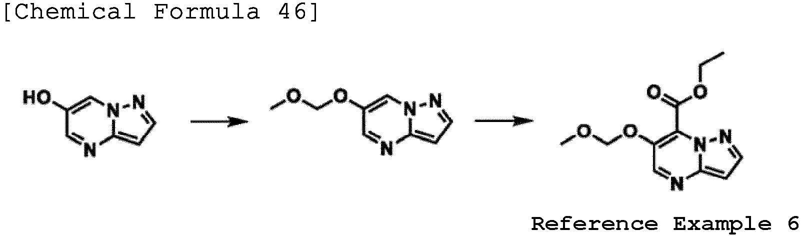

### a) Production of 6-(methoxymethoxy)pyrazolo[1,5-a]pyrimidine

To a solution of pyrazolo[1,5-a]pyrimidine-6-ol (1.50 g) and DIEA (3.83 mL) in THF (50.0 mL) was added MOMCl (1.25 mL) under ice-cooling, and the mixture was stirred at room temperature for 12 hr. To the reaction solution was added saturated aqueous sodium hydrogencarbonate solution, and the mixture was subjected to extraction with ethyl acetate. The organic layer was dried over anhydrous sodium sulfate, and filtered, and the solvent was evaporated under reduced pressure. The residue was purified by silica gel column chromatography (hexane/ethyl acetate) to give the title compound (1.10 g).
LC-MS;[M+H]⁺ 180.1/Rt (min) 0.539 (Measurement Condition A)

### b) Production of ethyl 6-(methoxymethoxy)pyrazolo[1,5-a]pyrimidine-7-carboxylate (Reference Example 6)

To a solution of diisopropylamine (87.0 uL) in THF (5.00 mL) was added dropwise n-BuLi (1.58 M, 0.389 mL) at -78°C, and the mixture was stirred at 0°C for 15 min. To the reaction solution was added dropwise a THF solution of 6-(methoxymethoxy)pyrazolo[1,5-a]pyrimidine (100 mg), and the mixture was stirred at -78°C for 30 min. Chloroethyl formate (70.0 uL) was added thereto, and the mixture was reacted for additional 2 hr. To the reaction solution was added saturated aqueous ammonium chloride solution, and the mixture was subjected to extraction with ethyl acetate. The organic layer was dried over anhydrous sodium sulfate, and filtered to give Reference Example 6 (52.0 mg).
LC-MS;[M+H]⁺ 252.2/Rt (min) 0.741 (Measurement Condition A)

### Reference Examples 7 to 32:

The compounds of Reference Examples 7 to 32 shown in the following tables were obtained by a method similar to those described in Reference Examples 1 to 5 using the corresponding raw material compounds. The mark # in the table means "Measurement Condition".

**[Table 1-1]**

| Ref. No. | Chemical Structure | LC-MS; [M+H]⁺ Retention time (min) | # |
|---|---|---|---|
| 7 | 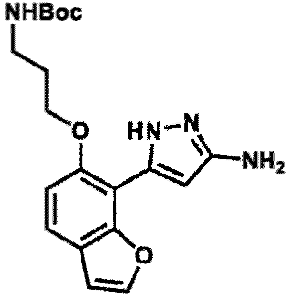 | 374.3/0.94 1 | A |
| 8 | 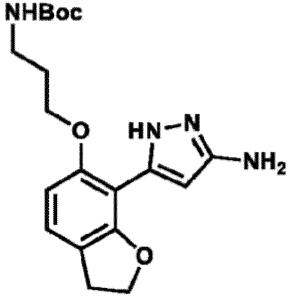 | 375.3/0.71 7 | A |
| 9 | 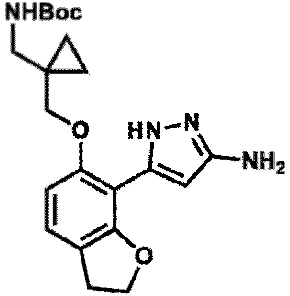 | 401.0/0.77 5 | A |

**[Table 1-2]**

| | | | |
|---|---|---|---|
| 10 | 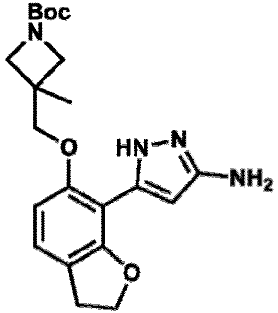 | 402.4/0.88 7 | A |
| 11 | 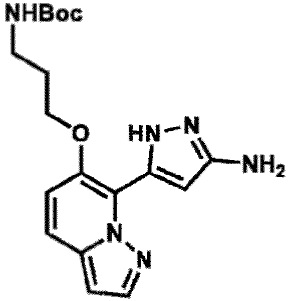 | 373.3/0.76 3 | A |
| 12 | 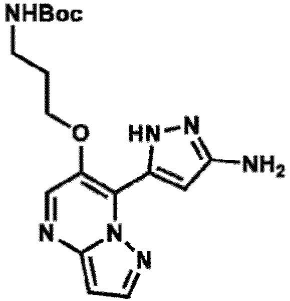 | 374.3/0.75 0 | A |
| 13 | 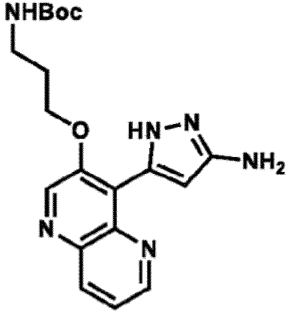 | 385.3/0.64 8 | A |

**[Table 1-3]**

| | | | |
|---|---|---|---|
| 14 | 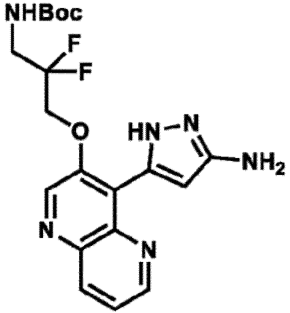 | 421.4/0.7 7 2 | A |
| 15 | 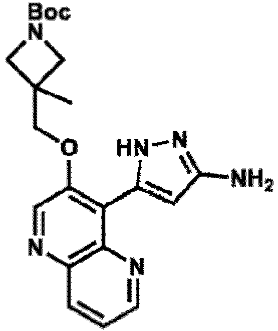 | 411.4/0.74 0 | A |
| 16 | 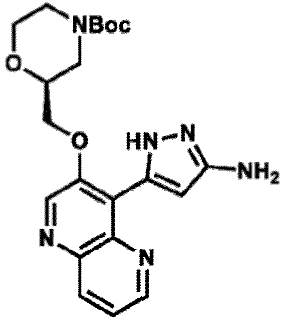 | 427.4/0.75 5 | A |

**[Table 1-4]**

| | | | |
|---|---|---|---|
| 17 | 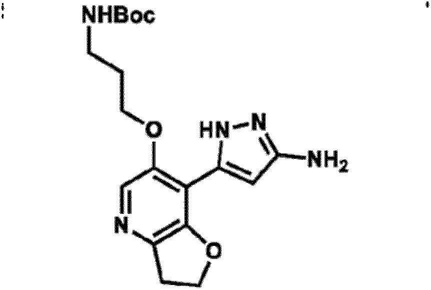 | 376.3/0.62 8 | A |
| 18 | 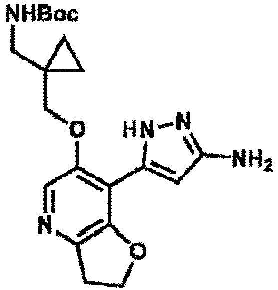 | 40.2.4/0.67 4 | A |
| 19 | 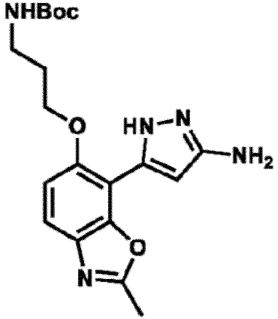 | 388.3/0.73 7 | A |
| 20 | 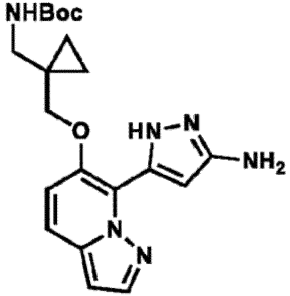 | 399.3/0.81 5 | A |

**[Table 1-5]**

| | | | |
|---|---|---|---|
| 21 | 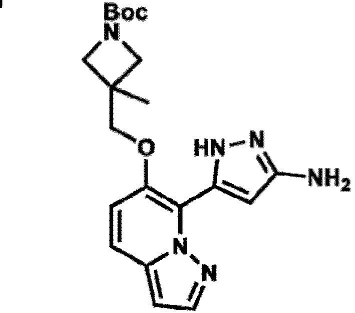 | 399.3/0.87 0. | A |
| 22 | 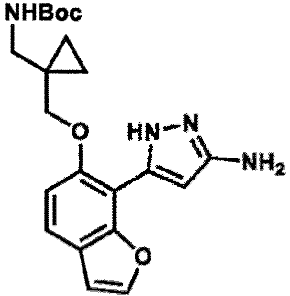 | 399.3/1.13 6 | A |
| 23 | 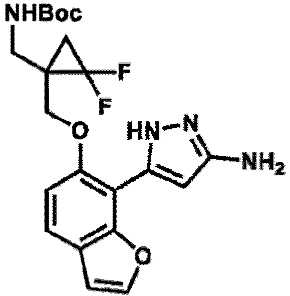 | 435.2/1.14 8 | A |
| 24 | 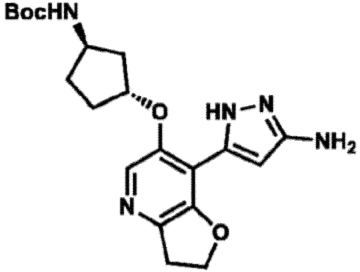 | 40.2/1.254 | B |

**[Table 1-6]**

| | | | |
|---|---|---|---|
| 25 | 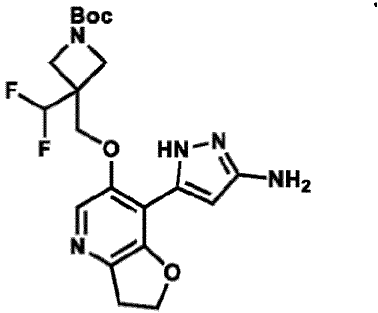 | 438/1.453 | B |
| 26 | 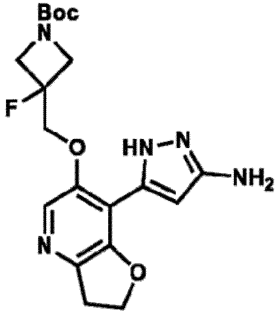 | 406/1.374 | B |
| 27 | 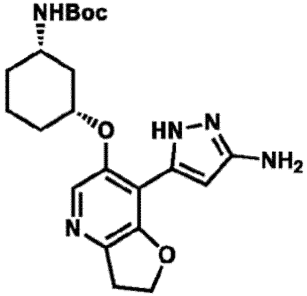 | 416/1.479 | B |
| 28 | 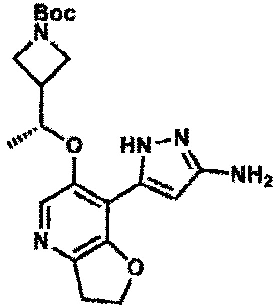 | 402/1.362 | B |

**[Table 1-7]**

| | | | |
|---|---|---|---|
| 29 | 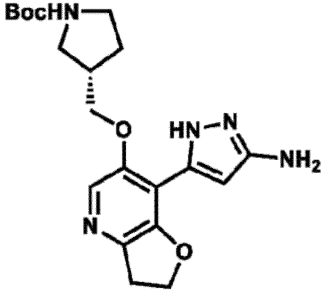 | 402/1.386 | B |
| 30 | 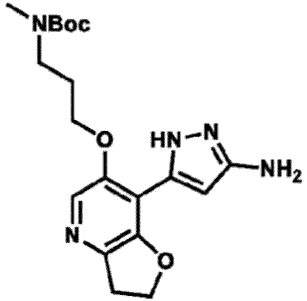 | 390/1.231 | B |
| 31 | 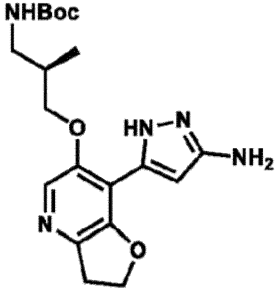 | 390/1.308 | B |
| 32 | 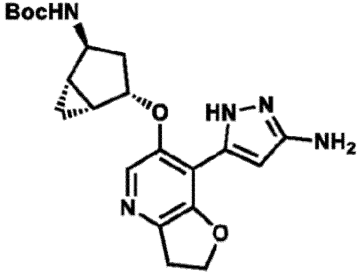 | 414/1.355 | B |

**[Table 1-8]**

| | | | |
|---|---|---|---|
| 33 | 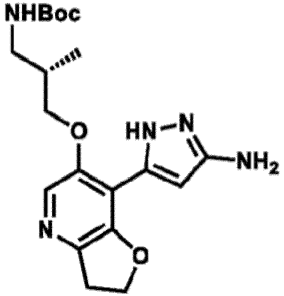 | 390/1.294 | B |
| 34 | 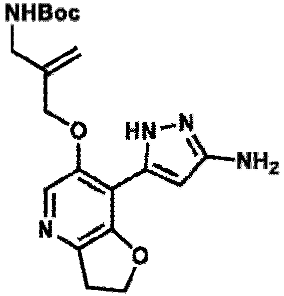 | 388/1.230 | B |
| 35 | 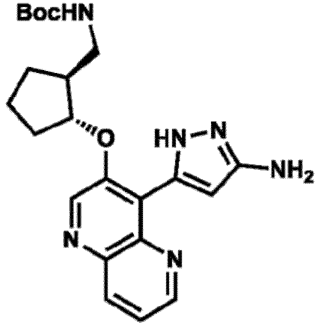 | 411.2/1.0.0 6 | A |

**[Table 1-9]**

| | | | |
|---|---|---|---|
| 36 | 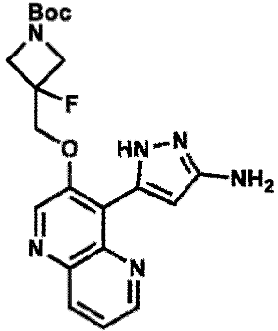 | 416.2/0.87 | A |
| 37 | 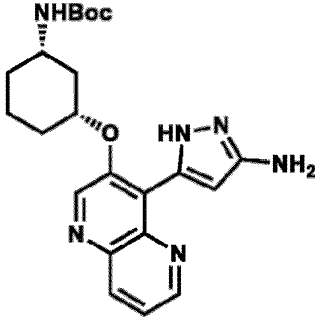 | 425.3/0.9 7 8 | A |
| 38 | 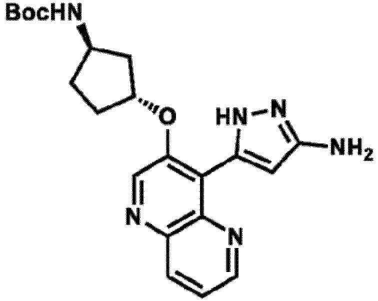 | 411.3/0.94 1 | A |

**[Table 1-10]**

| | | | |
|---|---|---|---|
| 39 | 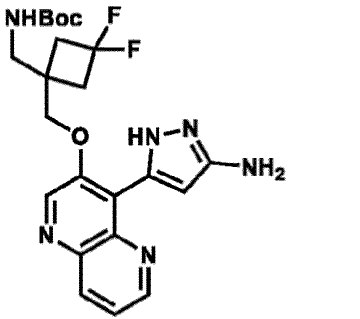 | 461.3/1.09 6 | A |

### Reference Example 20:

### tert-butyl {3-[(8-{3-[(5-cyanopyrazin-2-yl)amino]-1H-pyrazol-5-yl}quinolin-7-yl)oxy]propyl}carbamate

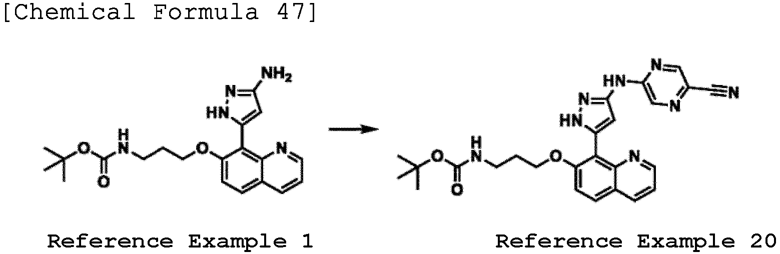

### Production of tert-butyl {3-[(8-{3-[(5-cyanopyrazin-2-yl)amino]-1H-pyrazol-5-yl}quinolin-7-yl)oxy]propyl}carbamate (Reference Example 20)

To a solution of Reference Example 1 (219 mg) in DMSO (2.50 mL) were added 4-ethylmorpholine (0.108 mL) and 5-chloropyrazine-2-carbonitrile (96.0 mg) at room temperature, and the mixture was stirred at 80°C for 2 hr. The reaction solution was ice-cooled, ice-cooled methanol (7.50 mL) was added thereto, and the mixture was stirred at 0°C for additional 1 hr. The resulting precipitate was collected by filtration, and washed with ice-cooled methanol, and the obtained solid was dried to give Reference Example 20 (81.6 mg).
LC-MS;[M+H]⁺ 487.4/Rt (min) 0.891

### Reference Example 21:

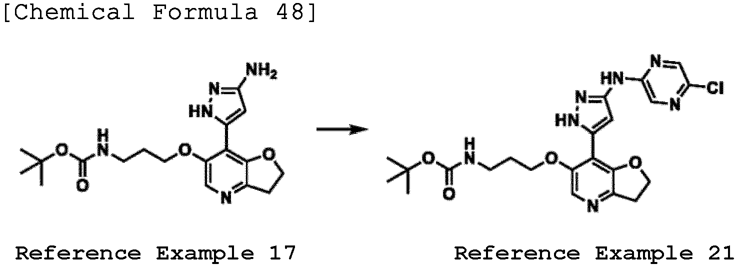

### Production of (Reference Example 21)

To a solution of Reference Example 17 (600 mg) in 1,4-dioxane (10.0 mL) were added 2,5-dichloropyrazine (319 µL), tris(dibenzylideneacetone)dipalladium(0) (146 mg), 4,5-bis(diphenylphosphino)-9,9-dimethylxanthene (185 mg) and cesium carbonate (1041 mg) at room temperature, and the mixture was stirred under microwave irradiation at 150°C for 2 hr. To the reaction solution was added water, and the mixture was subjected to extraction with ethyl acetate. The organic layer was washed with saturated brine, dried over anhydrous sodium sulfate, and filtered, and the solvent was evaporated under reduced pressure. The residue was purified by amino silica gel column chromatography (hexane/ethyl acetate) to give the title compound as a crude product.
LC-MS;[M+H]⁺ 488.2/Rt (min) 1.022 (Measurement Condition A)

### Reference Examples 22 to 57:

The compounds of Reference Examples 22 to 57 shown in the following tables were obtained by a method similar to that described in Reference Example 20 using the corresponding raw material compounds. The mark # in the table means "Measurement Condition".

**[Table 2-1]**

| Ref. No. | Chemical Structure | LC-MS; [M+H]⁺ Retention time (min) | # |
|---|---|---|---|
| 22 | 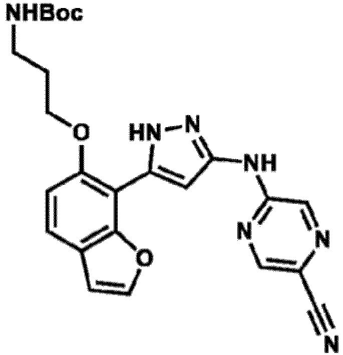 | 476.3/1.0 38 | A |
| 23 | 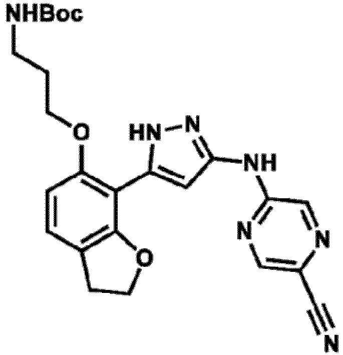 | 478.3/1.0 79 | A |
| 24 | 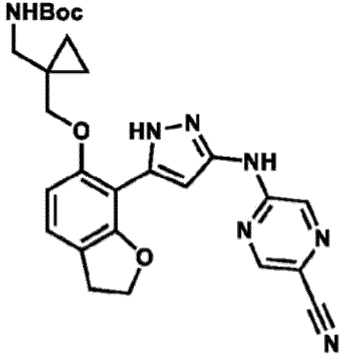 | 504.4/1.0 45 | A |

**[Table 2-2]**

| | | | |
|---|---|---|---|
| 25 | 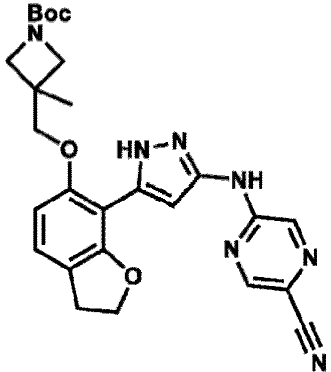 | 504.4/1.0 71 | A |
| 26 | 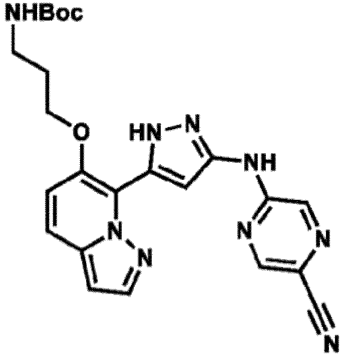 | 476.4/1.0 11 | A |
| 27 | 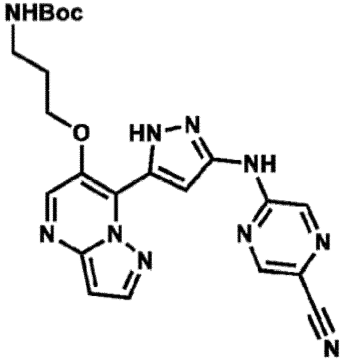 | 477.4/0.9 26 | A |

**[Table 2-3]**

| | | | |
|---|---|---|---|
| 28 | 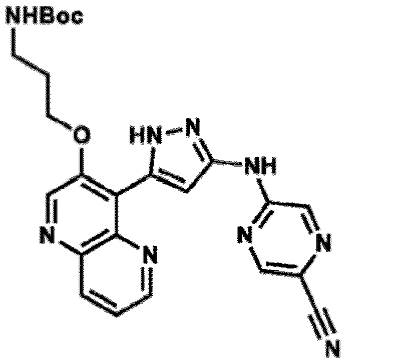 | 488.4/0.9 12 | A |
| 29 | 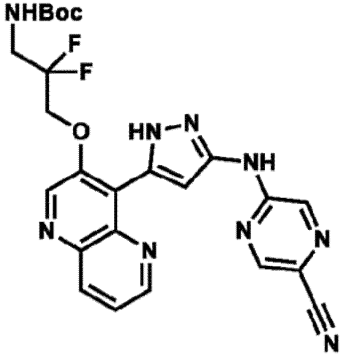 | 524.4/0.9 12 | A |
| 30 | 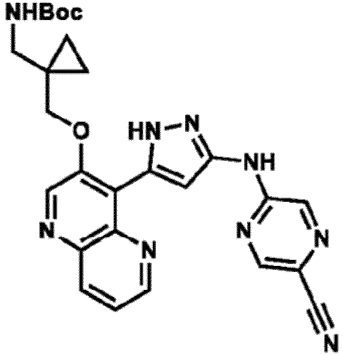 | 514.4/0.9 46 | A |

**[Table 2-4]**

| | | | |
|---|---|---|---|
| 31 | 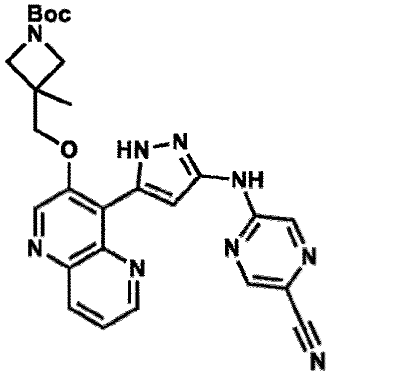 | 514.4/0.9 75 | A |
| 32 | 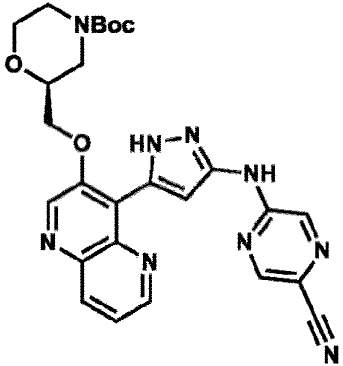 | 530.4/4.9 36 | A |
| 33 | 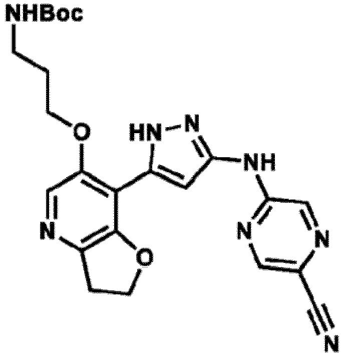 | 479.4/0.8 29 | A |

**[Table 2-5]**

| | | | |
|---|---|---|---|
| 31 | 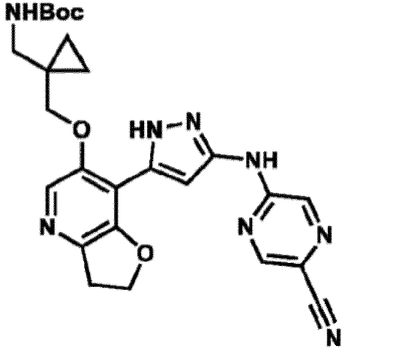 | 505.4/0.8 67 | A |
| 35 | 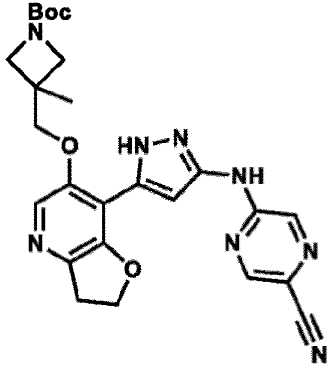 | 505.4/0.8 97 | A |
| 36 | 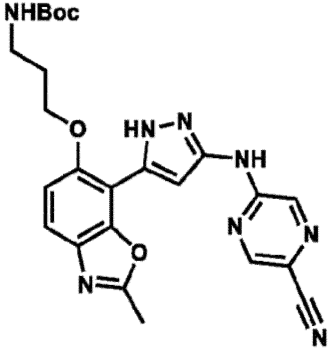 | 491.4/0.9 17 | A |

**[Table 2-6]**

| | | | |
|---|---|---|---|
| 37 | 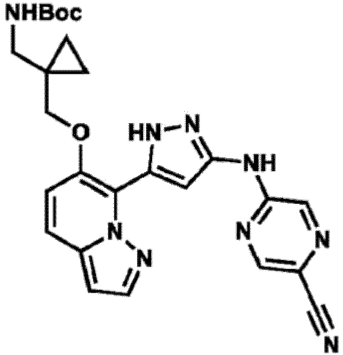 | 502.3/1.0 06 | A |
| 38 | 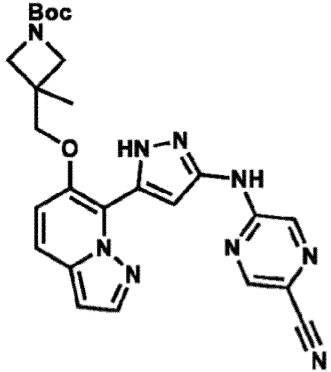 | 502.3/1.0 88 | A |
| 39 | 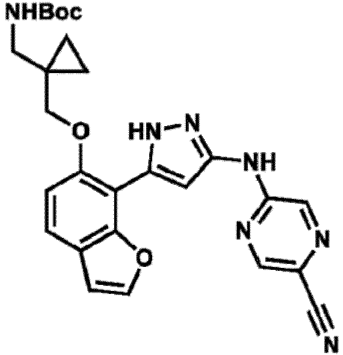 | 502.4/1.3 68 | A |

**[Table 2-7]**

| | | | |
|---|---|---|---|
| 40 | 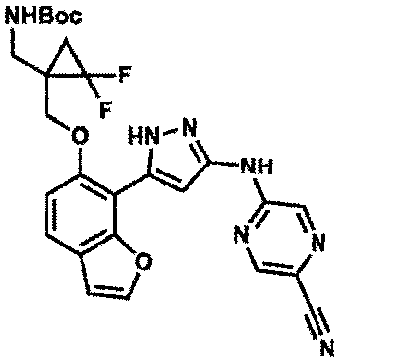 | 538.2/1.3 58 | A |
| 41 | 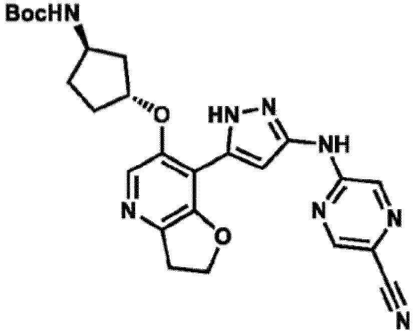 | 505.4/0.8 75 | A |
| 42 | 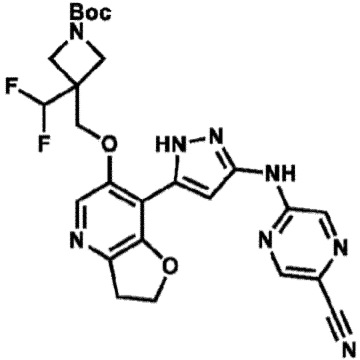 | 541.3/0.9 15 | A |

**[Table 2-8]**

| | | | |
|---|---|---|---|
| 43 | 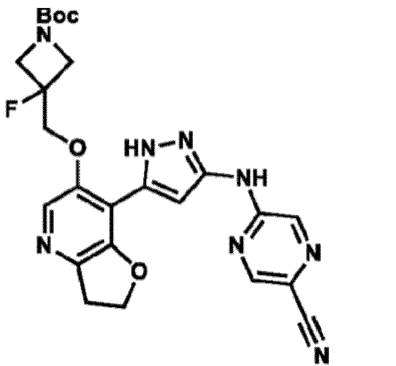 | 509.2/0.8 83 | A |
| 44 | 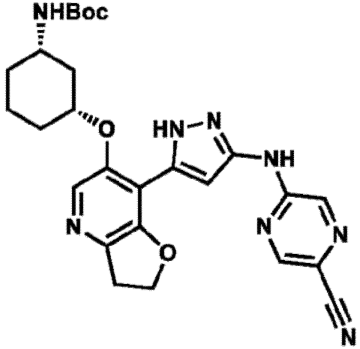 | 519.3/0.9 28 | A |
| 45 | 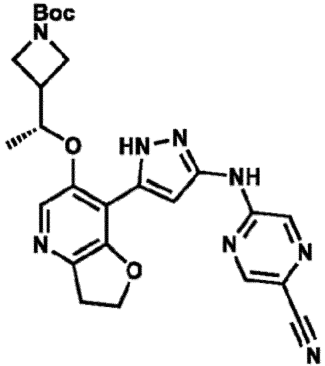 | 505.3/0.8 97 | A |

**[Table 2-9]**

| | | | |
|---|---|---|---|
| 46 | 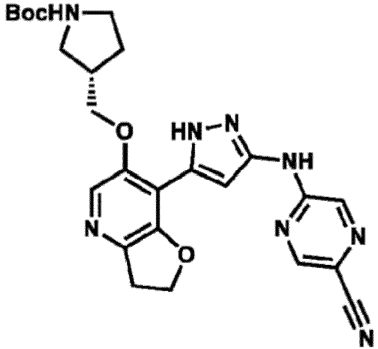 | 505.3/0.9 14 | A |
| 47 | 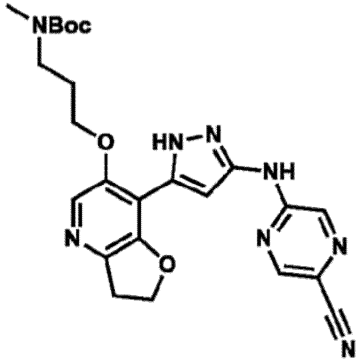 | 493.3/0.8 83 | A |
| 48 | 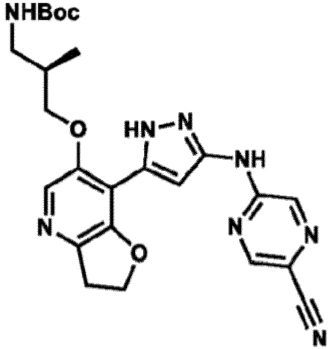 | 493.3/0.8 80 | A |

**[Table 2-10]**

| | | | |
|---|---|---|---|
| 49 | 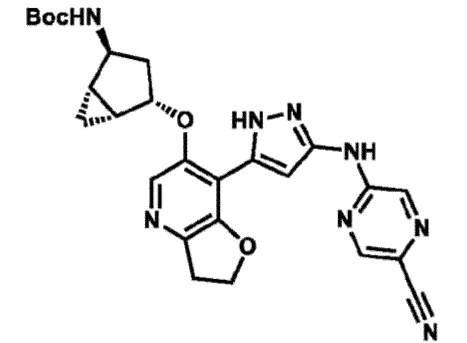 | 517.3/0.9 03 | A |
| 50 | 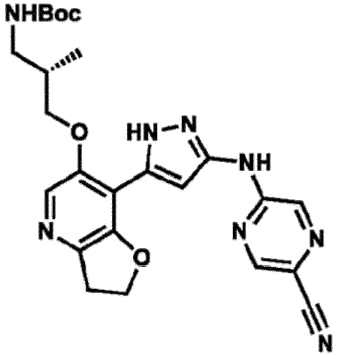 | 493.3/0.8 83 | A |
| 51 | 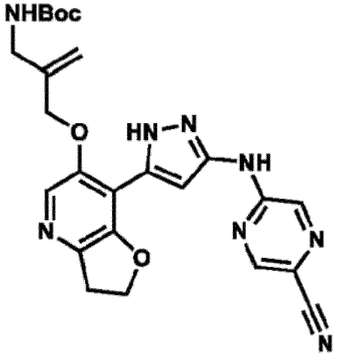 | 491.4/0.8 57 | A |

**[Table 2-11]**

| | | | |
|---|---|---|---|
| 52 | 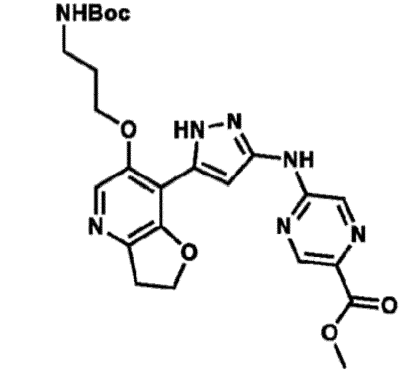 | 512.2/0.9 53 | A |
| 53 | 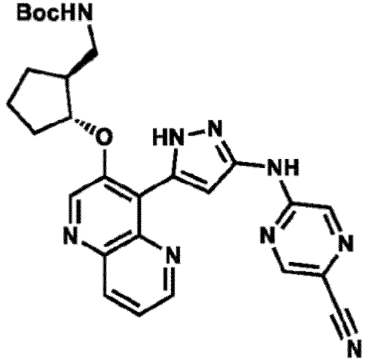 | 528.3/1.1 38 | A |
| 54 | 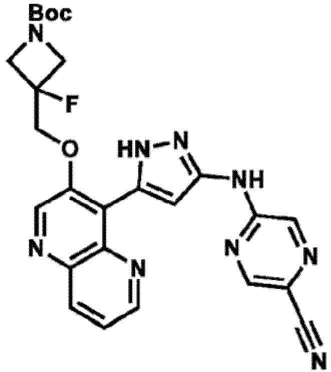 | 518.2/0.8 19 | A |

**[Table 2-12]**

| | | | |
|---|---|---|---|
| 55 | 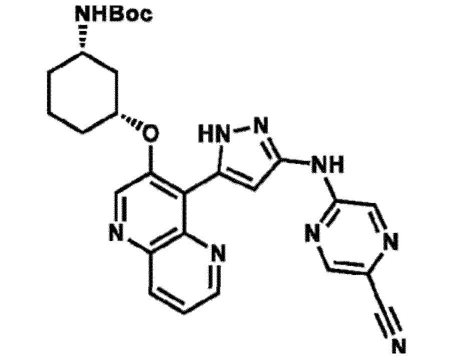 | 528.3/1.1 99 | A |
| 56 | 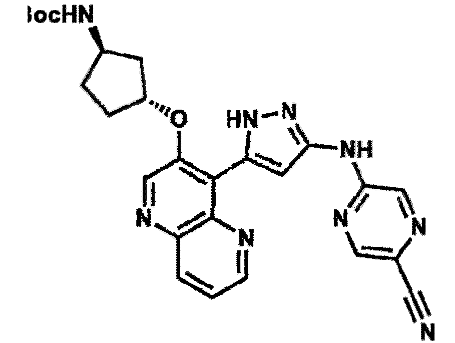 | 514.3/1.1 66 | A |
| 57 | 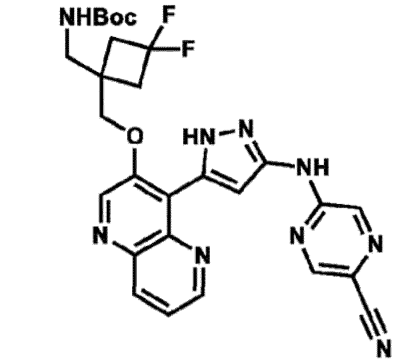 | 564.3/0.9 68 | A |

### Example 1:

### 5-({5-[7-(3-aminopropoxy)quinolin-8-yl]-1H-pyrazol-3-yl}amino)pyrazine-2-carbonitrile

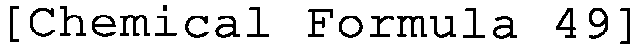

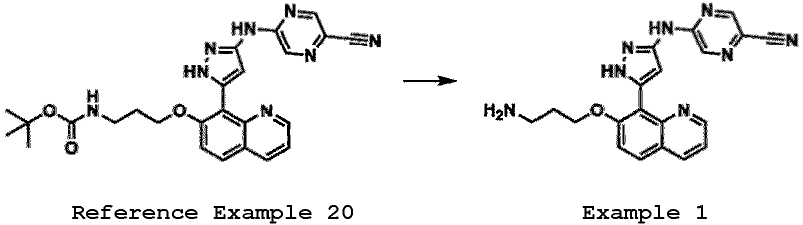

tert-Butyl {3-[(8-{3-[(5-cyanopyrazin-2-yl)amino]-1H-pyrazol-5-yl}quinolin-7-yl)oxy]propyl}carbamate (79.4 mg) obtained in Reference Example 20 was dissolved in formic acid (3.00 mL), and the solution was stirred at room temperature for 2 hr. The solvent was evaporated under reduced pressure, the residue was suspended in ethyl acetate, and the resulting precipitate was collected by filtration. The solid collected by filtration was suspended in ethanol/water (2:1, 6 mL), and the solution was heated to 90°C. 2M aqueous tripotassium phosphate solution (0.10 mL) was added thereto, and the mixture was stirred under ice-cooled at 0°C for 30 min. The resulting solid was collected by filtration, and washed with water, ice-cooled ethanol and diethyl ether, and the obtained solid was dried to give Example 1 (51.8 mg).
LC-MS;[M+H]⁺ 387.2/Rt (min) 0.552

¹H-NMR (DMSO-D6) δ: 8.96 (1H, dd, J = 4.3, 1.8 Hz), 8.68 (1H, d, J=1.2 Hz), 8.51 (1H, br s), 8.40 (1H, dd, J = 8.5, 1.8 Hz), 8.02 (1H, d, J =9.1Hz), 7.69 (1H, d, J = 9.1 Hz), 7.48 (1H, dd, J = 8.5, 4.3 Hz), 7.41 (1H, brs),4.36 (2H, t,J = 6.1 Hz), 2.83 (2H, t, J = 6.4 Hz), 1.97-1.91 (2H, m).

### Examples 2 to 38:

The compounds of Examples 2 to 38 shown in the following tables were obtained by a method similar to that described in Example 1 using the corresponding raw material compounds. The mark # in the table means "Measurement Condition".

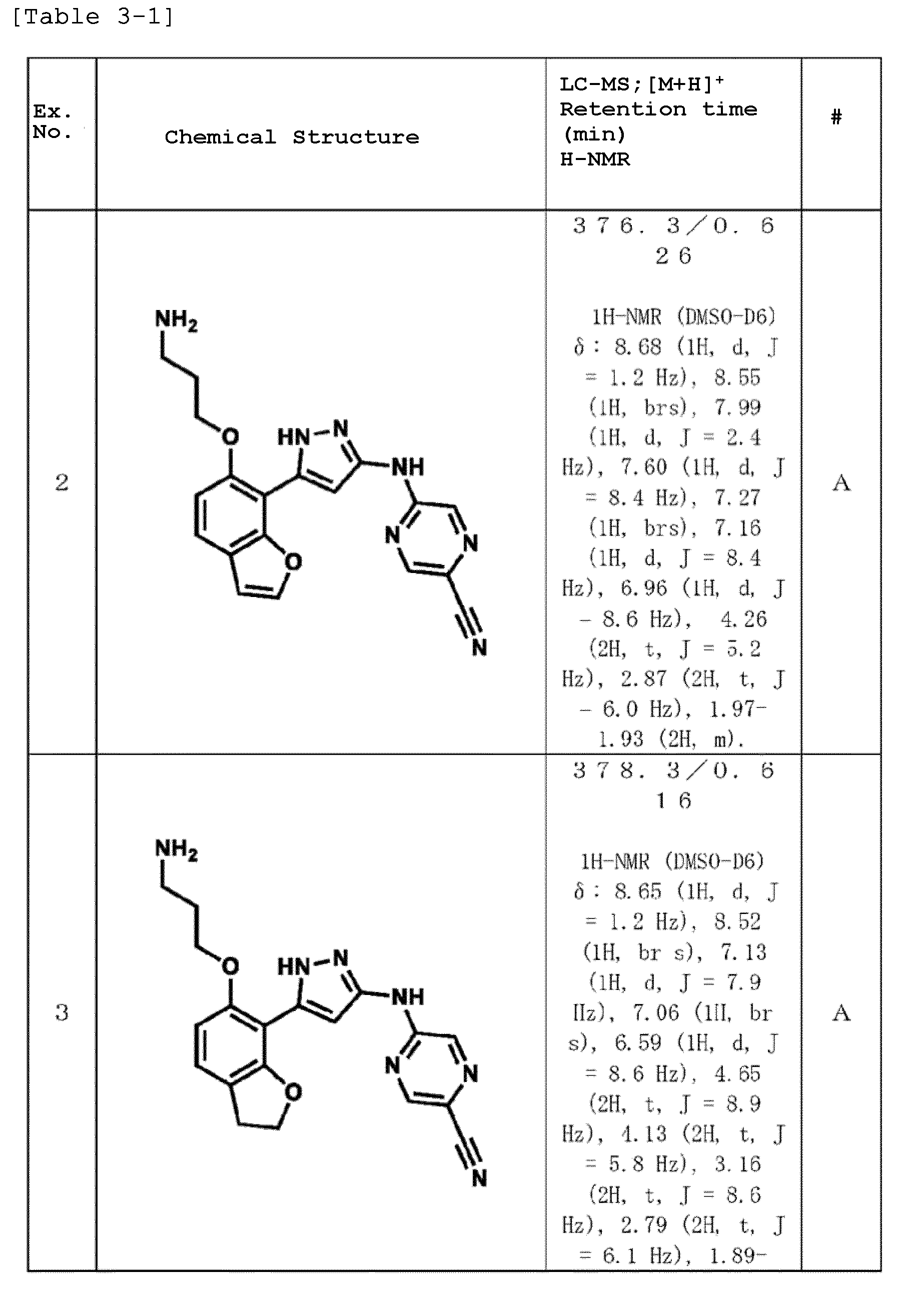

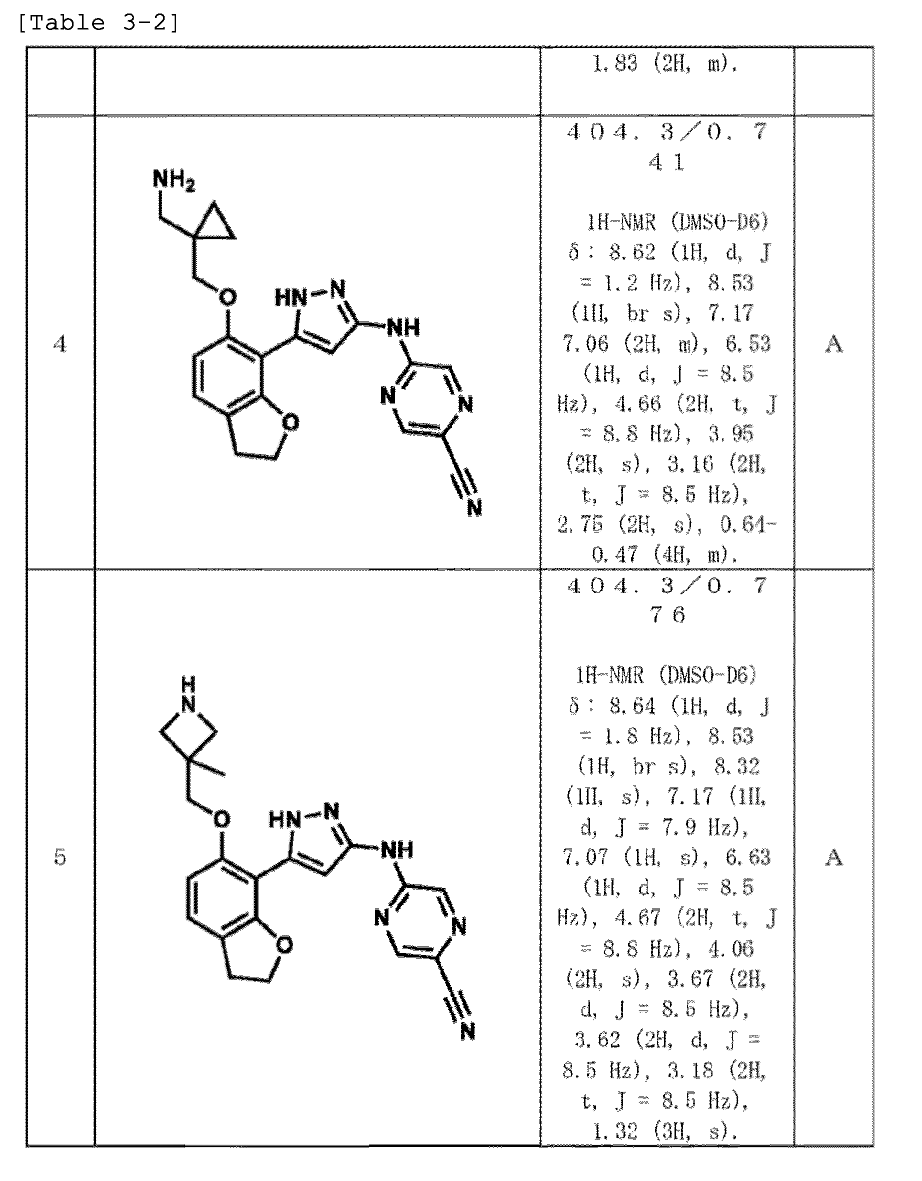

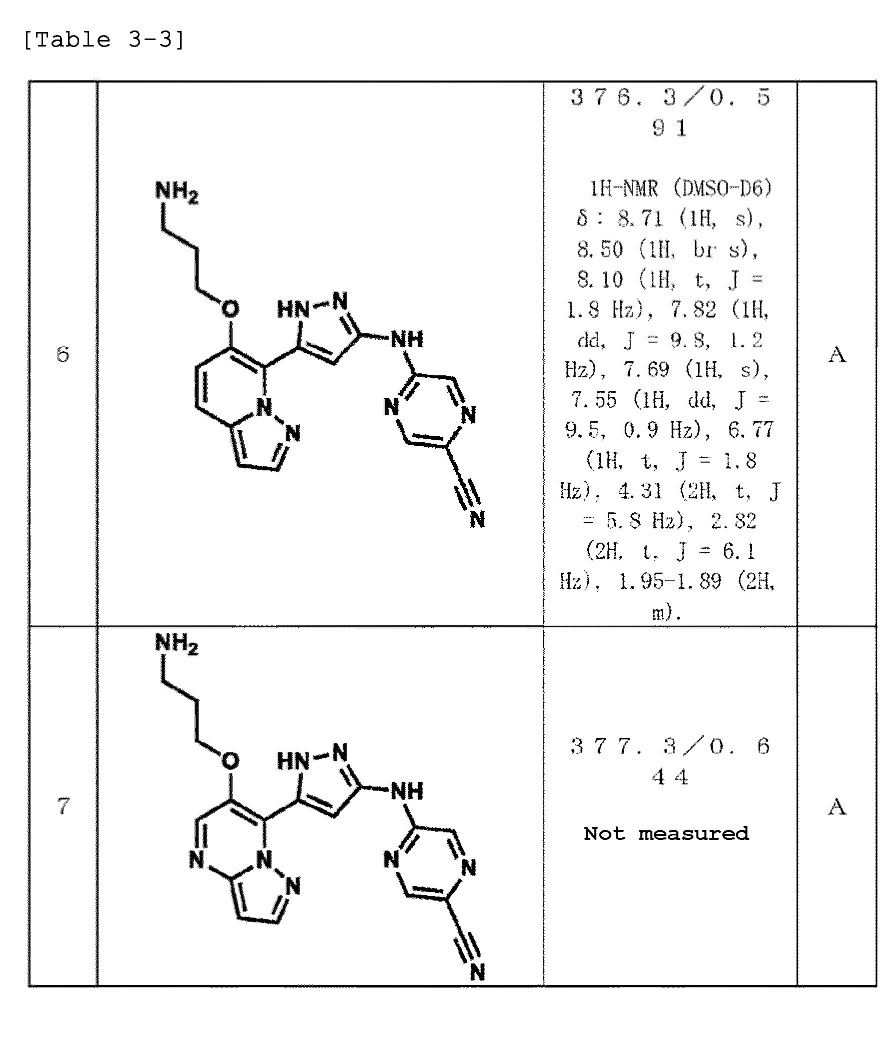

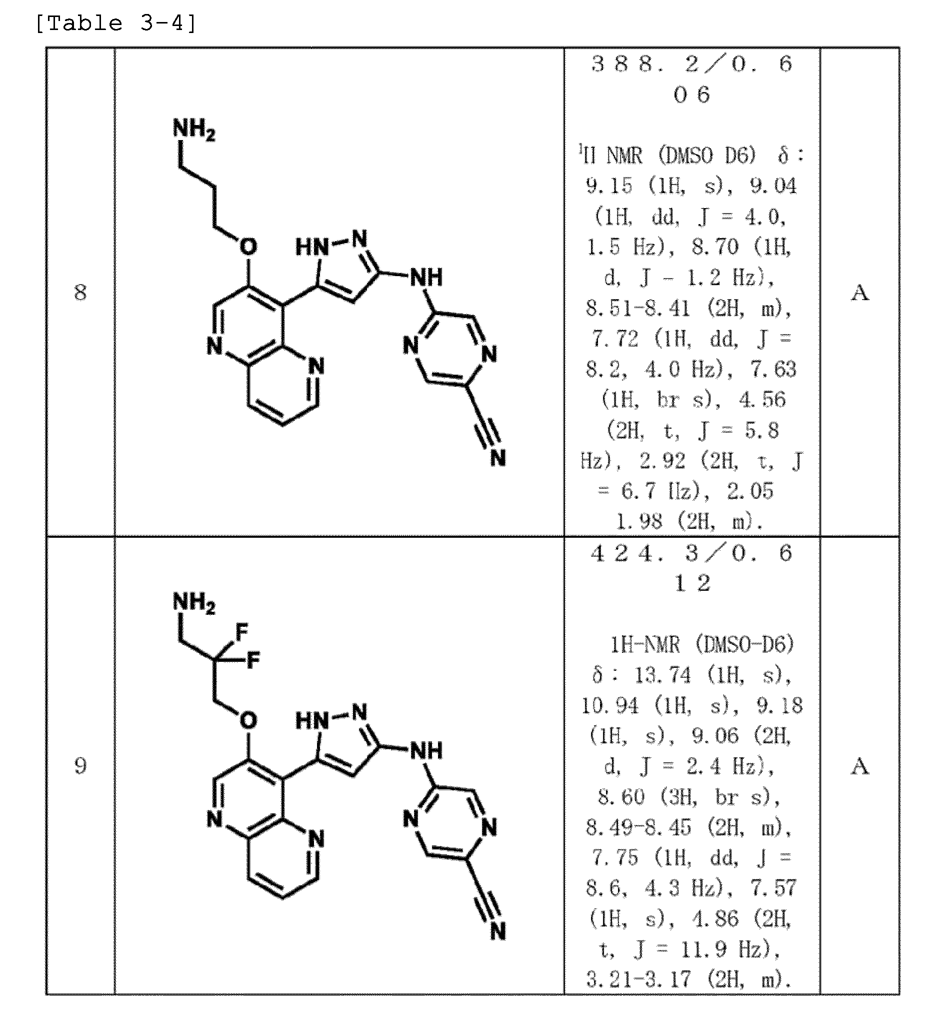

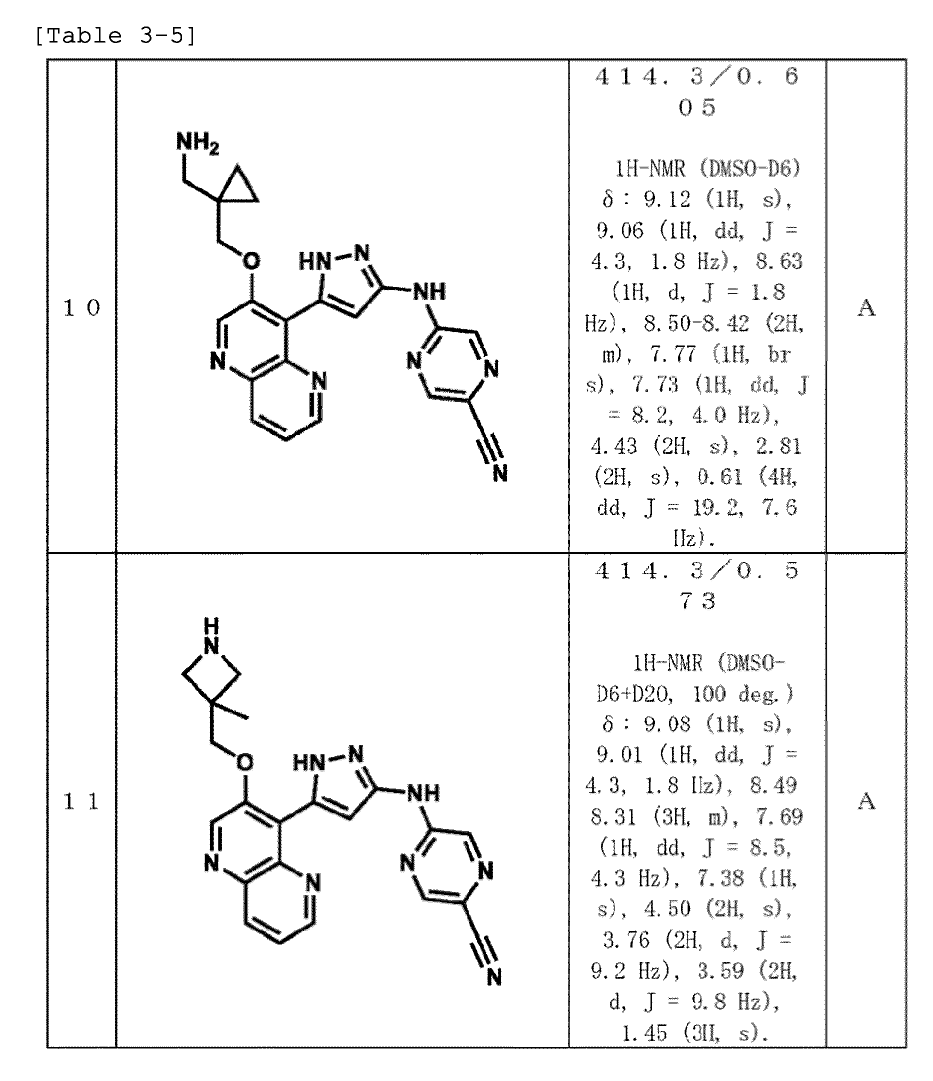

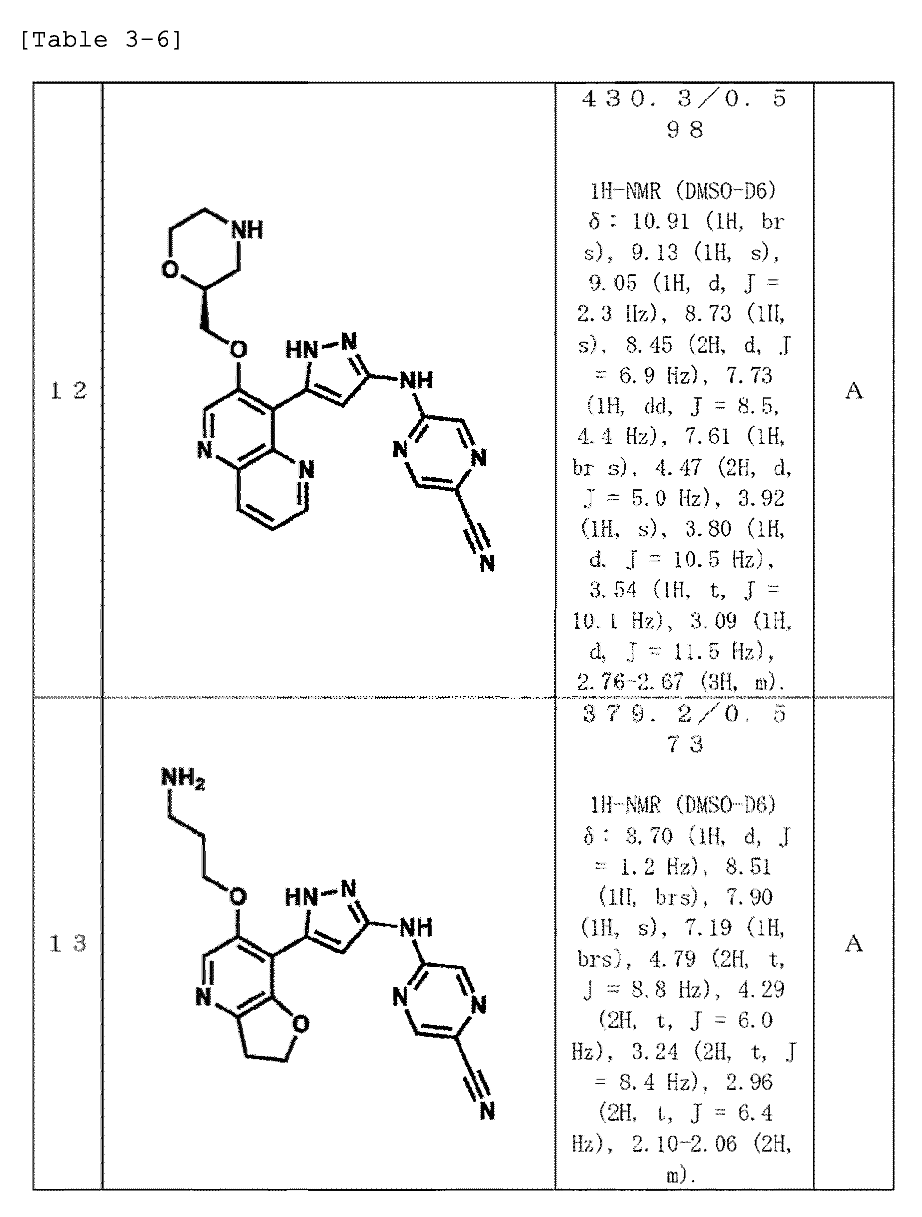

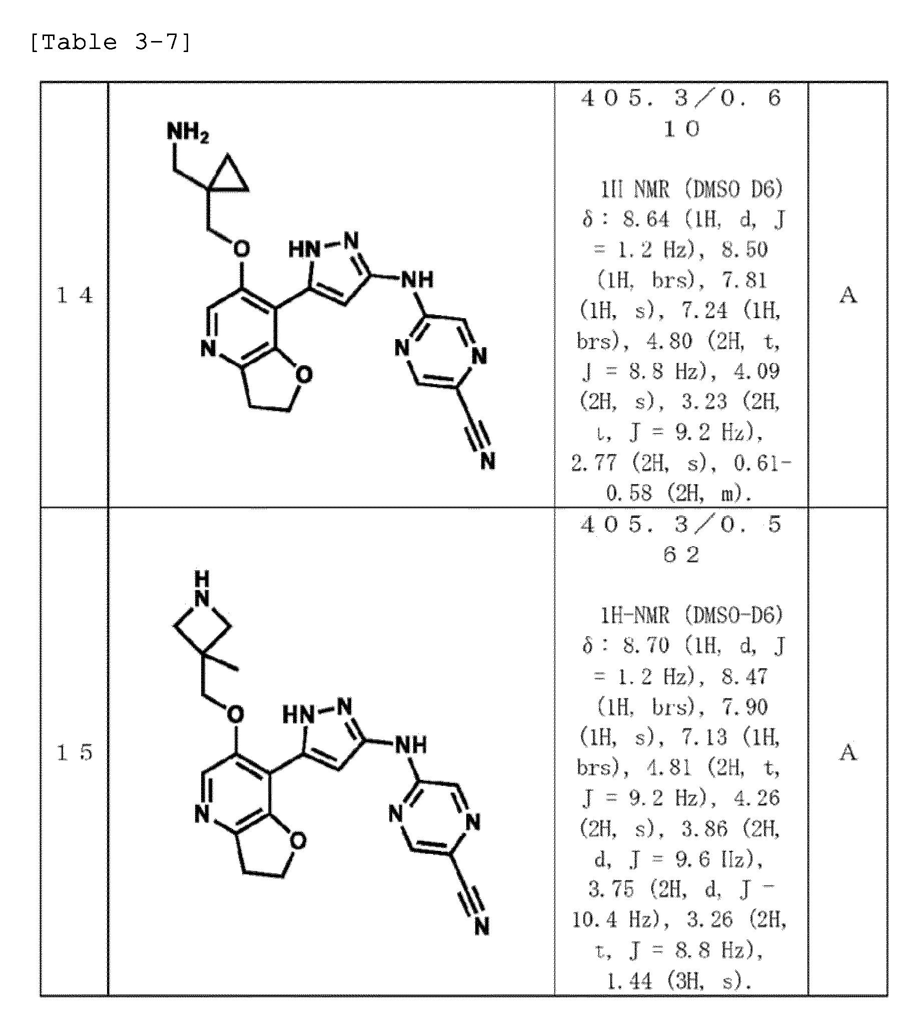

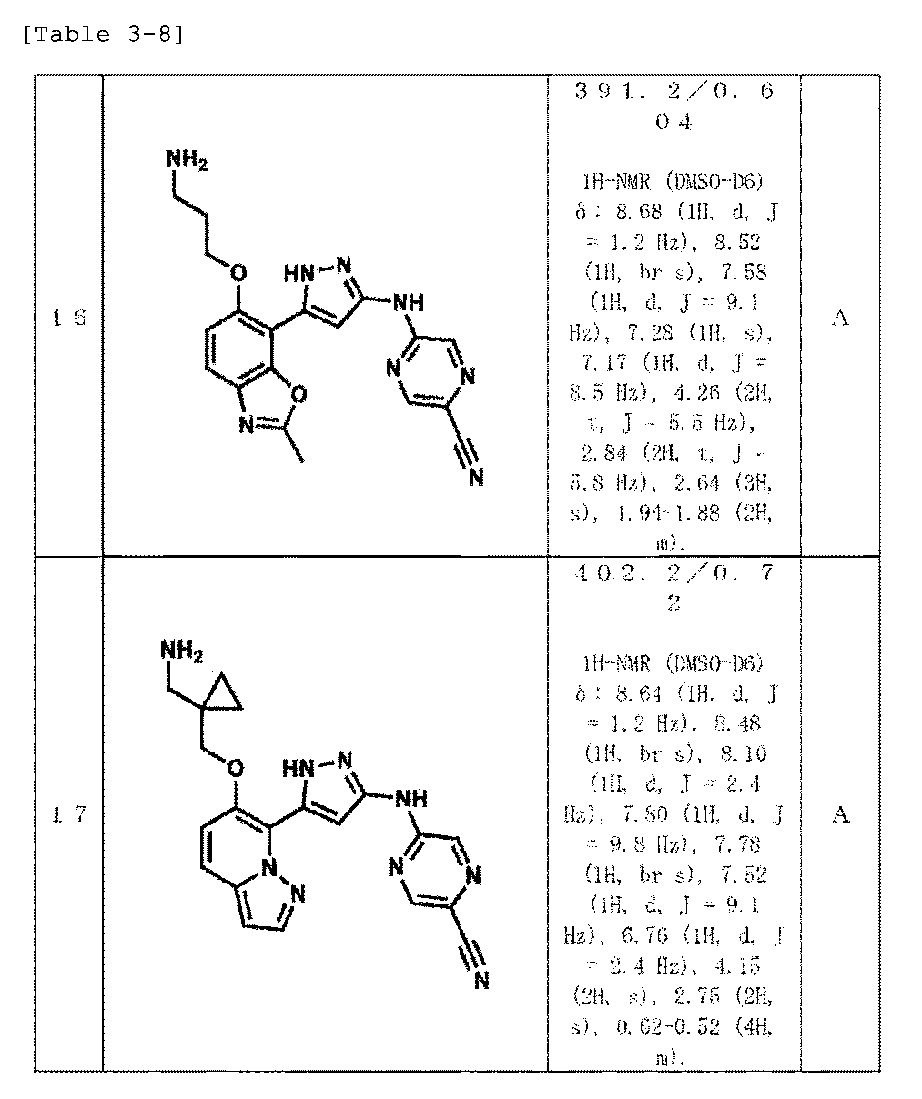

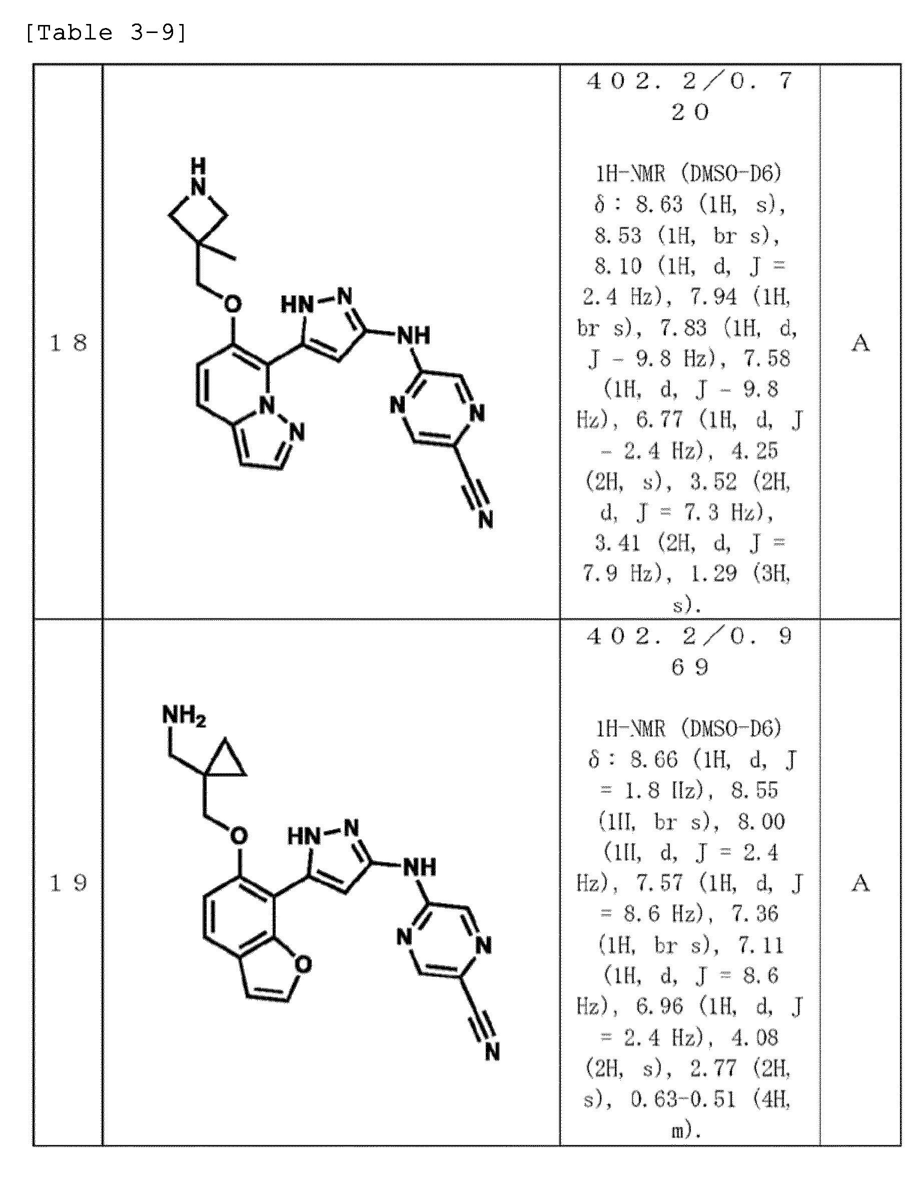

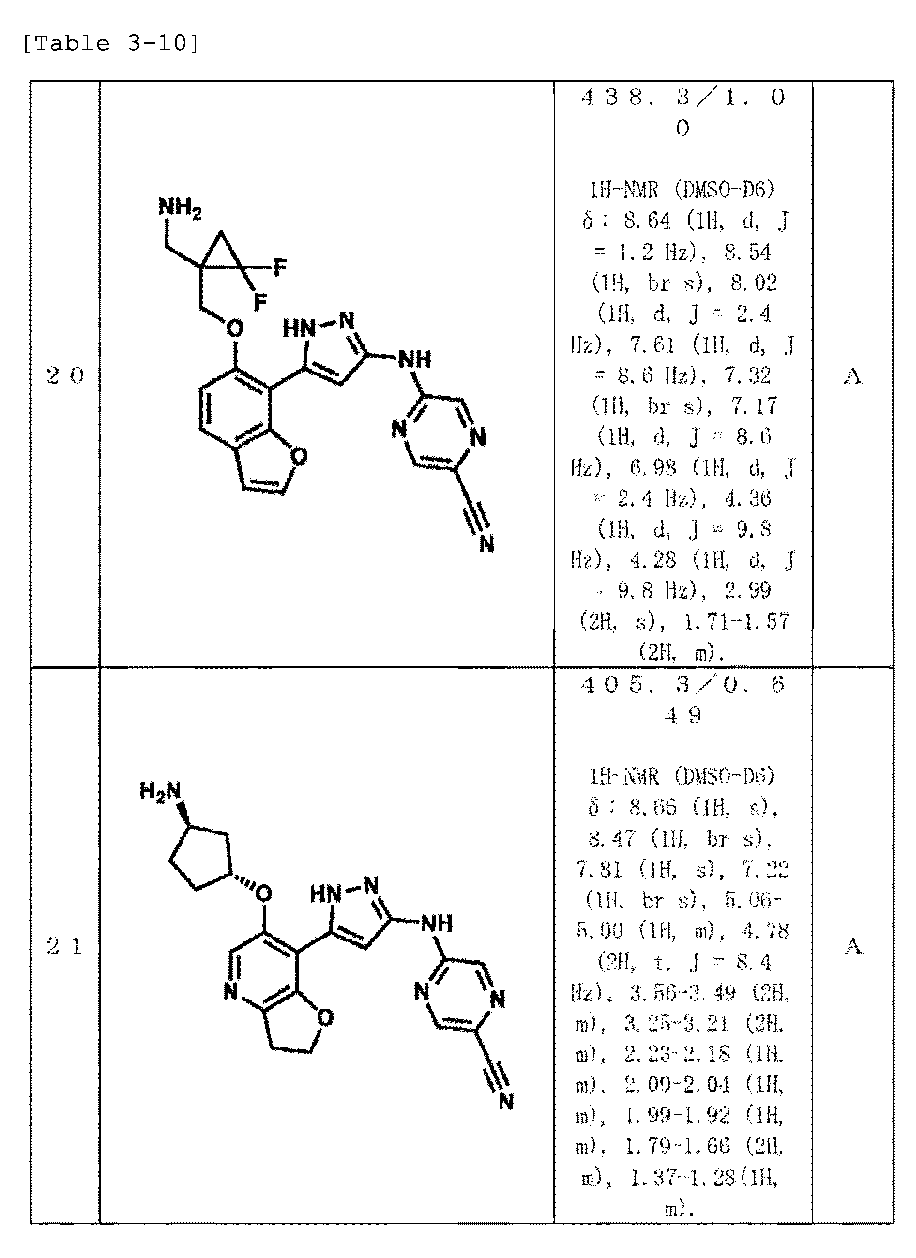

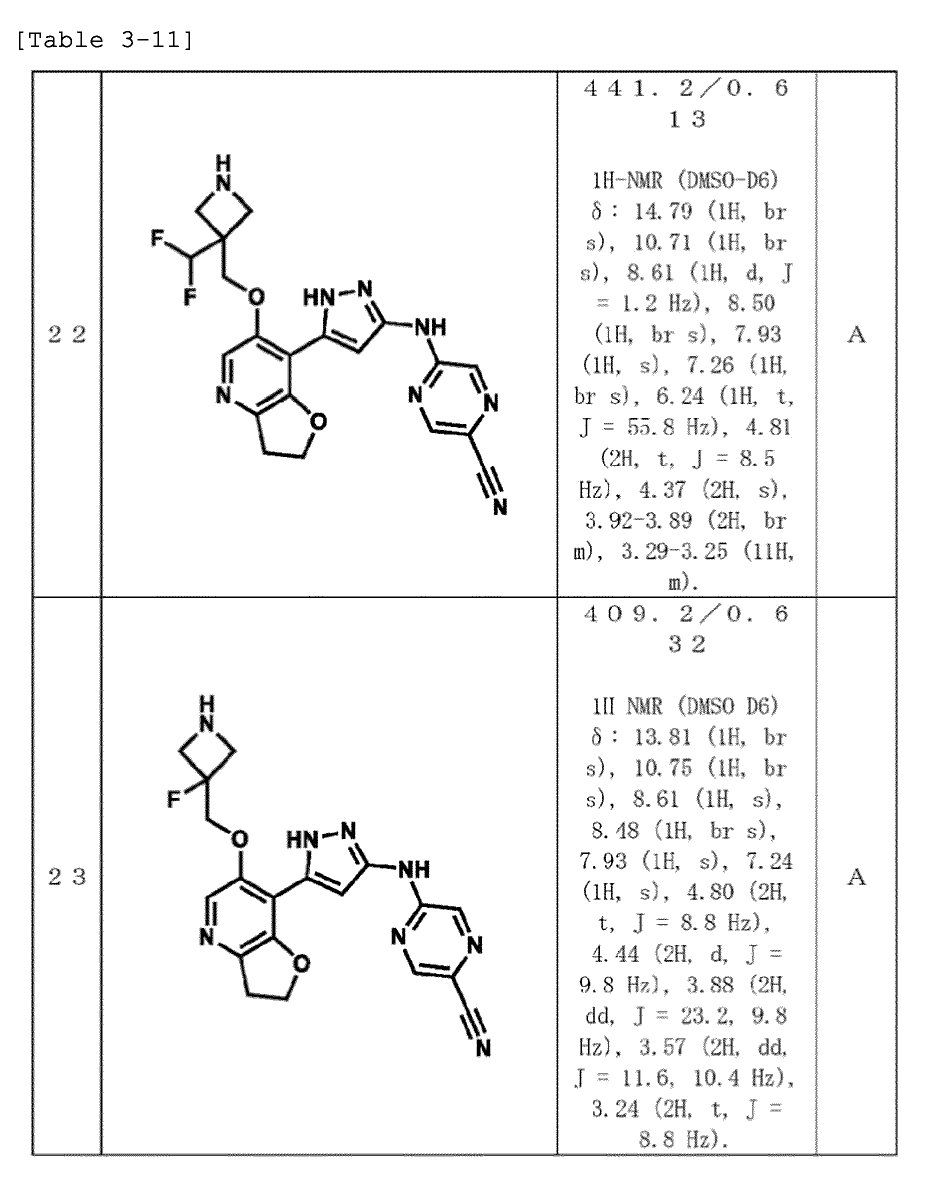

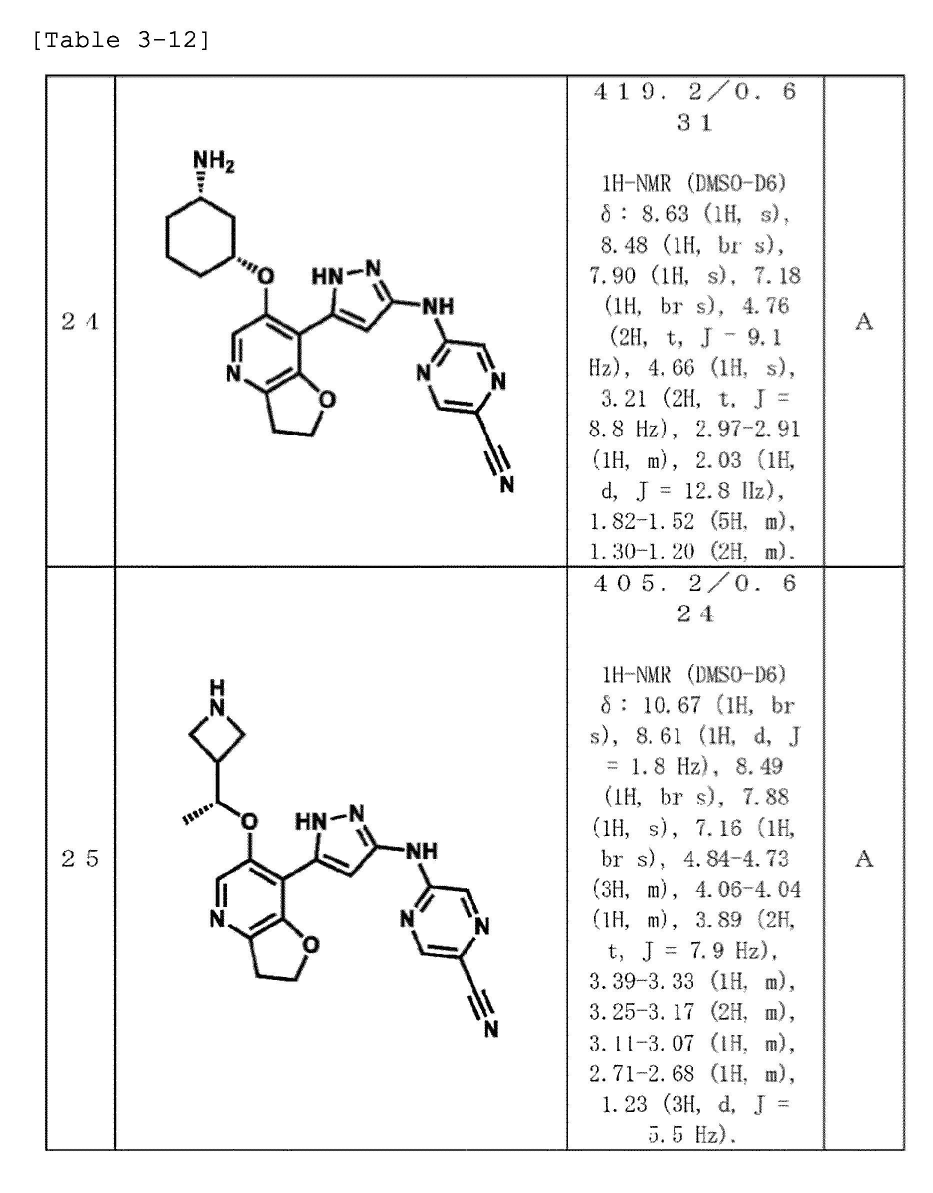

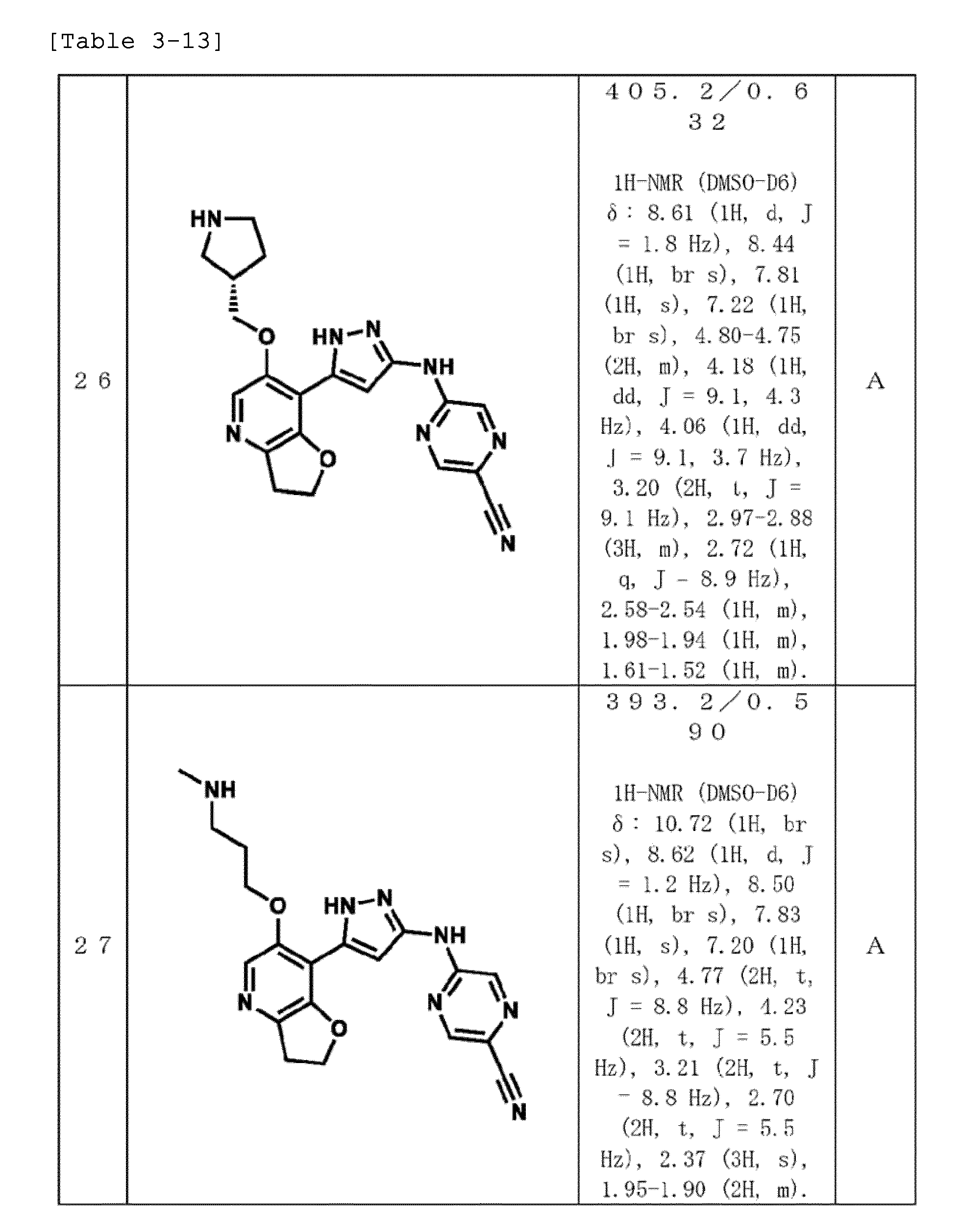

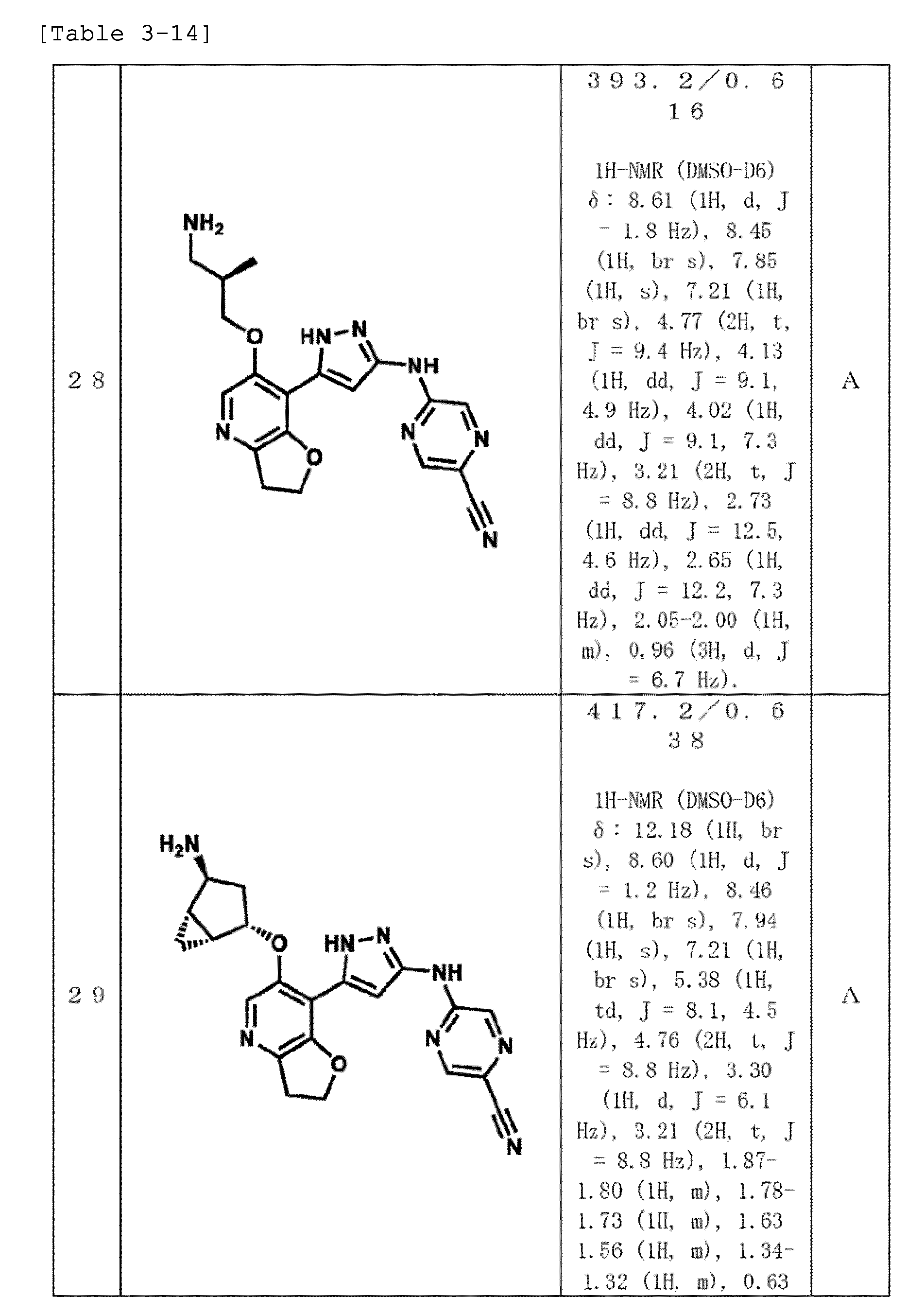

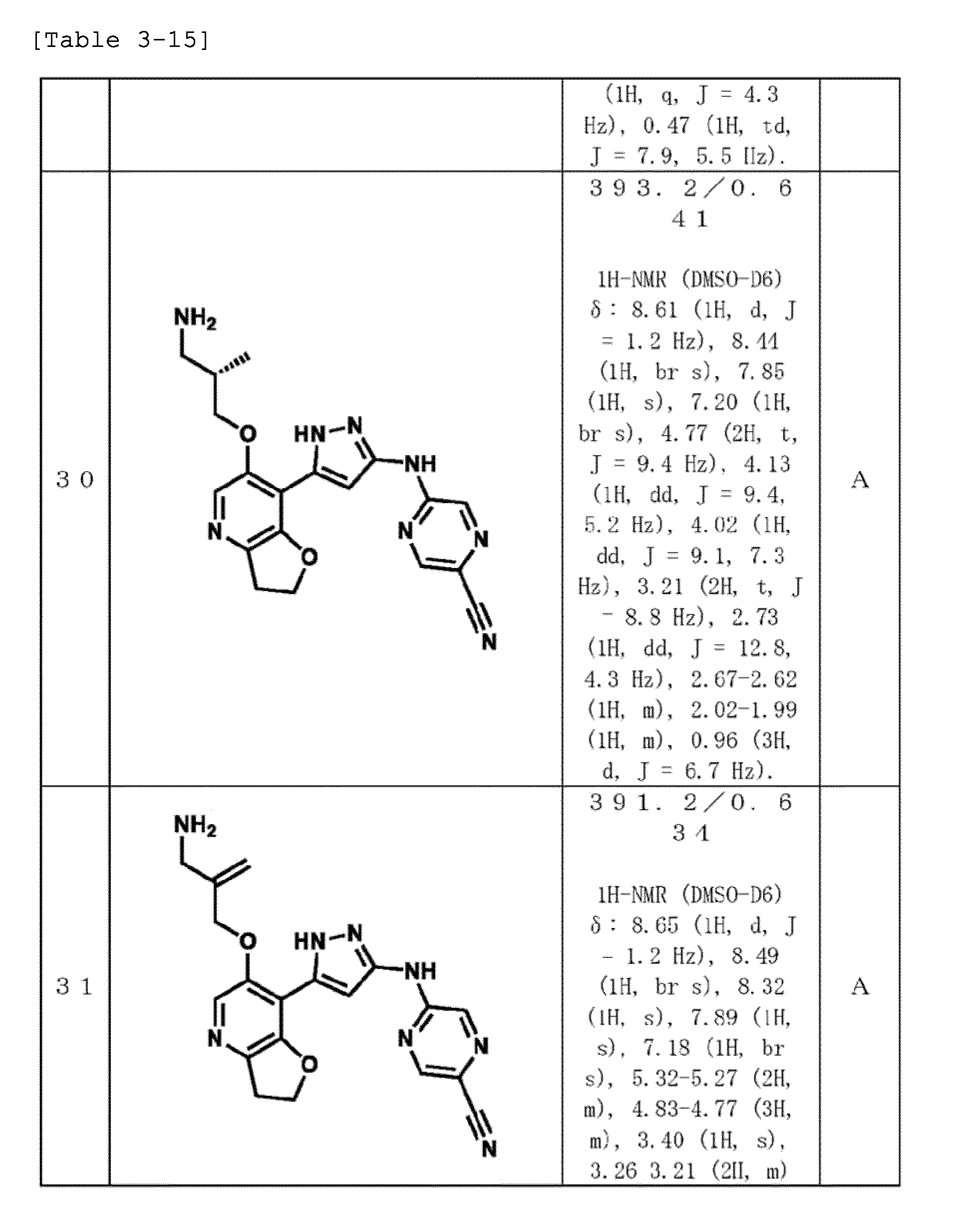

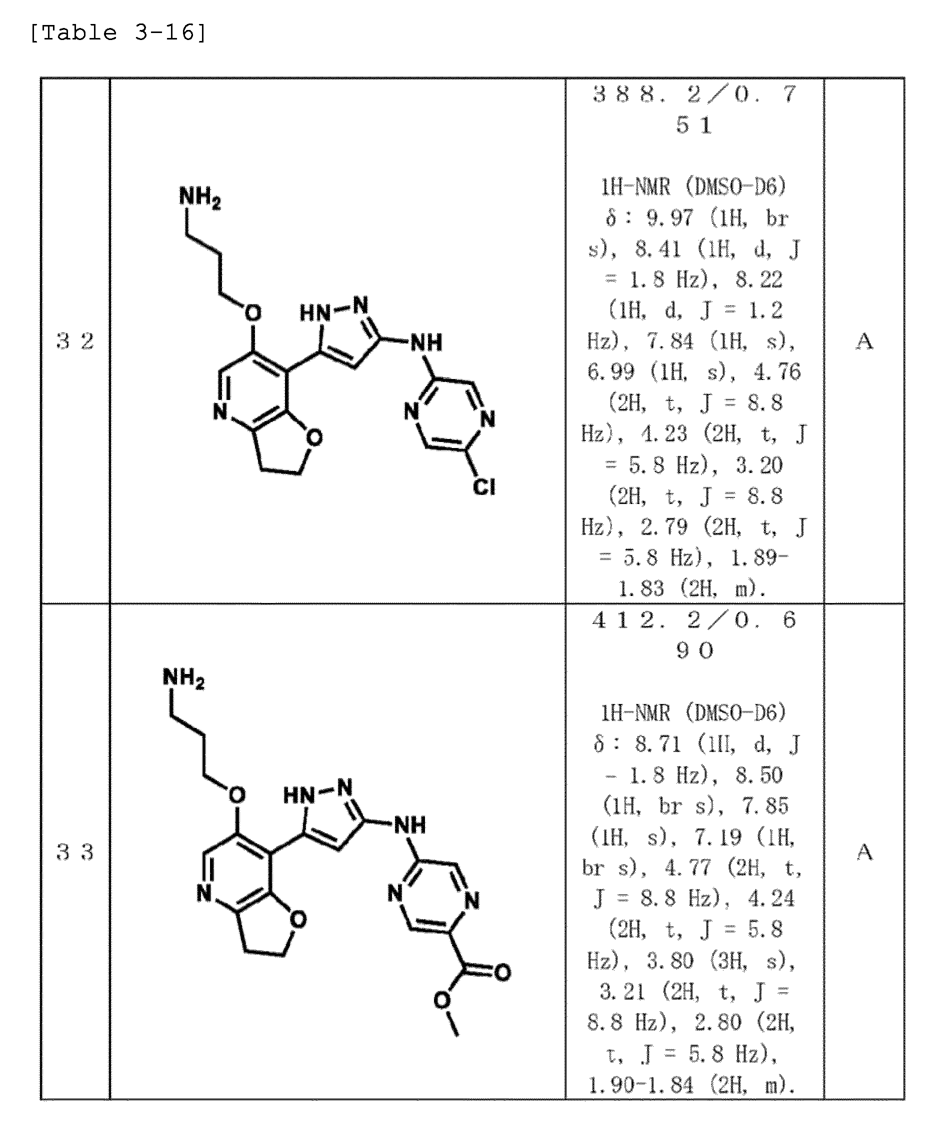

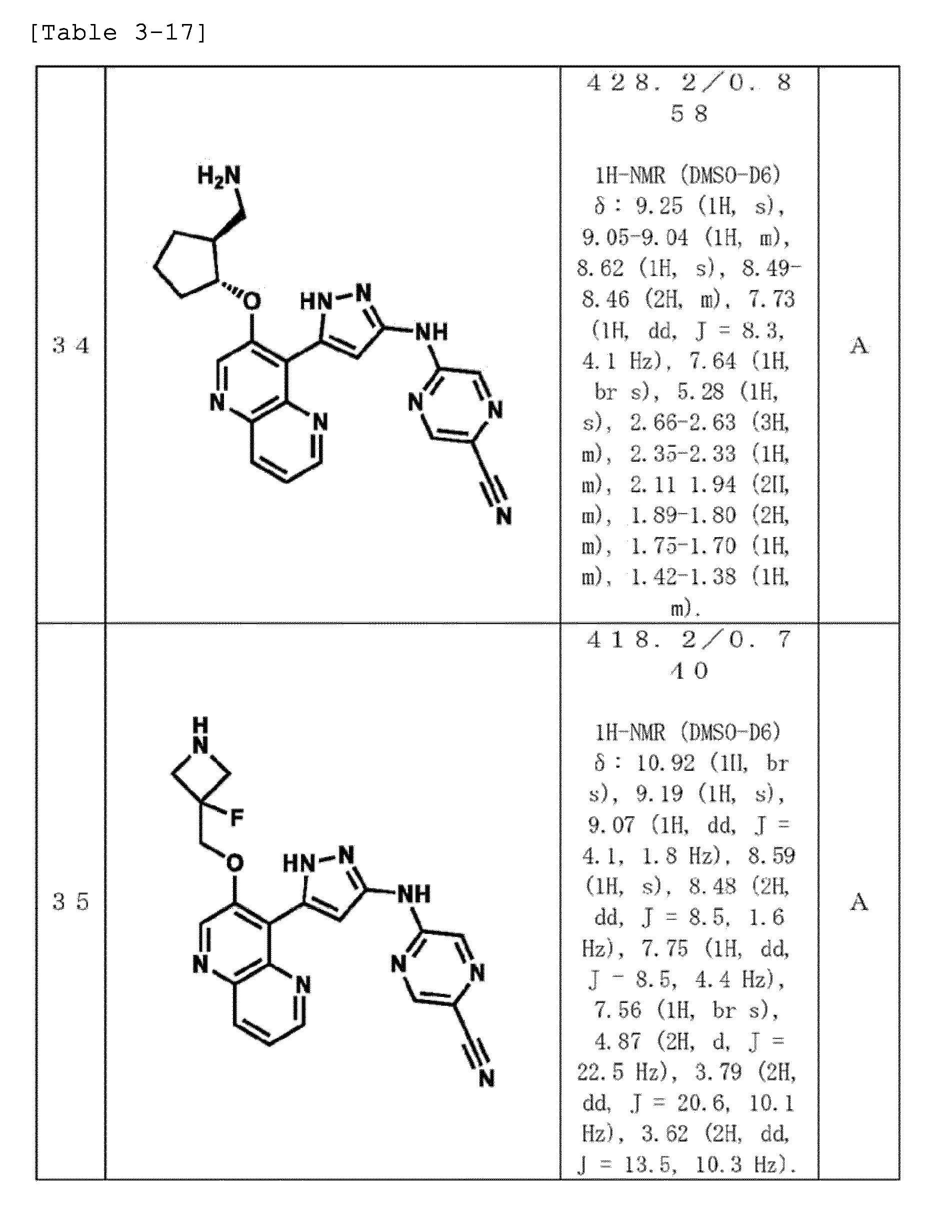

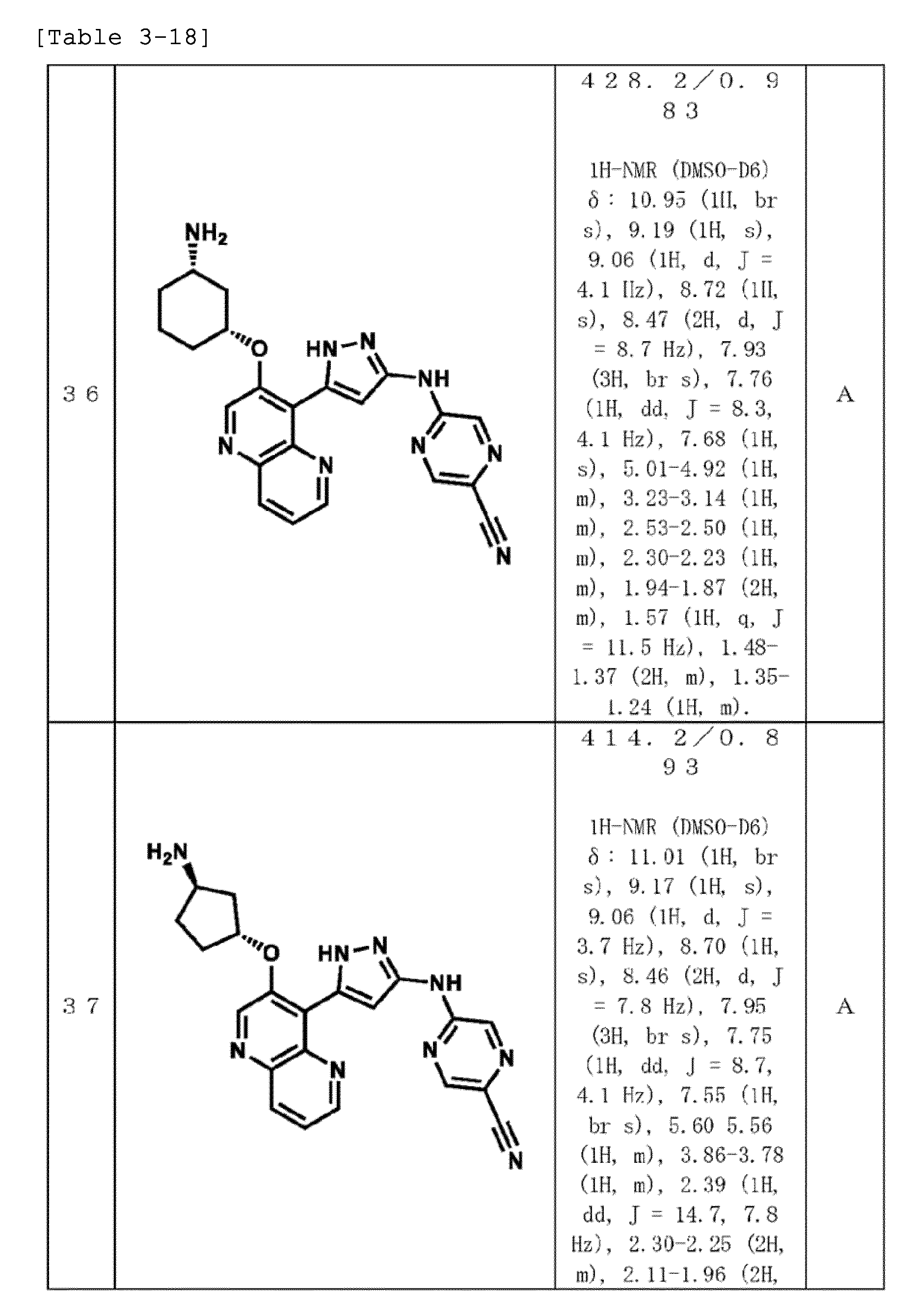

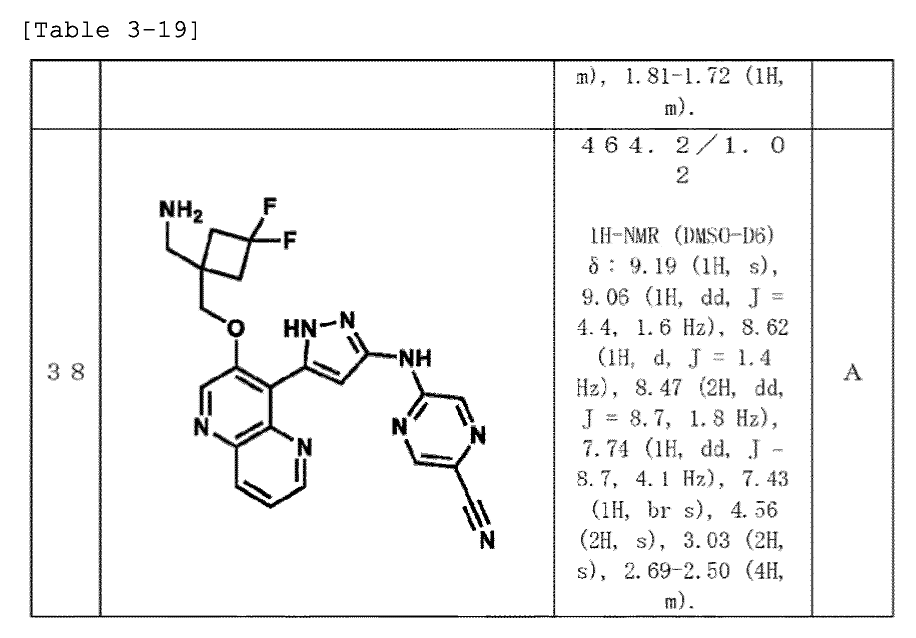

### Test Examples

### Test Example 1: CHK1 inhibitory activity test

IMAP TR-FRET Screening Express Kit (R8160) was obtained from Molecular Devices. CHK1 kinase (02-117, Carna Bio), FAM-labeled CHKltide (R7185, Molecular Devices), and ATP were diluted with an assay buffer to the final concentration of 4 ug/mL, 2 µM, and 20 µM, respectively. FAM-PKAtide (R7255, Molecular Devices) and FAM-Phospho-PKAtide (R7304, Molecular Devices) were admixed after dilution, and calibration standard systems for phosphorylation levels from 0 to 100% were prepared. 5 µL of a compound dissolved in a 0.4% DMSO solution was added to a 384 well plate. A compound study group to which 5 µL of each of CHK1, CHKltide, and ATP was added, and a standard group to which 20 µL of standard was added were prepared, and subjected to a kinase reaction for 3 hours at 30°C. Subsequently, 60 µL of Binding Reagent (80% Buffer A, 20% Buffer B, 1:600 Binding Reagent, 1:400 Tb-Donor) was added for a binding reaction for 2 hours at room temperature. SpectraMax Paradigm (Molecular Devices) was used to obtain fluorescence intensity of 520 nm or 490 nm as of 340 nm excitation. The standard was used to compute the phosphorylation level of CHKltide, and the kinase activity was found by using the equation described below while assuming the phosphorylation level of the DMSO treated group as 100%, and the IC₅₀ value corresponding to the concentration of the evaluated compound indicating kinase activity of 50% was calculated.$Kinase inhibition% = 100 - A/B \times 100$
A: signal in the presence of evaluated compound
B: signal in negative control (DMSO treated group)

The test shown in Test Example 1 was conducted on the compound obtained in the Examples and Prexasertib purchased from MedChemExpress (the supplier is the same in the following Test Examples). The IC₅₀ values (nM) for the test results for each tested substance are shown in the following table.

**[Table 4-1]**

| Example | IC₅₀ (nM) |
|---|---|
| Prexasertib | 0.54 |
| 1 | 0.37 |
| 2 | 0.25 |
| 3 | 0.16 |
| 4 | 0.27 |
| 5 | 0.23 |
| 6 | 0.21 |
| 7 | 1.71 |
| 8 | 0.50 |
| 9 | 0.64 |
| 10 | 0.34 |
| 11 | 0.39 |
| 12 | 0.38 |
| 13 | 0.42 |
| 14 | 0.27 |
| 15 | 0.23 |
| 16 | 0.45 |
| 17 | 0.27 |
| 18 | 0.34 |
| 19 | 0.26 |
| 20 | 0.20 |
| 21 | 0.37 |
| 22 | 0.39 |
| 23 | 0.30 |
| 24 | 0.31 |
| 25 | 0.43 |
| 26 | 0.34 |
| 27 | 0.22 |
| 28 | 0.43 |
| 29 | 0.33 |
| 30 | 0.43 |
| 31 | 0.31 |
| 32 | 0.33 |

**[Table 4-2]**

| | |
|---|---|
| 33 | 9.28 |
| 34 | 0.75 |
| 35 | 0.60 |
| 36 | 0.46 |
| 37 | 0.49 |
| 38 | 0.93 |

As shown in the above table, the compounds of Examples 1 to 6, 8, 10 to 32, 36 and 37 exhibited a more potent effect of suppressing CHK1 activity than Prexasertib.

### Test Example 2: Cell growth suppression experiment

ES-2 cells were obtained from the American Type Culture Collection (ATCC). These cells were cultured at 37°C in the presence of 5% CO₂ in a McCoy's 5a medium containing 10% fetal bovine serum and 1% penicillin/streptomycin.

500 cells were seeded for each well in a 384-well plate, and the evaluated compound was added to the final DMSO concentration of 0.1% to culture the cells for 2 days. After completion of the culture, the cell viability rate was calculated using CellTiter-Glo (registered trademark) 3D Reagent (Promega, G968B). The IC₅₀ values corresponding to the concentration of the evaluated compound indicating cell growth suppression ratio of 50% was calculated from the viability rate curve.

The test shown in Test Example 2 was conducted on the compounds obtained in Examples and Prexasertib. The concentrations at which 50% of cell growth is suppressed of each test compound (IC₅₀ value; nM) are shown in the following table.

**[Table 5-1]**

| Example | IC₅₀ (nM) |
|---|---|
| Prexasertib | 8.55 |
| 1 | 4.90 |
| 2 | 2.90 |
| 3 | 4.00 |
| 4 | 9.10 |
| 5 | 6.70 |
| 6 | 6.24 |
| 7 | 469 |
| 8 | 7.02 |
| 9 | 4.70 |
| 10 | 6.60 |
| 11 | 14.8 |
| 12 | 23.2 |
| 13 | 20.0 |
| 14 | 3.72 |
| 15 | 7.20 |
| 16 | 489 |
| 17 | 3.70 |
| 18 | 4.70 |
| 19 | 4.60 |
| 20 | 16.3 |
| 21 | 11.8 |
| 22 | 13.7 |
| 23 | 6.80 |
| 24 | 12.7 |
| 25 | 6.40 |
| 26 | 9.40 |
| 27 | 8.50 |
| 28 | 6.40 |
| 29 | 29.4 |
| 30 | 14.8 |
| 31 | 13.8 |

**[Table 5-2]**

| | |
|---|---|
| 32 | 21.5 |
| 33 | >1000 |
| 34 | 5.50 |
| 35 | 8.00 |
| 36 | 6.00 |
| 37 | 6.80 |
| 38 | 8.10 |

As shown in the above table, the compounds of Examples 1 to 6, 8 to 15, 17 to 32, 34 to 37 and 38 exhibited an excellent cell growth suppression effect that is equivalent to that of Prexasertib.

### Test Example 3: hERG current blocking test

The test compounds were added to cultured hERG (human Ether-a-go-go Related Gene) gene stably expressing CHO cell line cells to achieve 0.27 to 100 µM. The hERG current under electric potential stimulation was measured using Qube384 (Sophion Bioscience) to calculate the concentration at which each test compound suppresses 50% hERG current (IC₅₀ value; µM).

A test shown in Test Example 3 was conducted on the compounds obtained in the Examples and Prexasertib. A value from dividing IC₅₀ of hERG obtained in Test Example 3 by IC₅₀ of ES-2 cell growth inhibition obtained in Test Example 2 was calculated as hERG/ES-2 for the compound of each Example. The results are shown in the following table.

**[Table 6-1]**

| Example | hERG inhibition IC₅₀ (µM) | hERG /ES-2 |
|---|---|---|
| Prexasertib | 3.30 | 386 |
| 1 | 4.40 | 898 |
| 2 | 0.500 | 172 |
| 3 | 0.530 | 133 |
| 4 | 0.300 | 33 |
| 5 | <2.70 | <403 |
| 6 | 1.30 | 209 |
| 7 | 30.3 | 65 |
| 8 | 41.1 | 5853 |
| 9 | >10.0 | >3704 |
| 10 | 10.8 | 1636 |
| 11 | 16.7 | 1128 |
| 12 | 1.00 | 43 |
| 13 | >100 | >5002 |
| 14 | >10.0 | >2689 |
| 15 | 3.90 | 542 |
| 16 | 38.5 | 79 |
| 17 | <0.30 | <81.1 |
| 18 | 0.30 | 63.8 |
| 19 | <0.30 | <65.2 |
| 20 | 0.40 | 24.5 |
| 21 | >10 | >848 |
| 22 | 4.5 | 329 |
| 23 | 4.2 | 618 |
| 24 | 4.8 | 378 |
| 25 | 11.7 | 1828 |
| 26 | >10 | >1064 |
| 27 | 8.5 | 1000 |
| 28 | >10 | >1563 |
| 29 | >10 | >340 |
| 30 | >10 | >676 |
| 31 | 11.9 | 862 |

**[Table 6-2]**

| | | |
|---|---|---|
| 32 | 11.2 | 521 |
| 33 | >30 | N.D. |
| 34 | 1.40 | 255 |
| 35 | >10 | >1250 |
| 36 | 4.30 | 717 |
| 37 | 4.70 | 691 |
| 38 | >10 | >1235 |

As shown in the above table, the compounds of Examples 1, 8, 9, 10, 11, 13, 14, 21, 25, 26, 27, 28, 31, 35 and 38 have a difference value of 800-fold or greater between the IC₅₀ of ES-2 cell growth inhibition and the IC₅₀ of hERG.

The compounds of Examples 8, 9, 10, 13, 14, 25 and 28 have a difference value of 1500-fold or greater between the IC₅₀ of ES-2 cell growth inhibition and the IC₅₀ of hERG. The difference values for these five compounds were more than three times greater than that for Prexasertib. Therefore, these compounds have heterogeneous effects with a high safety profile.

In particular, the compounds of Examples 8 and 13 have a difference value of 5000-fold or greater between the IC₅₀ of ES-2 cell growth inhibition and the IC₅₀ of hERG. The difference values for these two compounds were more than 12-fold greater than that for Prexasertib. Therefore, these compounds have heterogeneous effects with a high safety profile.

### Test Example 4. Liposome encapsulation test

Liposome encapsulation tests were conducted on representative Example compounds and Prexasertib.

6.48 g of hydrogenated soybean phosphatidylcholine (COATSOME NC-21E, NOF Corporation), 2.32 g of cholesterol (Sigma) and 2.06 g of distearoylphosphatidylethanolamine-methoxypolyethylene glycol 2000 (SUNBRIGHT DSPE-020CN, NOF Corporation) were dissolved in 560 mL of t-butyl alcohol heated to 65°C. The solution was frozen with dry ice/acetone bath and t-butyl alcohol was then removed by evaporation under reduced pressure to obtain a lipid mixture.

200 mL of 250 mM ammonium sulfate solution was added to the lipid mixture. The resulting mixture was heated to 65°C and dispersed with a homogenizer (ULTRA-TURRAX, IKA) to obtain a crude liposome dispersion. The crude liposome dispersion was further dispersed with a high-pressure homogenizer (Nano-Mizer NM2, Yoshida Kikai) at a pressure of 100 MPa to obtain a liposome with a mean particle size (Z-average) of about 80 nm. A dialysis cassette (Slide-A-Lyzer G2 Dialysis Cassettes 20K MWCO, Thermo Scientific) was used to replace the outer aqueous phase of the liposome with a 10 mM L-histidine buffer/10% sucrose solution (pH 6.5) to obtain an empty liposome solution. The solution was filtered through a 0.22 um membrane filter, and a 10 mM L-histidine buffer/10% sucrose solution (pH 6.5) was added to adjust the total lipid concentration to be 75 mM (75 µmol/mL) or 50 mM (50 µmol/mL). The prepared amount was increased or decreased appropriately.

2 to 3 mg of the test compound was weighed out. 1 mL of the empty liposome solution with a total lipid concentration of 50 mM was added. An aqueous 1 mol/L hydrochloric acid or 1 mol/L sodium hydroxide solution was added to adjust the pH to 5.5 to 7. The mixture was heated for 10 to 30 minutes in a 65°C water bath and then cooled with ice. Insoluble substances, when present, were removed by centrifugation for 5 minutes at 15,000× g.

The liposome encapsulation ratio was calculated as follows.

100 µL of liposome solution was passed through an ultrafiltration filter (Amicon Ultra, 100K, 0.5 mL, Merck), and centrifuged at 4°C at 15,000 × g for 10 minutes. The concentration of the compound in the filtrate after ultrafiltration was measured by HPLC as the unencapsulated compound concentration.

The liposome solution was diluted with a trifluoroacetic acid/water/methanol mixture (0.1/25/75) and was left standing for 10 minutes or longer at 5°C. The solution was centrifuged for 5 minutes at 15,000× g to remove insoluble substances. The compound concentration in the supernatant was measured by HPLC as the compound concentration in the liposome solution.

The encapsulation ratio and the encapsulation efficiency were calculated by the following equations. Encapsulation ratio (%) = (compound concentration in liposome solution - unencapsulated compound concentration) × 100/compound concentration in liposome solution Encapsulation efficiency (%) = (compound concentration in liposome solution - unencapsulated compound concentration) × 100/concentration upon compound introduction

HPLC measurement conditions are the following.

### HPLC conditions

Column: Acquity UPLC BEH C18, 1.7 um, 50 × 2.1 mm
Column temperature: 40°C
Mobile Phase:
   A: 0.1% trifluoroacetic acid-containing water
   B: acetonitrile
A/B(min): 95/5(0)→0/100(3.5)→0/100(4)→95/5(4.01)→95/5(5)
Flow Rate: 0.8 mL/min
Detection: UV visible detector, measured wavelength 254 nm
Injected volume: 2 to 5 µL

**[Table 7]**

| Compound | Concentration upon compound introduction (mg/mL) | Compound concentration in liposome solution (mg/mL) | Encapsulation ratio (%) | Encapsulation efficiency (%) |
|---|---|---|---|---|
| Prexasertib | 2 | 2.16 | 99.8 | 107.7 |
| Example 1 | 2 | 2.02 | 91.6 | 92.8 |
| Example 2 | 2 | 1.90 | 100.0 | 94.8 |
| Example 6 | 2 | 1.88 | 100.0 | 94.0 |
| Example 8 | 2 | 1.79 | 100.0 | 89.6 |
| Example 10 | 2 | 1.80 | 100.0 | 90.1 |
| Example 13 | 3 | 2.92 | 100.0 | 97.3 |
| Example 14 | 2 | 1.94 | 99.9 | 97.0 |
| Example 25 | 3 | 2.93 | 99.8 | 97.4 |
| Example 27 | 3 | 2.75 | 100.0 | 91.6 |
| Example 28 | 2 | 2.00 | 99.9 | 99.9 |
| Example 36 | 3 | 3.18 | 100.0 | 106.1 |
| Example 38 | 2 | 1.96 | 100.0 | 97.9 |

It was confirmed that Prexasertib and Examples 1, 2, 6, 8, 10, 13, 14, 25, 27, 28, 36 and 38 can achieve a high encapsulation efficiency of 80% or higher.

### Test Example 5. Pharmacokinetic test

A solution formulation of Prexasertib and liposome formulations of Examples 1, 2, 6, 8, 10, 13, 14, 25, 27, 28, 36, 38 and Prexasertib were intravenously administrated to mice to measure the concentration of the test compound in blood.

The solution formulation used was prepared by dissolving the compound in a 10 mM glycine/5% mannitol solution (pH 2) and then filtering the solution through a 0.22 µm membrane filter.

The liposome formulation used was prepared by preparing a liposome encapsulating a test compound by the similar method to Test Example 4, replacing the outer aqueous phase of the liposome with a 10 mM L-histidine buffer/10% sucrose solution (pH 6.5) by using gel filtration column PD-10 (GE Healthcare), then filtering the solution through a 0.22 µm membrane filter, and adding a 10 mM L-histidine buffer/10% sucrose solution (pH 6.5) to adjust the concentration.

### <Administration Test>

The solution formulation or liposome formulation was instantaneously and intravenously administered into 7-week-old female BALB/c mice. Blood was collected over time up to 72 hours after administration from the jugular vein under no anesthesia. Blood immediate after collection, to which 4 times the volume of methanol was added, was centrifuged, and the concentration of the test compound in the obtained supernatant was quantified by LC-MS/MS.

LC-MS/MS measurement conditions are the following.
HPLC: Prominence system (Shimadzu Corporation)
MS/MS: 4000 QTRAP (SCIEX)
Column: Cadenza CD-C18, 3 um, 50 × 2 mm (Imtakt
Corporation) Column temperature: 40°C
Mobile phase:
   A: 0.1% formic acid-containing water
   B: 0.1% formic acid-containing acetonitrile
A/B(min) : 90/10(0)→10/90(2.5)→10/90(3.5)→90/10(3.6)→90/10(5.0)
Flow rate: 0.4 mL/min
Detection: ESI (positive mode)
Injected volume: 0.1 to 5 µL

The test results are shown in the following tables. In the tables, "mean" refers to the mean, and "S.D." refers to the standard deviation. Table 8 to Table 21 show the plasma test compound concentrations from 0 hours to 72 hours after administration.

**[Table 8]**

| Solution formulation of Prexasertib 1.0 mg/kg (i.v.) | Plasma compound concentration (ng/mL) | | | | |
|---|---|---|---|---|---|
| | 1 | 2 | 3 | mean | S.D. |
| 0.08 hr | 245 | 277 | 160 | 227 | 61 |
| 0.25 hr | 138 | 112 | 140 | 130 | 16 |
| 0.5 hr | 93.6 | 139 | 90 | 108 | 27 |
| 1.0 hr | 51.2 | 44.3 | 64.9 | 53.5 | 11 |
| 6.0 hr | N.D. | 9.95 | 5.46 | 5.14 | 5.0 |
| 24.0 hr | N.D. | N.D. | N.D. | N.D. | N.A. |
| 48.0 hr | N.D. | N.D. | N.D. | N.D. | N.A. |
| 72.0 hr | N.D. | N.D. | N.D. | N.D. | N.A. |

**[Table 9]**

| Liposome formulation of Prexasertib 1.0 mg/kg (i.v.) | Plasma compound concentration (ng/mL) | | | | |
|---|---|---|---|---|---|
| | 1 | 2 | 3 | mean | S.D. |
| 0.08 hr | 28200 | 24800 | 27400 | 26800 | 1778 |
| 0.25 hr | 23200 | 26600 | 25200 | 25000 | 1709 |
| 0.5 hr | 26200 | 26100 | 23800 | 25367 | 1358 |
| 1.0 hr | 20200 | 24600 | 21000 | 21933 | 2344 |
| 6.0 hr | 12600 | 13000 | 11200 | 12267 | 945 |
| 24.0 hr | 1610 | 2290 | 3630 | 2510 | 1028 |
| 48.0 hr | 211 | 320 | 232 | 254 | 58 |
| 72.0 hr | 7.98 | 11.3 | 21.8 | 13.7 | 7.2 |

**[Table 10]**

| Liposome formulation of Example 1 1.0 mg/kg (i.v.) | Plasma compound concentration (ng/mL) | | | | |
|---|---|---|---|---|---|
| | 1 | 2 | 3 | mean | S.D. |
| 0.08 hr | 37900 | 40400 | 44100 | 40800 | 3119 |
| 0.25 hr | 42200 | 42800 | 39600 | 41533 | 1701 |
| 0.5 hr | 34100 | 36700 | 34300 | 35033 | 1447 |
| 1.0 hr | 31100 | 33900 | 33900 | 32967 | 1617 |
| 6.0 hr | 21500 | 23200 | 20500 | 21733 | 1365 |
| 24.0 hr | 12300 | 12300 | 11800 | 12133 | 289 |
| 48.0 hr | 4270 | 5140 | 3810 | 4407 | 675 |
| 72.0 hr | 1060 | 1860 | 1730 | 1550 | 429 |

**[Table 11]**

| Liposome formulation of Example 2 1.0 mg/kg (i.v.) | Plasma compound concentration (ng/mL) | | | | |
|---|---|---|---|---|---|
| | 1 | 2 | 3 | mean | S.D. |
| 0.08 hr | 30300 | 28800 | 29000 | 29367 | 814 |
| 0.25 hr | 28700 | 29700 | 25500 | 27967 | 2194 |
| 0.5 hr | 26200 | 25500 | 25700 | 25800 | 361 |
| 1.0 hr | 24100 | 23400 | 20700 | 22733 | 1795 |
| 6.0 hr | 9960 | 11700 | 9860 | 10507 | 1035 |
| 24.0 hr | 976 | 1030 | 889 | 965 | 71 |
| 48.0 hr | 168 | 104 | 125 | 132 | 32.6 |
| 72.0 hr | N.D. | N.D. | N.D. | N.D. | N.A. |

**[Table 12]**

| Liposome formulation of Example 6 1.0 mg/kg (i.v.) | Plasma compound concentration (ng/mL) | | | | |
|---|---|---|---|---|---|
| | 1 | 2 | 3 | mean | S.D. |
| 0.08 hr | 35300 | 32200 | 28900 | 32133 | 3201 |
| 0.25 hr | 27700 | 31000 | 33600 | 30767 | 2957 |
| 0.5 hr | 29700 | 27900 | 26400 | 28000 | 1652 |
| 1.0 hr | 25100 | 26300 | 26300 | 25900 | 693 |
| 6.0 hr | 18300 | 15700 | 13200 | 15733 | 2550 |
| 24.0 hr | 5910 | 5410 | 6570 | 5963 | 581.8 |
| 48.0 hr | 2600 | 2050 | 2610 | 2420 | 320 |
| 72.0 hr | 218 | 85.0 | 271 | 191 | 96 |

**[Table 13]**

| Liposome formulation of Example 8 1.0 mg/kg (i.v.) | Plasma compound concentration (ng/mL) | | | | |
|---|---|---|---|---|---|
| | 1 | 2 | 3 | mean | S.D. |
| 0.08 hr | 41400 | 43800 | 40800 | 42000 | 1587 |
| 0.25 hr | 39700 | 34100 | 35500 | 36433 | 2914 |
| 0.5 hr | 37500 | 38000 | 37900 | 37800 | 265 |
| 1.0 hr | 30500 | 27900 | 28600 | 29000 | 1345 |
| 6.0 hr | 18200 | 18600 | 19400 | 18733 | 611 |
| 24.0 hr | 9880 | 8370 | 11500 | 9917 | 1565 |
| 48.0 hr | 4540 | 4600 | 4730 | 4623 | 97.1 |
| 72.0 hr | 1710 | 1010 | 2230 | 1650 | 612 |

**[Table 14]**

| Liposome formulation of Example 10 1.0 mg/kg (i.v.) | Plasma compound concentration (ng/mL) | | | | |
|---|---|---|---|---|---|
| | 1 | 2 | 3 | mean | S.D. |
| 0.08 hr | 29300 | 30200 | 29400 | 29633 | 493 |
| 0.25 hr | 44500 | 31300 | 35300 | 37033 | 6769 |
| 0.5 hr | 25600 | 28900 | 26200 | 26900 | 1758 |
| 1.0 hr | 30500 | 28900 | 29400 | 29600 | 819 |
| 6.0 hr | 16800 | 18000 | 17300 | 17367 | 603 |
| 24.0 hr | 5300 | 4520 | 3280 | 4367 | 1019 |
| 48.0 hr | 717 | 1110 | 1340 | 1056 | 315 |
| 72.0 hr | 80.3 | 47.8 | 30.9 | 53.0 | 25.1 |

**[Table 15]**

| Liposome formulation of Example 13 1.0 mg/kg (i.v.) | Plasma compound concentration (ng/mL) | | | | |
|---|---|---|---|---|---|
| | 1 | 2 | 3 | mean | S.D. |
| 0.08 hr | 32500 | 29800 | 38000 | 33433 | 4179 |
| 0.25 hr | 27500 | 32300 | 32600 | 30800 | 2862 |
| 0.5 hr | 25800 | 26600 | 28400 | 26933 | 1332 |
| 1.0 hr | 26700 | 20300 | 24400 | 23800 | 3242 |
| 6.0 hr | 18300 | 12600 | 14100 | 15000 | 2955 |
| 24.0 hr | 7270 | 6540 | 8910 | 7573 | 1214 |
| 48.0 hr | 4260 | 3080 | 4310 | 3883 | 696 |
| 72.0 hr | 66.2 | 83.0 | 551 | 233 | 275 |

**[Table 16]**

| Liposome formulation of Example 14 1.0 mg/kg (i.v.) | Plasma compound concentration (ng/mL) | | | | |
|---|---|---|---|---|---|
| | 1 | 2 | 3 | mean | S.D. |
| 0.08 hr | 46200 | 34000 | 38100 | 39433 | 6208 |
| 0.25 hr | 36000 | 31900 | 36500 | 34800 | 2524 |
| 0.5 hr | 39100 | 29200 | 34100 | 34133 | 4950 |
| 1.0 hr | 31500 | 26000 | 27400 | 28300 | 2858 |
| 6.0 hr | 24800 | 20600 | 18300 | 21233 | 3296 |
| 24.0 hr | 3760 | 4360 | 4730 | 4283 | 490 |
| 48.0 hr | 785 | 523 | 490 | 599 | 162 |
| 72.0 hr | 51.7 | 63.4 | 57.8 | 57.6 | 5.9 |

**[Table 17]**

| Liposome formulation of Example 25 1.0 mg/kg (i.v.) | Plasma compound concentration (ng/mL) | | | | |
|---|---|---|---|---|---|
| | 1 | 2 | 3 | mean | S.D. |
| 0.08 hr | 24500 | 23000 | 32700 | 26733 | 5221 |
| 0.25 hr | 27200 | 23000 | 23400 | 24533 | 2318 |
| 0.5 hr | 21900 | 21300 | 27400 | 23533 | 3362 |
| 1.0 hr | 21000 | 19300 | 19600 | 19967 | 907 |
| 6.0 hr | 10800 | 10600 | 11200 | 10867 | 306 |
| 24.0 hr | 3750 | 3530 | 3040 | 3440 | 363 |
| 48.0 hr | 771 | 339 | 593 | 568 | 217 |
| 72.0 hr | 10.7 | N.D | N.D | N.D | N.A. |

**[Table 18]**

| Liposome formulation of Example 27 1.0 mg/kg (i.v.) | Plasma compound concentration (ng/mL) | | | | |
|---|---|---|---|---|---|
| | 1 | 2 | 3 | mean | S.D. |
| 0.08 hr | 35600 | 33200 | 32000 | 33600 | 1833 |
| 0.25 hr | 30000 | 31000 | 33300 | 31433 | 1692 |
| 0.5 hr | 30300 | 24100 | 28200 | 27533 | 3153 |
| 1.0 hr | 26500 | 22400 | 24300 | 24400 | 2052 |
| 6.0 hr | 13000 | 15800 | 16800 | 15200 | 1970 |
| 24.0 hr | 5330 | 5520 | 5800 | 5550 | 236 |
| 48.0 hr | 1800 | 1420 | 2190 | 1803 | 385 |
| 72.0 hr | 157 | 17.3 | 333 | 169 | 158 |

**[Table 191**

| Liposome formulation of Example 28 1.0 mg/kg (i.v.) | Plasma compound concentration (ng/mL) | | | | |
|---|---|---|---|---|---|
| | 1 | 2 | 3 | mean | S.D. |
| 0.08 hr | 38700 | 44900 | 34400 | 39333 | 5279 |
| 0.25 hr | 25300 | 35100 | 29100 | 29833 | 4941 |
| 0.5 hr | 33200 | 34500 | 27300 | 31667 | 3837 |
| 1.0 hr | 21300 | 30200 | 25600 | 25700 | 4451 |
| 6.0 hr | 16900 | 18300 | 17700 | 17633 | 702 |
| 24.0 hr | 4420 | 5120 | 4680 | 4740 | 354 |
| 48.0 hr | 1160 | 1660 | 1310 | 1377 | 257 |
| 72.0 hr | 346 | 119 | 73.4 | 179 | 146 |

**[Table 20]**

| Liposome formulation of Example 36 1.0 mg/kg (i.v.) | Plasma compound concentration (ng/mL) | | | | |
|---|---|---|---|---|---|
| | 1 | 2 | 3 | mean | S.D. |
| 0.08 hr | 35600 | 33200 | 32000 | 33600 | 1833 |
| 0.25 hr | 30000 | 31000 | 33300 | 31433 | 1692 |
| 0.5 hr | 30300 | 24100 | 28200 | 27533 | 3153 |
| 1.0 hr | 26500 | 22400 | 24300 | 24400 | 2052 |
| 6.0 hr | 13000 | 15800 | 16800 | 15200 | 1970 |
| 24.0 hr | 5330 | 5520 | 5800 | 5550 | 236 |
| 48.0 hr | 1800 | 1420 | 2190 | 1803 | 385 |
| 72.0 hr | 157 | 17.3 | 333 | 169 | 158 |

**[Table 21]**

| Liposome formulation of Example 38 1.0 mg/kg (i.v.) | Plasma compound concentration (ng/mL) | | | | |
|---|---|---|---|---|---|
| | 1 | 2 | 3 | mean | S.D. |
| 0.08 hr | 38300 | 39300 | 31400 | 36333 | 4302 |
| 0.25 hr | 34000 | 38100 | 34900 | 35667 | 2155 |
| 0.5 hr | 35200 | 31700 | 31400 | 32767 | 2113 |
| 1.0 hr | 25900 | 30900 | 31000 | 29267 | 2915 |
| 6.0 hr | 15800 | 16600 | 15800 | 16067 | 462 |
| 24.0 hr | 835 | 1250 | 1080 | 1055 | 209 |
| 48.0 hr | 74.5 | 86.6 | 62.0 | 74.4 | 12.3 |
| 72.0 hr | N.D. | N.D. | N.D. | N.D. | N.A. |

Table 22 shows the AUC calculated by the trapezoidal method from 0 hours after administration to a point in time (t) at which the plasma test compound concentration was able to be quantified for AUC associated with the liposome formulations of Examples 1, 2, 6, 8, 10, 13, 14, 25, 27, 28, 36, 38 and Prexasertib.

**[Table 22]**

| Example | AUC₀₋ₜ (ng·hr/mL) |
|---|---|
| Prexasertib | 236309 |
| 1 | 755083 |
| 2 | 187143 |
| 6 | 430431 |
| 8 | 685073 |
| 10 | 377973 |
| 13 | 487964 |
| 14 | 396818 |
| 25 | 255400 |
| 27 | 397200 |
| 28 | 396900 |
| 36 | 397200 |
| 38 | 251800 |

The above results confirmed high blood retention upon intravenous administration of the liposome formulations prepared by liposome encapsulation of Examples 1, 6, 8, 10, 13, 14, 27, 28, 36 and 38. This result shows that 72 hours of sustained exposure was achieved, which is important for maximizing the efficacy of Prexasertib reported in a non-clinical trial. Therefore, Examples 1, 6, 8, 10, 13, 14, 27, 28, 36 and 38 have an excellent pharmacokinetic profile and are very useful as an anticancer agent.

### Test Example 6. Drug efficacy evaluation test using tumor-bearing mice transplanted with ES-2 cells

Anti-tumor effect was evaluated by using a solution formulation of Prexasertib, and liposome formulations encapsulating Examples 8, 13 and 28.

The solution formulation of Prexasertib used was prepared by dissolving Prexasertib in a 10 mM glycine/5% mannitol solution (pH 2) containing 20% sulfobutylether-β-cyclodextrin and then filtering the mixture through a 0.22 µm membrane filter.

The liposome formulations were prepared by the following method. An empty liposome solution with a total lipid concentration of 75 mM was obtained by the similar method to Test Example 4, where the outer aqueous phase was a 10 mM L-histidine buffer solution/9.4% sucrose solution (pH 6.5). A 10 mM L-histidine buffer solution/9.4% sucrose solution (pH 6.5) was added to the test compound to disperse the compound, and the empty liposome solution was added thereto to the compound concentration of 2 mg/mL and the total lipid concentration of 50 mM, and the pH was adjusted to 6 to 7. The mixture was warmed for 10 minutes in a 65°C water bath, and then cooled with ice. The mixture was filtered with a 0.22 um membrane filter.

ES-2 cells (ATCC) were intradermally transplanted at 1 × 10⁶ cells/mouse into the flank region in 4- to 7-week-old BALB/c-nu/nu mice (CAnN.Cg-Foxnlnu/CrlCrlj, female, Charles River Laboratories Japan). After confirming engraftment of the ES-2 cells 5 to 14 days post-transplantation, the solution formulation was intravenously administered at a dose of 30 mg/kg or the liposome formulation was intravenously administered at a dose of 4.5 mg/kg, or 10 mg/kg once a week. The tumor volume was measured over time from the start of administration to evaluate the tumor volume reduction from administration of the compound. The tumor volume was calculated by the following equation by using the minor axis and major axis of tumor measured with an electronic caliper (Mitutoyo). $\begin{matrix}Tumor volume \left[{mm}^{3}\right] = \\ 0.5 \times {\left(minor axis \left[mm\right]\right)}^{2} \times major axis \left[mm\right]\end{matrix}$

A control administration group administered with only a solvent and an administration group of the compound of the present disclosure were compared. T/C was calculated from the following equation to evaluate the anti-tumor effect. The complete tumor regression (CR) individual ratio at the end of administration of the solution formulation or liposome formulation was also recorded. For the control administration group, an empty liposome solution with total lipid concentration of 50 mM was used. T/C (%) = (Tumor volume at the end of administration in administration group of the compound of the present disclosure - Tumor volume at the start of administration in administration group of the compound of the present disclosure)/ (Tumor volume at the end of administration in control administration group - Tumor volume at the start of administration in control administration group) × 100

Table 18 shows the T/C (%) and CR individual ratio for tumor-bearing mice transplanted with ES-2 cells at each dosage and dosing period for the test compound.

**[Table 23]**

| Test | Route of administration | Dosage (mg/kg) | Dosing period (days) | Schedule (times/week) | T/C (%) | CR (%) |
|---|---|---|---|---|---|---|
| Solution formulation of Prexasertib | Intravenous | 30 (maximum tolerable dose) | 10 | 1 | 54 | 0 |
| Liposome formulation of Example 8 | Intravenous | 4.5 | 10 | 1 | -15 | 0 |
| Liposome formulation of Example 8 | Intravenous | 10 | 10 | 1 | -26 | 33 |
| Liposome formulation of Example 13 | Intravenous | 4.5 | 10 | 1 | 80 | 0 |
| Liposome formulation of Example 13 | Intravenous | 10 | 10 | 1 | 9 | 0 |
| Liposome formulation of Example 28 | Intravenous | 4.5 | 10 | 1 | -25 | 0 |
| Liposome formulation of Example 28 | Intravenous | 10 | 10 | 1 | -26 | 0 |

In the drug efficacy evaluation test using tumor-bearing mice, the liposome-formulated Examples 8, 13 and 28 achieved tumor regression, which could not be achieved by the maximum tolerable dose of solution-formulated Prexasertib, in an evaluation test using the dosage of 10 mg/kg. From the results of this test, Examples 8, 13 and 28 are promising compounds exhibiting excellent anti-tumor effects, and therefore have exceptional effects.

### Test Example 7. Minimum effective dose evaluation test using tumor-bearing mice transplanted with ES-2 cells

Using a solution formulation of Prexasertib, and liposome formulations encapsulating Examples 8 and 28, a minimum effective dose evaluation test was conducted on tumor-bearing mice transplanted with ES-2 cells by the similar method to Test Example 6.

The solution formulation of Prexasertib used was prepared by dissolving Prexasertib in a 10 mM glycine/5% mannitol solution (pH 2) containing 20% sulfobutylether-β-cyclodextrin and then filtering the mixture through a 0.22 um membrane filter.

The liposome formulations of Examples 8 and 28 were prepared by the following method. An empty liposome solution with a total lipid concentration of 75 mM was obtained by the similar method to Test Example 4, where the outer aqueous phase was a 10 mM L-histidine buffer solution/9.4% sucrose solution (pH 6.5). A 10 mM L-histidine buffer solution/9.4% sucrose solution (pH 6.5) was added to the test compound to disperse the compound, and the empty liposome solution was added thereto to the compound concentration of 2 mg/mL and the total lipid concentration of 50 mM, and the pH was adjusted to 6 to 7. The mixture was warmed for 10 minutes in a 65°C water bath, and then cooled with ice. The mixture was filtered with a 0.22 um membrane filter.

The following table shows the T/C (%) and CR individual ratio for tumor-bearing mice transplanted with ES-2 cells at each dosage and dosing period for the test compound.

**[Table 24]**

| Test | Route of administration | Dosage (mg/kg) | Dosing period (days) | Schedule (times/week) | T/C (%) | CR (%) |
|---|---|---|---|---|---|---|
| Solution formulation of Prexasertib | Intravenous | 30 (maximum tolerable dose) | 10 | 1 | 54 | 0 |
| Liposome formulation of Example 8 | Intravenous | 1 | 12 | 1 | 55 | 0 |
| Liposome formulation of Example 8 | Intravenous | 2 | 12 | 1 | 19 | 0 |
| Liposome formulation of Example 8 | Intravenous | 4.5 | 12 | 1 | -3 | 0 |
| Liposome formulation of Example 28 | Intravenous | 1 | 12 | 1 | 44 | 0 |
| Liposome formulation of Example 28 | Intravenous | 2 | 12 | 1 | -3 | 25 |
| Liposome formulation of Example 28 | Intravenous | 4.5 | 12 | 1 | -14 | 75 |

In the drug efficacy evaluation test using tumor-bearing mice, the liposome-formulated Examples 8 and 28 achieved tumor regression effects equivalent to or greater than the maximum tolerable dose of solution-formulated Prexasertib, in a dosage of 1 mg/kg. From the results of this test, Examples 8 and 28 are promising compounds exhibiting excellent anti-tumor effects at low doses, and therefore have exceptionally remarkable tumor regression effects.

### Test Example 8. Measurement of neutrophil count using tumor-bearing mice transplanted with ES-2 cells

ES-2 cells (ATCC) were intradermally transplanted at 1 × 10⁶ cells/mouse into the flank region in 4- to 7-week-old BALB/c-nu/nu mice (CAnN.Cg-Foxnlnu/CrlCrlj, female, The Jackson Laboratory Japan, Inc.). After confirming engraftment of the ES-2 cells 5 to 18 days post-transplantation, the liposome formulation encapsulating Prexasertib and the liposome formulations encapsulating Examples 8 and 28 were intravenously administered once at each dose. Blood was collected after administration to measure the neutrophil count. An empty liposome solution prepared by the similar method to Test Example 4 was used for comparison in this test.

The liposome formulation of Prexasertib was prepared by the following method. An empty liposome solution with a total lipid concentration of 75 mM was obtained by the similar method to Test Example 4, where the outer aqueous phase was a 10 mM L-histidine buffer solution/10% sucrose solution (pH 6.5). Prexasertib was added thereto at concentration of 3 mg/mL, and the pH was adjusted to 6 to 7. The mixture was warmed for 15 minutes in a 65°C water bath, and then cooled with ice. The mixture was filtered with a 0.22 um membrane filter.

The liposome formulations of Examples 8 and 28 were prepared by the following method. An empty liposome solution with a total lipid concentration of 75 mM was obtained by the similar method to Test Example 4, where the outer aqueous phase was a 10 mM L-histidine buffer solution/9.4% sucrose solution (pH 6.5). A 10 mM L-histidine buffer solution/9.4% sucrose solution (pH 6.5) was added to the test compound to disperse the compound, and the empty liposome solution was added thereto to the compound concentration of 2 mg/mL and the total lipid concentration of 50 mM, and the pH was adjusted to 6 to 7. The mixture was warmed for 10 minutes in a 65°C water bath, and then cooled with ice. The mixture was filtered with a 0.22 µm membrane filter.

A control administration group administered with the empty liposome solution and administration groups administered with the liposome-formulated Examples 8 and 28 and the liposome-formulated Prexasertib were compared. The ratio of residual neutrophils was calculated from the following equation to evaluate the safety of each formulation. Ratio of residual neutrophilis (%) = (neutrophil count in administration group)/(neutrophil count in control administration group) × 100

The following table shows the ratio of residual neutrophils for tumor-bearing mice transplanted with ES-2 cells, and the anti-tumor effect evaluated by the similar method to Test Example 6. TGI was used as an index of anti-tumor effect, and it was calculated from the following equation. TGI (%) = {1-(average tumor volume of administration group)/(average tumor volume of control administration group)} × 100

**[Table 25]**

| | | | Drug efficacy evaluation | | hemotoxicological evaluation | |
|---|---|---|---|---|---|---|
| Test | Route of administration | Dosage (mg/kg) | Dosing regimen | TGI (%) | Number of doses | Ratio of residual neutrophils (%) |
| Liposome formulation of Prexasertib | Intravenous | 7.5 | Once a week | 87.5 | 1 | 4 |
| Liposome formulation of Example 8 | Intravenous | 4.5 | Once a week | 90.4 | 1 | 69 |
| Liposome formulation of Example 28 | Intravenous | 4.5 | Once a week | 97.6 | 1 | 29 |

The liposome-formulated Prexasertib has a neutrophil residual of 4% at a dosage of 7.5 mg/kg, with a simultaneous observation of anti-tumor effect and hematotoxicity. Meanwhile, the liposome-formulated Examples 8 and 28 show stronger anti-tumor effects than the liposome-formulated Prexasertib at a dosage of 4.5 mg/kg, with a higher neutrophil residual and reduced hematologic toxic side effects. From the results of this test, the liposome-formulated Examples 8 and 28 are superior compounds with a combination of higher safety and efficacy than the liposome-formulated Prexasertib, with exceptionally remarkable and heterogeneous effects.

### Test Example 9. Cell growth suppression test on various cancer cell lines

HT1080 cells (derived from soft tissue sarcoma) were cultured at 37°C in the presence of 5% CO₂ in an EMEM medium containing 10% fetal bovine serum and 1% penicillin/streptomycin. SJCRH30 cells (derived from soft tissue sarcoma) and BxPC3 cells (derived from pancreatic cancer) were cultured at 37°C in the presence of 5% CO₂ in a McCoy's 5a medium containing 10% fetal bovine serum and 1% penicillin/streptomycin. SW948 cells (derived from colon cancer) were cultured at 37°C in air in a L-15 medium containing 10% fetal bovine serum and 1% penicillin/streptomycin.

The cells were seeded for each well in a 96-well plate at 70-90% confluency 96 hours after seeding, the evaluated compound was added to the final DMSO concentration of 0.1%, and the cells were cultured for 3 days. After completion of the culture, the cell viability rate was calculated using CellTiter-Glo (registered trademark) 3D Reagent (Promega, G968B). The IC₅₀ values corresponding to the concentration of the evaluated compound indicating cell growth suppression ratio of 50% was calculated from the viability rate curve.

The test shown in Test Example 2 was conducted on the compounds obtained in Examples and Prexasertib. The concentrations at which 50% of cell growth is suppressed of each test compound (IC₅₀ value; nM) are shown in the following table.

**[Table 26]**

| | HT1080 | SJCRH30 | BxPC3 | SW948 |
|---|---|---|---|---|
| Prexasertib | 14.9 | 4.9 | 9.5 | 38.9 |
| Example 8 | 9.1 | <3.0 | 7.0 | 22.3 |
| Example 28 | <3.0 | <3.0 | <3.0 | 5.0 |

As shown in the above table, the compounds of Examples 8 and 28 exhibited stronger cell growth suppression effects than Prexasertib against cancer cell lines derived from soft tissue sarcoma, pancreatic cancer and colon cancer. From the results of this test, the compounds of Examples 8 and 28 have exceptionally remarkable cancer cell growth suppression effects at least against soft tissue sarcoma and pancreatic cancer.

### Test Example 10. Human bone marrow cell colony formation test (hemotoxicological evaluation test)

The test can be evaluated, for example, by the following method. Frozen cells of human bone marrow CD34 positive hematopoietic stem cells are thawed a 9:1 mixture of a Methocult Express medium and a RPMI 1640 medium containing 10% fetal bovine serum and 1% penicillin/streptomycin. 5000 cells are seeded per well in a 6 well plate and cultured overnight at 37°C in the presence of 5% CO₂. The evaluated compound is added to the final DMSO concentration of 0.1% or 100 nM, and the cells are cultured for 48 hours. The cells are collected and washed with PBS. 1/6 of the amount of cells is seeded in a 12 well plate and cultured for about 2 weeks at 37°C in the presence of 5% CO₂. After completion of culture, the number of colonies formed is calculated using Thiazolyl blue tetrazolium bromide (MTT).

### Test Example 11. Kinase selectivity test

The selectivity for various wild-type kinases can be evaluated by HotSpot Full Panel Kinase profiling (Reaction Biology).
Buffer solution: 20 mM HEPES, pH 7.5, 10 mM MgCl2, 2 mM MnCl2 (as needed), 1 mM EGTA, 0.02% Brij35, 0.02 mg/mL BSA, 0.1 mM Na3VO4, 2 mM DTT, 1% DMSO
ATP concentration: 10 µM
Reaction time: 2 hr
ATP conversion rate in 2 hr: 5 to 20%
compound concentration: 100 nM

### Test Example 12. Cytotoxicity test

Cytotoxicity tests for various cells can be evaluated, for example, using OncoPanel Cell-Based Profiling Service (Eurofins) under the following conditions.

Cells are seeded into 384-well plates. Test compound is diluted 3.16 times starting from 10 mM to prepare a dilution series at 10 points. The diluted compounds and DMSO are added 24 hours after cell seeding. The cells are fixed 3 days after compound addition, and the nuclei are stained and detected. Data for compound-treated group in each cell are normalized with 0.1% DMSO-treated vehicle data.

The compound represented by formula (6) has a CHK1 inhibitory activity equivalent to or greater than that of Prexasertib (Test Example 1), and exhibits a cell growth suppression effect equivalent to or greater than that of Prexasertib (Test Example 2), and also has the characteristic of lower cardiotoxicity than Prexasertib (Test Example 3), and therefore has an exceptionally remarkable and heterogeneous effect. Furthermore, the compound encompassed by formula (6) can be efficiently encapsulated in a liposome by a simple encapsulation procedure (Test Example 4). The compound represented by formula (6) formulated in a liposome exhibits good pharmacokinetics (Test Example 5), and has an exceptionally remarkable effect of exhibiting an excellent anti-tumor effect in tumor-bearing mice transplanted with ES-2 cells (Test Examples 6 and 7). The compound represented by formula (6) formulated in a liposome has such an excellent anti-tumor effect, and also has a heterogeneous effect of a significantly reduced risk of hematologic toxicity compared to the liposome-formulated Prexasertib (Test Example 8). In addition, the compound represented by formula (6) exhibits a stronger cell growth suppression effect than Prexasertib against cancer cell lines derived from soft tissue sarcoma and pancreatic cancer, and therefore has an exceptional cancer cell growth suppression effect (Test Example 9).

From the above, the compound represented by formula (6) has particularly excellent efficacy and safety among the compounds represented by formula (1), and therefore, has an exceptionally remarkable and heterogeneous effect in that it can exhibit an excellent anti-tumor effect with high safety when formulated in a liposome.

The compound of Example 8, which is a representative compound encompassed by formula (4), exhibits a CHK1 inhibitory activity and a cell growth suppression effect equivalent to or greater than that of Prexasertib (Test Examples 1 and 2). In addition, the compound of Example 8 has a difference value of 5000-fold or greater between cell growth inhibition and hERG inhibition, and has an exceptionally remarkable and heterogeneous effect of extremely lower cardiotoxicity than Prexasertib. Furthermore, the compound of Example 8 can be efficiently encapsulated in a liposome by a simple encapsulation procedure, (Test Example 4), and the liposome formulation exhibits good pharmacokinetics (Test Example 5). The compound of Example 8 formulated in a liposome has an exceptional effect of exhibiting an excellent anti-tumor effect in tumor-bearing mice transplanted with ES-2 cells (Test Examples 6 and 7). The compound of Example 8 formulated in a liposome has a heterogeneous effect of a significantly reduced risk of hematologic toxicity compared to the liposome-formulated Prexasertib (Test Example 8). Furthermore, the compound of Example 8 exhibits a stronger cell growth suppression effect than Prexasertib against cancer cell lines derived from soft tissue sarcoma and pancreatic cancer, and therefore has an exceptionally remarkable cancer cell growth suppression effect (Test Example 9).

From the above, the compound of Example 8 has particularly excellent efficacy and safety among the compounds represented by formula (4), and therefore, has an exceptionally remarkable and heterogeneous effect in that it can exhibit an excellent anti-tumor effect with high safety when formulated in a liposome.

The compounds of Examples 13 and 28, which are representative compounds encompassed by formula (6), have a CHK1 inhibitory activity equivalent to or greater than that of Prexasertib (Test Example 1). The compound of Example 13 exhibits a cell growth suppression effect equivalent to that of Prexasertib, and the compound of Example 28 exhibits a cell growth suppression effect equivalent to or greater than that of Prexasertib (Test Example 2). In addition, the compounds of Examples 13 and 28 have a difference value of 1500-fold or greater between cell growth inhibition and hERG inhibition of 1500 times or more, and have an exceptionally remarkable and heterogeneous effect of extremely lower cardiotoxicity than Prexasertib (Test Example 3). Furthermore, the compounds of Examples 13 and 28 can be efficiently encapsulated in a liposome by a simple encapsulation procedure (Test Example 4), and the liposome formulations exhibit good pharmacokinetics (Test Example 5). The compounds of Examples 13 and 28 formulated in liposomes have an exceptionally remarkable effect of exhibiting an excellent anti-tumor effect in tumor-bearing mice transplanted with ES-2 cells (Test Examples 6 and 7). The compound of Example 28 formulated in a liposome has a heterogeneous effect of a significantly reduced risk of hematologic toxicity compared to the liposome-formulated Prexasertib (Test Example 8). Furthermore, the compound of Example 28 exhibits a stronger cell growth suppression effect than Prexasertib against cancer cell lines derived from soft tissue sarcoma and pancreatic cancer, and therefore has an exceptional cancer cell growth suppression effect (Test Example 9).

From the above, the compounds of Examples 13 and 28 have particularly excellent efficacy and safety among the compounds represented by formula (6), and therefore, have exceptionally remarkable and heterogeneous effects in that they can exhibit an excellent anti-tumor effect with high safety when formulated in liposomes.

As described above, the present disclosure is exemplified by the use of its preferred embodiments. It is understood that the scope of the present disclosure should be interpreted solely based on the Claims. The present application claims priority to Japanese Patent Application Nos. 2021-210843 (filed on December 24, 2021) and 2022-87175 (filed on May 27, 2022). It is understood that the entire content thereof is incorporated herein by reference. It is also understood that any patent, any patent application, and any references cited herein should be incorporated herein by reference in the same manner as the contents are specifically described herein.

### [Industrial Applicability]

The compounds of the present disclosure potently inhibit checkpoint kinase 1 (CHK1). These liposome formulations exhibit a potent anti-tumor effect based on CHK1 inhibitory action by achieving sustained drug exposure due to the sustained-release effect thereof. Therefore, the compound of the present disclosure and a pharmaceutically acceptable salt thereof, a pharmaceutical composition comprising the same, and a liposome formulation comprising the same are useful as a therapeutic agent or prophylactic agent for a pathological condition associated with CHK1.

## Claims

1. A compound represented by 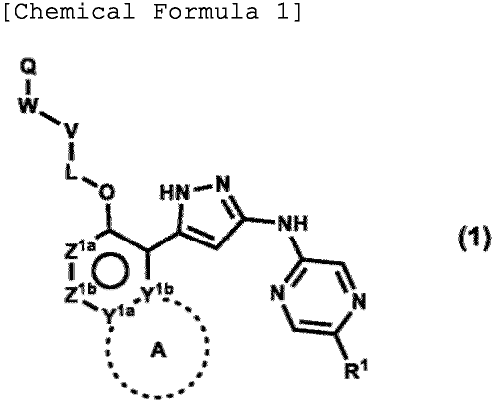
or a pharmaceutically acceptable salt thereof,
wherein
R¹ represents a hydrogen atom, a halogen atom, cyano, nitro, carboxyl, a sulfonic acid, a phosphoric acid, -OR², -SR², -COR³, -CO₂R³, -CONR⁴R⁵, -SO₂R³, -SO₂NR⁴R⁵, -OCOR³, -OCO₂R³, -OCONR⁴R⁵, -NR⁴R⁵, -NR⁶COR³, -NR⁶CO₂R³, - NR⁶CONR⁴R⁵, -NR⁶SO₂R³, -NR⁶SO₂NR⁴R⁵, optionally substituted C₁₋₆ alkyl, optionally substituted C₂₋₆ alkenyl, optionally substituted C₂₋₆ alkynyl, optionally substituted C₃₋₁₀ cycloalkyl, an optionally substituted 3- to 10-membered saturated heterocyclic group, optionally substituted C₆₋₁₀ aryl, or optionally substituted 5- to 12-membered heteroaryl,
R² represents a hydrogen atom or C₁₋₆ alkyl,
R³ represents C₁₋₆ alkyl,
R⁴, R⁵ and R⁶ represent each independently a hydrogen atom, or C₁₋₆ alkyl, wherein when R⁴ and R⁵ attached to the same nitrogen atom are both C₁₋₆ alkyl, then they may form a 3- to 8-membered nitrogen-containing saturated heterocycle together with the nitrogen atom to which each is attached,
Y^{1a} and Y^{1b} represent each independently a carbon atom, or a nitrogen atom,
Z^{1a} represents a nitrogen atom, or CR^{7a},
Z^{1b} represents a nitrogen atom, or CR^{7b},
R^{7a} and R^{7b} represent each independently a hydrogen atom, a halogen atom, cyano, nitro, carboxyl, a sulfonic acid, a phosphoric acid, -OR⁸, -SR⁸, -COR⁹, -CO₂R⁹, - CONR¹⁰R¹¹, -SO₂R⁹, -SO₂NR¹⁰R¹¹, -OCOR⁹, -OCO₂R⁹, -OCONR¹⁰R¹¹, - NR¹⁰R¹¹, -NR¹²COR⁹, -NR¹²CO₂R⁹, -NR¹²CONR¹⁰R¹¹, -NR¹²SO₂R⁹, - NR¹²SO₂NR¹⁰R¹¹, optionally substituted C₁₋₆ alkyl, optionally substituted C₂₋₆ alkenyl, optionally substituted C₂₋₆ alkynyl, optionally substituted C₃₋₁₀ cycloalkyl, an optionally substituted 3- to 10-membered saturated heterocyclic group, optionally substituted C₆₋₁₀ aryl, or optionally substituted 5- to 12-membered heteroaryl,
R⁸ represents a hydrogen atom or C₁₋₆ alkyl,
R⁹ represents C₁₋₆ alkyl,
R¹⁰, R¹¹ and R¹² represent each independently a hydrogen atom or C₁₋₆ alkyl, wherein when R¹¹ and R¹² attached to the same nitrogen atom are both C₁₋₆ alkyl, then they may form a 3- to 8-membered nitrogen-containing saturated heterocycle together with the nitrogen atom to which each is attached,
Ring A represents optionally substituted 3- to 10-membered cycloalkane, optionally substituted 6- to 10-membered arene, an optionally substituted 4- to 10-membered aromatic heterocycle, or an optionally substituted 4- to 10-membered non-aromatic heterocycle,
L represents a single bond or optionally substituted C₁₋₆ alkylene,
V represents a single bond, carbonyl, thiocarbonyl, optionally substituted C₂₋₆ alkenylene, optionally substituted C₃₋₁₀ cycloalkylene, optionally substituted C₃₋₁₀ cycloalkenylene, an optionally substituted 3- to 10-membered divalent saturated heterocyclic group, or - C(=NR¹³)-,
R¹³ represents a hydrogen atom, or C₁₋₆ alkyl,
W represents a single bond, or optionally substituted C₁₋₆ alkylene,
Q represents a hydrogen atom, or NHR¹⁴, and
R¹⁴ represents a hydrogen atom, optionally substituted C₁₋₆ alkyl, optionally substituted C₃₋₁₀ cycloalkyl, or an optionally substituted 3- to 10-membered saturated heterocyclic group.

2. The compound or the pharmaceutically acceptable salt thereof of claim 1, wherein the optionally substituted C₁₋₆ alkyl, the optionally substituted C₂₋₆ alkenyl, the optionally substituted C₂₋₆ alkynyl, the optionally substituted C₂₋₆ alkenylene, the optionally substituted C₃₋₁₀ cycloalkyl, the optionally substituted 3- to 10-membered saturated heterocyclic group, the optionally substituted 6- to 10-membered arene, the optionally substituted 4- to 10-membered aromatic heterocycle, the optionally substituted 3- to 10-membered cycloalkane, the optionally substituted 4- to 10-membered non-aromatic heterocycle, the optionally substituted C₆₋₁₀ aryl, the optionally substituted 5- to 12-membered heteroaryl, the optionally substituted C₁₋₆ alkylene, the optionally substituted C₃₋₁₀ cycloalkylene, the optionally substituted C₃₋₁₀ cycloalkenylene, or the optionally substituted 3- to 10-membered divalent saturated heterocyclic group in R¹, R^{7a}, R^{7b}, R¹⁴, Ring A, L, V, and W are each independently optionally substituted with 1 to 5 of the same or different substituents selected from the group consisting of
(1) a halogen atom,
(2) a hydroxyl group,
(3) C₆₋₁₀ aryl,
(4) 5- to 12-membered heteroaryl,
(5) C₁₋₆ alkyl,
(6) C₂₋₆ alkenyl,
(7) C₂₋₆ alkynyl,
(8) C₁₋₆ alkoxy,
(9) C₁₋₆ alkylthio
(10) C₃₋₁₀ cycloalkyl,
(11) a 3- to 10-membered saturated heterocyclic group,
(12) carboxyl,
(13) -COR¹⁵,
(14) -CO₂R¹⁵,
(15) -CONR¹⁶R¹⁷,
(16) -NR¹⁶R¹⁷,
(17) -NR¹⁸COR¹⁵,
(18) -NR¹⁸CO₂R¹⁵,
(19) -NR¹⁸SO₂R¹⁵,
(20) -NR¹⁸CONR¹⁶R¹⁷,
(21) -NR¹⁸SO₂NR¹⁶R¹⁷,
(22) -SO₂R¹⁵,
(23) -SO₂NR¹⁶R¹⁷,
(24) -OCOR¹⁵,
(25) -OCO₂R¹⁵,
(26) -OCONR¹⁶R¹⁷,
(27) a sulfonic acid,
(28) a phosphoric acid,
(29) cyano, and
(30) nitro,
wherein the groups represented by the (3) C₆₋₁₀ aryl, (4) 5- to 12-membered heteroaryl, (5) C₁₋₆ alkyl, (6) C₂₋₆ alkenyl, (7) C₂₋₆ alkynyl, (8) C₁₋₆ alkoxy, (9) C₁₋₆ alkylthio, (10) C₃₋₁₀ cycloalkyl and (11) a 3- to 10-membered saturated heterocyclic group are optionally substituted with 1 to 5 of the same or different substituents selected from the group consisting of
(a) a halogen atom,
(b) a hydroxyl group,
(c) C₆₋₁₀ aryl,
(d) 5- to 12-membered heteroaryl,
(e) C₁₋₆ alkyl,
(f) C₂₋₆ alkenyl,
(g) C₂₋₆ alkynyl,
(h) C₁₋₆ alkoxy,
(i) C₃₋₁₀ cycloalkyl,
(j) a 3- to 10-membered saturated heterocyclic group,
(k) carboxyl,
(l) -COR¹⁵,
(m) -CO₂R¹⁵,
(n) -CONR¹⁶R¹⁷,
(o) -NR¹⁶R¹⁷,
(p) -NR¹⁸COR¹⁵,
(q) -NR¹⁸SO₂R¹⁵,
(r) -SO₂R¹⁵,
(s) -SO₂NR¹⁶R¹⁷,
(t) a sulfonic acid,
(u) a phosphoric acid,
(v) cyano, and
(w) nitro,
R¹⁵ is C₁₋₆ alkyl, and when plural, then each is independent,
R¹⁶ and R¹⁷ represent each independently a hydrogen atom or C₁₋₆ alkyl, and when R¹⁶ or R¹⁷ is plural, then each of R¹⁶ or R¹⁷ may be the same or different, wherein when R¹⁶ and R¹⁷ attached to the same nitrogen atom are both C₁₋₆ alkyl, then they may form a 3- to 8-membered nitrogen-containing saturated heterocycle together with the nitrogen atom to which each is attached, and
R¹⁸ is a hydrogen atom or C₁₋₆ alkyl.

3. The compound or the pharmaceutically acceptable salt thereof of any one of claims 1 to 2, wherein R¹ is a hydrogen atom, a halogen atom, cyano, -OR², -CO₂R³, C₁₋₆ alkyl, C₃₋₁₀ cycloalkyl, or a 3- to 10-membered saturated heterocyclic group.

4. The compound or the pharmaceutically acceptable salt thereof of any one of claims 1 to 2, wherein R¹ is a hydrogen atom, a halogen atom, cyano, or -CO₂R³.

5. The compound or the pharmaceutically acceptable salt thereof of any one of claims 1 to 2, wherein R¹ is cyano.

6. The compound or the pharmaceutically acceptable salt thereof of any one of claims 1 to 5, wherein
R^{7a} and R^{7b} are each independently a hydrogen atom, a halogen atom, cyano, -OR⁸, -CO₂R⁹, -CONR¹⁰R¹¹, -NR¹⁰R¹¹, - NR¹²COR⁹, C₁₋₆ alkyl (the alkyl is optionally substituted with 1 to 3 of the same or different substituents selected from the group consisting of a fluorine atom, a chlorine atom, a bromine atom, a hydroxyl group, phenyl, 5- to 6-membered heteroaryl, C₁₋₆ alkoxy, C₃₋₇ cycloalkyl, a 3- to 7-membered saturated heterocyclic group, - CONR¹⁶R¹⁷, -NR¹⁶R¹⁷, and cyano), C₃₋₁₀ cycloalkyl (the cycloalkyl is optionally substituted with 1 to 3 of the same or different substituents selected from the group consisting of a fluorine atom, a chlorine atom, a bromine atom, a hydroxyl group, phenyl, 5- to 6-membered heteroaryl, C₁₋₆ alkyl, C₁₋₆ alkoxy, C₃₋₇ cycloalkyl, a 3-to 7-membered saturated heterocyclic group, -CONR¹⁶R¹⁷, - NR¹⁶R¹⁷, and cyano), a 3- to 10-membered saturated heterocyclic group (the saturated heterocyclic group is optionally substituted with 1 to 3 of the same or different substituents selected from the group consisting of a fluorine atom, a chlorine atom, a bromine atom, a hydroxyl group, phenyl, 5- to 6-membered heteroaryl, C₁₋₆ alkyl, C₁₋₆ alkoxy, C₃₋₇ cycloalkyl, a 3- to 7-membered saturated heterocyclic group, -CONR¹⁶R¹⁷, -NR¹⁶R¹⁷, and cyano), phenyl (the phenyl is optionally substituted with 1 to 3 of the same or different substituents selected from the group consisting of a fluorine atom, a chlorine atom, a bromine atom, a hydroxyl group, phenyl, 5- to 6-membered heteroaryl, C₁₋₆ alkyl, C₁₋₆ alkoxy, C₃₋₇ cycloalkyl, a 3- to 7-membered saturated heterocyclic group, -CONR¹⁶R¹⁷, -NR¹⁶R¹⁷, and cyano), or 5- to 6-membered heteroaryl (the heteroaryl is optionally substituted with 1 to 3 of the same or different substituents selected from the group consisting of a fluorine atom, a chlorine atom, a bromine atom, a hydroxyl group, phenyl, 5- to 6-membered heteroaryl, C₁₋₆ alkyl, C₁₋₆ alkoxy, C₃₋₇ cycloalkyl, a 3- to 7-membered saturated heterocyclic group, -CONR¹⁶R¹⁷, -NR¹⁶R¹⁷, and cyano), and
R¹⁶ and R¹⁷ represent each independently a hydrogen atom or C₁₋₆ alkyl, and when R¹⁶ or R¹⁷ is plural, then each of R¹⁶ or R¹⁷ may be the same or different, wherein when R¹⁶ and R¹⁷ attached to the same nitrogen atom are both C₁₋₆ alkyl, then they may form a 3- to 8-membered nitrogen-containing saturated heterocycle together with the nitrogen atom to which each is attached.

7. The compound or the pharmaceutically acceptable salt thereof of any one of claims 1 to 5, wherein
R^{7a} and R^{7b} are each independently
a hydrogen atom,
a fluorine atom,
a chlorine atom,
a bromine atom,
C₁₋₆ alkyl (the alkyl is optionally substituted with 1 to 2 of the same or different substituents selected from the group consisting of a fluorine atom, a hydroxyl group, C₁₋₃ alkoxy, -NR¹⁶R¹⁷, and cyano), or
5- to 6-membered heteroaryl (the heteroaryl is optionally substituted with 1 to 2 of the same or different substituents selected from the group consisting of a fluorine atom, a hydroxyl group, C₁₋₃ alkyl, C₁₋₃ alkoxy, - NR¹⁶R¹⁷, and cyano), and
R¹⁶ and R¹⁷ represent each independently a hydrogen atom or C₁₋₆ alkyl, and when R¹⁶ or R¹⁷ is plural, then each of R¹⁶ or R¹⁷ may be the same or different, wherein when R¹⁶ and R¹⁷ attached to the same nitrogen atom are both C₁₋₆ alkyl, then they may form a 3- to 8-membered nitrogen-containing saturated heterocycle together with the nitrogen atom to which each is attached.

8. The compound or the pharmaceutically acceptable salt thereof of any one of claims 1 to 7, wherein
Ring A is
3- to 10-membered cycloalkane (the cycloalkane is optionally substituted with 1 to 3 of the same or different substituents selected from the group consisting of a fluorine atom, a chlorine atom, a bromine atom, a hydroxyl group, phenyl, 5- to 6-membered heteroaryl, C₁₋₆ alkyl, C₁₋₆ alkoxy, C₃₋₇ cycloalkyl, a 3- to 7-membered saturated heterocyclic group, -CONR¹⁶R¹⁷, -NR¹⁶R¹⁷, and cyano),
6- to 10-membered arene (the arene is optionally substituted with 1 to 3 of the same or different substituents selected from the group consisting of a fluorine atom, a chlorine atom, a bromine atom, a hydroxyl group, phenyl, 5- to 6-membered heteroaryl, C₁₋₆ alkyl, C₁₋₆ alkoxy, C₃₋₇ cycloalkyl, a 3- to 7-membered saturated heterocyclic group, -CONR¹⁶R¹⁷, -NR¹⁶R¹⁷, and cyano),
a 4- to 10-membered aromatic heterocycle (the aromatic heterocycle is optionally substituted with 1 to 3 of the same or different substituents selected from the group consisting of a fluorine atom, a chlorine atom, a bromine atom, a hydroxyl group, phenyl, 5- to 6-membered heteroaryl, C₁₋₆ alkyl, C₁₋₆ alkoxy, C₃₋₇ cycloalkyl, a 3-to 7-membered saturated heterocyclic group, -CONR¹⁶R¹⁷, - NR¹⁶R¹⁷, and cyano), or
a 4- to 10-membered non-aromatic heterocycle (the non-aromatic heterocycle is optionally substituted with 1 to 3 of the same or different substituents selected from the group consisting of a fluorine atom, a chlorine atom, a bromine atom, a hydroxyl group, phenyl, 5- to 6-membered heteroaryl, C₁₋₆ alkyl, C₁₋₆ alkoxy, C₃₋₇ cycloalkyl, a 3-to 7-membered saturated heterocyclic group, -CONR¹⁶R¹⁷, - NR¹⁶R¹⁷, and cyano), and
R¹⁶ and R¹⁷ represent each independently a hydrogen atom or C₁₋₆ alkyl, and when R¹⁶ or R¹⁷ is plural, then each of R¹⁶ or R¹⁷ may be the same or different, wherein when R¹⁶ and R¹⁷ attached to the same nitrogen atom are both C₁₋₆ alkyl, then they may form a 3- to 8-membered nitrogen-containing saturated heterocycle together with the nitrogen atom to which each is attached.

9. The compound or the pharmaceutically acceptable salt thereof of any one of claims 1 to 7, wherein
Ring A is
a 4- to 10-membered aromatic heterocycle (the aromatic heterocycle is optionally substituted with 1 to 2 of the same or different substituents selected from the group consisting of a fluorine atom, a hydroxyl group, C₁₋₃ alkyl, C₁₋₃ alkoxy, -NR¹⁶R¹⁷, and cyano), or
a 4- to 10-membered non-aromatic heterocycle (the non-aromatic heterocycle is optionally substituted with 1 to 2 of the same or different substituents selected from the group consisting of a fluorine atom, a hydroxyl group, C₁₋₃ alkyl, C₁₋₃ alkoxy, -NR¹⁶R¹⁷, and cyano), and
R¹⁶ and R¹⁷ represent each independently a hydrogen atom or C₁₋₆ alkyl, and when R¹⁶ or R¹⁷ is plural, then each of R¹⁶ or R¹⁷ may be the same or different, wherein when R¹⁶ and R¹⁷ attached to the same nitrogen atom are both C₁₋₆ alkyl, then they may form a 3- to 8-membered nitrogen-containing saturated heterocycle together with the nitrogen atom to which each is attached.

10. The compound or the pharmaceutically acceptable salt thereof of any one of claims 1 to 9, wherein
L is
a single bond, or
C₁₋₆ alkylene (the alkylene is optionally substituted with 1 to 3 of the same or different substituents selected from the group consisting of a fluorine atom, a hydroxyl group, C₁₋₃ alkyl, C₁₋₃ alkoxy, -NR¹⁶R¹⁷, and cyano), and
R¹⁶ and R¹⁷ represent each independently a hydrogen atom or C₁₋₆ alkyl, and when R¹⁶ or R¹⁷ is plural, then each of R¹⁶ or R¹⁷ may be the same or different, wherein when R¹⁶ and R¹⁷ attached to the same nitrogen atom are both C₁₋₆ alkyl, then they may form a 3- to 8-membered nitrogen-containing saturated heterocycle together with the nitrogen atom to which each is attached.

11. The compound or the pharmaceutically acceptable salt thereof of any one of claims 1 to 10, wherein
V is
a single bond,
carbonyl,
thiocarbonyl,
C₂₋₆ alkenylene (the alkenylene is optionally substituted with 1 to 3 of the same or different substituents selected from the group consisting of a fluorine atom, a hydroxyl group, C₁₋₃ alkyl, C₁₋₃ alkoxy, -NR¹⁶R¹⁷, and cyano),
C₃₋₁₀ cycloalkylene (the cycloalkylene is optionally substituted with 1 to 3 of the same or different substituents selected from the group consisting of a fluorine atom, a hydroxyl group, C₁₋₃ alkyl, C₁₋₃ alkoxy, - NR¹⁶R¹⁷, and cyano),
C₃₋₁₀ cycloalkenylene (the cycloalkenylene is optionally substituted with 1 to 3 of the same or different substituents selected from the group consisting of a fluorine atom, a hydroxyl group, C₁₋₃ alkyl, C₁₋₃ alkoxy, - NR¹⁶R¹⁷, and cyano),
a 3- to 10-membered divalent saturated heterocyclic group (the saturated heterocyclic group is optionally substituted with 1 to 3 of the same or different substituents selected from the group consisting of a fluorine atom, a hydroxyl group, C₁₋₃ alkyl optionally substituted with 1 to 2 hydroxyl groups or fluorine atoms, C₁₋₃ alkoxy, -NR¹⁶R¹⁷, and cyano), or
-C(=NR¹³)-, and
R¹⁶ and R¹⁷ represent each independently a hydrogen atom or C₁₋₆ alkyl, and when R¹⁶ or R¹⁷ is plural, then each of R¹⁶ or R¹⁷ may be the same or different, wherein when R¹⁶ and R¹⁷ attached to the same nitrogen atom are both C₁₋₆ alkyl, then they may form a 3- to 8-membered nitrogen-containing saturated heterocycle together with the nitrogen atom to which each is attached.

12. The compound or the pharmaceutically acceptable salt thereof of any one of claims 1 to 11, wherein
W is
a single bond, or
C₁₋₆ alkylene (the alkylene is optionally substituted with 1 to 3 of the same or different substituents selected from the group consisting of a fluorine atom, a hydroxyl group, C₁₋₃ alkoxy, -NR¹⁶R¹⁷, and cyano), and
R¹⁶ and R¹⁷ represent each independently a hydrogen atom or C₁₋₆ alkyl, and when R¹⁶ or R¹⁷ is plural, then each of R¹⁶ or R¹⁷ may be the same or different, wherein when R¹⁶ and R¹⁷ attached to the same nitrogen atom are both C₁₋₆ alkyl, then they may form a 3- to 8-membered nitrogen-containing saturated heterocycle together with the nitrogen atom to which each is attached.

13. The compound or the pharmaceutically acceptable salt thereof of any one of claims 1 to 12, wherein
R¹⁴ is
a hydrogen atom,
C₁₋₆ alkyl (the alkyl is optionally substituted with 1 to 3 of the same or different substituents selected from the group consisting of a fluorine atom, a hydroxyl group, C₁₋₃ alkoxy, -NR¹⁶R¹⁷, and cyano),
C₃₋₁₀ cycloalkyl (the cycloalkyl is optionally substituted with 1 to 3 of the same or different substituents selected from the group consisting of a fluorine atom, a hydroxyl group, C₁₋₃ alkyl, C₁₋₃ alkoxy, -NR¹⁶R¹⁷, and cyano), or
a 3- to 10-membered saturated heterocyclic group (the saturated heterocyclic group is optionally substituted with 1 to 3 of the same or different substituents selected from the group consisting of a fluorine atom, a hydroxyl group, C₁₋₃ alkyl, C₁₋₃ alkoxy, -NR¹⁶R¹⁷, and cyano), and
R¹⁶ and R¹⁷ represent each independently a hydrogen atom or C₁₋₆ alkyl, and when R¹⁶ or R¹⁷ is plural, then each of R¹⁶ or R¹⁷ may be the same or different, wherein when R¹⁶ and R¹⁷ attached to the same nitrogen atom are both C₁₋₆ alkyl, then they may form a 3- to 8-membered nitrogen-containing saturated heterocycle together with the nitrogen atom to which each is attached.

14. The compound or the pharmaceutically acceptable salt thereof of claim 1, wherein formula (1) is represented by the following formula (2): 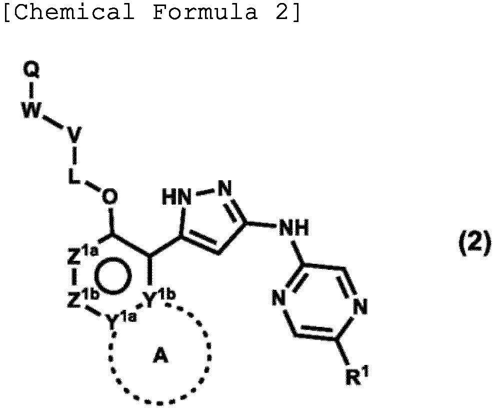 wherein
R¹ represents a hydrogen atom, a halogen atom, cyano, or -CO₂R³,
R³ represents C₁₋₆ alkyl,
Y^{1a} and Y^{1b} represent each independently a carbon atom, or a nitrogen atom,
Z^{1a} represents a nitrogen atom, or CR^{7a},
Z^{1b} represents a nitrogen atom, or CR^{7b},
R^{7a} and R^{7b} represent each independently
a hydrogen atom,
a fluorine atom,
a chlorine atom,
a bromine atom,
C₁₋₆ alkyl (the alkyl is optionally substituted with 1 to 2 of the same or different substituents selected from the group consisting of a fluorine atom, a hydroxyl group, C₁₋₃ alkoxy, -NR¹⁶R¹⁷, and cyano), or
5- to 6-membered heteroaryl (the heteroaryl is optionally substituted with 1 to 2 of the same or different substituents selected from the group consisting of a fluorine atom, a hydroxyl group, C₁₋₃ alkyl, C₁₋₃ alkoxy, - NR¹⁶R¹⁷, and cyano),
Ring A represents
a 5- to 6-membered aromatic heterocycle (the aromatic heterocycle is optionally substituted with 1 to 2 of the same or different substituents selected from the group consisting of a fluorine atom, a hydroxyl group, C₁₋₃ alkyl, C₁₋₃ alkoxy, -NR¹⁶R¹⁷, and cyano), or
a 5- to 8-membered non-aromatic heterocycle (the non-aromatic heterocycle is optionally substituted with 1 to 2 of the same or different substituents selected from the group consisting of a fluorine atom, a hydroxyl group, C₁₋₃ alkyl, C₁₋₃ alkoxy, -NR¹⁶R¹⁷, and cyano),
L represents
a single bond, or
C₁₋₆ alkylene (the alkylene is optionally substituted with 1 to 3 of the same or different substituents selected from the group consisting of a fluorine atom, a hydroxyl group, C₁₋₃ alkyl, C₁₋₃ alkoxy, -NR¹⁶R¹⁷, and cyano),
V represents
a single bond,
C₂₋₆ alkenylene (the alkenylene is optionally substituted with 1 to 3 of the same or different substituents selected from the group consisting of a fluorine atom, a hydroxyl group, C₁₋₃ alkyl, C₁₋₃ alkoxy, -NR¹⁶R¹⁷, and cyano),
C₃₋₁₀ cycloalkylene (the cycloalkylene is optionally substituted with 1 to 3 of the same or different substituents selected from the group consisting of a fluorine atom, a hydroxyl group, C₁₋₃ alkyl, C₁₋₃ alkoxy, - NR¹⁶R¹⁷, and cyano),
C₃₋₁₀ cycloalkenylene (the cycloalkenylene is optionally substituted with 1 to 3 of the same or different substituents selected from the group consisting of a fluorine atom, a hydroxyl group, C₁₋₃ alkyl, C₁₋₃ alkoxy, - NR¹⁶R¹⁷, and cyano), or
a 3- to 10-membered divalent saturated heterocyclic group (the saturated heterocyclic group is optionally substituted with 1 to 3 of the same or different substituents selected from the group consisting of a fluorine atom, a hydroxyl group, C₁₋₃ alkyl optionally substituted with 1 to 2 fluorine atoms, C₁₋₃ alkoxy, - NR¹⁶R¹⁷, and cyano),
W represents
a single bond, or
C₁₋₆ alkylene (the alkylene is optionally substituted with 1 to 3 of the same or different substituents selected from the group consisting of a fluorine atom, a hydroxyl group, C₁₋₃ alkoxy, -NR¹⁶R¹⁷, and cyano),
Q represents
a hydrogen atom, or
NHR¹⁴,
R¹⁴ represents
a hydrogen atom,
C₁₋₆ alkyl (the alkyl is optionally substituted with 1 to 3 of the same or different substituents selected from the group consisting of a fluorine atom, a hydroxyl group, C₁₋₃ alkoxy, -NR¹⁶R¹⁷, and cyano),
C₃₋₁₀ cycloalkyl (the cycloalkyl is optionally substituted with 1 to 3 of the same or different substituents selected from the group consisting of a fluorine atom, a hydroxyl group, C₁₋₃ alkyl, C₁₋₃ alkoxy, -NR¹⁶R¹⁷, and cyano), or
a 3- to 10-membered saturated heterocyclic group (the saturated heterocyclic group is optionally substituted with 1 to 3 of the same or different substituents selected from the group consisting of a fluorine atom, a hydroxyl group, C₁₋₃ alkyl, C₁₋₃ alkoxy, -NR¹⁶R¹⁷, and cyano), and
R¹⁶ and R¹⁷ represent each independently a hydrogen atom, or C₁₋₆ alkyl, and when R¹⁶ or R¹⁷ is plural, then each of R¹⁶ or R¹⁷ may be the same or different, wherein when R¹⁶ and R¹⁷ attached to the same nitrogen atom are both C₁₋₆ alkyl, then they may form a 3- to 8-membered nitrogen-containing saturated heterocycle together with the nitrogen atom to which each is attached.

15. The compound or the pharmaceutically acceptable salt thereof of claim 14, wherein R¹ is cyano.

16. The compound or the pharmaceutically acceptable salt thereof of any one of claims 1 to 15, wherein Z^{1a} is CR^{7a}.

17. The compound or the pharmaceutically acceptable salt thereof of any one of claims 1 to 16, wherein Z^{1b} is CR^{7b}.

18. The compound or the pharmaceutically acceptable salt thereof of any one of claims 1 to 16, wherein Z^{1b} is a nitrogen atom.

19. The compound or the pharmaceutically acceptable salt thereof of any one of claims 1 to 18, wherein R^{7a} and R^{7b} are each independently
a hydrogen atom,
a fluorine atom,
a chlorine atom,
a bromine atom, or
C₁₋₆ alkyl (the alkyl is optionally substituted with 1 to 2 of the same or different substituents selected from the group consisting of a fluorine atom, a hydroxyl group, and C₁₋₃ alkoxy) .

20. The compound or the pharmaceutically acceptable salt thereof of any one of claims 1 to 18, wherein R^{7a} and R^{7b} are each independently a hydrogen atom, a fluorine atom, a chlorine atom, a bromine atom, or C₁₋₃ alkyl.

21. The compound or the pharmaceutically acceptable salt thereof of any one of claims 1 to 18, wherein R^{7a} and R^{7b} are hydrogen atoms.

22. The compound or the pharmaceutically acceptable salt thereof of any one of claims 1 to 21, wherein L is a single bond, or
C₁₋₆ alkylene (the alkylene is optionally substituted with 1 to 2 of the same or different substituents selected from the group consisting of a fluorine atom, a hydroxyl group, C₁₋₃ alkyl, and cyano) .

23. The compound or the pharmaceutically acceptable salt thereof of any one of claims 1 to 22, wherein V is a single bond,
C₂₋₆ alkenylene (the alkenylene is optionally substituted with 1 to 3 of the same or different substituents selected from the group consisting of a fluorine atom, a hydroxyl group, C₁₋₃ alkyl, and cyano),
C₃₋₁₀ cycloalkylene (the cycloalkylene is optionally substituted with 1 to 3 of the same or different substituents selected from the group consisting of a fluorine atom, a hydroxyl group, C₁₋₃ alkyl, and cyano),
C₃₋₁₀ cycloalkenylene (the cycloalkenylene is optionally substituted with 1 to 3 of the same or different substituents selected from the group consisting of a fluorine atom, a hydroxyl group, C₁₋₃ alkyl, and cyano), or
a 3- to 10-membered divalent saturated heterocyclic group (the saturated heterocyclic group is optionally substituted with 1 to 3 of the same or different substituents selected from the group consisting of a fluorine atom, a hydroxyl group, C₁₋₃ alkyl optionally substituted with 1 to 2 fluorine atoms, and cyano).

24. The compound or the pharmaceutically acceptable salt thereof of any one of claims 1 to 23, wherein W is a single bond, or
C₁₋₆ alkylene (the alkylene is optionally substituted with 1 to 2 of the same or different substituents selected from the group consisting of a fluorine atom, a hydroxyl group, and cyano).

25. The compound or the pharmaceutically acceptable salt thereof of any one of claims 1 to 24, wherein R¹⁴ is a hydrogen atom, or
C₁₋₆ alkyl (the alkyl is optionally substituted with 1 to 2 of the same or different substituents selected from the group consisting of a fluorine atom, a hydroxyl group, and cyano).

26. The compound or the pharmaceutically acceptable salt thereof of any one of claims 1 to 25, wherein Q is a hydrogen atom,
NH₂, or
NMeH.

27. The compound or the pharmaceutically acceptable salt thereof of any one of claims 1 to 26, wherein Ring A is a 5- to 6-membered aromatic heterocycle (the aromatic heterocycle is optionally substituted with 1 to 2 of the same or different substituents selected from the group consisting of a fluorine atom, a hydroxyl group, C₁₋₃ alkyl, C₁₋₃ alkoxy, and cyano) .

28. The compound or the pharmaceutically acceptable salt thereof of any one of claims 1 to 26, wherein Ring A is a 5- to 8-membered non-aromatic heterocycle (the non-aromatic heterocycle is optionally substituted with 1 to 2 of the same or different substituents selected from the group consisting of a fluorine atom, a hydroxyl group, C₁₋₃ alkyl, C₁₋₃ alkoxy, and cyano) .

29. The compound or the pharmaceutically acceptable salt thereof of claim 1, wherein formula (1) is represented by the following formula (3): 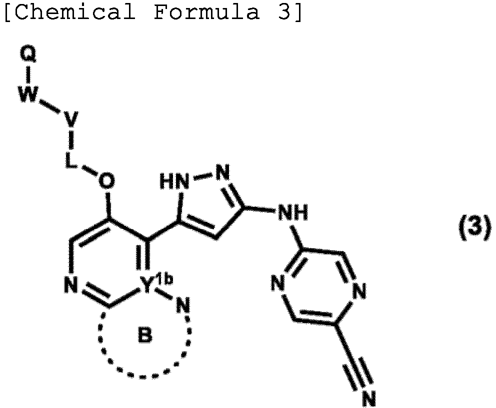 wherein
Y^{1b} represents
a carbon atom, or
a nitrogen atom,
Ring B represents a 5- to 6-membered aromatic heterocycle (the aromatic heterocycle is optionally substituted with 1 to 2 of the same or different substituents selected from the group consisting of a fluorine atom, a hydroxyl group, C₁₋₃ alkyl, C₁₋₃ alkoxy, and cyano),
L represents
a single bond, or
C₁₋₆ alkylene (the alkylene is optionally substituted with 1 to 2 substituents selected from the group consisting of a fluorine atom, a hydroxyl group, C₁₋₃ alkyl, and cyano),
V represents
a single bond,
C₂₋₆ alkenylene (the alkenylene is optionally substituted with 1 to 3 of the same or different substituents selected from the group consisting of a fluorine atom, a hydroxyl group, C₁₋₃ alkyl, and cyano),
C₃₋₁₀ cycloalkylene (the cycloalkylene is optionally substituted with 1 to 3 of the same or different substituents selected from the group consisting of a fluorine atom, a hydroxyl group, C₁₋₃ alkyl, and cyano),
C₃₋₁₀ cycloalkenylene (the cycloalkenylene is optionally substituted with 1 to 3 of the same or different substituents selected from the group consisting of a fluorine atom, a hydroxyl group, C₁₋₃ alkyl, and cyano), or
a 3- to 10-membered divalent saturated heterocyclic group (the saturated heterocyclic group is optionally substituted with 1 to 3 of the same or different substituents selected from the group consisting of a fluorine atom, a hydroxyl group, C₁₋₃ alkyl optionally substituted with 1 to 2 hydroxyl groups or fluorine atoms, and cyano),
W represents
a single bond, or
C₁₋₃ alkylene (the alkylene is optionally substituted with 1 to 2 substituents selected from the group consisting of a fluorine atom, a hydroxyl group, and cyano),
Q represents
a hydrogen atom, or
NHR¹⁴, and
R¹⁴ is
a hydrogen atom, or
C₁₋₃ alkyl (the alkyl is optionally substituted with one substituent selected from the group consisting of a fluorine atom, a hydroxyl group, and cyano).

30. The compound or the pharmaceutically acceptable salt thereof of claim 29, wherein Ring B is an unsubstituted 5- to 6-membered aromatic heterocycle.

31. The compound or the pharmaceutically acceptable salt thereof of claim 29 or 30, wherein Y^{1b} is a carbon atom.

32. The compound or the pharmaceutically acceptable salt thereof of any one of claims 1 to 31, wherein L is a single bond, or
C₁₋₆ alkylene (the alkylene is optionally substituted with 1 to 2 of the same or different substituents selected from the group consisting of a fluorine atom or C₁₋₃ alkyl).

33. The compound or the pharmaceutically acceptable salt thereof of any one of claims 1 to 32, wherein V is a single bond,
C₂₋₆ alkenylene (the alkenylene is optionally substituted with one substituent selected from the group consisting of a hydroxyl group, and C₁₋₃ alkyl),
C₃₋₁₀ cycloalkylene (the cycloalkylene is optionally substituted with 1 to 3 substituents selected from the group consisting of a fluorine atom, a hydroxyl group, and C₁₋₃ alkyl),
C₃₋₁₀ cycloalkenylene (the cycloalkenylene is optionally substituted with one substituent selected from the group consisting of a hydroxyl group, and C₁₋₃ alkyl), or
a 3- to 10-membered divalent saturated heterocyclic group (the saturated heterocyclic group is optionally substituted with one substituent selected from the group consisting of a fluorine atom, a hydroxyl group, and C₁₋₃ alkyl optionally substituted with 1 to 2 fluorine atoms).

34. The compound or the pharmaceutically acceptable salt thereof of any one of claims 1 to 33, wherein W is a single bond, or C₁₋₃ alkylene optionally substituted with 1 to 2 fluorine atoms.

35. The compound or the pharmaceutically acceptable salt thereof of any one of claims 1 to 34, wherein R¹⁴ is a hydrogen atom, or
C₁₋₆ alkyl.

36. The compound or the pharmaceutically acceptable salt thereof of any one of claims 1 to 35, wherein Q is a hydrogen atom, or
NH₂.

37. The compound or the pharmaceutically acceptable salt thereof of claim 1, wherein formula (1) is represented by the following formula (4): 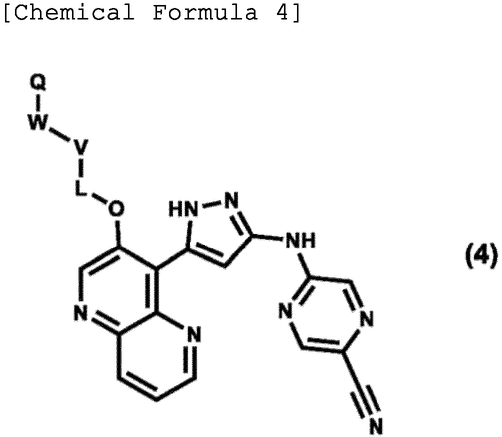 wherein
L represents
a single bond, or
C₁₋₆ alkylene (the alkylene is optionally substituted with 1 to 2 of the same or different substituents selected from the group consisting of a fluorine atom or C₁₋₃ alkyl),
V represents
a single bond,
C₂₋₆ alkenylene (the alkenylene is optionally substituted with one substituent selected from the group consisting of a hydroxyl group, and C₁₋₃ alkyl),
C₃₋₁₀ cycloalkylene (the cycloalkylene is optionally substituted with 1 to 3 substituents selected from the group consisting of a fluorine atom, a hydroxyl group, and C₁₋₃ alkyl),
C₃₋₁₀ cycloalkenylene (the cycloalkenylene is optionally substituted with one substituent selected from the group consisting of a hydroxyl group, and C₁₋₃ alkyl), or
a 3- to 10-membered divalent saturated heterocyclic group (the saturated heterocyclic group is optionally substituted with one substituent selected from the group consisting of a fluorine atom, a hydroxyl group, and C₁₋₃ alkyl optionally substituted with 1 to 2 fluorine atoms),
W represents
a single bond, or
C₁₋₃ alkylene optionally substituted with 1 to 2 fluorine atoms, and
Q represents
a hydrogen atom, or
NH₂.

38. The compound or the pharmaceutically acceptable salt thereof of any one of claims 1 to 28 and 37, wherein
V is
a single bond,
C₂₋₆ alkenylene,
C₃₋₁₀ cycloalkylene (the cycloalkylene is optionally substituted with 1 to 3 fluorine atoms),
C₃₋₁₀ cycloalkenylene, or
a 3- to 10-membered divalent saturated heterocyclic group (the saturated heterocyclic group is optionally substituted with one substituent selected from the group consisting of a fluorine atom, and C₁₋₃ alkyl optionally substituted with 1 to 2 fluorine atoms).

39. The compound or the pharmaceutically acceptable salt thereof of any one of claims 1 to 28 and 37 to 38, wherein Q is a hydrogen atom.

40. The compound or the pharmaceutically acceptable salt thereof of any one of claims 1 to 28 and 37 to 38, wherein Q is NH₂.

41. The compound or the pharmaceutically acceptable salt thereof of claim 1, wherein formula (1) is represented by the following formula (5): 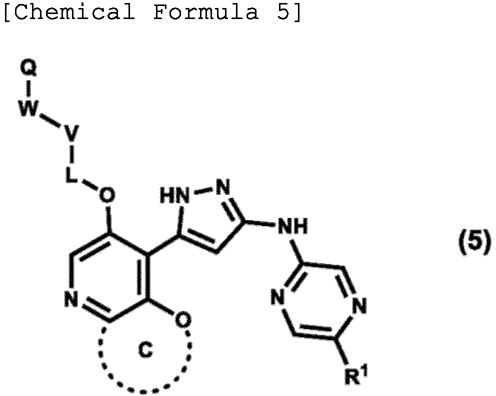 wherein
R¹ represents a hydrogen atom, a halogen atom, cyano, or -CO₂R³,
R³ represents C₁₋₆ alkyl,
Ring C represents a 5- to 8-membered non-aromatic heterocycle (the non-aromatic heterocycle is optionally substituted with 1 to 2 of the same or different substituents selected from the group consisting of a fluorine atom, a hydroxyl group, C₁₋₃ alkyl, C₁₋₃ alkoxy, and cyano),
L represents
a single bond, or
C₁₋₆ alkylene (the alkylene is optionally substituted with 1 to 2 substituents selected from the group consisting of a fluorine atom, a hydroxyl group, C₁₋₃ alkyl, and cyano),
V represents
a single bond,
C₂₋₆ alkenylene (the alkenylene is optionally substituted with 1 to 3 of the same or different substituents selected from the group consisting of a fluorine atom, a hydroxyl group, C₁₋₃ alkyl, and cyano),
C₃₋₁₀ cycloalkylene (the cycloalkylene is optionally substituted with 1 to 3 of the same or different substituents selected from the group consisting of a fluorine atom, a hydroxyl group, C₁₋₃ alkyl, and cyano),
C₃₋₁₀ cycloalkenylene (the cycloalkenylene is optionally substituted with 1 to 3 of the same or different substituents selected from the group consisting of a fluorine atom, a hydroxyl group, C₁₋₃ alkyl, and cyano), or
a 3- to 10-membered divalent saturated heterocyclic group (the saturated heterocyclic group is optionally substituted with 1 to 3 of the same or different substituents selected from the group consisting of a fluorine atom, a hydroxyl group, C₁₋₃ alkyl optionally substituted with 1 to 2 hydroxyl groups or fluorine atoms, and cyano),
W represents
a single bond, or
C₁₋₃ alkylene (the alkylene is optionally substituted with 1 to 2 substituents selected from the group consisting of a fluorine atom, a hydroxyl group, and cyano),
Q represents
a hydrogen atom, or
NHR¹⁴, and
R¹⁴ is
a hydrogen atom, or
C₁₋₃ alkyl (the alkyl is optionally substituted with one substituent selected from the group consisting of a fluorine atom, a hydroxyl group, and cyano).

42. The compound or the pharmaceutically acceptable salt thereof of claim 41, wherein R¹ is cyano.

43. The compound or the pharmaceutically acceptable salt thereof of claim 41 or 42, wherein Ring C is an unsubstituted 5- to 8-membered non-aromatic heterocycle.

44. The compound or the pharmaceutically acceptable salt thereof of any one of claims 1 to 28 and 41 to 43, wherein L is
a single bond, or
C₁₋₆ alkylene (the alkylene is optionally substituted with 1 to 2 of the same or different substituents selected from the group consisting of a fluorine atom, or C₁₋₃ alkyl).

45. The compound or the pharmaceutically acceptable salt thereof of any one of claims 1 to 28 and 41 to 44, wherein V is
a single bond,
C₂₋₆ alkenylene (the alkenylene is optionally substituted with one substituent selected from the group consisting of a hydroxyl group, and C₁₋₃ alkyl),
C₃₋₁₀ cycloalkylene (the cycloalkylene is optionally substituted with one substituent selected from the group consisting of a hydroxyl group, and C₁₋₃ alkyl),
C₃₋₁₀ cycloalkenylene (the cycloalkenylene is optionally substituted with one substituent selected from the group consisting of a hydroxyl group, and C₁₋₃ alkyl), or
a 3- to 10-membered divalent saturated heterocyclic group (the saturated heterocyclic group is optionally substituted with one substituent selected from the group consisting of a fluorine atom, a hydroxyl group, and C₁₋₃ alkyl optionally substituted with 1 to 2 hydroxyl groups or fluorine atoms).

46. The compound or the pharmaceutically acceptable salt thereof of any one of claims 1 to 28 and 41 to 45, wherein W is
a single bond, or
C₁₋₃ alkylene.

47. The compound or the pharmaceutically acceptable salt thereof of any one of claims 1 to 28 and 41 to 46, wherein R¹⁴ is
a hydrogen atom, or
C₁₋₆ alkyl.

48. The compound or the pharmaceutically acceptable salt thereof of any one of claims 1 to 28 and 41 to 47, wherein Q is
a hydrogen atom
NH₂, or
NMeH.

49. The compound or the pharmaceutically acceptable salt thereof of claim 1, wherein formula (1) is represented by the following formula (6): 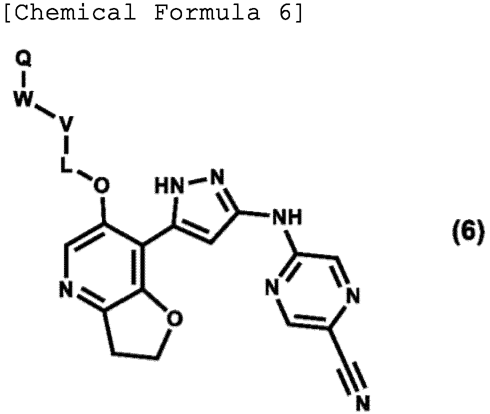 wherein
L represents
a single bond, or
C₁₋₆ alkylene (the alkylene is optionally substituted with 1 to 2 of the same or different substituents selected from the group consisting of a fluorine atom, or C₁₋₃ alkyl),
V represents
a single bond,
C₂₋₆ alkenylene (the alkenylene is optionally substituted with one substituent selected from the group consisting of a hydroxyl group, and C₁₋₃ alkyl),
C₃₋₁₀ cycloalkylene (the cycloalkylene is optionally substituted with one substituent selected from the group consisting of a hydroxyl group, and C₁₋₃ alkyl),
C₃₋₁₀ cycloalkenylene (the cycloalkenylene is optionally substituted with one substituent selected from the group consisting of a hydroxyl group, and C₁₋₃ alkyl), or
a 3- to 10-membered divalent saturated heterocyclic group (the saturated heterocyclic group is optionally substituted with one substituent selected from the group consisting of a fluorine atom, a hydroxyl group, and C₁₋₃ alkyl optionally substituted with 1 to 2 fluorine atoms),
W represents
a single bond, or
C₁₋₃ alkylene optionally substituted with 1 to 2 fluorine atoms, and
Q represents
a hydrogen atom
NH₂, or
NMeH.

50. The compound or the pharmaceutically acceptable salt thereof of any one of claims 1 to 28 and 49, wherein
V is
a single bond,
C₂₋₆ alkenylene,
C₃₋₁₀ cycloalkylene,
C₃₋₁₀ cycloalkenylene, or
a 3- to 10-membered divalent saturated heterocyclic group (the saturated heterocyclic group is optionally substituted with one substituent selected from the group consisting of a fluorine atom, and C₁₋₃ alkyl optionally substituted with 1 to 2 fluorine atoms).

51. The compound or the pharmaceutically acceptable salt thereof of any one of claims 1 to 28 and 49 to 50, wherein W is
a single bond, or
C₁₋₃ alkylene.

52. The compound or the pharmaceutically acceptable salt thereof of any one of claims 1 to 28 and 49 to 51, wherein Q is a hydrogen atom.

53. The compound or the pharmaceutically acceptable salt thereof of any one of claims 1 to 28 and 49 to 51, wherein Q is NH₂.

54. The compound or the pharmaceutically acceptable salt thereof of any one of claims 1 to 28 and 49 to 51, wherein Q is NHMe.

55. The compound or the pharmaceutically acceptable salt thereof of claim 1, wherein formula (1) is represented by the following formula (7): 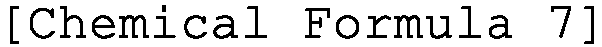 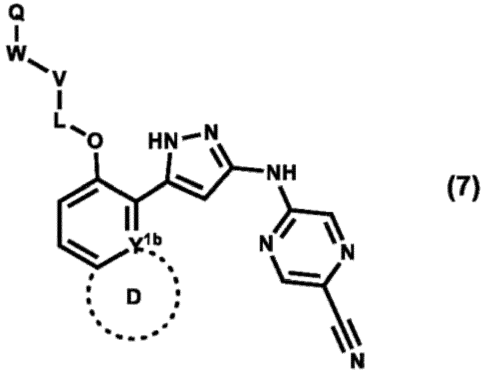 wherein
Y^{1b} represents
a carbon atom, or
a nitrogen atom,
Ring D represents
a 5- to 8-membered non-aromatic heterocycle, or
a 5- to 6-membered aromatic heterocycle (the non-aromatic heterocycle and aromatic heterocycle are optionally substituted with 1 to 2 of the same or different substituents selected from the group consisting of a fluorine atom, a hydroxyl group, C₁₋₃ alkyl, C₁₋₃ alkoxy, and cyano),
L represents
a single bond, or
C₁₋₆ alkylene (the alkylene is optionally substituted with 1 to 2 substituents selected from the group consisting of a fluorine atom, a hydroxyl group, C₁₋₃ alkyl, and cyano),
V represents
a single bond,
C₂₋₆ alkenylene (the alkenylene is optionally substituted with 1 to 3 of the same or different substituents selected from the group consisting of a fluorine atom, a hydroxyl group, C₁₋₃ alkyl, and cyano),
C₃₋₁₀ cycloalkylene (the cycloalkylene is optionally substituted with 1 to 3 of the same or different substituents selected from the group consisting of a fluorine atom, a hydroxyl group, C₁₋₃ alkyl, and cyano), C₃₋₁₀ cycloalkenylene (the cycloalkenylene is optionally substituted with 1 to 3 of the same or different substituents selected from the group consisting of a fluorine atom, a hydroxyl group, C₁₋₃ alkyl, and cyano), or
a 3- to 10-membered divalent saturated heterocyclic group (the saturated heterocyclic group is optionally substituted with 1 to 3 of the same or different substituents selected from the group consisting of a fluorine atom, a hydroxyl group, C₁₋₃ alkyl optionally substituted with 1 to 2 hydroxyl groups or fluorine atoms, and cyano),
W represents
a single bond, or
C₁₋₃ alkylene (the alkylene is optionally substituted with one substituent selected from the group consisting of a fluorine atom, a hydroxyl group, and cyano),
Q represents
a hydrogen atom, or
NHR¹⁴, and
R¹⁴ is
a hydrogen atom, or
C₁₋₃ alkyl (the alkyl is optionally substituted with one substituent selected from the group consisting of a fluorine atom, a hydroxyl group, and cyano).

56. The compound or the pharmaceutically acceptable salt thereof of claim 55, wherein Y^{1b} is a carbon atom.

57. The compound or the pharmaceutically acceptable salt thereof of claim 55, wherein Y^{1b} is a nitrogen atom.

58. The compound or the pharmaceutically acceptable salt thereof of any one of claims 55 to 57, wherein Ring D is a 5- to 8-membered non-aromatic heterocycle.

59. The compound or the pharmaceutically acceptable salt thereof of any one of claims 55 to 57, wherein Ring D is a 5- to 6-membered aromatic heterocycle.

60. The compound or the pharmaceutically acceptable salt thereof of any one of claims 1 to 28 and 55 to 59, wherein L is
a single bond, or
C₁₋₆ alkylene (the alkylene is optionally substituted with 1 to 2 of the same or different substituents selected from the group consisting of a fluorine atom, or C₁₋₃ alkyl).

61. The compound or the pharmaceutically acceptable salt thereof of any one of claims 1 to 28 and 55 to 60, wherein V is
a single bond,
C₂₋₆ alkenylene,
C₃₋₁₀ cycloalkylene (the cycloalkylene is optionally substituted with 1 to 3 fluorine atoms),
C₃₋₁₀ cycloalkenylene, or
a 3- to 10-membered divalent saturated heterocyclic group (the saturated heterocyclic group is optionally substituted with one substituent selected from the group consisting of a fluorine atom, and C₁₋₃ alkyl optionally substituted with 1 to 2 hydroxyl groups or fluorine atoms).

62. The compound or the pharmaceutically acceptable salt thereof of any one of claims 1 to 28 and 55 to 61, wherein W is
a single bond, or
C₁₋₃ alkylene.

63. The compound or the pharmaceutically acceptable salt thereof of any one of claims 1 to 28 and 55 to 62, wherein R¹⁴ is
a hydrogen atom, or
C₁₋₆ alkyl.

64. The compound or the pharmaceutically acceptable salt thereof of any one of claims 1 to 28 and 55 to 63, wherein Q is
a hydrogen atom, or
NH₂.

65. The compound or the pharmaceutically acceptable salt thereof of claim 1, wherein formula (1) is represented by the following formula (8): 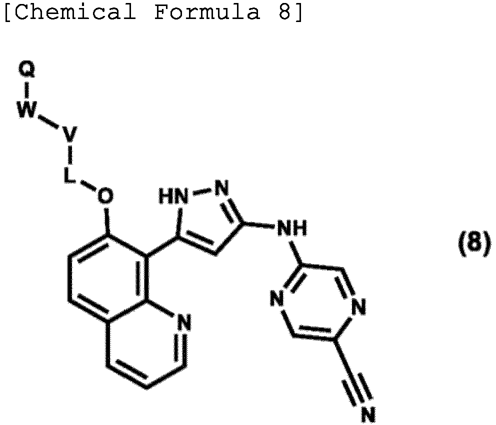 wherein
L represents
a single bond, or
C₁₋₆ alkylene (the alkylene is optionally substituted with 1 to 2 substituents selected from the group consisting of a fluorine atom, and C₁₋₃ alkyl),
V represents
a single bond,
C₂₋₆ alkenylene (the alkenylene is optionally substituted with one substituent selected from the group consisting of a hydroxyl group, and C₁₋₃ alkyl),
C₃₋₁₀ cycloalkylene (the cycloalkylene is optionally substituted with one substituent selected from the group consisting of a hydroxyl group, and C₁₋₃ alkyl),
C₃₋₁₀ cycloalkenylene (the cycloalkenylene is optionally substituted with one substituent selected from the group consisting of a hydroxyl group, and C₁₋₃ alkyl), or
a 3- to 10-membered divalent saturated heterocyclic group (the saturated heterocyclic group is optionally substituted with one substituent selected from the group consisting of a fluorine atom, a hydroxyl group, and C₁₋₃ alkyl optionally substituted with 1 to 2 fluorine atoms),
W represents
a single bond, or
C₁₋₃ alkylene optionally substituted with 1 to 2 fluorine atoms, and
Q represents
a hydrogen atom
NH₂, or
NHMe.

66. The compound or the pharmaceutically acceptable salt thereof of any one of claims 1 to 28 and 65, wherein L is C₁₋₆ alkylene.

67. The compound or the pharmaceutically acceptable salt thereof of any one of claims 1 to 28 and 65 to 66, wherein V is a single bond.

68. The compound or the pharmaceutically acceptable salt thereof of any one of claims 1 to 28 and 65 to 67, wherein W is
a single bond, or
C₁₋₃ alkylene.

69. The compound or the pharmaceutically acceptable salt thereof of any one of claims 1 to 28 and 65 to 68, wherein Q is NH₂.

70. The compound or the pharmaceutically acceptable salt thereof of claim 1, which is selected from the following compounds:
5-({5-[7-(3-aminopropoxy)quinolin-8-yl]-1H-pyrazol-3-yl}amino)pyrazine-2-carbonitrile,
5-({5-[6-(3-aminopropoxy)-1-benzofuran-7-yl]-1H-pyrazol-3-yl}amino)pyrazine-2-carbonitrile,
5-({5-[6-(3-aminopropoxy)-2,3-dihydro-1-benzofuran-7-yl]-1H-pyrazol-3-yl}amino)pyrazine-2-carbonitrile,
5-{[5-(6-{[1-(aminomethyl)cyclopropyl]methoxy}-2,3-dihydro-1-benzofuran-7-yl)-1H-pyrazol-3-yl]amino}pyrazine-2-carbonitrile,
5-[(5-{6-[(3-methylazetidin-3-yl)methoxy]-2,3-dihydro-1-benzofuran-7-yl}-1H-pyrazol-3-yl)amino]pyrazine-2-carbonitrile,
5-({5-[6-(3-aminopropoxy)pyrazolo[1,5-a]pyridin-7-yl]-1H-pyrazol-3-yl}amino)pyrazine-2-carbonitrile,
5-({5-[6-(3-aminopropoxy)pyrazolo[1,5-a]pyrimidin-7-yl]-1H-pyrazol-3-yl}amino)pyrazine-2-carbonitrile,
5-({5-[3-(3-aminopropoxy)-1,5-naphthyridin-4-yl]-1H-pyrazol-3-yl}amino)pyrazine-2-carbonitrile,
5-({5-[3-(3-amino-2,2-difluoropropoxy)-1,5-naphthyridin-4-yl]-1H-pyrazol-3-yl}amino)pyrazine-2-carbonitrile,
5-{[5-(3-{[1-(aminomethyl)cyclopropyl]methoxy}-1,5-naphthyridin-4-yl)-1H-pyrazol-3-yl]amino}pyrazine-2-carbonitrile,
5-[(5-f3-[(3-methylazetidin-3-yl)methoxy]-1,5-naphthyridin-4-yl}-1H-pyrazol-3-yl)amino]pyrazine-2-carbonitrile,
5-{[5-(3-{[(2S)-morpholin-2-yl]methoxy}-1,5-naphthyridin-4-yl)-1H-pyrazol-3-yl]amino}pyrazine-2-carbonitrile,
5-({5-[6-(3-aminopropoxy)-2,3-dihydrofuro[3,2-b]pyridin-7-yl]-1H-pyrazol-3-yl}amino)pyrazine-2-carbonitrile,
5-{[5-(6-{[1-(aminomethyl)cyclopropyl]methoxy}-2,3-dihydrofuro[3,2-b]pyridin-7-yl)-1H-pyrazol-3-yl]amino}pyrazine-2-carbonitrile,
5-[(5-{6-[(3-methylazetidin-3-yl)methoxy]-2,3-dihydrofuro[3,2-b]pyridin-7-yl}-1H-pyrazol-3-yl)amino]pyrazine-2-carbonitrile,
5-({5-[6-(3-aminopropoxy)-2-methyl-1,3-benzoxazol-7-yl]-1H-pyrazol-3-yl}amino)pyrazine-2-carbonitrile,
5-{[5-(6-{[1-(aminomethyl)cyclopropyl]methoxy}pyrazolo[1,5-a]pyridin-7-yl)-1H-pyrazol-3-yl]amino}pyrazine-2-carbonitrile,
5-[(5-{6-[(3-methylazetidin-3-yl)methoxy]pyrazolo[1,5-a]pyridin-7-yl}-1H-pyrazol-3-yl)amino]pyrazine-2-carbonitrile,
5-{[5-(6-{[1-(aminomethyl)cyclopropyl]methoxy}-1-benzofuran-7-yl)-1H-pyrazol-3-yl]amino}pyrazine-2-carbonitrile,
5-{[5-(6-{[1-(aminomethyl)-2,2-difluorocyclopropyl]methoxy]-1-benzofuran-7-yl)-1H-pyrazol-3-yl]amino}pyrazine-2-carbonitrile,
5-{[5-(6-{[(1R,3R)-3-aminocyclopentyl]oxy]-2,3-dihydrofuro[3,2-b]pyridin-7-yl)-1H-pyrazol-3-yl]amino}pyrazine-2-carbonitrile,
5-{[5-(6-{[3-(difluoromethyl)azetidin-3-yl]methoxy}-2,3-dihydrofuro[3,2-b]pyridin-7-yl)-1H-pyrazol-3-yl]amino}pyrazine-2-carbonitrile,
5-[(5-{6-[(3-fluoroazetidin-3-yl)methoxy]-2,3-dihydrofuro[3,2-b]pyridin-7-yl}-1H-pyrazol-3-yl)amino]pyrazine-2-carbonitrile,
5-{[5-(6-{[(1R,3S)-3-aminocyclohexyl]oxy]-2,3-dihydrofuro[3,2-b]pyridin-7-yl)-1H-pyrazol-3-yl]amino}pyrazine-2-carbonitrile,
5-[(5-{6-[(1R)-1-(azetidin-3-yl)ethoxy]-2,3-dihydrofuro[3,2-b]pyridin-7-yl}-1H-pyrazol-3-yl)amino]pyrazine-2-carbonitrile,
5-{[5-(6-{[(3S)-pyrrolidin-3-yl]methoxy}-2,3-dihydrofuro[3,2-b]pyridin-7-yl)-1H-pyrazol-3-yl]amino}pyrazine-2-carbonitrile,
5-[(5-{6-[3-(methylamino)propoxy]-2,3-dihydrofuro[3,2-b]pyridin-7-yl}-1H-pyrazol-3-yl)amino]pyrazine-2-carbonitrile,
5-[(5-{6-[(2R)-3-amino-2-methylpropoxy]-2,3-dihydrofuro[3,2-b]pyridin-7-yl}-1H-pyrazol-3-yl)amino]pyrazine-2-carbonitrile,
5-{[5-(6-{[(1R,2S,4S,5S)-4-aminobicyclo[3.1.0]hexan-2-yl]oxy}-2,3-dihydrofuro[3,2-b]pyridin-7-yl)-1H-pyrazol-3-yl]amino}pyrazine-2-carbonitrile,
5-[(5-{6-[(2S)-3-amino-2-methylpropoxy]-2,3-dihydrofuro[3,2-b]pyridin-7-yl}-1H-pyrazol-3-yl)amino]pyrazine-2-carbonitrile,
5-{[5-(6-{[2-(aminomethyl)prop-2-en-1-yl]oxy}-2,3-dihydrofuro[3,2-b]pyridin-7-yl)-1H-pyrazol-3-yl]amino}pyrazine-2-carbonitrile,
N-{5-[6-(3-aminopropoxy)-2,3-dihydrofuro[3,2-b]pyridin-7-yl]-1H-pyrazol-3-yl}-5-chloropyrazin-2-amine,
methyl 5-({5-[6-(3-aminopropoxy)-2,3-dihydrofuro[3,2-b]pyridin-7-yl]-1H-pyrazol-3-yl}amino)pyrazine-2-carboxylate,
5-{[5-(3-{[(1R,2S)-2-(aminomethyl)cyclopentyl]oxy}-1,5-naphthyridin-4-yl)-1H-pyrazol-3-yl]amino}pyrazine-2-carbonitrile,
5-[(5-13-[(3-fluoroazetidin-3-yl)methoxy]-1,5-naphthyridin-4-yl}-1H-pyrazol-3-yl)amino]pyrazine-2-carbonitrile,
5-{[5-(3-1[(1R,3S)-3-aminocyclohexyl]oxyl-1,5-naphthyridin-4-yl)-1H-pyrazol-3-yl]amino}pyrazine-2-carbonitrile,
5-{[5-(3-{[(1R,3R)-3-aminocyclopentyl]oxy}-1,5-naphthyridin-4-yl)-1H-pyrazol-3-yl]amino}pyrazine-2-carbonitrile, and
5-{[5-(3-{[1-(aminomethyl)-3,3-difluorocyclobutyl]methoxy]-1,5-naphthyridin-4-yl)-1H-pyrazol-3-yl]amino}pyrazine-2-carbonitrile.

71. The compound or the pharmaceutically acceptable salt thereof of claim 1, which is the following:
5-({5-[7-(3-aminopropoxy)quinolin-8-yl]-1H-pyrazol-3-yl}amino)pyrazine-2-carbonitrile,
5-({5-[3-(3-aminopropoxy)-1,5-naphthyridin-4-yl]-1H-pyrazol-3-yl}amino)pyrazine-2-carbonitrile,
5-({5-[3-(3-amino-2,2-difluoropropoxy)-1,5-naphthyridin-4-yl]-1H-pyrazol-3-yl}amino)pyrazine-2-carbonitrile,
5-{[5-(3-{[1-(aminomethyl)cyclopropyl]methoxy}-1,5-naphthyridin-4-yl)-1H-pyrazol-3-yl]amino}pyrazine-2-carbonitrile,
5-[(5-13-[(3-methylazetidin-3-yl)methoxy]-1,5-naphthyridin-4-yl}-1H-pyrazol-3-yl)amino]pyrazine-2-carbonitrile,
5-({5-[6-(3-aminopropoxy)-2,3-dihydrofuro[3,2-b]pyridin-7-yl]-1H-pyrazol-3-yl}amino)pyrazine-2-carbonitrile,
5-{[5-(6-{[1-(aminomethyl)cyclopropyl]methoxy}-2,3-dihydrofuro[3,2-b]pyridin-7-yl)-1H-pyrazol-3-yl]amino}pyrazine-2-carbonitrile,
5-[(5-{6-[(1R)-1-(azetidin-3-yl)ethoxy]-2,3-dihydrofuro[3,2-b]pyridin-7-yl}-1H-pyrazol-3-yl)amino]pyrazine-2-carbonitrile,
5-{[5-(6-{[(3S)-pyrrolidin-3-yl]methoxy}-2,3-dihydrofuro[3,2-b]pyridin-7-yl)-1H-pyrazol-3-yl]amino}pyrazine-2-carbonitrile,
5-[(5-{6-[3-(methylamino)propoxy]-2,3-dihydrofuro[3,2-b]pyridin-7-yl}-1H-pyrazol-3-yl)amino]pyrazine-2-carbonitrile,
5-[(5-{6-[(2R)-3-amino-2-methylpropoxy]-2,3-dihydrofuro[3,2-b]pyridin-7-yl}-1H-pyrazol-3-yl)amino]pyrazine-2-carbonitrile,
5-[(5-{3-[(3-fluoroazetidin-3-yl)methoxy]-1,5-naphthyridin-4-yl}-1H-pyrazol-3-yl)amino]pyrazine-2-carbonitrile, or
5-{[5-(3-{[1-(aminomethyl)-3,3-difluorocyclobutyl]methoxy]-1,5-naphthyridin-4-yl)-1H-pyrazol-3-yl]amino}pyrazine-2-carbonitrile.

72. The compound or the pharmaceutically acceptable salt thereof of claim 1, which is the following:
5-({5-[7-(3-aminopropoxy)quinolin-8-yl]-1H-pyrazol-3-yl}amino)pyrazine-2-carbonitrile,
5-({5-[3-(3-aminopropoxy)-1,5-naphthyridin-4-yl]-1H-pyrazol-3-yl}amino)pyrazine-2-carbonitrile,
5-{[5-(3-{[1-(aminomethyl)cyclopropyl]methoxy}-1,5-naphthyridin-4-yl)-1H-pyrazol-3-yl]amino}pyrazine-2-carbonitrile,
5-({5-[6-(3-aminopropoxy)-2,3-dihydrofuro[3,2-b]pyridin-7-yl]-1H-pyrazol-3-yl}amino)pyrazine-2-carbonitrile,
5-{[5-(6-{[1-(aminomethyl)cyclopropyl]methoxy}-2,3-dihydrofuro[3,2-b]pyridin-7-yl)-1H-pyrazol-3-yl]amino}pyrazine-2-carbonitrile,
5-[(5-{6-[(1R)-1-(azetidin-3-yl)ethoxy]-2,3-dihydrofuro[3,2-b]pyridin-7-yl}-1H-pyrazol-3-yl)amino]pyrazine-2-carbonitrile, or
5-[(5-{6-[(2R)-3-amino-2-methylpropoxy]-2,3-dihydrofuro[3,2-b]pyridin-7-yl}-1H-pyrazol-3-yl)amino]pyrazine-2-carbonitrile.

73. A liposome comprising the compound or the pharmaceutically acceptable salt thereof of any one of claims 1 to 72.

74. A pharmaceutical composition comprising the compound or the pharmaceutically acceptable salt thereof of any one of claims 1 to 72 as an active ingredient.

75. A pharmaceutical composition comprising a liposome encapsulating the compound or the pharmaceutically acceptable salt thereof of any one of claims 1 to 72.

76. The pharmaceutical composition of claim 75, wherein the liposome further comprises a phospholipid.

77. The pharmaceutical composition of claim 75 or 76, wherein the liposome comprises
(1) the compound or the pharmaceutically acceptable salt thereof of any one of claims 1 to 72, and
(2) a phospholipid.

78. The pharmaceutical composition of claim 75 or 76, wherein the phospholipid is one phospholipid selected from the group consisting of phosphatidylcholine, phosphatidylglycerol, phosphatidic acid, phosphatidylethanolamine, phosphatidylserine, phosphatidylinositol, sphingomyelin, soybean lecithin, egg yolk lecithin, hydrogenated egg yolk lecithin, and hydrogenated soybean lecithin, or a combination of two or more thereof.

79. The pharmaceutical composition of any one of claims 75 to 78, wherein the liposome further comprises a sterol.

80. The pharmaceutical composition of claim 79, wherein the sterol is a cholesterol.

81. The pharmaceutical composition of any one of claims 75 to 80, wherein the liposome further comprises a polymer-modified lipid.

82. The pharmaceutical composition of claim 81, wherein a polymer moiety of the polymer-modified lipid is polyethylene glycol, polypropylene glycol, polyvinyl alcohol, polyvinylpyrrolidone, methoxypolyethylene glycol, methoxypolypropylene glycol, methoxypolyvinyl alcohol, methoxypolyvinylpyrrolidone, ethoxypolyethylene glycol, ethoxypolypropylene glycol, ethoxypolyvinyl alcohol, ethoxypolyvinylpyrrolidone, propoxypolyethylene glycol, propoxypolypropylene glycol, propoxypolyvinyl alcohol, or propoxypolyvinylpyrrolidone.

83. The pharmaceutical composition of claim 81 or 82, wherein a lipid moiety of the polymer-modified lipid is phosphatidylethanolamine or diacylglycerol.

84. The pharmaceutical composition of any one of claims 81 to 83, wherein the polymer-modified lipid comprises polyethylene glycol, polypropylene glycol, polyvinyl alcohol, polyvinylpyrrolidone, methoxypolyethylene glycol, methoxypolypropylene glycol, methoxypolyvinyl alcohol, methoxypolyvinylpyrrolidone, ethoxypolyethylene glycol, ethoxypolypropylene glycol, ethoxypolyvinyl alcohol, ethoxypolyvinylpyrrolidone, propoxypolyethylene glycol, propoxypolypropylene glycol, propoxypolyvinyl alcohol, or propoxypolyvinylpyrrolidone as a polymer moiety, and comprises phosphatidylethanolamine or diacylglycerol as a lipid moiety.

85. The pharmaceutical composition of any one of claims 75 to 84, wherein the liposome comprises
(1) the compound or the pharmaceutically acceptable salt thereof of any one of claims 1 to 72,
(2) 40 to 70 mol% of a phospholipid,
(3) 30 to 50 mol% of a cholesterol, and
(4) 1 to 10 mol% of a polymer-modified lipid.

86. The pharmaceutical composition of any one of claims 75 to 85, wherein the liposome further comprises an additive selected from the group consisting of an inorganic acid, an inorganic acid salt, an organic acid, an organic acid salt, a saccharide, a buffer, an antioxidant, and a polymer.

87. A therapeutic agent and/or prophylactic agent for cancer, comprising the compound or the pharmaceutically acceptable salt thereof of any one of claims 1 to 72 as an active ingredient.

88. The therapeutic agent and/or prophylactic agent of claim 87, wherein the cancer is at least one type of cancer selected from the group consisting of acute leukemia, chronic lymphocytic leukemia, chronic myelogenous leukemia, myelodysplastic syndrome, polycythemia vera, malignant lymphoma, plasma cell tumor, multiple myeloma, brain tumor, head and neck cancer, esophageal cancer, thyroid cancer, small cell lung cancer, non-small cell lung cancer, thymoma/thymic carcinoma, breast cancer, gastric cancer, gallbladder/bile duct cancer, liver cancer, hepatocellular carcinoma, pancreatic cancer, colon cancer, rectal cancer, anal cancer, gastrointestinal stromal tumor, choriocarcinoma, endometrial cancer, cervical cancer, ovarian cancer, bladder cancer, urothelial cancer, renal cancer, renal cell cancer, prostate cancer, testicular tumor, testicular germ cell tumor, ovarian germ cell tumor, Wilms tumor, malignant melanoma, neuroblastoma, osteosarcoma, Ewing sarcoma, chondrosarcoma, soft tissue sarcoma, or skin cancer.

89. The compound or the pharmaceutically acceptable salt thereof of any one of claims 1 to 72, or the liposome of claim 73, or the pharmaceutical composition of claims 74 to 86, or the therapeutic agent and/or prophylactic agent of claim 87 or 88, for use in the treatment and/or prophylaxis of cancer.

90. The compound or the pharmaceutically acceptable salt thereof, or the pharmaceutical composition, or the liposome, or the therapeutic agent and/or prophylactic agent of claim 89, wherein the cancer is at least one type of cancer selected from the group consisting of acute leukemia, chronic lymphocytic leukemia, chronic myelogenous leukemia, myelodysplastic syndrome, polycythemia vera, malignant lymphoma, plasma cell tumor, multiple myeloma, brain tumor, head and neck cancer, esophageal cancer, thyroid cancer, small cell lung cancer, non-small cell lung cancer, thymoma/thymic carcinoma, breast cancer, gastric cancer, gallbladder/bile duct cancer, liver cancer, hepatocellular carcinoma, pancreatic cancer, colon cancer, rectal cancer, anal cancer, gastrointestinal stromal tumor, choriocarcinoma, endometrial cancer, cervical cancer, ovarian cancer, bladder cancer, urothelial cancer, renal cancer, renal cell cancer, prostate cancer, testicular tumor, testicular germ cell tumor, ovarian germ cell tumor, Wilms tumor, malignant melanoma, neuroblastoma, osteosarcoma, Ewing sarcoma, chondrosarcoma, soft tissue sarcoma, or skin cancer.

91. The compound or the pharmaceutically acceptable salt thereof of any one of claims 1 to 72, or the liposome of claim 73, for treating cancer, used in combination with a concomitant drug or a pharmaceutically acceptable salt thereof, wherein the concomitant drug is at least one selected from the group consisting of a hormonal therapy agent, a chemotherapeutic agent, an immunotherapeutic agent, and an agent that inhibits a cell growth factor and its receptor action.

92. The compound or the pharmaceutically acceptable salt thereof of any one of claims 1 to 72, or the liposome of claim 73, for treating cancer, used in combination with a concomitant drug or a pharmaceutically acceptable salt thereof, wherein the concomitant drug is at least one selected from the group consisting of a 5-FU type drug, Cytarabine, Doxorubicin hydrochloride, Gemcitabine, Methotrexate, Pemetrexed, Etoposide, Irinotecan, Topotecan, Cisplatin, Carboplatin, Oxaliplatin, Paclitaxel, Docetaxel, ionizing radiation, Bevacizumab, liposomal Doxorubicin, Rucaparib, Olaparib, Niraparib, Trabectedin, Pazopanib, Pembrolizumab, Nivolumab, Ipilimumab, Durvalumab, Avelumab, Atezolizumab, Larotrectinib, Entrectinib, nab-Paclitaxel, Erlotinib, liposomal Irinotecan, Leucovorin, Cetuximab, Eribulin, Ifosfamide, and Dacarbazine.

93. The compound or the pharmaceutically acceptable salt thereof of any one of claims 1 to 72, or the liposome of claim 73, for treating cancer, used in combination with a concomitant drug or a pharmaceutically acceptable salt thereof, wherein the concomitant drug is at least one selected from the group consisting of a 5-FU type drug, Irinotecan, Gemcitabine, Cisplatin, Carboplatin, Oxaliplatin, Paclitaxel, ionizing radiation, Rucaparib, Olaparib, Niraparib, Pembrolizumab, and Nivolumab.

94. A pharmaceutical composition comprising the compound or the pharmaceutically acceptable salt thereof of any one of claims 1 to 72, or the pharmaceutical composition of any one of claims 74 to 86, in combination with a concomitant drug, wherein the concomitant drug is at least one selected from the group consisting of a hormonal therapy agent, a chemotherapeutic agent, an immunotherapeutic agent, and an agent that inhibits a cell growth factor and its receptor action.

95. A pharmaceutical composition comprising the compound or the pharmaceutically acceptable salt thereof of any one of claims 1 to 72, or the pharmaceutical composition of any one of claims 74 to 86, in combination with a concomitant drug, wherein the concomitant drug is at least one selected from the group consisting of a 5-FU type drug, Cytarabine, Doxorubicin hydrochloride, Gemcitabine, Methotrexate, Pemetrexed, Etoposide, Irinotecan, Topotecan, Cisplatin, Carboplatin, Oxaliplatin, Paclitaxel, Docetaxel, ionizing radiation, Bevacizumab, liposomal Doxorubicin, Rucaparib, Olaparib, Niraparib, Trabectedin, Pazopanib, Pembrolizumab, Nivolumab, Ipilimumab, Durvalumab, Avelumab, Atezolizumab, Larotrectinib, Entrectinib, nab-Paclitaxel, Erlotinib, liposomal Irinotecan, Leucovorin, Cetuximab, Eribulin, Ifosfamide, and Dacarbazine.
